(19) Europäisches Patentamt
European Patent Office
Office européen des brevets

(11) EP 4 292 596 A1

(12) EUROPEAN PATENT APPLICATION
published in accordance with Art. 153(4) EPC

(43) Date of publication:
**20.12.2023 Bulletin 2023/51**

(21) Application number: **22752281.0**

(22) Date of filing: **09.02.2022**

(51) International Patent Classification (IPC):
***A61K 31/519*** *(2006.01)* ***A61K 31/522*** *(2006.01)*
***A61P 35/00*** *(2006.01)*

(52) Cooperative Patent Classification (CPC):
**A61K 31/519; A61K 31/522; A61P 35/00**

(86) International application number:
**PCT/CN2022/075642**

(87) International publication number:
**WO 2022/171121 (18.08.2022 Gazette 2022/33)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **10.02.2021 CN 202110184081**
**13.04.2021 CN 202110410557**
**27.01.2022 CN 202210099148**

(71) Applicant: **Curon Biopharmaceutical (Shanghai) Co., Limited**
**Shanghai 201203 (CN)**

(72) Inventors:
- **LU, Rongzhen**
  **Shanghai 201203 (CN)**
- **CHEN, Zhihong**
  **Shanghai 201203 (CN)**

(74) Representative: **Hoffmann Eitle**
**Patent- und Rechtsanwälte PartmbB**
**Arabellastraße 30**
**81925 München (DE)**

Remarks:
The complete document including Reference Table(s) and the Sequence Listing(s) can be downloaded from the EPO website

# (54) METHOD AND COMBINATION FOR TREATING TUMORS

(57) The present application relates to a pharmaceutical combination comprising a phosphoinositide 3-kinase (PI3K) inhibitor and a second therapeutic agent, wherein the second therapeutic agent is an immune checkpoint inhibitor, a TGFβ inhibitor, a bifunctional immune checkpoint/TGFβ inhibitors or combinations thereof. The present application also provides a method of treatment comprising administering the combination, and the use of the drug combination for treating tumors



Processed by Luminess, 75001 PARIS (FR)

## Description

## Field

The present application relates to the field of biomedicine, in particular to a method and combination for treating tumors.

## Background

The phosphoinositide 3-kinase (PI3K) signaling pathway is one of the most highly mutated systems in human cancers. PI3Ks are members of a unique and conserved family of intracellular lipid kinases that phosphorylate the 3'-OH group on phosphatidylinositols or phosphoinositides. The PI3K family includes 15 kinases with distinct substrate specificities, expression patterns, and regulatory patterns. Class I PI3Ks (p110α, p110β, p110δ, and p110γ) are normally activated by tyrosine kinases or G-protein-coupled receptors to generate (3,4,5)-phosphatidylinositol triphosphate (PIP3), which engages downstream effectors, such as those in the AKT/PDK1 pathway, mTOR, Tec family kinases and Rho family GTPases. By synthesizing phosphatidylinositol 3-diphosphate (PI(3)P) and (3,4)-phosphatidylinositol diphosphate (PI(3,4)P2), Class II and III PI3Ks plays a key role in intracellularly transport. PI3Ks are protein kinases that control cell growth (mTORCl) or monitor genome integrity (ATM, ATR, DNA-PK, and hSmg-1).

There are four mammalian subtypes of class I PI3Ks: PI3K-alpha, β, delta (class Ia PI3Ks) and PI3K-gamma (class Ib PI3Ks). These enzymes catalyze the production of PIP3, leading to the activation of downstream effector pathways important for cell survival, differentiation and function. PI3K-α and PI3K-β are ubiquitously expressed and are important mediators of signal transduction from cell surface receptors. PI3K-α is the most commonly mutated isoform in cancer and plays a role in insulin signaling and glucose homeostasis (Knight et al Cell (2006) 125(4):733-47; Vanhaesebroeck et al Current Topic Microbiol. Immunol. (2010) 347:1-19). PI3K-β is activated in phosphatase and tensin homolog (PTEN)-deficient cancers. Both subtypes are cancer targets for small molecule therapies in development.

PI3K-δ and -γ are preferentially expressed in leukocytes and play a key role in leukocyte function. These subtypes also contribute to the development and maintenance of hematological malignancies (Vanhaesebroeck et al. Current Topic Microbiol. Immunol. (2010) 347:1-19; Clayton et al. J Exp Med. (2002) 196(6):753-19 63; Fung-Leung Cell Signal. (2011) 23(4):603-8; Okkenhaug et al. Science (2002) 297(5583):1031-34). PI3K-delta is activated by cellular receptors (eg, receptor tyrosine kinases) through interaction with the Sarc homology 2 (SH2) domain of the PI3K regulatory subunit (p85) or through direct interaction with RAS.

Transforming growth factor β (TGFβ) is a potent cytokine that has a significant effect on the immune system. The major function of TGFβ in the immune system is to maintain tolerance and initial immune response to foreign pathogens. Three isoforms of TGF[β] have been identified in mammals, TGFβ1, TGFβ2 and TGFβ3, with TGFβ1 being the predominant isoform. TGFβ is secreted in a latent form, and only a small fraction of the total TGFβ secreted is activated under physiological conditions. Most of the biological effects of TGFβ are realized through the binding of TGFβ to receptor ALK5 and TGFβ receptor II (TGFβR2). Specifically, active TGFβ dimers can bind tetrameric ALK5 and TGFβR2 complexes to initiate signal transduction. ALK5 is not required for initial binding of TGFβ but is required for signal transduction.

Programmed death receptor 1 (programmed death 1, PD-1) is a member of the CD28 superfamily. PD-1 is expressed in activated T cells, B cells and myeloid cells, and it has two ligands, namely programmed death ligand 1 (PD-L1) and PD-L2. PD-L1 interacts with the receptor PD-1 on T cells and plays an important role in the negative regulation of the immune response. The expression of PD-L1 protein can be detected in many human tumor tissues. The microenvironment of the tumor site can induce the expression of PD-L1 on tumor cells. The expressed PD-L1 is conducive to the occurrence and growth of tumors, and induces anti-tumor Apoptosis of T cells. PD-1/PD-L1 pathway inhibitors can block the combination of PD-1 and PD-L1, block negative regulatory signals, restore the activity of T cells, and enhance the immune response.

Transforming growth factor-β (transforming growth factor-β, TGF-β) belongs to the TGF-β superfamily that regulates cell growth and differentiation. TGF-β deliver signals by a heterotetrameric receptor complex consisting of two type I and two type II transmembrane serine/threonine kinase receptors.

TGFβ can affect many cellular functions, such as cell proliferation, differentiation, cell-cell and cell-matrix adhesion, cell motility, and activation of lymphocytes. (For a review of the role of TGFβ in regulating immune responses, see Li et al. (2006) Annu. Rev. Immunol. 24:99-146.) Furthermore, TGFβ is believed to induce or mediate the progression of many diseases, such as osteoporosis disease, hypertension, atherosclerosis, cirrhosis, fibrotic disease of the kidney, liver, and lung, and tumor progression. In animal models of certain diseases, such as diabetic nephropathy, glomerulonephritis, cyclosporin-mediated kidney injury, and systemic lupus erythematosus (SLE), TGFβ can enhance end-organ damage caused by chronic inflammation, while TGFβ Antagonists can effectively reduce this damage (Border et al. (1990) Nature346:371-374; Border et al. (1992) Nature 360:361-364; Isaka et al. (1999) KidneyInt.55:465-475; Sharma

et al. Diabetes 45: 522; Xin et al. (2004) Transplantation 15: 1433; Benigni et al. (2003) J. Am. Soc. Nephrol. 14: 1816). In terms of cancer, TGFβ may have a direct inhibitory effect on malignant cells, and may increase the production or activity of a series of tumor growth factors and angiogenesis factors.

In the treatment of cancer, it has long been recognized that chemotherapy brings high toxicity and can lead to negative effects on drug-resistant cancer cells. Even with therapies that target overexpressed or activated proteins associated with tumor survival and growth, cancer cells mutate to reduce or escape reliance on the pathway targeted by the therapy and exploit other pathways to continue Survive. Tumor immunotherapy mainly improves the immunogenicity of tumor cells and the sensitivity to effector cell killing through immunological principles and methods, stimulates and enhances the body's anti-tumor immune response, and uses immune cells and effector molecules to infuse the host body to cooperate with the body. The immune system kills tumors and inhibits tumor growth. Reducing the toxicity and resistance of tumor drugs, and reducing the side effects are still urgent problems to be solved in tumor treatment.

## Summary

The object of the present application is to provide combinations and methods comprising a PI3K inhibitor and a selected second therapeutic agent. In certain embodiment, a combination of a PI3K inhibitor and a second therapeutic agent selected from one or more of the following has been found to be synergistic in treating tumors (e.g., in reducing cancer cell growth or viability, or both) : Inhibitors of immune checkpoints, TGFβ inhibitors, or combinations thereof. The combination of a PI3K inhibitor and a selected second therapeutic agent may allow for a desired lower dose of the PI3K inhibitor, second therapeutic agent, or both, while achieving the same therapeutic effect as compared to monotherapy doses apply. In certain embodiment, the combination can allow for less frequent administration of the PI3K inhibitor, the second therapeutic agent, or both than when the PI3K inhibitor or the second therapeutic agent is administered as a monotherapy. Such a combination may provide an advantageous effect, e.g., in reducing, preventing, delaying and/or reducing the occurrence of one or more of side effects, toxicity or resistance otherwise associated with the administration of higher doses of the agents.

In one aspect, the present application is to provide a pharmaceutical combination comprising a phosphoinositide 3-kinase (PI3K) inhibitor and a second therapeutic agent, wherein the second therapeutic agent is an immune checkpoint inhibitor, a TGFβ inhibitor, a bifunctional immune checkpoint/ TGFβ inhibitors or combinations thereof.

In certain embodiment, the immune checkpoint inhibitor comprises an agent capable of blocking the interaction between programmed death 1 (PD-1) and programmed death ligand 1 (PD-L1) .

In certain embodiment, the immune checkpoint inhibitor comprises a programmed death ligand 1 (PD-L1) and/or programmed death 1 (PD-1 ) inhibitor.

In certain embodiment, the bifunctional immune checkpoint/TGFβ inhibitor comprises a PD-L1/TGFβ dual inhibitor, or a PD-1/TGFβ dual inhibitor.

In certain embodiment, the combination comprises:

1) the combination of PD-L1 inhibitor and PI3K inhibitor;
2) the combination of TGFβ inhibitor and PI3K inhibitor;
3) the combination of PD-1 inhibitor, TGFβ inhibitor and PI3K inhibitor;
4) the combination of PD-L1 inhibitor, TGFβ inhibitor and PI3K inhibitor;
5) the combination of PD-L1/TGFβ dual inhibitor and PI3K inhibitor; or
6) the combination of PD-1/TGFβ dual inhibitor and PI3K inhibitor.

In certain embodiment, the immune checkpoint inhibitor comprises a nucleic acid, an antibody or an antigen-binding fragment thereof, a fusion protein or a chemical compound.

In certain embodiment, wherein the inhibitors of immune checkpoints include nucleic acids(e.g. dsRNA, siRNA or sh RNA), polypeptide(e.g. soluble ligands, antibodies or antigen-binding fragments thereof, immuneoadhesins, fusion proteins, oligopeptides and other molecules) or compound.

In certain embodiment, said antibody is selected from the group consisting of: human antibody, humanized antibody, chimeric antibody, multispecific antibody, monoclonal antibody and polyclonal antibody.

In certain embodiment, the antigen-binding fragment is selected from Fab, Fab', $F(ab')_2$, Fv, scFv, bispecific antibody, Fd, dAb, VHH, large antibody and complementarity determining region (CDR) fragments.

In certain embodiment, the antibody or antigen-binding fragment thereof comprises a heavy chain variable region (VH) comprising HCDR1, HCDR2 and HCDR3.

In certain embodiment, the antibody or antigen-binding fragment thereof further comprises or does not comprise a CH1 domain.

In certain embodiment, the antibody or antigen-binding fragment thereof further comprises or does not comprise CH2 and CH3 domains.

In certain embodiment, the antibody or antigen-binding fragment thereof further comprises an antibody heavy chain constant region.

In certain embodiment, the antibody heavy chain constant region is a human antibody heavy chain constant region.

In certain embodiment, the human antibody heavy chain constant region is selected from constant regions derived from the group consisting of IgG1, IgG2, IgG3, IgG4 and variants thereof.

In certain embodiment, the antibody or antigen-binding fragment thereof further comprises a light chain variable region (VL) comprising LCDR1, LCDR2, LCDR3.

In certain embodiment, the antibody or antigen-binding fragment thereof further comprises a light chain constant region (CL).

In certain embodiment, the immune checkpoint inhibitor comprises an anti-PD-L1 antibody or an antigen-binding fragment thereof, or an anti-PD-1 antibody or an antigen-binding fragment thereof.

In certain embodiment, the anti-PD-L1 antibody or antigen-binding fragment thereof comprises a heavy chain variable region of an antibody, wherein the heavy chain variable region comprises HCDR1, HCDR2, HCDR3, which has at least about 80% sequence identity to HCDR1, HCDR2, HCDR3, respectively, of atezolizumab, avelumab, BMS-936559, MPDL3280A (RG7446), or durvalumab (MEDI-4736).

In certain embodiment, the anti-PD-L1 antibody or antigen-binding fragment thereof comprises an antibody heavy chain variable region, wherein the heavy chain variable region is separately associated with The heavy chain variable regions of the following molecules have at least about 80% sequence identity: atezolizumab, avelumab, BMS-936559, MPDL3280A, or durvalumab.

In certain embodiment, the anti-PD-L1 antibody or antigen-binding fragment thereof comprises a heavy chain of an antibody, wherein the heavy chain is respectively associated with a heavy chain of the following molecule having at least about 80% sequence identity: Atezolizumab, Avelumab, BMS-936559, MPDL3280A, or durvalumab.

In certain embodiment, the anti-PD-L1 antibody or antigen-binding fragment thereof further comprises an antibody light chain variable region, wherein the light chain variable region comprises LCDR1, LCDR2, LCDR3, each of which has at least about 80% sequence identity to LCDR1, LCDR2, LCDR3, respectively, of atezolizumab, avelumab, BMS-936559, MPDL3280A, or durvalumab.

For example, the anti-PD-L1 antibody or antigen-binding fragment thereof comprises a heavy chain variable region and a light chain variable region of an antibody, wherein the heavy chain variable region comprises HCDR1, HCDR2, HCDR3, wherein the light chain can be The variable regions comprise LCDR1, LCDR2, LCDR3, each of which has at least about 80% (e.g., about 85%, 90%, or 95%) sequence identity to HCDR1, HCDR2, HCDR3, LCDR1, LCDR2, LCDR3, respectively, of: Atezolizumab, Avelumab, BMS-936559, MPDL3280A (RG7446), or durvalumab (MEDI4736).

In certain embodiment, wherein the light chain variable regions each have at least about 80% (e.g., about 85%, 90%, or 95%) sequence identity to the light chain variable regions of: Atezolizumab, Avelumab, BMS-936559, MPDL3280A or durvalumab.

For example, the anti-PD-L1 antibody or antigen-binding fragment thereof comprises a heavy chain variable region and a light chain variable region of the antibody, each of which has at least about 80% (e.g., about 85%, 90%, or 95%) sequence identity: Atezolizumab, Avelumab, BMS-936559, MPDL3280A (RG7446), or durvalumab (MEDI4736).

In certain embodiment, wherein the anti-PD-L1 antibody or antigen-binding fragment thereof further comprises a light chain of the antibody, wherein the light chains are at least about 80% (e.g. about 85% identical) to the light chains of the following molecules, respectively , 90% or 95%) sequence identity: Atezolizumab, Avelumab, BMS-936559, MPDL3280A or durvalumab.

For example, the anti-PD-L1 antibody or antigen-binding fragment thereof comprises a heavy chain and a light chain of the antibody, each of which has at least about 80% (e.g. about 85%, 90%, or 95%) sequence identity: Atezolizumab, Avelumab, BMS-936559, MPDL3280A (RG7446), or durvalumab (MEDI4736).

In certain embodiment, wherein the anti-PD-L1 antibody or antigen-binding fragment thereof comprises: Atezolizumab, Avelumab, BMS-936559, MPDL3280A or durvalumab, or an antigen-binding fragment thereof, or a variant or biosimilar thereof, or its combination.

In certain embodiment, wherein the anti-PD-L1 antibody or antigen-binding fragment thereof comprises a heavy chain variable region of an antibody, wherein the heavy chain variable region comprises one or more heavy chain variable region CDRs (HCDRs) selected from the group consisting of: (a) HCDR1 having at least about 70% sequence identity to the amino acid sequence shown in SEQ ID NO: 1; (b) having at least about 70% sequence identity to SEQ ID NO:2 HCDR2 of sequence identity; and (c) HCDR3 having at least about 70% sequence identity to SEQ ID NO:3.

In certain embodiment, wherein the anti-PD-L1 antibody or antigen-binding fragment thereof comprises an antibody heavy chain variable region, wherein the heavy chain variable region comprises one or more HCDRs selected from the group consisting of: (a) HCDR1 having the amino acid sequence shown in SEQ ID NO: 1 or having no more than 2 amino acid differences from the amino acid sequence shown in SEQ ID NO: 1 obtained through amino acid addition, elimination or substitution reactions HCDR1; (b) HCDR2 having the amino acid sequence shown in SEQ ID

NO: 2 or HCDR2 having no more than 2 amino acid differences from the amino acid sequence shown in SEQ ID NO: 2 obtained through amino acid addition, elimination or substitution reactions; and (c) HCDR3 having the amino acid sequence shown in SEQ ID NO: 3 or HCDR3 having no more than 2 amino acid differences from the amino acid sequence shown in SEQ ID NO: 3 obtained through amino acid addition, elimination or substitution reactions.

In certain embodiment, wherein the anti-PD-L1 antibody or antigen-binding fragment thereof comprises a heavy chain variable region of an antibody, wherein the heavy chain variable region comprises HCDR1, HCDR2, HCDR3, the said HCDR1 comprises an amino acid sequence with at least about 70% sequence identity to the amino acid sequence shown in SEQ ID NO: 1, said HCDR2 comprises an amino acid sequence with at least about 70% sequence identity to the amino acid sequence shown in SEQ ID NO: 2, wherein the HCDR3 comprises an amino acid sequence having at least about 70% sequence identity to the amino acid sequence set forth in SEQ ID NO:3.

In certain embodiment, wherein the anti-PD-L1 antibody or antigen-binding fragment thereof comprises a heavy chain variable region of an antibody, wherein the heavy chain variable region comprises HCDR1, HCDR2, HCDR3, the said HCDR1 comprises the amino acid sequence shown in SEQ ID NO: 1 or an amino acid sequence obtained by amino acid addition, elimination or substitution reaction with no more than 2 amino acid differences from the amino acid sequence shown in SEQ ID NO: 1; HCDR2 comprises the amino acid sequence shown in SEQ ID NO: 2 or an amino acid sequence obtained by amino acid addition, elimination or substitution reaction with no more than 2 amino acid differences from the amino acid sequence shown in SEQ ID NO: 2; the HCDR3 comprising the amino acid sequence shown in SEQ ID NO: 3 or an amino acid sequence obtained by amino acid addition, elimination or substitution reaction and having no more than 2 amino acid differences from the amino acid sequence shown in SEQ ID NO: 3.

In certain embodiment, wherein the anti-PD-L1 antibody or antigen-binding fragment thereof comprises an antibody heavy chain variable region, wherein the heavy chain variable region comprises an amino acid sequence selected from the group consisting of: (a) the amino acid sequence set forth in SEQ ID NO: 4; (b) an amino acid sequence at least about 85%, 90%, 95% or 99% sequence identity to the amino acid sequence set forth in SEQ ID NO: 4; and (c) an amino acid sequence having 1 or more differences from the amino acid sequence shown in SEQ ID NO: 4 obtained by addition, elimination or substitution reaction.

In certain embodiment, wherein the TGFβ inhibitor binds to TGFβ1, TGFβ2 or TGFβ3.

In certain embodiment, wherein the TGFβ inhibitor binds to TGFβ or transforming growth factor β receptor (TGFβR).

In certain embodiment, wherein the TGFβ inhibitor binds to TGFβ type I receptor (TGFβRI), TGFβ type II receptor (TGFβRII) or TGFβ type III receptor (TGFβRIII).

In certain embodiment, wherein the TGFβ inhibitor comprises: 1) an anti-TGFβ antibody or an antigen-binding fragment thereof; 2) an anti-TGFβR antibody or an antigen-binding fragment thereof; 3) small molecule TGFβ inhibitors; 4) proteins comprising transforming growth factor β receptor II (TGFβRII) or functionally active fragments thereof, or variants or biosimilars thereof, or combinations thereof.

In certain embodiment, wherein said anti-TGFβ antibody or antigen-binding fragment thereof comprises an antibody heavy chain variable region, wherein said heavy chain variable region comprises HCDR1, HCDR2, HCDR3, each of which has at least about 80% sequence identity with HCDR1, HCDR2, and HCDR3 of the fresolimumab molecule.

In certain embodiment, wherein the anti-TGFβ antibody or its antigen-binding fragment comprises an antibody heavy chain variable region, wherein the heavy chain variable region has at least about 80% sequence identity to the heavy chain variable region of Fresolimumab.

In certain embodiment, wherein the anti-TGFβ antibody or its antigen-binding fragment comprises an antibody heavy chain, wherein the heavy chain has at least about 80% sequence identity to the heavy chain of Fresolimumab.

In certain embodiment, wherein said anti-TGFβ antibody or antigen-binding fragment thereof comprises an antibody's light chain variable region, wherein said light chain variable region comprises LCDR1, LCDR2 , LCDR3, each of which has at least about 80% sequence identity with LCDR1, LCDR2, and LCDR3 of the Fresolimumab.

In certain embodiment, wherein the anti-TGFβ antibody or its antigen-binding fragment comprises an antibody light chain variable region, wherein the light chain variable region has at least about 80% sequence identity to the light chain variable region.

In certain embodiment, wherein the anti-TGFβ antibody, or antigen-binding fragment thereof, comprises a light chain of an antibody, wherein the light chain has at least about 80% sequence identity with the light chain of a Fresolimumab molecule.

In certain embodiment, wherein the anti-TGFβ antibody or antigen-binding fragment thereof comprises: Fresolimumab, or an antigen-binding fragment thereof, or a variant or biosimilar thereof.

In certain embodiment, wherein the small molecule TGFβ inhibitor comprises the following compound: SB525334, SD-208, SB431542, LY2109761, LY2157299, GW788388, RepSox, SIS3, LDN-193189, EW-7197, LY364947, or a combination thereof.

In certain embodiment, wherein the TGFβRII comprises a polypeptide having a wild-type human TGFβ receptor type 2 isoform A sequence(For example, the amino acid sequence of NCBI reference sequence (Ref Seq) accession

number NP_001020018), or a polypeptide having a wild-type human TGFβ receptor type 2 isoform B sequence(For example, the amino acid sequence of NCBI reference sequence (Ref Seq) accession number NP_003233), or a polypeptide having a sequence substantially identical to an amino acid sequence thereof.

In certain embodiment, wherein said TGFβ RII or its functionally active fragment comprises: human TGFβRII extracellular domain (ECD), any part of NCBI Ref Seq NO. NP_001020018 or NCBI Ref Seq NO. NP_003233, or an amino acid sequence substantially identical to sequence.

In certain embodiment, wherein the TGFβRII or a functionally active fragment thereof comprises:

(a) the amino acid sequence of SEQ ID NO: 6;
(b) an amino acid sequence having at least about 85% sequence identity to the amino acid sequence set forth in SEQ ID NO: 6; Or
(c) has an amino acid sequence in which one or more amino acids are added, deleted and/or substituted as compared to the amino acid sequence shown in SEQ ID NO: 6.

In certain embodiment, wherein the bifunctional immune checkpoint/TGFβ inhibitor is a fusion protein.

In certain embodiment, wherein the PD-L1/TGFβ dual inhibitor or PD-1/TGFβdual inhibitor is a fusion protein.

In certain embodiment, wherein the PD-L1/TGFβ dual inhibitor comprises a PD-L1 targeting moiety and a TGFβ receptor domain comprising transforming growth factor beta receptor II (TGF β RII) or a functionally active fragment thereof.

In certain embodiment, wherein the PD-L1//TGF β dual inhibitor comprises a polypeptide, wherein the polypeptide comprises at least: (i) a heavy chain variable region of an anti-PD-L1 antibody; and (ii) TGFβII or a functionally active fragment thereof.

In certain embodiment, wherein the anti-PD-L1 antibody heavy chain variable region comprises HCDR1, HCDR2, HCDR3; Wherein the HCDR1 comprises an amino acid sequence having at least about 70% sequence identity to the amino acid sequence set forth in SEQ ID NO: 1, the HCDR2 comprises an amino acid sequence having at least about 70% sequence identity to the amino acid sequence set forth in SEQ ID NO: 2, and the HCDR3 comprises an amino acid sequence having at least about 70% sequence identity to the amino acid sequence set forth in SEQ ID NO: 3; or wherein the HCDR1, HCDR2, HCDR3 have at least about 80% sequence identity to the HCDR1, HCDR2, HCDR3, respectively, of Atezolizumab, Avelumab, BMS-936559, MPDL3280A (RG7446), or durvalumab (MEDI-4736).

In certain embodiment, wherein the anti-PD-L1 antibody heavy chain variable region comprises HCDR1, HCDR2, HCDR3, and the HCDR1 comprises the amino acid sequence shown in SEQ ID NO: 1 or an amino acid sequence obtained by amino acid addition, elimination or substitution reaction and having no more than 2 amino acid differences from the amino acid sequence shown in SEQ ID NO: 1; The HCDR2 comprises the amino acid sequence represented by SEQ ID NO: 2 or an amino acid sequence obtained by amino acid addition, elimination or substitution reaction and having no more than 2 amino acid differences from the amino acid sequence represented by SEQ ID NO: 2; the HCDR3 comprises an amino acid sequence represented by SEQ ID NO: 3 or an amino acid sequence obtained by an amino acid addition, elimination or substitution reaction and having no more than 2 amino acid differences from the amino acid sequence represented by SEQ ID NO: 3.

In certain embodiment, wherein the anti-PD-L1 antibody heavy chain variable region comprises an amino acid sequence selected from the group consisting of: (a) the amino acid sequence set forth in SEQ ID NO: 4; (b) an amino acid sequence having at least about 85%, 90%, 95%, or 99% sequence identity to the amino acid sequence set forth in SEQ ID NO: 4; (c) an amino acid sequence having 1 or more differences from the amino acid sequence shown in SEQ ID NO: 4 obtained by addition, elimination or substitution; And (d) an amino acid sequence having at least about 80% sequence identity to the heavy chain variable region of Atezolizumab, Avelumab, BMS-936559, MPDL3280A, or durvalumab.

In certain embodiment, wherein the TGFβRII or a functionally active fragment thereof comprises:

(a) the amino acid sequence of SEQ ID NO: 6;
(b) an amino acid sequence having at least about 85% sequence identity to the amino acid sequence set forth in SEQ ID NO: 6; Or
(c) has an amino acid sequence in which one or more amino acids are added, deleted and/or substituted as compared to the amino acid sequence shown in SEQ ID NO: 6.

In certain embodiment, wherein the polypeptide further comprises a linker that links the C-terminus of the heavy chain variable region of the anti-PD-L1 antibody or antigen-binding fragment thereof to the N-terminus of the TGFβRII or functionally active fragment thereof.

In certain embodiment, said polypeptide further comprising CH2, CH3 domains, said polypeptide comprising the heavy chain variable region (VH) of anti-PD-L1 antibody, CH2, CH3 domains and TGF βRII or a functionally active

fragment thereof in sequence from N-terminus to C-terminus, said CH3 domain having its C-terminus connected to the N-terminus of said TGF βRII or a functionally active fragment thereof by a linker.

In certain embodiment, wherein the CH2, CH3 domains are derived from IgG. For example, said CH2, CH3 are derived from IgG1, IgG2, IgG3 or IgG4.

In certain embodiment, wherein the linker is a peptide linker.

In certain embodiment, wherein the amino acid sequence of the peptide linker is $(G_4S)_x$ , wherein x is any integer from 3 to 6.

In certain embodiment, wherein the peptide linker comprises an amino acid sequence selected from the group consisting of: (a) the amino acid sequence set forth in SEQ ID NO: 5; (b) an amino acid sequence having at least about 85%, 90%, 95%, or 99% sequence identity to the amino acid sequence set forth in SEQ ID NO: 5; And (c) an amino acid sequence having 1 or more differences from the amino acid sequence shown in SEQ ID NO: 5 obtained by addition, elimination or substitution reaction.

In certain embodiment, wherein the polypeptide comprises an amino acid sequence selected from the group consisting of: (a) the amino acid sequence set forth in SEQ ID NO: 7; (b) an amino acid sequence having at least about 85%, 90%, 95%, or 99% sequence identity to the amino acid sequence set forth in SEQ ID NO: 7; (c) an amino acid sequence having 1 or more differences from the amino acid sequence shown in SEQ ID NO: 7 obtained by addition, elimination or substitution reactions.

In certain embodiment, wherein the PD-L1//TGFβ dual inhibitor comprises two aforementioned polypeptides.

In certain embodiment, wherein the PD-L1/TGFβ dual inhibitor comprises a first polypeptide and a second polypeptide, wherein the first polypeptide comprises at least: (i) a heavy chain of an anti-PD-L1 antibody variable region; and (ii) TGFβRII or a functionally active fragment thereof; wherein the second polypeptide comprises at least the light chain variable region of an anti-PD-L1 antibody; wherein the heavy chain variable region of the first polypeptide and the light chain variable regions of the second polypeptide are capable of specifically binding to PD-L1.

In certain embodiment, wherein the first polypeptide further comprises a linker, said linker connects the C-terminus of the heavy chain variable region of the anti-PD-L1 antibody or antigen-binding fragment thereof with the TGFβRII or the N-terminus of its functionally active fragments.

In certain embodiment, wherein the PD-L1/TGFβ dual inhibitor comprises a first polypeptide and a second polypeptide, wherein, the first polypeptide comprises the heavy chain variable region (VH) of the anti-PD-L1 antibody, the CH1 domain, and TGFβRII or its functionally active fragment sequentially from the N-terminus to the C-terminus, and the C-terminus of the CH1 domain is connected to the TGFβRII or its functionally active fragment N-terminus is connected by a linker; and the second polypeptide comprises a light chain variable region (VL) and a light chain constant region (CL) of an anti-PD-L1 antibody from the N-terminus to the C-terminus ).

In certain embodiment, wherein the said linker is peptide linker.

In certain embodiment, wherein the first polypeptide has at least about 80% sequence identity to a polypeptide having the same function as: M7824 or SHR-1701.

In certain embodiment, wherein the second polypeptide has at least about 80% sequence identity to a polypeptide having the same function as: M7824 or SHR-1701.

In certain embodiment, wherein the PD-L1/TGFβ dual inhibitor comprises M7824, SHR-1702, or variant or biosimilar or their combination.

In certain embodiment, wherein the PI3K inhibitor is PI3K8/y dual inhibitor.

In certain embodiment, wherein the said PI3K inhibitors comprise compounds as formula(IA-I), (IA-11), (IA-III), (IA-IV):

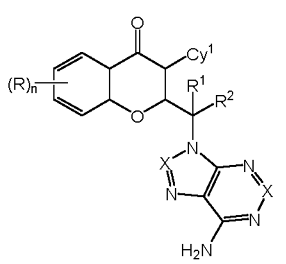
(IA-I)

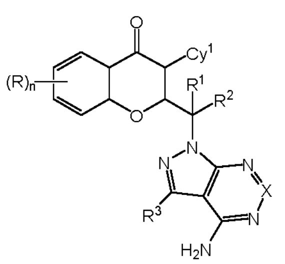
(IA-II)

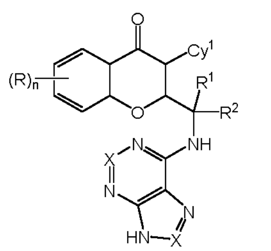
(IA-III)

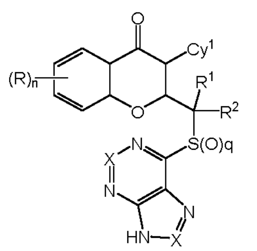
(IA-IV)

or their enantiomers, mixtures of enantiomers, mixtures of two or more diastereoisomers, isotopic variants, pharmaceutically acceptable salts, solvates, hydrates or prodrug, wherein,

each occurrence of R is independently selected from hydrogen, halogen, $-OR^a$, CN, substituted or unsubstituted $C_{1-6}$ alkyl, substituted or unsubstituted $C_{2-6}$ alkenyl, substituted or unsubstituted $C_{2-6}$ alkynyl, substituted or unsub-

stituted $C_{3-8}$ cycloalkyl, and substituted or unsubstituted heterocyclic group;

$R^1$ and $R^2$ may be the same or different and are independently selected from hydrogen, halogen, and substituted or unsubstituted $C_{1-6}$ alkyl, or both $R^1$ and$R^2$ directly bound to a common atom, may be joined to form an oxo group (=O) or a substituted or unsubstituted saturated or unsaturated 3-10 member ring, including the carbon atom to which $R^1$ and $R^2$ are bound, which may optionally include one or more heteroatoms which may be the same or different and are selected from O, $NR^a$ and S;

$Cy^1$ is selected from substituted or unsubstituted $C_{3-8}$ cycloalkyl, substituted or unsubstituted 3 to 15 membered heterocyclic groups having at least one heteroatom selected from N, O and S, substituted or unsubstituted $C_{6-20}$ aryl and substituted or unsubstituted 5 to 14 membered heteroaryl monocyclic groups with one or more heteroatoms selected from N, O and S;

each occurrence of $R^a$ may be the same or different and are independently selected from hydrogen, halogen, hydroxy, cyano, substituted or unsubstituted $C_{1-6}$alkyl, - $NR^cR^d$ , wherein $R^c$ and $R^d$ are independently hydrogen, halogen, hydroxy, cyano, substituted or unsubstituted $C_{1-6}$alkyl, and $C_1$-$_6$alkoxy, and -$OR^c$ , wherein $R^c$ is substituted or unsubstituted $C_{1-6}$alkyl ,

n is an integer from 1 to 4;

q is 0, 1 or 2;

each occurrence of X is independently selected from $CR^3$ or N;

and each occurrence of $R^3$ is independently selected from hydrogen, hydroxy, halogen, carboxyl, cyano, nitro, substituted or unsubstituted $C_{1-8}$ alkyl, substituted or unsubstituted $C_{1-8}$ alkoxy, substituted or unsubstituted $C_{2-10}$ alkenyl, substituted or unsubstituted $C_{2-10}$ alkynyl, substituted or unsubstituted $C_{6-20}$ aryl, substituted or unsubstituted $C_{6-20}$ aryl $C_{1-8}$alkyl, substituted or unsubstituted $C_{3-20}$ cycloalkyl,

substituted or unsubstituted $C_{3-20}$ cycloalkyl $C_{1-8}$ alkyl, substituted or unsubstituted $C_{3-8}$ cycloalkenyl $C_{1-8}$ alkyl, substituted or unsubstituted $C_{3-8}$ cycloalkene, substituted or unsubstituted 5-14-membered heteroaryl with one or more heteroatoms selected from N, O and S, substituted or unsubstituted with one or more heteroatoms selected from N, O, S 5-14 membered heteroaryl $C_{1-8}$ alkyl, substituted or unsubstituted 3 to 15 membered heterocycles having at least one heteroatom selected from N, O and S, substituted or unsubstituted 3 to 15 membered heterocyclyl $C_{1-8}$ alkyl rings having at least one heteroatom selected from N, O and S, substituted or unsubstituted guanidine, -$COOR^x$, -$C(O)R^x$, -$C(S)R^x$, -$C(O)NR^xR^y$, -$C(O)ONR^xR^y$, -$NR^yR^z$, -$NR^xCONR^yR^z$, - $N(R^x)SOR^y$, - $N(R^x)SO_2R^y$, -(=N-$N(R^x)R^y$), - $NR^xC(O)OR^y$, -$NR^xR^y$, -$NR^xC(O)R^y$-, - $NR^xC(S)R^y$ -$NR^xC(S)NR^yR^z$, -$SONR^xR^y$-, -$S0_2NR^xR^y$-, -$OR^x$, -$OR^xC(O)NR^yR^z$, - $OR^xC(O)OR^y$-, -$OC(O)R^x$, -$OC(O)NR^xR^y$, - $R^xNR^yC(O)R^z$, -$R^xOR^y$, -$R^xC(O)ORY$, - $R^xC(O)NR^yR^z$, -$R^xC(O)R^x$, -$R^xOC(O)R^y$, -$SR^x$, -$SOR^x$, - $SO_2R^x$, -$ONO_2$,

wherein $R^x$, $R^y$ and $R^z$ in each of the above groups can be hydrogen, substituted or unsubstituted $C_{1-8}$ alkyl, substituted or unsubstituted $C_{1-8}$ alkoxy, substituted or unsubstituted $C_{2-10}$ alkenyl, substituted or unsubstituted $C_{2-12}$ alkynyl, substituted or unsubstituted $C_{6-20}$ aryl, substituted or unsubstituted $C_{6-20}$ aryl $C_{1-8}$ alkyl , substituted or unsubstituted $C_{3-20}$ cycloalkyl, substituted or unsubstituted $C_{3-20}$ cycloalkyl$C_{1-8}$ alkyl, substituted or unsubstituted $C_{3-8}$ cycloalkenyl, substituted or unsubstituted 5 to 14 membered heteroaryl with one or more heteroatoms selected from N, O and S, substituted or unsubstituted heteroaryl with one or more heteroatoms selected from N, O and S atomic 5 to 14 membered heteroaryl $C_{1-8}$ alkyl, substituted or unsubstituted 3 to 15 membered heterocycle having at least one heteroatom selected from N, O and S, substituted or unsubstituted 3 to 15 membered heterocyclyl $C_{1-8}$ alkyl ring having at least one heteroatom selected from N, O and S or substituted or unsubstituted amino, or any two of $R^x$, $R^y$ and $R^z$ may be joined to form a substituted or unsubstituted saturated or unsaturated 3-10 membered ring, which may optionally include heteroatoms which may be the same or different and are selected from O, $NR^x$ or S, wherein $R^x$ is hydrogen or substituted or unsubstituted alkyl.

In certain embodiment, wherein the PI3K inhibitor comprises compounds as formula (IA-V):

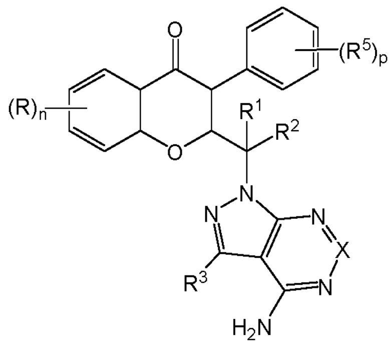

(IA-V)

or their enantiomers, mixtures of enantiomers, mixtures of two or more diastereoisomers, isotopic variants, pharmaceutically acceptable salts, solvates, hydrates or prodrug, wherein

each occurrence of R is independently selected from hydrogen, halogen, -$OR^a$, CN, substituted or unsubstituted $C_{1-6}$ alkyl, substituted or unsubstituted $C_{2-6}$ alkenyl, substituted or unsubstituted $C_{2-6}$ alkynyl, substituted or unsubstituted $C_{3-8}$ cycloalkyl, and substituted or unsubstituted heterocyclic group;

$R^1$ and $R^2$ may be the same or different and are independently selected from hydrogen, halogen, and substituted or unsubstituted $C_{1-6}$ alkyl, or both $R^1$ and$R^2$ directly bound to a common atom, may be joined to form an oxo group (=O) or a substituted or unsubstituted saturated or unsaturated 3-10 member ring, including the carbon atom to which $R^1$ and $R^2$ are bound, which may optionally include one or more heteroatoms which may be the same or different and are selected from O, $NR^a$ and S;

each occurrence of X is independently selected from $CR^3$ or N; and

each occurrence of $R^3$ is independently selected from hydrogen, hydroxy, halogen, carboxyl, cyano, nitro, substituted or unsubstituted $C_{1-8}$ alkyl, substituted or unsubstituted $C_{1-8}$ alkoxy, substituted or unsubstituted $C_{2-10}$ alkenyl, substituted or unsubstituted $C_{2-10}$ alkynyl, substituted or unsubstituted $C_{6-20}$ aryl, substituted or unsubstituted $C_{6-20}$ aryl $C_{1-8}$alkyl, substituted or unsubstituted $C_{3-20}$ cycloalkyl, substituted or unsubstituted $C_{3-20}$ cycloalkyl $C_{1-8}$ alkyl, substituted or unsubstituted $C_{3-8}$ cycloalkenyl $C_{1-8}$ alkyl, substituted or unsubstituted $C_{3-8}$ cycloalkene, substituted or unsubstituted 5-14-membered heteroaryl with one or more heteroatoms selected from N, O and S, substituted or unsubstituted with one or more heteroatoms selected from N, O, S 5-14 membered heteroaryl $C_{1-8}$ alkyl, substituted or unsubstituted 3 to 15 membered heterocycles having at least one heteroatom selected from N, O and S, substituted or unsubstituted 3 to 15 membered heterocyclyl $C_{1-8}$ alkyl rings having at least one heteroatom selected from N, O and S, substituted or unsubstituted guanidine, - $COOR^x$, -$C(O)R^x$, -$C(S)R^x$, -$C(O)NR^xR^y$, -$C(O)ONR^xR^y$, -$NR^yR^z$, -$NR^xCONR^yR^z$, - $N(R^x)SOR^y$, - $N(R^x)SO_2R^y$, -(=N-$N(R^x)R^y$), - $NR^xC(O)OR^y$, -$NR^xR^y$, -$NR^xC(O)R^y$-, - $NR^xC(S)R^y$ -$NR^xC(S)NR^yR^z$, -$SONR^xR^y$-, -$S0_2NR^xR^y$-, -$OR^x$, -$OR^xC(O)NR^yR^z$, - $OR^xC(O)OR^y$-, -$OC(O)R^x$, -$OC(O)NR^xR^y$, - $R^xNR^yC(O)R^z$, -$R^xOR^y$, -$R^xC(O)ORY$, - $R^xC(O)NR^yR^z$, -$R^xC(0)R^x$, -$R^xOC(O)R^y$, -$SR^X$, -$SOR^x$, - $SO_2R^x$, -$ONO_2$, wherein $R^x$, $R^y$ and $R^z$ in each of the above groups can be hydrogen, substituted or unsubstituted $C_{1-8}$ alkyl, substituted or unsubstituted $C_{1-8}$ alkoxy, substituted or unsubstituted $C_{2-10}$ alkenyl, substituted or unsubstituted $C_{2-12}$ alkynyl, substituted or unsubstituted $C_{6-20}$ aryl, substituted or unsubstituted $C_{6-20}$ aryl $C_{1-8}$ alkyl , substituted or unsubstituted $C_{3-20}$ cycloalkyl, substituted or unsubstituted $C_{3-20}$ cycloalkyl$C_{1-8}$ alkyl, substituted or unsubstituted $C_{3-8}$ cycloalkenyl, substituted or unsubstituted 5 to 14 membered heteroaryl with one or more heteroatoms selected from N, O and S, substituted or unsubstituted heteroaryl with one or more heteroatoms selected from N, O and S atomic 5 to 14 membered heteroaryl $C_{1-8}$ alkyl, substituted or unsubstituted 3 to 15 membered heterocycle having at least one heteroatom selected from N, O and S, substituted or unsubstituted 3 to 15 membered heterocyclyl $C_{1-8}$ alkyl ring having at least one heteroatom selected from N, O and S or substituted or unsubstituted amino, or any two of $R^x$, $R^y$ and $R^z$ may be joined to form a substituted or unsubstituted saturated or unsaturated 3-10 membered ring, which may optionally include heteroatoms which may be the same or different and are selected from O, $NR^x$ or S, wherein $R^x$ is hydrogen or substituted or unsubstituted alkyl,

each occurrence of $R^5$ is hydrogen, $C_{1-6}$ alkyl or halogen,

n is 0, 1, 2, 3 or 4; and

Pis 0, 1, 2, 3, 4 or 5.

In certain embodiment, wherein the PI3K inhibitor comprises compounds as formula (IA-VI):

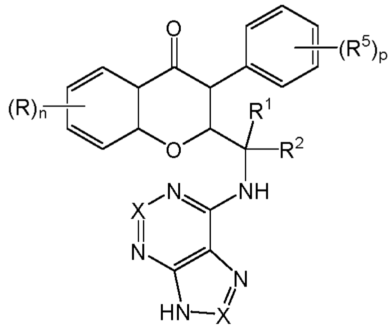

(IA-VI)

or their enantiomers, mixtures of enantiomers, mixtures of two or more diastereoisomers, isotopic variants, pharmaceutically acceptable salts, solvates, hydrates or prodrug, wherein
each occurrence of R is independently selected from hydrogen, halogen, -$OR^a$, CN, substituted or unsubstituted $C_{1-6}$ alkyl, substituted or unsubstituted $C_{2-6}$ alkenyl, substituted or unsubstituted $C_{2-6}$ alkynyl, substituted or unsubstituted $C_{3-8}$ cycloalkyl, and substituted or unsubstituted heterocyclic group;
$R^1$ and $R^2$ may be the same or different and are independently selected from hydrogen, halogen, and substituted or unsubstituted $C_{1-6}$ alkyl, or both $R^1$ and$R^2$ directly bound to a common atom, may be joined to form an oxo group (=O) or a substituted or unsubstituted saturated or unsaturated 3-10 member ring, including the carbon atom to which $R^1$ and $R^2$ are bound, which may optionally include one or more heteroatoms which may be the same or different and are selected from O, $NR^a$ and S;
each occurrence of X is independently selected from $CR^3$ or N; and
each occurrence of $R^3$ is independently selected from hydrogen, hydroxy, halogen, carboxyl, cyano, nitro, substituted or unsubstituted $C_{1-8}$ alkyl, substituted or unsubstituted $C_{1-8}$ alkoxy, substituted or unsubstituted $C_{2-10}$ alkenyl, substituted or unsubstituted $C_{2-10}$ alkynyl, substituted or unsubstituted $C_{6-20}$ aryl, substituted or unsubstituted $C_{6-20}$ aryl $C_{1-8}$alkyl, substituted or unsubstituted $C_{3-20}$ cycloalkyl, substituted or unsubstituted $C_{3-20}$ cycloalkyl $C_{1-8}$ alkyl, substituted or unsubstituted $C_{3-8}$ cycloalkenyl $C_{1-8}$ alkyl, substituted or unsubstituted $C_{3-8}$ cycloalkene, substituted or unsubstituted 5-14-membered heteroaryl with one or more heteroatoms selected from N, O and S, substituted or unsubstituted with one or more heteroatoms selected from N, O, S 5-14 membered heteroaryl $C_{1-8}$ alkyl, substituted or unsubstituted 3 to 15 membered heterocycles having at least one heteroatom selected from N, O and S, substituted or unsubstituted 3 to 15 membered heterocyclyl $C_{1-8}$ alkyl rings having at least one heteroatom selected from N, O and S, substituted or unsubstituted guanidine, - $COOR^x$, -$C(O)R^x$, -$C(S)R^x$, -$C(O)NR^xR^y$, -$C(O)ONR^xR^y$, -$NR^yR^z$, -$NR^xCONR^yR^z$, - $N(R^x)SOR^y$, - $N(R^x)SO_2R^y$, -(=N-$N(R^x)R^y$), - $NR^xC(O)OR^y$, -$NR^xR^y$, -$NR^xC(O)R^y$-, - $NR^xC(S)R^y$ -$NR^xC(S)NR^yR^z$, -$SONR^xR^y$-, -$S0_2NR^xR^y$-, -$OR^x$, -$OR^xC(O)NR^yR^z$, - $OR^xC(O)OR^y$, -$OC(O)R^x$, -$OC(O)NR^xR^y$, - $R^XNR^yC(O)R^z$, -$R^xOR^y$, -$R^xC(O)ORY$, - $R^xC(O)NR^yR^z$, -$R^xC(0)R^x$, -$R^xOC(O)R^y$, -$SR^x$, -$SOR^x$, - $SO_2R^x$, -$ONO_2$, wherein $R^x$, $R^y$ and $R^z$ in each of the above groups can be hydrogen, substituted or unsubstituted $C_{1-8}$ alkyl, substituted or unsubstituted $C_{1-8}$ alkoxy, substituted or unsubstituted $C_{2-10}$ alkenyl, substituted or unsubstituted $C_{2-12}$ alkynyl, substituted or unsubstituted $C_{6-20}$ aryl, substituted or unsubstituted $C_{6-20}$ aryl $C_{1-8}$ alkyl , substituted or unsubstituted $C_{3-20}$ cycloalkyl, substituted or unsubstituted $C_{3-20}$ cycloalkyl$C_{1-8}$ alkyl, substituted or unsubstituted $C_{3-8}$ cycloalkenyl, substituted or unsubstituted 5 to 14 membered heteroaryl with one or more heteroatoms selected from N, O and S, substituted or unsubstituted heteroaryl with one or more heteroatoms selected from N, O and S atomic 5 to 14 membered heteroaryl $C_{1-8}$ alkyl, substituted or unsubstituted 3 to 15 membered heterocycle having at least one heteroatom selected from N, O and S, substituted or unsubstituted 3 to 15 membered heterocyclyl $C_{1-8}$ alkyl ring having at least one heteroatom selected from N, O and S or substituted or unsubstituted amino, or any two of $R^x$, $R^y$ and $R^z$ may be joined to form a substituted or unsubstituted saturated or unsaturated 3-10 membered ring, which may optionally include heteroatoms which may be the same or different and are selected from O, $NR^x$ or S, wherein $R^x$ is hydrogen or substituted or unsubstituted alkyl,
each occurrence of $R^5$ is hydrogen, $C_{1-6}$ alkyl or halogen,
n is 0, 1, 2, 3 or 4; and
Pis 0, 1, 2, 3, 4 or 5.

In certain embodiment, wherein R is hydrogen, halogen, substituted or unsubstituted $C_{1-6}$ alkyl or $OR^a$.
In certain embodiment, wherein $R^a$ is alkyl.
In certain embodiment, wherein $Cy^1$ is selected from:

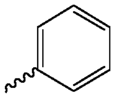 、 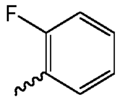 、 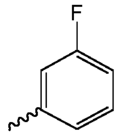 、 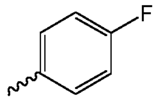 、 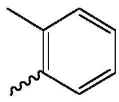 or 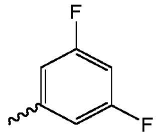 .

In certain embodiment, wherein $R^1$ and $R^2$ each independently represent hydrogen or substituted or unsubstituted $C_{1-6}$ alkyl.

In certain embodiment, wherein $R^3$ is iodo, cyano, substituted unsubstituted alkyl, substituted or unsubstituted alkynyl, substituted or unsubstituted aryl, substituted or unsubstituted heteroaryl.

In certain embodiment, wherein X is $CR^3$ and each occurrence of $R^3$ is independently hydrogen, halogen, hydroxyl or $NH_2$.

In certain embodiment,wherein $R^3$ is hydrogen.

In certain embodiment, wherein said PI3K inhibitors comprise:

2-[(6-Amino-9H-purin-9-yl) methyl]-6-bromo-3-phenyl-4H-chromen-4-one;
6-Bromo-2-(morpholinomethyl)-3-phenyl-4H-chromen-4-one;
6-Bromo-2-(morpholinomethyl)-3-phenyl-4H-chromen-4-one hydrochloride;
2-[(6-Amino-9H-purin-9-yl) methyl]-3-phenyl-4H-chromen-4-one;
2-(Morpholinomethyl)-3-phenyl-4H-chromen-4-one;
2-(Morpholinomethyl)-3-phenyl-4H-chromen-4-one hydrochloride;
2-[(1H-Benzo[d]imidazol-1-yl) methyl]-6-bromo-3-phenyl-4H-chromen-4-one;
6-Bromo-2-[(4-methyl-1H-benzo[d]imidazol-1-yl) methyl]-3-phenyl-4H-chromen-4-one;
2-[(1 H-benzo [d] imidazol- 1 -yl) methyl] -3-phenyl-4H-chromen-4-one;
2-[(4-methyl- 1 H-benzo [d] imidazol- 1 -yl)methyl] -3-phenyl-4H-chromen-4-one;
2-[(6-Chloro-9H-purin-9-yl)methyl]-3-phenyl-4H-chromen-4-one;
6-Bromo-2-[(6-chloro-9H-purin-9-yl)methyl]-3-phenyl-4H-chromen-4-one;
2-((9H-Purin-6-ylthio)methyl)-3-phenyl-4H-chromen-4-one;
2- [(1 H-Imidazol- 1 -yl)methyl] -3-phenyl-4H-chromen-4-one ;
2-[(9H-Purin-6-ylthio)methyl]-6-bromo-3-phenyl-4H-chromen-4-one;
2-((4-Amino-1H-pyrazolo[3,4-d]pyrimidin-1-yl)methyl)-6-bromo-3-phenyl-4H-chromen-4-one ;
2-[(6-Amino-9H-purin-9-yl)methyl]-6-bromo-3-(4-fluorophenyl)-4H-chromen-4-one ;
2- [(6-Arnino-9H-purin-9-yl)methyl]-3-(4-fluorophenyl)-4H-chromen-4-one ; 6-Bromo-3-(4-fluorophenyl)-2-(morpholinomethyl)-4H-chromen-4-one ; 6-Bromo-3-(4-fluorophenyl)-2-(morpholinomethyl)-4H-chromen-4-one hydro chloride;
3- (4-fluorophenyl)-2-(morpholinomethyl)-4H-chromen-4-one ; 3-(4-fluorophenyl)-2-(morpholinomethyl)-4H-chromen-4-one hydrochloride ;
2-[(6-Amino-9H-purin-9-yl)methyl]-6-bromo-3-o-tolyl-4H-chromen-4-one;
7-[(6-Bromo-4-oxo-3-phenyl-4H-chromen-2-yl)methyl]-1,3-dimethyl-1H-purine- 2,6(3H,7H)-dione;
2-(1-(6-Amino-9H-purin-9-yl)ethyl)-6-bromo-3-phenyl-4H-chromen-4-one;
2-(l-(9H-Purin-6-ylthio)ethyl)-6-bromo-3-phenyl-4H-chromen-4-one;
2-( 1 -(6-Amino-9H-purin-9-yl)ethyl)-3-phenyl-4H-chromen-4-one;
(S)-2-( 1 -(9H-purin-6-ylamino)ethyl)-6-bromo-3-phenyl-4H-chromen-4-one ;
2-((9H-purin-6-ylamino)methyl)-6-bromo-3-phenyl-4H-chromen-4-one ;
2-( 1 -(4-amino- 1 H-pyrazolo [3 ,4-d]pyrimidin- 1 -yl)ethyl)-6-bromo-3-phenyl-4H-chromen-4- one ;
2-((6-Amino-9H-purin-9-yl)methyl)-6-methoxy-3-phenyl-4H-chromen-4-one;
2-(1-(6-Amino-9H-purin-9-yl)ethyl)-6-bromo-3-(2-fluorophenyl)-4H-chromen-4-one;
2-((6-Amino-9H-purin-9-yl)methyl)-6-bromo-3-(2-fluorophenyl)-4H-chromen-4-one ;
2-( 1 -(4-Amino- 1 H-pyrazolo [3,4-d]pyrimidin- 1 -yl)ethyl)-3 -phenyl-4H-chromen-4-one ;
2-( 1 -(6-Amino-9H-purin-9-yl) propyl)-3-phenyl-4H-chromen-4-one; 2-(l-(6-Amino-9H-purin-9-yl)ethyl)-3 -(3 -fluorophenyl)-4H-chromen-4-one;
2-((6-Amino-9H-purin-9-yl)methyl)-3-(2-fluorophenyl)-4H-chromen-4-one ;
2-( 1 -(6-Amino-9H-purin-9-yl)ethyl)-3-(2-fluorophenyl)-4H-chromen-4-one ;
2-(1-(6-Amino-9H-purin-9-yl)propyl)-3-(2-fluorophenyl)-4H-chromen-4-one;
2-(l-(6-amino-9H-purin-9-yl)propyl)-3-(3-fluorophenyl)-4H-chromen-4-one ;
2-(l-(6-Amino-9H-purin-9-yl)propyl)-3-(4-fluorophenyl)-4H-chromen-4-one ;
2-(l-(6-amino-9H-purin-9-yl)propyl)-6-fluoro-3-phenyl-4H-chromen-4-one;
2-(l-(6-Amino-9H-purin-9-yl)ethyl)-3-(4-fluorophenyl)-4H-chromen-4-one;

2-(l-(6-Amino-9H-purin-9-yl)ethyl)-6-fluoro-3-phenyl-4H-chromen-4-one;
2-(1-(4-Amino-3-(3-methoxyphenyl)-1H-pyrazolo[3,4-d]pyrimidin-1-yl)ethyl)-3-phenyl-4H-chromen-4-one;
2-( 1 -(4-Amino-3-(3-hydroxyphenyl)- 1 H-pyrazolo [3 ,4-d]pyrimidin- 1 -yl)ethyl)-3 -phenyl-4H- chromen-4-one;
2-((9H-purin-6-ylamino)methyl)-3-phenyl-4H-chromen-4-one;
2-( 1 -(6-Amino-9H-purin-9-yl)ethyl)-3-o-tolyl-4H-chromen-4-one;
2-((9H-purin-6-ylamino)methyl)-3-(2-fluorophenyl)-4H-chromen-4-one;
2-((9H-purin-6-ylamino)methyl)-3-(3-fluorophenyl)-4H-chromen-4-one;
(S)-2-(1-(9H-purin-6-ylamino)ethyl)-3-(3-fluorophenyl)-4H-chromen-4-one;
2-(1-(6-amino-9H-purin-9-yl)ethyl)-6-fluoro-3-(2-fluorophenyl)-4H-chromen-4-one;
2-(1-(6-Amino-9H-purin-9-yl)ethyl)-3-(3,5-difluorophenyl)-4H-chromen-4-one;
2-(1-(6-amino-9H-purin-9-yl)ethyl)-6-fluoro-3-(3-fluorophenyl)-4H-chromen-4-one;
2-(1-(4-amino-3-(3-methoxyphenyl)-1H-pyrazolo[3,4-d]pyrimidin-1-yl)ethyl)-3-(3-fluorophenyl)-4H-chromen-4-one;
2-( 1 -(4-amino-3-(3-hydroxyphenyl)- 1 H-pyrazolo [3, 4-d] pyrimidin-l-yl) ethyl)-3-(3-fluorophenyl)-4H-chromen-4-one;
2-((4-Amino-3-(3-methoxyphenyl)-1H-pyrazolo [3, 4-d] pyrimidin-l-yl) methyl)-3-phenyl- 4H-chromen-4-one;
2-((4-Amino-3-(3-hydroxyphenyl)-1H-pyrazolo [3, 4-d] pyrimidin-l-yl) methyl)-3-phenyl- 4H-chromen-4-one;
2-((4-amino-3-(3-methoxyphenyl)-1H-pyrazolo [3, 4-d] pyrimidin-l-yl) methyl)-3-(3-fluorophenyl)-4H-chromen-4-one;
2-((4-amino-3-(3-hydroxyphenyl)-lH-pyrazolo [3, 4-d] pyrimidin-1-yl) methyl)-3-(3-fluorophenyl)-4H-chromen-4-one;
(R)-2-( 1 -(9H-purin-6-ylamino) ethyl)-3-(3-fluorophenyl)-4H-chromen-4-one;
(S)-2-(l-(9H-purin-6-ylamino) ethyl)-6-fluoro-3-phenyl-4H-chromen-4-one;
2-((4-Amino-3-iodo-1H-pyrazolo [3, 4-d] pyrimidin-1-yl) methyl)-3-phenyl-4H-chromen-4-one;
2-(1-(4-amino-3-iodo-1H-pyrazolo [3, 4-d] pyrimidin-l-yl) ethyl)-3-phenyl-4H-chromen-4- one;
2-(1-(4-amino-3-iodo-1H-pyrazolo [3, 4-d] pyrimidin-l-yl) ethyl)-3-(3-fluorophenyl)-4H-chromen-4-one;
2-((4-amino-3-iodo-1H-pyrazolo [3, 4-d] pyrimidin-l-yl) methyl)-6-fluoro-3-phenyl-4H-chromen-4-one;
2-(l-(4-amino-3-iodo-lH-pyrazolo [3, 4-d] pyrimidin-l-yl) ethyl)-6-fluoro-3-phenyl-4H-chromen-4-one;
2-(l-(4-amino-3-iodo-lH-pyrazolo [3, 4-d] pyrimidin-l-yl) ethyl)-6-fluoro-3-(3- fluorophenyl)-4H-chromen-4-one;
2-(l-(4-ammo-3-iodo-lH-pyrazolo [3, 4-d] pyrimidin-l-yl) propyl)-3-(3-fluorophenyl)-4H-chromen-4-one;
2-((4-amino-3-(pyridin-3-yl)-lH-pyrazolo [3, 4-d] pyrimidin-l-yl) methyl)-3-phenyl-4H-chromen-4-one;
2-((4-amino-3-(3-hydroxyprop-l-ynyl)-lH-pyrazolo [3, 4-d] pyrimidin-l-yl) methyl)-3- phenyl-4H-chromen-4-one;
2-((4-amino-3-(lH-pyrazol-4-yl)-lH-pyrazolo [3, 4-d] pyrimidin-l-yl) methyl)-3-phenyl-4H-chromen-4-one;
2-((4-amino-3-(3-(hydroxymethyl) phenyl)- lH-pyrazolo [3, 4-d] pyrimidin-l-yl) methyl)-3-phenyl-4H-chromen-4-one;
2-((4-amino-3-(lH-indazol-4-yl)-lH-pyrazolo [3, 4-d] pyrimidin-l-yl) methyl)-3-phenyl-4H-chromen-4-one;
2-((4-amino-3-(3-fluorophenyl)-lH-pyrazolo [3, 4-d] pyrimidin-l-yl) methyl)-3-phenyl-4H-chromen-4-one
2-((4-amino-3-(3-hydroxypropyl)-l H-pyrazolo [3, 4-d] pyrimidin-l-yl) methyl)-3-phenyl- 4H-chromen-4-one;
N-(3-(4-amino-l-((4-oxo-3-phenyl-4H-chromen-2-yl) methyl)- lH-pyrazolo [3, 4-d] pyrimidin-3-yl) phenyl) acetamide;
2-((4-amino-3-(3-fluoro-5-methoxyphenyl)-l H-pyrazolo [3, 4-d] pyrimidin-l-yl) methyl)-3-phenyl-4H-chromen-4-one;
2-((4-amino-3-(3-fluoro-5-hydroxyphenyl)-lH-pyrazolo [3, 4-d] pyrimidin-l-yl) methyl)-3-phenyl-4H-chromen-4-one;
2-((4-amino-3-(3-fluoro-5-methoxyphenyl)- 1 H-pyrazolo [3, 4-d] pyrimidin-l-yl) methyl)-3-(3-fluorophenyl)-4H-chromen-4-one;
2-((4-amino-3-(3-fluoro-5-hydroxyphenyl)-lH-pyrazolo [3, 4-d] pyrimidin-l-yl) methyl)-3- (3-fluorophenyl)-4H-chromen-4-one;
2-( 1 -(4-amino-3-( 1 H-pyrazol-4-yl)- 1 H-pyrazolo [3, 4-d] pyrimidin-l-yl) ethyl)-3-phenyl-4H- chromen-4-one;
2-(l-(4-amino-3-(lH-indazol-6-yl)-lH-pyrazolo [3, 4-d] pyrimidin-l-yl) ethyl)-3-phenyl-4H-chromen-4-one;
2-(l-(4-amino-3-(3-hydroxy-3-methylbut-l-ynyl)-lH-pyrazolo [3, 4-d] pyrimidin-l-yl) ethyl)-3-phenyl-4H-chromen-4-one;
2-( 1 -(4-amino-3-( 1 H-pyrazol-4-yl)- 1 H-pyrazolo [3, 4-d] pyrimidin-l-yl) ethyl)-3-(3-fluorophenyl)-4H-chromen-4-one;
(S)-2-( 1 -(9H-purin-6-ylamino) ethyl)-3-phenyl-4H-chromen-4-one;
(S)-2-( 1 -(9H-purin-6-ylamino) ethyl)-6-fluoro-3-(3-fluorophenyl)-4H-chromen-4-one;
2-((4-amino-3-(lH-indazol-6-yl)-lH-pyrazolo [3, 4-d] pyrimidin-l-yl) methyl)-3-phenyl-4H-chromen-4-one;
2-(l-(4-amino-3-(3-fluoro-5-methoxyphenyl)-lH-pyrazolo [3, 4-d] pyrimidin-l-yl) ethyl)-3- (3-fluorophenyl)-4H-chromen-4-one;
2-(l-(4-amino-3-(3-fluoro-5-hydroxyphenyl)-lH-pyrazolo [3, 4-d] pyrimidin-l-yl) ethyl)-3- (3-fluorophenyl)-4H-chromen-4-one;
2-(l-(4-amino-3-(lH-indazol-4-yl)-lH-pyrazolo [3, 4-d] pyrimidin-l-yl) ethyl)-3-(3-fluorophenyl)-4H-chromen-4-one;
2-(l-(4-amino-3-(3, 5-dimethyl-lH-pyrazol-4-yl)-lH-pyrazolo [3, 4-d] pyrimidin-l-yl) ethyl)-3-(3-fluorophenyl)-4H-chromen-4-one;

2-(l-(4-amino-3-(3-methyl-lH-indazol-6-yl)-lH-pyrazolo [3, 4-d] pyrimidin-l-yl) ethyl)-3- (3-fluorophenyl)-4H-chromen-4-one;
2-(l-(4-amino-3-(lH-indazol-6-yl)-lH-pyrazolo [3, 4-d] pyrimidin-l-yl) ethyl)-3-(3-fluorophenyl)-4H-chromen-4-one;
2-(l-(4-amino-3-(2-(hydroxymethyl) phenyl)- lH-pyrazolo [3, 4-d] pyrimidin-l-yl) ethyl)-3- (3-fluorophenyl)-4H-chromen-4-one;
2-(l-(4-amino-3-(4-fluoro-3-methoxyphenyl)-lH-pyrazolo [3, 4-d] pyrimidin-l-yl) ethyl)-3- (3-fluorophenyl)-4H-chromen-4-one;
2-(l-(4-amino-3-(4-fluoro-3-hydroxyphenyl)-lH-pyrazolo [3, 4-d] pyrimidin-l-yl) ethyl)-3- (3-fluorophenyl)-4H-chromen-4-one;
2-(l-(4-amino-3-(3-hydroxyprop-l-ynyl)-lH-pyrazolo [3, 4-d] pyrimidin-l-yl) ethyl)-3-(3-fluorophenyl)-4H-chromen-4-one;
2-(l-(4-amino-3-(3-fluoro-4-methoxyphenyl)-lH-pyrazolo [3, 4-d] pyrimidin-l-yl) ethyl)-3- (3-fluorophenyl)-4H-chromen-4-one;
2-(l-(4-amino-3-(3-fluoro-4-hydroxyphenyl)-lH-pyrazolo [3, 4-d] pyrimidin-l-yl) ethyl)-3- (3-fluorophenyl)-4H-chromen-4-one;
2-(l-(4-amino-3-(3-chloro-5-methoxyphenyl)-lH-pyrazolo [3, 4-d] pyrimidin-l-yl) ethyl)-3- (3-fluorophenyl)-4H-chromen-4-one;
2-(l-(4-amino-3-(3-chloro-5-hydroxyphenyl)-lH-pyrazolo [3, 4-d] pyrimidin-l-yl) ethyl)-3- (3-fluorophenyl)-4H-chromen-4-one;
2- (l-(4-amino-3-(3-(trifluoromethoxy) phenyl)- lH-pyrazolo [3, 4-d] pyrimidin-l-yl) ethyl)-3- (3-fluorophenyl)-4H-chromen-4-one;
2-(l-(4-amino-3-(4-methoxyphenyl)-lH-pyrazolo [3, 4-d] pyrimidin-l-yl) ethyl)-3-(3-fluorophenyl)-4H-chromen-4-one;
2-(l-(4-amino-3-(4-hydroxyphenyl)-lH-pyrazolo [3, 4-d] pyrimidin-l-yl) ethyl)-3-(3-fluorophenyl)-4H-chromen-4-one;
2-((6-amino-9H-purin-9-yl) methyl)-3 -(3 -fluorophenyl)-4H-chromen-4-one;
2-(l-(4-amino-3-(4-fluoro-2-methoxyphenyl)-lH-pyrazolo [3, 4-d] pyrimidin-l-yl) ethyl)-3- (3-fluorophenyl)-4H-chromen-4-one;
2-(l-(4-amino-3-(4-fluoro-2-hydroxyphenyl)-lH-pyrazolo [3, 4-d] pyrimidin-l-yl) ethyl)-3- (3-fluorophenyl)-4H-chromen-4-one;
2-((4-amino-3-(3-aminophenyl)-lH-pyrazolo [3, 4-d] pyrimidin-l-yl) methyl)-3-phenyl-4H-chromen-4-one;
2-((4-amino-3-(3-methyl-lH-indazol-6-yl)-lH-pyrazolo [3, 4-d] pyrimidin-l-yl) methyl)-3-phenyl-4H-chromen-4-one;
2-(1-(4-amino-3-(2-aminopyrimidin-5-yl)-1H-pyrazolo [3, 4-d] pyrimidin-l-yl) ethyl)-3-(3-fluorophenyl)-4H-chromen-4-one;
2-(l-(4-amino-3-(lH-indol-6-yl)-lH-pyrazolo [3, 4-d] pyrimidin-l-yl) ethyl)-3-(3-fluorophenyl)-4H-chromen-4-one;
2-(l-(4-amino-3-(4-chloro-3-methoxyphenyl)-lH-pyrazolo [3, 4-d] pyrimidin-l-yl) ethyl)-3- (3-fluorophenyl)-4H-chromen-4-one;
2-(l-(4-amino-3-(4-chloro-3-hydroxyphenyl)-lH-pyrazolo [3, 4-d] pyrimidin-l-yl) ethyl)-3- (3-fluorophenyl)-4H-chromen-4-one;
2-(l-(4-amino-3-(2-chloro-5-methoxyp enyl)-lH-pyrazolo [3, 4-d] pyrimidin-l-yl) ethyl)-3- (3-fluorophenyl)-4H-chromen-4-one;
2-(l-(4-amino-3-(2-chloro-5-hydroxyphenyl)-lH-pyrazolo [3, 4-d] pyrimidin-l-yl) ethyl)-3- (3-fluorophenyl)-4H-chromen-4-one;
2-(l-(4-amino-3-(3, 4-dimethoxyphenyl)-lH-pyrazolo [3, 4-d] pyrimidin-l-yl) ethyl)-3-(3-fluorophenyl)-4H-chromen-4-one;
2-(l-(4-amino-3-(3, 4-dihydroxyphenyl)- 1 H-pyrazolo [3, 4-d] pyrimidin-l-yl) ethyl)-3-(3-fluorophenyl)-4H-chromen-4-one;
2-((4-amino-3-(3-methyl- 1 H-indazol-6-yl)- 1 H-pyrazolo [3, 4-d] pyrimidin-l-yl) methyl)-3-(3-fluorophenyl)-4H-chromen-4-one;
2-(l-(4-amino-3-(lH-indol-5-yl)-lH-pyrazolo [3, 4-d] pyrimidin-l-yl) ethyl)-3-(3-fluorophenyl)-4H-chromen-4-one;
2-(l-(4-Amino-3-(3-methyl-lH-indol-5-yl)-lH-pyrazolo [3, 4-d] pyrimidin-l-yl) ethyl)-3-(3-fluorophenyl)-4H-chromen-4-one;
tert-butyl-(5-(4-amino-l-(l-(3-(3-fluorophenyl)-4-oxo-4H-chromen-2-yl) ethyl)- 1 H-pyrazolo [3, 4-d] pyrimidin-3-yl) thiophen-2-yl) methylcarbamate
2-(l-(4-amino-3-(5-(aminomethyl) thiophen-2-yl)-lH-pyrazolo [3, 4-d] pyrimidin-l-yl) ethyl)-3 -(3 -fluorophenyl)-4H-chromen-4-one;
2-((4-amino-3-(3-methyl- 1 H-indazol-6-yl)- 1 H-pyrazolo [3, 4-d] pyrimidin-l-yl) methyl)-6-fluoro-3-phenyl-4H-chromen-4-one;
2-( 1 -(4-amino-3-(3-methyl- 1 H-indazol-6-yl)- 1 H-pyrazolo [3, 4-d] pyrimidin-l-yl) ethyl)-3-phenyl-4H-chromen-4-one;

2-( 1 -(4-amino-3-(3-methyl- 1 H-indazol-6-yl)- 1 H-pyrazolo [3, 4-d] pyrimidin-l-yl) ethyl)-6-fluoro-3-phenyl-4H-chromen-4-one;
2-( 1 -(4-amino-3-(3-methyl- lH-indazol-5-yl)- 1 H-pyrazolo [3, 4-d] pyrimidin-l-yl) ethyl)-3-(3-fluorophenyl)-4H-chromen-4-one;
N-(4-(4-amino-l-(l-(3-(3-fluorophenyl)-4-oxo-4H-chromen-2-yl) ethyl)- 1 H-pyrazolo [3, 4-d] pyrimidin-3-yl) phenyl) acetamide;
2-(l-(4-amino-3-(4-aminophenyl)-l H-pyrazolo [3, 4-d] pyrimidin-l-yl) ethyl)-3-(3-fluorophenyl)-4H-chromen-4-one;
2-(l-(4-amino-3-(3-methyl-lH-indazol-6-yl)-lH-pyrazolo [3, 4-d] pyrimidin-l-yl) ethyl)-6-fluoro-3 -(3 -fluorophenyl)-4H-chromen-4-one;
2- (l-(4-amino-3-(2, 3-dihydrobenzofuran-5-yl)-l H-pyrazolo [3, 4-d] pyrimidin-l-yl) ethyl)-3-(3-fluorophenyl)-4H-chromen-4-one;
2-(l-(4-amino-3-(3-ethyl-lH-indazol-6-yl)-lH-pyrazolo [3, 4-d] pyrimidin-l-yl) ethyl)-3-(3-fluorophenyl)-4H-chromen-4-one;
2-( 1 -(4-amino-3-(3-methyl- 1 H-indol-6-yl)- 1 H-pyrazolo [3, 4-d] pyrimidin-l-yl) ethyl)-3-(3- fluorophenyl)-4H-chromen-4-one;
2-( 1 -(4-amino-3-(2-methoxypyrimidin-5-yl)- 1 H-pyrazolo [3, 4-d] pyrimidin-l-yl) ethyl)-3-(3-fluorophenyl)-4H-chromen-4-one;
4- (4-amino- 1-( 1 -(3-(3-fluorophenyl)-4-oxo-4H-chromen-2-yl) ethyl)- 1 H-pyrazolo [3, 4-d] pyrimidin-3-yl) thiophene-2-carbaldehyde;
2-(l-(4-amino-3-(5-(hydroxymethyl) thiophen-3-yl)-lH-pyrazolo [3, 4-d] pyrimidin-l-yl) ethyl)-3-(3-fluorophenyl)-4H-chromen-4-one;
2-(l-(4-amino-3-(2-methyl-lH-benzo[d]imidazol-5-yl)-lH-pyrazolo [3, 4-d] pyrimidin-l-yl) ethyl)-3-(3-fluorophenyl)-4H-chromen-4-one;
2-( 1 -(4-amino-3-(3-methyl- 1 H-indazol-6-yl)- 1 H-pyrazolo [3, 4-d] pyrimidin-l-yl) propyl)-3- (3-fluorophenyl)-4H-chromen-4-one;
2-(l-(4-amino-3-(3-methyl-lH-indol-6-yl)-lH-pyrazolo [3, 4-d] pyrimidin-l-yl) ethyl)-3-(3-fluorophenyl)-4H-chromen-4-one;
2-((6-amino-9H-purin-9-yl) methyl)-6-fluoro-3-phenyl-4H-chromen-4-one;
2-((6-amino-9H-purin-9-yl) methyl)-6-fluoro-3 -(3 -fluorophenyl)-4H-chromen-4-one;
2-((4-amino-3-(3-fluoro-5-methoxyphenyl)-lH-pyrazolo [3, 4-d] pyrimidin-l-yl) methyl)-6-fluoro-3 -(3 -fluorophenyl)-4H-chromen-4-one;
2-((4-amino-3-(3-fluoro-5-hydroxyphenyl)-lH-pyrazolo [3, 4-d] pyrimidin-l-yl) methyl)-6-fluoro-3 -(3 -fluorophenyl)-4H-chromen-4-one;
2-(l-(4-amino-3-(3-methoxyphenyl)-lH-pyrazolo [3, 4-d] pyrimidin-l-yl) ethyl)-6-fluoro-3- (3-fluorophenyl)-4H-chromen-4-one;
2-(l-(4-amino-3-(3-hydroxyphenyl)-lH-pyrazolo [3, 4-d] pyrimidin-l-yl) ethyl)-6-fluoro-3- (3-fluorophenyl)-4H-chromen-4-one;
2-((9H-purin-6-ylamino) methyl)-6-fluoro-3-(3-fluorophenyl)-4H-chromen-4-one;
2-((9H-purin-6-ylamino) methyl)-6-fluoro-3-phenyl-4H-chromen-4-one;
(R)-2-( 1 -(9H-purin-6-ylamino) ethyl)-6-fluoro-3-(3-fluorophenyl)-4H-chromen-4-one;
2-((4-amino-3-(lH-pyrazol-4-yl)-lH-pyrazolo [3, 4-d] pyrimidin-l-yl) methyl)-6-fluoro-3-phenyl-4H-chromen-4-one;
2-(l-(4-amino-3-(3, 5-difluoro-4-methoxyphenyl)-lH-pyrazolo [3, 4-d] pyrimidin-l-yl) ethyl)-3-(3-fluorophenyl)-4H-chromen-4-one;
2-(l-(4-amino-3-(3, 5-difluoro-4-hydroxyphenyl)-lH-pyrazolo [3, 4-d] pyrimidin-l-yl) ethyl)-3-(3-fluorophenyl)-4H-chromen-4-one;
2-((4-amino-3-(3, 5-difluoro-4-methoxyphenyl)-lH-pyrazolo [3, 4-d] pyrimidin-l-yl) methyl)-6-fluoro-3-(3-fluorophenyl)-4H-chromen-4-one;
2-((4-amino-3-(3, 5-difluoro-4-hydroxyphenyl)-lH-pyrazolo [3, 4-d] pyrimidin-l-yl) methyl)-6-fluoro-3-(3-fluorophenyl)-4H-chromen-4-one;
(+)-2-(l-(4-amino-3-(3-methyl-lH-indazol-6-yl)-lH-pyrazolo [3, 4-d] pyrimidin-l-yl) ethyl)- 3-(3-fluorophenyl)-4H-chromen-4-one;
(-)-2-(l-(4-amino-3-(3-methyl-lH-indazol-6-yl)-lH-pyrazolo [3, 4-d] pyrimidin-l-yl) ethyl)- 3-(3-fluorophenyl)-4H-chromen-4-one;
2-(l-(4-amino-3-(3, 5-dimethoxyphenyl)-lH-pyrazolo [3, 4-d] pyrimidin-l-yl) ethyl)-3-(3-fluorophenyl)-4H-chromen-4-one;
2-(l-(4-amino-3-(4-methoxy-3, 5-dimethylphenyl)-lH-pyrazolo [3, 4-d] pyrimidin-l-yl) ethyl)-3 -(3 -fluorophenyl)-4H-chromen-4-one;
2- (l-(4-amino-3-(2-fluoro-5-isopropoxyphenyl)-lH-pyrazolo [3, 4-d] pyrimidin-l-yl) ethyl)-3- (3-fluorophenyl)-4H-chromen-4-one;
2-(l-(4-amino-3-(2, 3-dihydrobenzo[b] [1, 4] dioxin-6-yl)-lH-pyrazolo [3, 4-d] pyrimidin-l- yl) ethyl)-3-(3-fluorophenyl)-4H-chromen-4-one;
2-(l-(4-amino-3-(l-benzyl-lH-pyrazol-4-yl)-lH-pyrazolo [3, 4-d] pyrimidin-l-yl) ethyl)-3- (3-fluorophenyl)-4H-chromen-4-one;
2-(l-(4-amino-3-(2-methylpyridin-4-yl)-lH-pyrazolo [3, 4-d] pyrimidin-l-yl) ethyl)-3-(3-fluorophenyl)-4H-chromen-4-one;
2-(l-(4-amino-3-(3, 4-dihydro-2H-benzo[b] [1, 4] dioxepin-7-yl)-lH-pyrazolo [3, 4-d] pyrimidin-l-yl) ethyl)-3-(3-fluorophenyl)-4H-chromen-4-one;
2-(l-(4-amino-3-(6-morpholinopyridin-3-yl)-lH-pyrazolo [3, 4-d] pyrimidin-l-yl) ethyl)-3- (3-fluorophenyl)-4H-chromen-4-one;
2-(l-(4-amino-3-(dibenzo [b, d] furan-4-yl)-lH-pyrazolo [3, 4-d] pyrimidin-l-yl) ethyl)-3-(3-fluorophenyl)-4H-chromen-4-one;
2-(l-(4-amino-3-(4-phenoxyphenyl)-lH-pyrazolo [3, 4-d] pyrimidin-l-yl) ethyl)-3-(3-fluorophenyl)-4H-chromen-4-one;
2-(l-(4-amino-3-(4-(benzyloxy)-3-chlorophenyl)-lH-pyrazolo [3, 4-d] pyrimidin-l-yl) ethyl)-3-(3-fluorophenyl)-4H-chromen-4-one;
2- (l-(4-amino-3-(3-chloro-4-isopropoxyphenyl)-lH-pyrazolo [3, 4-d] pyrimidin-l-yl) ethyl)-3- (3-fluorophenyl)-4H-chromen-4-one;
2-(l-(4-amino-3-(3-(dimethylamino) phenyl)- lH-pyrazolo [3, 4-d] pyrimidin-l-yl) ethyl)-3- (3-fluorophenyl)-4H-chromen-4-one;
2-(l-(4-amino-3-(4-ethoxy-3-fluorophenyl)-lH-pyrazolo [3, 4-d] pyrimidin-l-yl) ethyl)-3-(3-fluorophenyl)-4H-chromen-4-one;
2-(l-(4-amino-3-(4-isopropoxyphenyl)-lH-pyrazolo [3, 4-d] pyrimidin-l-yl) ethyl)-3-(3-fluorophenyl)-4H-chromen-4-one;
2- (l-(4-amino-3-(4-(trifluoromethoxy) phenyl)- lH-pyrazolo [3, 4-d] pyrimidin-l-yl) ethyl)-3- (3-fluorophenyl)-4H-chromen-4-one;
2-(l-(3-(4-acetylphenyl)-4-amino-lH-pyrazolo [3, 4-d] pyrimidin-l-yl) ethyl)-3-(3-fluorophenyl)-4H-chromen-4-one;
2-(l-(4-amino-3-(4-(benzyloxy) phenyl)- lH-pyrazolo [3, 4-d] pyrimidin-l-yl) ethyl)-3-(3-fluorophenyl)-4H-chromen-4-one;
2-(l-(4-amino-3-(4-(dimethylamino) phenyl)- lH-pyrazolo [3, 4-d] pyrimidin-l-yl) ethyl)-3- (3-fluorophenyl)-4H-chromen-4-one;
2-(l-(4-amino-3-(4-(methylsulfonyl) phenyl)- lH-pyrazolo [3, 4-d] pyrimidin-l-yl) ethyl)-3- (3-fluorophenyl)-4H-chromen-4-one;
2-( 1 -(4-amino-3-(3-ethoxyphenyl)- 1 H-pyrazolo [3, 4-d] pyrimidin-l-yl) ethyl)-3-(3-fluorophenyl)-4H-chromen-4-one;
2-(l-(4-amino-3-(benzo[b]thiophen-2-yl)-lH-pyrazolo [3, 4-d] pyrimidin-l-yl) ethyl)-3-(3-fluorophenyl)-4H-chromen-4-one;
2-(l-(4-amino-3-(5-chlorothiophen-2-yl)-lH-pyrazolo [3, 4-d] pyrimidin-l-yl) ethyl)-3-(3-fluorophenyl)-4H-chromen-4-one;
2-(l-(4-amino-3-(3, 5-dimethylisoxazol-4-yl)-lH-pyrazolo [3, 4-d] pyrimidin-l-yl) ethyl)-3- (3-fluorophenyl)-4H-chromen-4-one;
2-(l-(4-amino-3-(3-propoxyphenyl)-lH-pyrazolo [3, 4-d] pyrimidin-l-yl) ethyl)-3-(3-fluorophenyl)-4H-chromen-4-one;
2~(l-(4-amino-3-(furan-2-yl)-lH-pyrazolo [3, 4-d] pyrimidin-l-yl) ethyl)-3-(3-fluorophenyl)-4H-chromen-4-one;
2-(l-(4-amino-3-(4-ethoxyphenyl)-lH-pyrazolo [3, 4-d] pyrimidin-l-yl) ethyl)-3-(3-fluorophenyl)-4H-chromen-4-one;
2-(l-(4-amino-3-(3-chloro-4-methoxyphenyl)-lH-pyrazolo [3, 4-d] pyrimidin-l-yl) ethyl)-3- (3-fluorophenyl)-4H-chromen-4-one;
2- (l-(4-amino-3-(3-fluoro-4-isopropoxyphenyl)-lH-pyrazolo [3, 4-d] pyrimidin-l-yl) ethyl)-3- (3-fluorophenyl)-4H-chromen-4-one;
2-(l-(4-amino-3-(6-fluoropyridin-3-yl)-lH-pyrazolo [3, 4-d] pyrimidin-l-yl) ethyl)-3-(3-fluorophenyl)-4H-chromen-4-one;
2-(l-(4-amino-3-(pyrimidin-5-yl)-lH-pyrazolo [3, 4-d] pyrimidin-l-yl) ethyl)-3-(3-fluorophenyl)-4H-chromen-4-one;
2-(l-(4-amino-3-(3-(methoxymethyl) phenyl)- lH-pyrazolo [3, 4-d] pyrimidin-l-yl) ethyl)-3- (3-fluorophenyl)-4H-chromen-4-one;
2-(l-(4-amino-3-(6-hydroxynaphthalen-2-yl)-lH-pyrazolo [3, 4-d] pyrimidin-l-yl) ethyl)-3- (3-fluorophenyl)-4H-chromen-4-one;
2-( 1 -(4-amino-3-(3-isopropoxyphenyl)- lH-pyrazolo [3, 4-d] pyrimidin-l-yl) ethyl)-3-(3-fluorophenyl)-4H-chromen-4-one;
2-(l-(4-amino-3-(l-methyl-lH-pyrazol-4-yl)-lH-pyrazolo [3, 4-d] pyrimidin-l-yl) ethyl)-3- (3-fluorophenyl)-4H-chromen-4-one;

6-Fluoro-3-(3-fluorophenyl)-2-(l-(4-methoxyphenylamino) ethyl)-4H-chromen-4-one;
2-(l-(4-Chloro-7H-pyrrolo [2, 3-d] pyrimidin-7-yl) emyl)-3-(3-fluorophenyl)-4H-chromen-4-one; 2-(l-(4-Chloro-lH-pyrazolo [3, 4-b] pyridin-l-yl) ethyl)-3-(3-fluorophenyl)-4H-chromen-4-one;
2-(l-(4-Chloro-lH-pyrazolo [3, 4-d] pyrimidin-l-yl) ethyl)-3-(3-fluorophenyl)-4H-chromen-4- one;
2-(l-(4-Chloro-5H-pyrrolo [3, 2-d] pyrimidin-5-yl) ethyl)-3-(3-fluorophenyl)-4H-chromen-4-one;
2-(l-(4-amino-3-(l, 3-dimethyl-lH-indazol-6-yl)-lH-pyrazolo [3, 4-d] pyrimidin-l-yl) ethyl)-3-(3-fluorophenyl)-4H-chromen-4-one;
2-(l-(4-amino-3-(2, 3-dimethyl-2H-indazol-6-yl)-lH-pyrazolo [3, 4-d] pyrimidin-l-yl) ethyl)-3-(3-fluorophenyl)-4H-chromen-4-one;
2-( 1 -(4-amino-3-(6-methoxynaphthalen-2-yl)- 1 H-pyrazolo [3, 4-d] pyrimidin-l-yl) ethyl)-3-(3-fluorophenyl)-4H-chromen-4-one;
2-(l-(4-amino-3-(benzo[b]thiophen-3-yl)-lH-pyrazolo [3, 4-d] pyrimidin-l-yl) ethyl)-3-(3-fluorophenyl)-4H-chromen-4-one;
2- (l-(4-amino-3-(2, 4-dimethoxypyrimidin-5-yl)-l H-pyrazolo [3, 4-d] pyrimidin-l-yl) ethyl)-3- (3-fluorophenyl)-4H-chromen-4-one;
2- ( 1 -(4-amino-3-(6-ethoxynaphthalen-2-yl)- 1 H-pyrazolo [3, 4-d] pyrimidin-l-yl) ethyl)-3-(3- fluorophenyl)-4H-chromen-4-one;
3- (4-amino-l-(l-(3-(3-fluorophenyl)-4-oxo-4H-chromen-2-yl) ethyl)- 1 H-pyrazolo [3, 4-d] pyrimidin-3-yl)-N-cyclopropylbenzamide;
2-(l-(4-amino-3-(3-(morpholine-4-carbonyl) phenyl)- 1 H-pyrazolo [3, 4-d] pyrimidin-l-yl) ethyl)-3-(3-fluorophenyl)-4H-chromen-4-one;
2-(l-(4-amino-3-(3-(difluoromethoxy) phenyl)- 1 H-pyrazolo [3, 4-d] pyrimidin-l-yl) ethyl)-3-(3-fluorophenyl)-4H-chromen-4-one;
5- (4-amino-l-(l-(3-(3-fluorophenyl)-4-oxo-4H-chromen-2-yl) ethyl)- 1 H-pyrazolo [3, 4-d] pyrimidin-3-yl) furan-2-carbaldehyde and pharmaceutically acceptable salts thereof;
or their enantiomers, mixtures of enantiomers, mixtures of two or more diastereoisomers, isotopic variants, pharmaceutically acceptable salts, solvates, hydrates or pro-drug.

In certain embodiment, wherein said PI3K inhibitor comprise: 2-(1-(9H-purin--6-ylamino) propyl group)-3-(3-fluorophenyl)-4H-chromen-4-one or its enantiomers, mixtures of enantiomers, mixtures of two or more diastereoisomers, isotopic variants, pharmaceutically acceptable salts, solvates, hydrates or prodrugs.

In certain embodiment, wherein said PI3K inhibitor comprise: (S)-2-(1-(9H-purin--6-ylamino) propyl group)-3-(3-fluorophenyl)--4H-chromen--4-one or its pharmaceutically acceptable salts, solvates, hydrates or prodrugs.

In certain embodiment, wherein said pharmaceutical composition comprises a PD-L1/TGFβ dual inhibitor and a PI3K inhibitor.

In certain embodiment, wherein the PD-L1/TGFβ dual inhibitor comprises an anti-PD-L1 antibody or an antigen-binding fragment thereof and TGFβRII or a functionally active fragment thereof.

In certain embodiment, wherein the PD-L1/TGFβ dual inhibitor comprises two polypeptides comprising an amino acid sequence selected from the group consisting of: (a) the amino acid sequence shown in SEQ ID NO :7; (b) an amino acid sequence having at least 85%, 90%, 95% or 99% sequence identity with the amino acid sequence shown in SEQ ID NO: 7; (c) an amino acid sequence having one or more differences from the amino acid sequence shown in SEQ ID NO:7 obtained by addition, elimination or substitution reaction.

In certain embodiment, wherein the PI3K inhibitor is a dual PI3K δ/γ inhibitor.

In certain embodiment, wherein the PI3K inhibitor is (S)-2-(1-(9H-purin--6-ylamino) propyl group)-3-(3-fluorophenyl)--4H-chromen--4-one or its pharmaceutically acceptable salts, solvates, hydrates or prodrugs.

In certain embodiment, wherein the said PD-L1/TGFβ dual inhibitor and the PI3K inhibitor are not intermixed in the pharmaceutical combination.

In certain embodiment, wherein the second therapeutic agent and the PI3K inhibitor are each independently present in separate containers.

In another aspect, the present application provides a pharmaceutical composition, comprising the aforementioned PI3K inhibitor and a second therapeutic agent, and optionally one or more pharmaceutically acceptable carriers or excipients.

In certain embodiment, wherein the second therapeutic agent is the aforementioned PD-L1/TGFβ dual inhibitor.

In certain embodiment, the PI3K inhibitor is the aforementioned PI3K8/y dual inhibitor.
In certain embodiment, wherein said second therapeutic agent and said PI3K inhibitor are present in a single or separate dosage form.

In another aspect, the present application provides a method for treating and/or preventing tumors, which comprises administering to a subject in need: an effective amount of the aforementioned pharmaceutical combination

or the aforementioned pharmaceutical composition.

In certain embodiment, the method includes administering to a subject in need: an effective amount of the aforementioned PI3K inhibitor and the aforementioned immune checkpoint inhibitor.

In certain embodiment, the method comprises administering to a subject in need: an effective amount of the aforementioned PI3K inhibitor and the aforementioned TGFβ inhibitor. In certain embodiment, the method includes administering to a subject in need: an effective amount of the aforementioned PI3K inhibitor, the aforementioned TGFβ inhibitor, and the aforementioned immune checkpoint inhibitor.

In certain embodiment, the method includes administering to a subject in need: an effective amount of the aforementioned PI3K inhibitor and the aforementioned PD-L1/TGFβ dual inhibitor.

In certain embodiment, the PI3K inhibitor and the PD-L1/TGFβ dual inhibitor are administered simultaneously.

In a certain embodiment, the PI3K inhibitor is administered after the PD-L1/TGFβ dual inhibitor.

In a certain embodiment, the PI3K inhibitor is administered before the PD-L1/TGFβ dual inhibitor.

In certain embodiment, the present invention also includes administering an effective amount of the aforementioned small molecule TGFβ inhibitor to a subject in need.

In certain embodiment, wherein the tumor includes solid tumors and non-solid tumors.

In certain embodiment, wherein the tumor comprises a tumor with abnormal expression of PI3K.

In certain embodiment, wherein the tumor comprises a tumor with abnormal expression of PI3K.

In certain embodiment, wherein the tumor comprises a tumor with abnormal expression of PD-L1.

In certain embodiment, wherein the tumor comprises a tumor with abnormal expression of TGFβ.

In certain embodiment, the tumor comprises gastrointestinal tumors, melanoma, or lymphoma.

In certain embodiment, wherein said tumor has delayed resistance to said PI3K inhibitor.

In certain embodiment, wherein the tumor in the subject has prolonged remission.

In certain embodiment, wherein the subject experiences a complete remission of the tumor.

In certain embodiment, wherein the level of minimal residual disease (MRD) is reduced.

In another aspect, the present application provides the use of the aforementioned pharmaceutical combination or the aforementioned pharmaceutical composition in the preparation of a medicament for treating and/or preventing tumors.

For example, the use of the aforementioned PI3K inhibitors and the aforementioned immune checkpoint inhibitors in the manufacture of drugs for treating and/or preventing tumors. For example, the use of the aforementioned PI3K inhibitor and the aforementioned TGFβ inhibitor in the manufacture of medicaments for treating and/or preventing tumors. For example, the use of the aforementioned PI3K inhibitors, the aforementioned immune checkpoint inhibitors and the aforementioned TGFβ inhibitors in the manufacture of drugs for treating and/or preventing tumors.

In another aspect, the present application provides the aforementioned pharmaceutical combination or the aforementioned pharmaceutical composition for treating and/or preventing tumors.

In another aspect, the present application provides a method for inhibiting tumor growth, the said method comprising exposing the tumor to the aforementioned pharmaceutical combination or the aforementioned pharmaceutical composition.

In certain embodiment, the contacting is in an in vitro or ex vivo environment.

In certain embodiment, the method comprises exposing the tumor to an aforementioned PI3K inhibitor and an aforementioned inhibitor of an immune checkpoint. In certain embodiment, the method comprises contacting the tumor with the aforementioned PI3K inhibitor and the aforementioned TGFβ inhibitor. In certain embodiment, the method comprises contacting the tumor with an aforementioned PI3K inhibitor, an aforementioned inhibitor of an immune checkpoint, and an aforementioned TGFβ inhibitor.

In another aspect, the present application provides a method of inhibiting tumor growth, the method comprising exposing the tumor to fresh PI3K and fresh PD-L1/TGFβ dual inhibitor.

In another aspect, the present application provides a kit, which includes: (1) a first container, and the aforementioned PI3K inhibitor located in the first container; (2) a second container, and he aforementioned PD-L1/TGFβ dual inhibitors in the second container.

In another aspect, the present application provides a kit, which includes: (1) a first container, and the aforementioned PI3K inhibitor located in the first container; (2) a second container, and the aforementioned immune checkpoint inhibitor or the aforementioned TGFβ inhibitor in the second container.

In certain embodiment, the kit further includes (3) a third container, and the aforementioned immune checkpoint inhibitor or the aforementioned TGFβ inhibitor located in the third container, the medicament in the third container different from the medicament in the second container.

In certain embodiment, the dosage form of the medicine in the kit is an oral dosage form or an injection dosage form.

In certain embodiment, the kit further includes instructions.

Those the skilled person in the field can easily perceive other aspects and advantages of the present application

from the following detailed description. In the following detailed description, only exemplary embodiment of the present application are shown and described. As those skilled in the field will appreciate, the content of the present application enables those skilled in the art to make changes to the specific embodiment which are disclosed without departing from the spirit and scope of the invention to which this application relates. Correspondingly, the drawings and descriptions in the specification of the present application are only exemplary rather than restrictive.

**Description of Drawings**

The particular features of the invention to which this application relates are set forth in the appended claims. The features and advantages of the invention to which this application relates can be better understood with reference to the exemplary embodiment described in detail hereinafter and the accompanying drawings. A brief description of the accompanying drawings is as follows:

Figure 1 shows the results of combined administration of CN401 (Tenalisib) (150mg/kg) and CN202 (WBP1126) (5mg/kg) in the A20 mouse B-cell lymphoma animal model.
Figure 2 shows the results of combined administration of CN401 (Tenalisib) (150mg/kg) and CN202 (WBP1126) (15mg/kg) in the A20 mouse B-cell lymphoma animal model.
Figure 3 shows the results of combined administration of CN401 (Tenalisib) (150mg/kg) and CN202 (WBP1126) (5mg/kg) (15mg/kg) in the A20 mouse B-cell lymphoma animal model.
Figure 4 shows the results of combined administration of CN202 (WBP1126) (5mg/kg) and CN401 (Tenalisib) (150mg/kg) in the A20 mouse B-cell lymphoma animal model.
Figure 5 shows the results of combined administration of CN202 (WBP1126) (5mg/kg) and Alpelisib (50mg/kg) in the A20 mouse B-cell lymphoma animal model.
Figure 6 shows the results of combined administration of CN401 (Tenalisib) (150mg/kg) and anti-PD-L1 antibody (Atezolizumab) (5mg/kg) in the A20 mouse B-cell lymphoma animal model.
Figure 7 shows the effect of CN401 (Tenalisib) (150mg/kg), CN202 (WBP1126) (15mg/kg) and nab-paclitaxel (nab-pac) (10mg/kg) on mouse EMT-60 (mouse breast cancer cells) tumor growth curve and body weight growth curve.

**Embodiment**

The implementation mode of the invention of the application is described below by specific examples, and those who are familiar with this technology can easily understand other advantages and effects of the invention of the application from the content disclosed in this description.

**Terms defination**

In the present application, "phosphoinositide 3-kinase (PI3K) inhibitor" or "PI3K inhibitor" generally refers to any inhibitor of PI3K. PI3Ks are members of a unique and conserved family of intracellular lipid kinases that phosphorylate the 3'-OH group on phosphatidylinositols or phosphoinositides. The PI3K family includes kinases with distinct substrate specificities, expression patterns, and regulatory patterns (see, e.g., Katso et al., 2001, Annu. Rev. Cell Dev. Biol. 17, 615-675; Foster, F.M. et al., 2003, J Cell Sci 116, 3037-3040). Class I PI3Ks (e.g., p110$\alpha$, p110$\beta$, p110$\gamma$, and p110$\delta$ are normally activated by tyrosine kinases or G-protein-coupled receptors to generate PIP3, which binds downstream mediators such as those in the Akt/PDK1 pathway, mTOR, Tec family Kinases and Rho family GTPases. Class II PI3Ks (e.g. PI3K-C2$\alpha$, PI3K-C2$\beta$, PI3K-C2$\gamma$) and class III PI3Ks (e.g.Vps34) play a key role in intracellular trafficking by the synthesis of PI(3)P and PI(3,4)P2. Specific exemplary PI3K inhibitors are disclosed herein. For example, PI3K inhibitors inhibit PI3K-alpha, PI3K-beta, PI3K-gamma, and PI3K-delta subtypes or combinations thereof.
For example, the PI3K inhibitor can be Tenalisib, a dual PI3K delta/gamma inhibitor, 2-(1-(9H-purin-6-ylamino)propyl)-3-(3-fluorophenyl)-4H-chromene-4 -ketone, its structural formula is as follows:

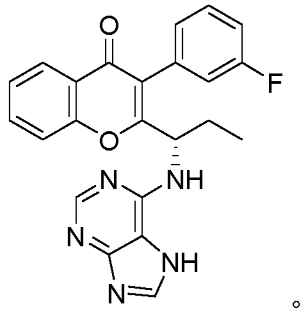

For example, the PI3K inhibitor can be the PI3K inhibitor Alpelisib, (2S)-1-N-[4-methyl-5-[2-(1,1,1-trifluoro-2-methylpropan-2-yl )pyridin-4-yl]-1,3-thiazol-2-yl]pyrrolidine-1,2-dicarboxamide, its structural formula is as follows:

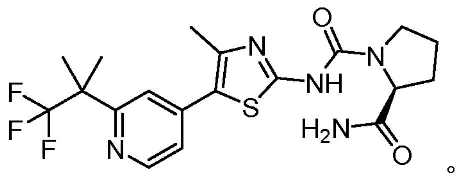

In certain embodiment, a PI3K inhibitor generally refers to a compound that inhibits one or more PI3K subtype with an IC50 of less than about 1000 nM, less than about 900 nM, less than about 800 nM, less than about 700 nM, less than about 600 nM, less than about 500 nM, Less than about 400 nM, less than about 300 nM, less than about 200 nM, less than about 100 nM, less than about 75 nM, less than about 50 nM, less than about 25 nM, less than about 20 nM, less than about 15 nM, less than about 10 nM, less than about 10 nM, less than about 5 nM, or less than about 1nM.

The term "alkyl" refers to a straight or branched hydrocarbon chain radical consisting solely of carbon and hydrogen atoms, containing no unsaturation, having from one to eight carbon atoms, and which is attached to the rest of the molecule by a single bond, e.g., methyl, ethyl, n-propyl, 1-methylethyl (isopropyl), n-butyl, n-pentyl, and 1,1-dimethylethyl (t-butyl). The term "(C1-6)alkyl" refers to an alkyl group as defined above having up to 6 carbon atoms.

The term "alkenyl" refers to an aliphatic hydrocarbon group containing a carbon-carbon double bond and which may be a straight or branched or branched chain having about 2 to about 10 carbon atoms, e.g., ethenyl, 1-propenyl, 2-propenyl (allyl), iso- propenyl, 2-methyl- 1-propenyl, 1-butenyl, and 2-butenyl. The term "(C2-6)alkenyl" refers to an alkenyl group as defined above having up to 6 carbon atoms.

The term "alkynyl" refers to a straight or branched chain hydrocarbyl radical having at least one carbon-carbon triple bond, and having in the range of 2 to up to 12 carbon atoms (with radicals having in the range of 2 to up to 10 carbon atoms presently being preferred) e.g., ethynyl, propynyl, and butnyl. The term "(C2-6) alkynyl" refers to an alkynyl group as defined above having up to 6 carbon atoms.

The term "alkoxy" denotes an alkyl, cycloalkyl, or cycloalkylalkyl group as defined above attached via an oxygen linkage to the rest of the molecule. The term "substituted alkoxy" refers to an alkoxy group where the alkyl constituent is substituted (i.e., -0-(substituted alkyl) wherein the term "substituted alkyl" is the same as defined above for "alkyl". For example "alkoxy" refers to the group -O-alkyl, including from 1 to 8 carbon atoms of a straight, branched, cyclic configuration and combinations thereof attached to the parent structure through oxygen. Examples include methoxy, ethoxy, propoxy, isopropoxy, cyclopropyloxy, and cyclohexyloxy.

The term "cycloalkyl" denotes a non-aromatic mono or multicyclic ring system of about 3 to 12 carbon atoms such as cyclopropyl, cyclobutyl, cyclopentyl, and cyclohexyl. Examples of multicyclic cycloalkyl groups include perhydronaphthyl, adamantyl and norbornyl groups, bridged cyclic groups, and sprirobicyclic groups, e.g., sprio (4,4) non- 2-yl. The term "(C3-8) cycloalkyl" refers to a cycloalkyl group as defined above having up to 8 carbon atoms.

The term "cycloalkylalkyl" refers to a cyclic ring-containing radical containing in the range of about 3 up to 8 carbon atoms directly attached to an alkyl group which are then attached to the main structure at any carbon from the alkyl group that results in the creation of a stable structure such as cyclopropylmethyl, cyclobutylethyl, and cyclopentylethyl.

The term "cycloalkenyl" refers to cyclic ring-containing radicals containing in the range of about 3 up to 8 carbon atoms with at least one carbon-carbon double bond such as cyclopropenyl, cyclobutenyl, and cyclopentenyl. The term "cycloalkenylalkyl" refers to a cycloalkenyl group directly attached to an alkyl group which are then attached to the main structure at any carbon from the alkyl group that results in the creation of a stable structure.

The term "aryl" refers to aromatic radicals having in the range of 6 up to 20 carbon atoms such as phenyl, naphthyl, tetrahydronaphthyl, indanyl, and biphenyl.

The term "arylalkyl" refers to an aryl group as defined above directly bonded to an alkyl group as defined above, e.g., $-CH_2C_6H_5$ and $-C_2H_5C_6H_5$.

The term "heterocyclic ring" refers to a non-aromatic 3 to 15 member ring radical which consists of carbon atoms and at least one heteroatom selected from nitrogen, phosphorus, oxygen and sulfur. For purposes of this invention, the heterocyclic ring radical may be a mono-, bi-, tri- or tetracyclic ring system, which may include fused, bridged or spiro ring systems, and the nitrogen, phosphorus, carbon, oxygen or sulfur atoms in the heterocyclic ring radical may be optionally oxidized to various oxidation states. In addition, the nitrogen atom may be optionally quatemized. The heterocyclic ring radical may be attached to the main structure at any heteroatom or carbon atom that results in the creation of a stable structure.

The term "heterocyclyl" refers to a heterocylic ring radical as defined above. The heterocylcyl ring radical may be attached to the main structure at any heteroatom or carbon atom that results in the creation of a stable structure.

The term "heterocyclylalkyl" refers to a heterocylic ring radical as defined above directly bonded to an alkyl group. The heterocyclylalkyl radical may be attached to the main structure at carbon atom in the alkyl group that results in the creation of a stable structure. Examples of such heterocycloalkyl radicals include, but are not limited to, dioxolanyl, thienyl[1,3]dithianyl, decahydroisoquinolyl, imidazolinyl, imidazolidinyl, isothiazolidinyl, isoxazolidinyl, morpholinyl, octahydroindolyl, octahydroisoindolyl, 2- oxopiperazinyl, 2-oxopiperidinyl, 2-oxopyrrolidinyl, oxazolidinyl, piperidinyl, piperazinyl, 4- piperidonyl, pyrrolidinyl, pyrazolidinyl, quinuclidinyl, thiazolidinyl, tetrahydrofuryl, trithianyl, tetrahydropyranyl, thiomorpholinyl, thiamorpholinyl, 1 -oxo- thiomorpholinyl, and 1 , 1 -dioxo-thiomorpholinyl.

The term "heteroaryl" refers to an optionally substituted 5 to 14 member aromatic ring having one or more heteroatoms selected from N, O, and S as ring atoms. The heteroaryl may be a mono-, bi- or tricyclic ring system. Examples of such "heterocyclic ring" or "heteroaryl" radicals include, but are not limited to, oxazolyl, thiazolyl, imidazolyl, pyrrolyl, furanyl, pyridinyl, pyrimidinyl, pyrazinyl, benzofuranyl, indolyl, benzothiazolyl, benzoxazolyl, carbazolyl, quinolyl , isoquinolyl, azetidinyl, acridinyl, benzodioxolyl, benzodioxanyl, benzofuranyl, carbazolyl, cinnolinyl, dioxolanyl, indolizinyl, naphthyridinyl, perhydroazepinyl, phenazinyl, phenothiazinyl, phenoxazinyl, phthalazinyl, pteridinyl, purinyl, quinazolinyl, quinoxalinyl, tetrazoyl, tetrahydroisoquinolyl, piperidinyl, piperazinyl, 2-oxopiperazinyl, 2-oxopiperidinyl, 2-oxopyrrolidinyl, 2-oxoazepinyl, azepinyl, 4- piperidonyl, pyrrolidinyl, pyridazinyl, oxazolinyl, oxazolidinyl, triazolyl, indanyl, isoxazolyl, isoxazolidinyl, morpholinyl, thiazolinyl, thiazolidinyl, isothiazolyl, quinuclidinyl, isothiazolidinyl, isoindolyl, indolinyl, isoindolinyl, octahydroindolyl, octahydroisoindolyl, decahydroisoquinolyl, benzimidazolyl, thiadiazolyl, benzopyranyl, tetrahydrofuryl, tetrahydropyranyl, thienyl, benzothienyl, thiamorpholinyl, thiamorpholinyl sulfoxide, thiamorpholinyl sulfone, dioxaphospholanyl, oxadiazolyl, chromanyl, and isochromanyl. The heteroaryl ring radical may be attached to the main structure at any heteroatom or carbon atom that results in the creation of a stable structure. The term "substituted heteroaryl" also includes ring systems substituted with one or more oxide (-0-) substituents, such as pyridinyl N-oxides.

The term "heteroarylalkyl" refers to a heteroaryl ring radical as defined above directly bonded to an alkyl group. The heteroarylalkyl radical may be attached to the main structure at any carbon atom from alkyl group that results in the creation of a stable structure.

The term "cyclic ring" refers to a cyclic ring containing 3 to 10 carbon atoms.

In the present application, the term "immune checkpoint" generally refers to a group of molecules on the cell surface of CD4 cells and CD8 T cells. These molecules can effectively act as "brakes" to downregulate or suppress anti-tumor immune responses. Immune checkpoint molecules include but are not limited to programmed cell death 1 (PD-1), programmed cell death ligand 1 (PD-L1), programmed cell death ligand 2 (PD-L2), lymphocyte activation gene- 3 (LAG-3; also known as CD223), galectin-3, B and T lymphocyte attenuator (BTLA), T-cell membrane protein 3 (TIM3), galectin-9 (GAL9), B7 -H1, B7-H3, B7-H4, T-cell immune receptors with Ig and ITIM domains (TIGIT/Vstm3/WUCAM/VSIG9), V-domain Ig inhibitor of T-cell activation (VISTA), Glucocorticoid-induced tumor necrosis factor receptor-related (GITR) protein, herpes virus entry medium (HVEM), OX40, CD27, CD28, CD80, CD86, CD137, CGEN-15001T, CGEN-15022, CGEN-15027, CGEN- 15049, CGEN-15052, and CGEN-15092.

In the present application, the term "inhibitor of an immune checkpoint" generally refers to a molecule that inhibits, reduces or interferes with the activity of an inhibitory checkpoint molecule. Without being bound by a particular theory, inhibitory checkpoint molecules downregulate immune responses (eg, T-cell activation) by delivering negative signals to T cells upon their binding by ligands or counter-receptors. In certain embodiment, the checkpoint inhibitors used with the methods and compositions provided herein can directly inhibit the activity of inhibitory checkpoint molecules or reduce the expression of inhibitory checkpoint molecules or interfere with inhibitory checkpoint molecules and interaction of binding partners (eg, ligands).

In the present application, "inhibitors of immune checkpoints" include, but are not limited to, proteins, polypeptides, peptides, antisense oligonucleotides, antibodies, antibody fragments, or RNA molecules (e.g. that target the expression of inhibitory checkpoint molecules). inhibitory RNA molecules). Its inhibitory effect can be carried out at the DNA, RNA or protein level. For example, inhibitory nucleic acids (eg, dsRNA, siRNA, or shRNA) can be used to inhibit the expression of inhibitory molecules. In other embodiment, the inhibitor of the inhibitory signal is a polypeptide that

binds to an immune checkpoint, e.g., a soluble ligand (e.g., PD-1-Ig), an antibody or an antigen-binding fragment thereof; e.g., with PD-1, PD -An antibody or fragment thereof that binds to L1, PD-L2, CTLA4, TIM3, LAG3, VISTA, BTLA, TIGIT, LAIR1, CD160, 2B4 and/or TGFRβ or a combination thereof.

In the present application, the term"TGFβRII" or"TGFβ Receptor II" is meant a polypeptide having the wild-type human TGFβ Receptor Type 2 Isoform A sequence (e.g., the amino acid sequence of NCBI Reference Sequence (RefSeq) Accession No. NP_001020018 (SEQ ID NO.8)), or a polypeptide having the wild-type human TGFβ Receptor Type 2 Isoform B sequence (e.g., the amino acid sequence of NCBI RefSeq Accession No. NP_003233 (SEQ ID NO.9)) or having a sequence substantially identical the amino acid sequence of SEQ ID NO.8 or of SEQ ID NO. 9. The TGFβRII may retain at least 0.1%, 0.5%, 1%, 5%, 10%, 25%, 35%, 50%, 75%, 90%, 95%, or 99% of the TGFβ-binding activity of the wild-type sequence.

In the present application, the term"fragment of TGFβRII capable of binding TGFβ" is meant any portion of NCBI RefSeq Accession No. NP_001020018 (SEQ ID NO.8) or of NCBI RefSeq Accession No. NP_003233 (SEQ ID NO.9), or a sequence substantially identical to SEQ ID NO.8 or SEQ ID NO.9 that is at least 20 (e.g., at least 30, 40, 50, 60, 70, 80, 90, 100, 110, 120, 130, 140, 150, 160, 175, or 200) amino acids in length that retains at least some of the TGFβ-binding activity (e.g., at least 0.1%, 0.5%, 1%, 5%, 10%, 25%, 35%, 50%, 75%, 90%, 95%, or 99%) of the wild-type receptor or of the corresponding wild-type fragment. Typically such fragment is a soluble fragment. An exemplary such fragment is a TGFβRII extra-cellular domain having the sequence of SEQ ID NO: 6

In the present application, the term"substantially identical" is meant a polypeptide exhibiting at least 50%, desirably 60%, 70%, 75%, or 80%, more desirably 85%, 90%, or 95%, and most desirably 99% amino acid sequence identity to a reference amino acid sequence. The length of comparison sequences will generally be at least 10 amino acids, desirably at least 15 contiguous amino acids, more desirably at least 20, 25, 50, 75, 90, 100, 150, 200, 250, 300, or 350 contiguous amino acids, and most desirably the full-length amino acid sequence. For example, suitable algorithms for determining percent sequence identity and percent sequence similarity may be the BLAST and BLAST 2.0 algorithms, respectively, see Altschul et al. (1977) Nucleic Acids Res. 25:3389 and Altschul et al. (1990) J. Mol. Biol. 215 :403.

In the present application, the term "TGFβ inhibitor" generally refers to substances that inhibit the production and signal transduction of TGFβ signal molecules, and the inhibitors can be small molecular compounds or macromolecular compounds (such as antibodies). Exemplary small molecule TGFβ inhibitors include SB525334, SD-208, SB431542, LY2109761, LY2157299 (Galunisertib), GW788388, RepSox, SIS3, LDN-193189, EW-7197, and LY364947, among others.

| Compound | Chemical name |
|---|---|
| SB525334 | 6-(2-(Tert-Butyl)-5-(6-methylpyridin-2-yl)-1H-imidazol-4-yl)quinoxaline |
| SD-208 | 2-(5-Chloro-2-fluorophenyl)-N-(pyridin-4-yl)pteridin-4-amine |
| SB431542 | 4-(4-(Benzo[d][1,3]dioxol-5-yl)-5-(pyridin-2-yl)-1H-imidazol-2-yl)benzamide |
| LY2109761 | 4-(2-((4-(2-(Pyridin-2-yl)-5,6-dihydro-4H-pyrrolo[1,2-b]pyrazol-3-yl)quinolin-7-yl)oxy)ethyl) morpholine |
| LY2157299 | 4-[2-(6-methylpyridin-2-yl)-5,6-dihydro-4H-pyrrolo[1,2-b]pyrazol-3-yl]quinoline-6-carboxamide |
| GW788388 | N-(oxan-4-yl)-4-[4-(5-pyridin-2-yl-1H-pyrazol-4-yl)pyridin-2-yl]benzamide |
| RepSox | 2-[5-(6-methylpyridin-2-yl)-1H-pyrazol-4-yl]-1,5-naphthyridine |
| SIS3 | (E)-1-(6,7-dimethoxy-3,4-dihydro-1H-isoquinolin-2-yl)-3-(1-methyl-2-phenylpyrrolo[2,3-b] pyridin-3-yl)prop-2-en-1-one;hydrochloride |
| LDN-193189 | 4-[6-(4-piperazin-1-ylphenyl)pyrazolo[1,5-a]pyrimidin-3-yl]quinoline |
| EW-7197 | 2-fluoro-N-[[5-(6-methylpyridin-2-yl)-4-([1,2,4]triazolo[1,5-a]pyridin-6-yl)-1H-imidazol-2-yl] methyl]aniline |
| LY364947 | 4-(5-pyridin-2-yl-1H-pyrazol-4-yl)quinoline |

In the present application, the term "antibody" generally refers to immunoglobulins that are reactive to a specified protein or peptide or fragment thereof. Antibodies may be antibodies from any class, including but not limited to IgG, IgA, IgM, IgD and IgE, and antibodies from any subclass (e.g., IgGl, IgG2, IgG3, and IgG4). The antibody may have a heavy chain constant region selected from the group consisting of, for example, IgG1, IgG2, IgG3, or IgG4. The antibody may also have a light chain selected from the group consisting of, for example, kappa (kappa) or lambda (lambda). The antibody of the application can be derived from any species.

In the present application, the term "antigen-binding fragment" generally refers to a portion of an antibody molecule, which comprises an amino acid responsible for the specific binding between the antibody and the antigen. The portion of the antigen that is specifically recognized and bound by the antibody is referred to as the "epitope" as described above. The antigen binding domain may typically comprise an antibody light chain variable region (VL) and an antibody heavy chain variable region (VH); However, it is not necessary to contain both. The Fd fragment, for example, has two VH regions and generally retains some antigen binding function of the entire antigen-binding domain. Examples of antigen-binding fragments of an antibody include (1) a Fab fragment, a monovalent fragment having VL, VH, constant light chain (CL) and CH1 domain; (2) F (ab ') 2 fragment, a divalent fragment of two Fab fragments connected by disulfide bridge of the hinge region; (3) Fd fragments having two VH and CH1 domains; (4) Fv fragment with VL and VH domain of antibody single arm; (5) dAb fragment (Ward et al., " Binding Actida of a Repertoire ofSingle Immunoglobulin. variable Domains Secreted From Escherichia coli, Nature 341: 544-546 (1989), which is incorporated herein by reference), which has a VH domain; (6) a separate complementarity determining region (CDR); (7) single chain Fv (scFv), such as from scFV-library. Although the two domains of the Fv fragment, VL and VH, are encoded by an independent gene, they can be joined by a recombinant method using a synthetic linker; the synthetic linker is prepared into a single protein chain (called single chain Fv (scFv)) wherein VL and VH regions are paired to form monovalent molecules (referred to as single chain Fv (scFv)) (see e.g. Huston et al., Protein Engineering of Antibody Binding Sites: Recovery of SpecificActivity in an Anti-Digoxin Single-Chain Fv Analogue Produced in Escherichiacoli, " Proc. Natl. Acad. Sci. USA 85: 5879-5883 (1988)); and (8) VHH, VHH refers to a camel (camel, single-peak camel, llama, alpaca, etc.) heavy chain antibody of variable antigen-binding domain (see Nguyen V. K. et al., 2000, The EMBO Journal, 19, 921-930; Muyldermans S., 2001, J Biotechnol, 74, 277-302, and the Vanlandschoot P. et al., 2011, Antiviral Research 92, 389-407). VHH also can be referred to as Nanobody (Nanobody) (Nb) and/or single domain antibody. These antibody fragments are obtained using conventional techniques known to those of ordinary skill in the art, and the function of the fragments is evaluated in the same manner as the intact antibody.

In the present application, the term "variable region" or "variable domain" generally refers to a domain of an antibody heavy or light chain involved in the binding of an antibody to an antigen. In the present application, the term "variable" generally refers to some part of the sequence of the variable domain of the antibody is strong, forming various specific antibody binding and specificity of the specific antigen. The variability is non-uniformly distributed throughout the variable region of the antibody, which is concentrated in three sections of light chain variable region and heavy chain variable region, called complementarity determining region (CDR) or hypervariable region (HVR), respectively is LCDR1, LCDR2, LCDR3, HCDR1, HCDR2 and HCDR3. A more highly conserved portion in the variable domain is referred to as a framework region (FR). The variable domains of the natural heavy chain and the light chain each comprise four FR regions (H-FR1, H-FR2, H-FR3, H-FR4, L-FR1, L-FR2, L-FR3 and L-FR4), most of which are in the beta-folded configuration, and are connected by three CDR structural ring regions. the CDRs in each strand are closely adjacent together through the FR region and form the antigen binding site of the antibody together with the CDRs from the other strand.

In the present application, the variable region of the antibody can be encoded by a variety of methods or CDRs of a divided antibody, such as Kabat numbering scheme and definition rule (see, Kabat et al., Immunology protein sequence, Fifth Edition, United States National Institution of Health, Besseda, based on sequence variability. Maryland (1991)), Chothia numbering scheme based on structural ring region position and defining rule (see, A1-Lazikani et al., JMol Biol 273: 927-48, 1997); IMGT numbering scheme and definition rule of efranc et al based V gene based on the amino acid sequence, and Honneger's numbering scheme (AHo's), Martin numbering scheme, Gelfand numbering scheme and so on, can refer to Mathieu Dondelinger et al., Understanding the Significance and Imperial of Antibody Numbering and Antigen-Binding Surface/Residue Definition, Front.Immunol., 16 October 2018.

In the present application, "peptide linker" generally refers to the amino acid sequence through which the amino acid sequences of different structural domains in the PD-L1/TGFβ dual inhibitor of the present application are connected to each other. An essential technical feature of this peptide linker is that the peptide linker does not contain any polymerization activity. Preferred amino acid residues for peptide linkers include Gly, Ser and Thr, characterized by a length of 5 to 25 amino acid residues. Suitable peptide linkers include those described in US Patent No. 4,751,180 and 4,935,233 or WO88/09344. A preferred embodiment of the peptide linker is characterized by the amino acid sequence Gly-Gly-Gly-Gly-Ser, ie Gly4Ser, or a polymer thereof, ie (Gly4Ser)x, where x is an integer 1 or greater. The characteristics of such peptide linkers, including not promoting secondary structure, are known in the art and described, for example, in Dall'Acqua et al. (Biochem. (1998) 37, 9266-9273), Cheadle et al. (Mol Immunol ( 1992) 29, 21-30) and Raag and Whitlow (FASEB (1995) 9(1), 73-80). Peptide linkers that also do not promote any secondary structure are preferred. Linkage of the domains to each other is provided, eg by genetic engineering, as described in the Examples. Methods for preparing fused and operably linked bispecific single chain constructs and expressing them in mammalian cells or bacteria are well known in the art (e.g. WO99/54440 or Sambrook et al., Molecular Cloning: A Laboratory Manual, Cold Spring Harbor Laboratory Press, Cold Spring Harbor, New York, 2001).

In the present application, the terms "antagonist" and "inhibitor" are used interchangeably, and generally refer to a compound or compound capable of reducing or inhibiting the biological function of a target protein or polypeptide,

such as reducing or inhibiting the activity or expression of a target protein or polypeptide. potion. Inhibitors need not completely abolish the biological function of the target protein or polypeptide, and In certain embodiment, reduce activity by at least 50%, 60%, 70%, 80%, 90%, 95%, or 99%. While some antagonists of the present application specifically interact with (e.g., bind to) a target, inhibit the biological activity of the target protein or polypeptide by interacting with other members of a signal transduction pathway that includes the target protein or polypeptide Compounds of are also specifically included in this definition. Nonlimiting examples of biological activities inhibited by antagonists include those associated with the development, growth or spread of tumors, or undesired immune responses manifested in autoimmune diseases.

In the present application, the term "effective amount" or "therapeutically effective amount" generally refers to the amount of the compound or pharmaceutical composition described in the application that is sufficient to achieve the intended application described below, including but not limited to disease treatment. The therapeutically effective amount can vary according to: the intended application (in vivo or in vitro); or the subject and the condition to be treated, for example, the weight and age of the subject, the severity of the condition; the mode of administration, etc., which can be determined by ordinary skill in the art Personnel are easily identified. The terms also apply to doses that will induce a specific response in target cells, such as platelet adhesion and/or cell migration. The specific dosage will vary according to, for example, the particular compound chosen, the dosing regimen followed, whether it is administered in combination with other agents, the time of administration, the tissue to which it is administered, and the physical delivery system by which it is delivered.

In the present application, the term "in vivo" generally refers to events that occur within the body of a subject.

In the present application, the term "in vitro" generally refers to events that occur outside the body of a subject. For example, an in vitro assay includes any assay performed outside of a subject. In vitro assays include cell-based assays in which live or dead cells are employed. In vitro assays also include cell-free assays, in which intact cells are not used

In the present application, the term "combination therapy" or "combination" generally refers to the use of more than one compound or agent to treat a particular disorder or condition. For example, a PI3K inhibitor can be administered in combination with at least one additional therapeutic agent. "Combination" is not intended to imply that the other therapy and the PI3K inhibitor must be administered and/or formulated for delivery together, but such methods of delivery are within the scope of this application. The PI3K inhibitor can be administered simultaneously with, prior to (e.g., 5 minutes, 15 minutes, 30 minutes, 45 minutes, 1 hour, 2 hours, 4 hours, 6 hours, 12 hours, 24 hours) the one or more additional agents hours, 48 hours, 72 hours, 96 hours, 1 week, 2 weeks, 3 weeks, 4 weeks, 5 weeks, 6 weeks, 8 weeks, 12 weeks or 16 weeks before) or after (for example, 5 minutes, 15 minutes, 30 minutes, 45 minutes, 1 hour, 2 hours, 4 hours, 6 hours, 12 hours, 24 hours, 48 hours, 72 hours, 96 hours, 1 week, 2 weeks, 3 weeks, 4 weeks, 5 weeks , 6 weeks, 8 weeks, 12 weeks or 16 weeks later) administration. Generally, each therapeutic agent will be administered at a dosage and/or on a schedule established for that particular agent. Other therapeutic agents can be administered with the PI3K inhibitors provided herein in a single composition or separately in different compositions. This application also considers higher combinations, such as triple therapy of PI3K inhibitor + PD-L1 inhibitor + TGF$\beta$ inhibitor.

Combination therapies term "administering" generally refers to introducing the drug combination into the body of a subject by any route of introduction or delivery. Any method known to those skilled in the art for contacting cells, organs or tissues with the drug combination may be employed. Including but not limited to intraarterial, intranasal, intraperitoneal, intravenous, intramuscular, subcutaneous transdermal or oral. The daily dose may be divided into one, two or more doses in suitable form to be administered at one, two or more times during a certain period of time.

In certain embodiment, the term "administering" includes administering two or more agents to a subject such that the agents and/or metabolites thereof are present in the subject simultaneously or substantially simultaneously. In certain embodiment, co-administration of a PI3K inhibitor with an additional anticancer agent (both components hereinafter referred to as "two active agents") refers to any administration of the two active agents, either separately or together, Wherein the two active agents are administered as part of a suitable dosage regimen aimed at obtaining the benefits of combination therapy. Thus, the two active agents may be administered as part of the same pharmaceutical composition or in separate pharmaceutical compositions. The additional agent may be administered before, simultaneously with, or after the administration of the PI3K inhibitor, or in some combination thereof. When a PI3K inhibitor is administered to a patient at repeated intervals, for example, during a standard course of treatment, the additional agent may be administered before, simultaneously with, or after each administration of the PI3K inhibitor, or in some combination thereof, or in a relative PI3K inhibiting The doses are administered at different intervals of treatment, or administered as a single dose before, at any time during the course of treatment with the PI3K inhibitor, or after the course of treatment with the PI3K inhibitor. In certain embodiment, the first agent can be administered before (e.g., 5 minutes, 15 minutes, 30 minutes, 45 minutes, 1 hour, 2 hours, 4 hours, 6 hours, 12 hours, 24 hours) the administration of the second therapeutic agent. , 48 hours, 72 hours, 96 hours, 1 week, 2 weeks, 3 weeks, 4 weeks, 5 weeks, 6 weeks, 8 weeks, or 12 weeks ago), substantially at the same time or after (for example, 5 minutes, 15 minutes, 30 minutes, 45 minutes, 1 hour, 2 hours, 4 hours, 6 hours, 12 hours, 24 hours, 48 hours, 72 hours, 96 hours, 1 week, 2 weeks, 3 weeks, 4 weeks, 5 weeks,

6 weeks , 8 weeks or 12 weeks later) administration.

In the present application, "monotherapy" refers to the use of an agent (for example, as a single compound or agent) alone (also referred to herein as "single (alone)"), for example in the absence of a second active ingredients to treat the same indication, for example cancer. In this context, the term "monotherapy" includes the use of a PI3K single agent or a second agent alone in the treatment of cancer.

In the present application, the term "synergy" or "synergistic" includes the additive effect of the combination of two or more agents that is higher than the individual effects of the two or more agents. In certain embodiment, synergy or synergistic effect refers to the beneficial effect of two or more agents used in combination, e.g., in a pharmaceutical composition or in a method of treatment. In certain embodiment, one or more beneficial effects are achieved by using a PI3K inhibitor in combination with a second therapeutic agent (e.g., one or more second therapeutic agents) described herein.

In certain embodiment, synergy means that a lower dose of one or both agents is required to achieve an effect. For example, a combination may provide a selected effect, such as a therapeutic effect, when at least one agent is administered at a lower dose than that required to achieve the same therapeutic effect when the agent is administered as a monotherapy. In certain embodiment, the combination of a PI3K inhibitor (e.g., a PI3K inhibitor) and a second agent (as described herein) allows the PI3K inhibitor to achieve the same therapeutic effect than when the PI3K inhibitor is administered as monotherapy. Administer at lower doses as required.

In certain embodiment, the synergistic effect reduces, prevents, delays or reduces the occurrence or likelihood of one or more side effects, toxicity, resistance otherwise associated with the administration of at least one of the agents.

In certain embodiment, the synergistic effect reduces resistance to at least one of the agents (e.g., reduces the measure of resistance or reduces the likelihood of developing resistance) or delays the development of resistance to at least one of the agents produce.

In certain embodiment, the synergistic effect is a reduction in minimal residual disease (MRD). In certain embodiment, the combination of a PI3K inhibitor (e.g., a PI3K inhibitor described herein) and a second agent (e.g., a second agent described herein) is effective to reduce MRD in a subject, e.g., low levels previously measured in a subject (e.g., levels measured prior to administration of the combination). In certain embodiment, the combination of a PI3K inhibitor and a second agent is effective to reduce MRD in a subject below levels observed during or after treatment with a monotherapy comprising, for example, a PI3K inhibitor or a second agent. Monotherapy with any of the agents. In certain embodiment, the MRD is reduced below levels observed during treatment with a monotherapy comprising a PI3K inhibitor. In certain embodiment, the MRD is reduced below the level observed during treatment with the monotherapy comprising the second agent. In certain embodiment, the combination is effective to reduce the level of MRD to a preselected cutoff value (e.g., 1 malignant cell in 100 normal cells, 1 malignant cell in 1000 normal cells, or 10,000 normal cells 1 malignant tumor cell per cell, or 1 malignant tumor cell per 100,000 normal cells). In certain embodiment, the preselected cutoff value is 1 malignant cell in 1000 normal cells. In certain embodiment, the preselected cutoff value is 1 malignant cell in 100,000 normal cells.

In certain embodiment, synergy refers to a PI3K inhibitor (e.g., a PI3K inhibitor or a pharmaceutically acceptable form thereof) and a second therapeutic agent (e.g., one or more additional therapeutic agents or a pharmaceutically acceptable form thereof). The combination produces a higher therapeutic effect than the additive effect of the PI3K inhibitor and the second agent.

In the present application, the term "pharmaceutically acceptable" generally refers to one or more non-toxic substances that do not interfere with the effectiveness of the biological activity of the active ingredient. Such formulations will generally contain salts, buffers, preservatives, compatible carriers and optionally other therapeutic agents.

In the present application, "pharmaceutically acceptable form" of a compound includes, but is not limited to, pharmaceutically acceptable salts, hydrates, solvates, isomers, prodrugs and isotopelabeled derivatives of the disclosed compounds. In certain embodiment, "pharmaceutically acceptable form" includes, but is not limited to, pharmaceutically acceptable salts, isomers, prodrugs, and isotopically labeled derivatives of the disclosed compounds.

In certain embodiment, the pharmaceutically acceptable form is a pharmaceutically acceptable salt. As used herein, the term "pharmaceutically acceptable salt" refers to those salts which are, within the scope of sound medical judgment, suitable for use in contact with the tissues of subjects without undue toxicity, irritation, allergic response and the like, and are commensurate with a reasonable benefit/risk ratio. Pharmaceutically acceptable salts are well known in the art. For example, Berge et al. describes pharmaceutically acceptable salts in detail v& J. Pharmaceutical Sciences (1977) 66: 1-19. Pharmaceutically acceptable salts of the compounds provided herein include those derived from suitable inorganic and organic acids and bases. Examples of pharmaceutically acceptable, nontoxic acid addition salts are salts of an amino group formed with inorganic acids such as hydrochloric acid, hydrobromic acid, phosphoric acid, sulfuric acid and perchloric acid or with organic acids such as acetic acid, oxalic acid, maleic acid, tartaric acid, citric acid, succinic acid or malonic acid or by using other methods used in the art such as ion exchange. Other pharmaceutically acceptable salts include adipate, alginate, ascorbate, aspartate, benzenesulfonate, besylate, benzoate, bisulfate, borate, butyrate, camphorate, camphorsulfonate, citrate, cyclopentanepropionate, digluconate, dodecylsulfate, ethanesulfonate,

formate, fumarate, glucoheptonate, glycerophosphate, gluconate, hemisulfate, heptanoate, hexanoate, hydroiodide, 2-hydroxy-ethanesulfonate, lactobionate, lactate, laurate, lauryl sulfate, malate, maleate, malonate, methanesulfonate, 2-naphthalenesulfonate, nicotinate, nitrate, oleate, oxalate, palmitate, pamoate, pectinate, persulfate, 3-phenylpropionate, phosphate, picrate, pivalate, propionate, stearate, succinate, sulfate, tartrate, thiocyanate, p-toluenesulfonate, undecanoate, valerate salts, and the like. In certain embodiment, organic acids from which salts can be derived include, for example, acetic acid, propionic acid, glycolic acid, pyruvic acid, oxalic acid, maleic acid, malonic acid, succinic acid, fumaric acid, tartaric acid, citric acid, benzoic acid, cinnamic acid, mandelic acid, methanesulfonic acid, ethanesulfonic acid, p-toluenesulfonic acid, salicylic acid, and the like.

In certain embodiment, the pharmaceutically acceptable form is a pharmaceutically acceptable salt. As used herein, the term "pharmaceutically acceptable salt" refers to those salts which are, within the scope of sound medical judgment, suitable for use in contact with the tissues of subjects without undue toxicity, irritation, allergic response and the like, and are commensurate with a reasonable benefit/risk ratio. Pharmaceutically acceptable salts are well known in the art. For example, Berge et al. describes pharmaceutically acceptable salts in detail v& J. Pharmaceutical Sciences (1977) 66: 1-19. Pharmaceutically acceptable salts of the compounds provided herein include those derived from suitable inorganic and organic acids and bases. Examples of pharmaceutically acceptable, nontoxic acid addition salts are salts of an amino group formed with inorganic acids such as hydrochloric acid, hydrobromic acid, phosphoric acid, sulfuric acid and perchloric acid or with organic acids such as acetic acid, oxalic acid, maleic acid, tartaric acid, citric acid, succinic acid or malonic acid or by using other methods used in the art such as ion exchange. Other pharmaceutically acceptable salts include adipate, alginate, ascorbate, aspartate, benzenesulfonate, besylate, benzoate, bisulfate, borate, butyrate, camphorate, camphorsulfonate, citrate, cyclopentanepropionate, digluconate, dodecylsulfate, ethanesulfonate, formate, fumarate, glucoheptonate, glycerophosphate, gluconate, hemisulfate, heptanoate, hexanoate, hydroiodide, 2-hydroxy-ethanesulfonate, lactobionate, lactate, laurate, lauryl sulfate, malate, maleate, malonate, methanesulfonate, 2-naphthalenesulfonate, nicotinate, nitrate, oleate, oxalate, palmitate, pamoate, pectinate, persulfate, 3-phenylpropionate, phosphate, picrate, pivalate, propionate, stearate, succinate, sulfate, tartrate, thiocyanate, p-toluenesulfonate, undecanoate, valerate salts, and the like. In certain embodiment, organic acids from which salts can be derived include, for example, acetic acid, propionic acid, glycolic acid, pyruvic acid, oxalic acid, maleic acid, malonic acid, succinic acid, fumaric acid, tartaric acid, citric acid, benzoic acid, cinnamic acid, mandelic acid, methanesulfonic acid, ethanesulfonic acid, p-toluenesulfonic acid, salicylic acid, and the like.

In certain embodiment, the term "solvate" refers to compounds that further include a stoichiometric or non-stoichiometric amount of solvent bound by non-covalent intermolecular forces. The solvate can be of a disclosed compound or a pharmaceutically acceptable salt thereof. Where the solvent is water, the solvate is a "hydrate". Pharmaceutically acceptable solvates and hydrates are complexes that, for example, can include 1 to about 100, or 1 to about 10, or 1 to about 2, about 3 or about 4, solvent or water molecules. It will be understood that the term "compound" as used herein encompasses the compound and solvates of the compound, as well as mixtures thereof.

In certain embodiment, the term "prodrug" refers to compounds that are transformed in vivo to yield a disclosed compound or a pharmaceutically acceptable form of the compound. A prodrug can be inactive when administered to a subject, but is converted in vivo to an active compound, for example, by hydrolysis (e.g., hydrolysis in blood). In certain cases, a prodrug has improved physical and/or delivery properties over the parent compound. Prodrugs are typically designed to enhance pharmaceutically and/or pharmacokinetic ally based properties associated with the parent compound. The prodrug compound often offers advantages of solubility, tissue compatibility or delayed release in a mammalian organism (see, e.g., Bundgard, H., Design of Prodrugs (1985), pp. 7-9, 21-24 (Elsevier, Amsterdam). A discussion of prodrugs is provided in Higuchi, T., et al., "Pro-drugs as Novel Delivery Systems," A. C. S. Symposium Series, Vol. 14, and in Bioreversible Carriers in Drug Design, ed. Edward B. Roche, American Pharmaceutical Association and Pergamon Press, 1987, both of which are incorporated in full by reference herein. Exemplary advantages of a prodrug can include, but are not limited to, its physical properties, such as enhanced water solubility for parenteral administration at physiological pH compared to the parent compound, or it enhances absorption from the digestive tract, or it can enhance drug stability for long-term storage.

The term "prodrug" is also meant to include any covalently bonded carriers, which release the active compound in vivo when such prodrug is administered to a subject. Prodrugs of an active compound, as described herein, can be prepared by modifying functional groups present in the active compound in such a way that the modifications are cleaved, either in routine manipulation or in vivo, to the parent active compound. Prodrugs include compounds wherein a hydroxy, amino or mercapto group is bonded to any group that, when the prodrug of the active compound is administered to a subject, cleaves to form a free hydroxy, free amino or free mercapto group, respectively. Examples of prodrugs include, but are not limited to, acetate, formate and benzoate derivatives of an alcohol or acetamide, formamide and benzamide derivatives of an amine functional group in the active compound and the like. Other examples of prodrugs include compounds that comprise -NO, -N02, -ONO, or - ON02 moieties. Prodrugs can typically be prepared using well-known methods, such as those described in Burger’s Medicinal Chemistry and Drug Discovery, 172-178, 949-982 (Manfred E. Wolff ed., 5th ed., 1995), and Design of Prodrugs (H. Bundgaard ed., Elselvier, New York, 1985).

For example, if a disclosed compound or a pharmaceutically acceptable form of the compound contains a carboxylic acid functional group, a prodrug can comprise a pharmaceutically acceptable ester formed by the replacement of the hydrogen atom of the acid group with a group such as $(C_1\text{-}C_8)$alkyl, $(C_2\text{-} C_{12})$alkanoyloxymethyl, l-(alkanoyloxy)ethyl having from 4 to 9 carbon atoms, 1 - methyl- l-(alkanoyloxy)-ethyl having from 5 to 10 carbon atoms, alkoxycarbonyloxymethyl having from 3 to 6 carbon atoms, 1- (alkoxycarbonyloxy)ethyl having from 4 to 7 carbon atoms, 1 -methyl-l-(alkoxycarbonyloxy)ethyl having from 5 to 8 carbon atoms, N-(alkoxycarbonyl)aminomethyl having from 3 to 9 carbon atoms, l-(N-(alkoxycarbonyl)amino)ethyl having from 4 to 10 carbon atoms, 3-phthalidyl, 4-crotonolactonyl, gamma-butyrolacton-4-yl, di-N,N-$(C_1\text{-}C_2)$alkylamino$(C_2\text{-}C_3)$alkyl (such as β-dimethylaminoethyl), carbamoyl-$(C_1\text{-}C_2)$alkyl, N,N-di$(C_1\text{-}C_2)$alkylcarbamoyl-$(C_1\text{-}C_2)$alkyl and piperidino-, pyrrolidino- or mo holino$(C_2\text{-}C_3)$alkyl.

Similarly, if a disclosed compound or a pharmaceutically acceptable form of the compound contains an alcohol functional group, a prodrug can be formed by the replacement of the hydrogen atom of the alcohol group with a group such as $(C_1\text{-}C_6)$alkanoyloxymethyl, l-($(C_1\text{-}C_6)$alkanoyloxy)ethyl, 1 -methyl- 1 -($(C_1\text{-}C_6)$alkanoyloxy)ethyl $(C_1\text{-}C_6)$alkoxycarbonyloxymethyl, N-(Ci-C6)alkoxycarbonylaminomethyl, succinoyl, $(C_1\text{-}C_6)$alkanoyl, a-amino$(C_1\text{-}C_4)$alkanoyl, arylacyl and a-aminoacyl, or a-aminoacyl-a-aminoacyl, where each a-aminoacyl group is independently selected from the naturally occurring L-amino acids, $P(O)(OH)_2$, $-P(O)(O(C_1\text{-}C_6)alkyl)_2$ or glycosyl (the radical resulting from the removal of a hydroxyl group of the hemiacetal form of a carbohydrate).

If a disclosed compound or a pharmaceutically acceptable form of the compound incorporates an amine functional group, a prodrug can be formed by the replacement of a hydrogen atom in the amine group with a group such as R-carbonyl, RO-carbonyl, NRR'-carbonyl where R and R' are each independently $(C_1\text{-}C_{10})$alkyl, $(C_3\text{-}C_7)$cycloalkyl, benzyl, a natural α-aminoacyl or natural a-aminoacyl-natural α-aminoacyl,-C(OH)C(O)OY$^1$ wherein $Y^1$ is H, $(C_1\text{-}C_6)$alkyl or benzyl, - C(OY$^2$)Y$^3$ wherein $Y^2$ is $(C_1\text{-}C_4)$ alkyl and $Y^3$ is $(C_1\text{-}C_6)$alkyl, carboxy$(C_1\text{-}C_6)$alkyl, amino$(C_1\text{-}C_4)$alkyl or mono-N- or di-N,N-$(C_1\text{-}C_6)$alkylaminoalkyl,-C(Y$^4$)Y$^5$ wherein $Y^4$ is H or methyl and $Y^3$ is mono-N-or di-N,N-$(C_1\text{-}C_6)$alkylamino, morpholino, piperidin-1-yl or pyrrolidin-1-yl.

In certain embodiment, "isomers" are different compounds that have the same molecular formula. "Stereoisomers" are isomers that differ only in the way the atoms are arranged in space. As used herein, the term "isomer" includes any and all geometric isomers and stereoisomers. For example, "isomers" include geometric double bond cis- and trans-isomers, also termed E- and Z-isomers; R- and S-enantiomers; diastereomers, (d)-isomers and (l)-isomers, racemic mixtures thereof; and other mixtures thereof, as falling within the scope of this disclosure.

"Enantiomers" are a pair of stereoisomers that are non-superimposable mirror images of each other. A mixture of a pair of enantiomers in any proportion can be known as a "racemic" mixture. The term "(±)" is used to designate a racemic mixture where appropriate. "Diastereoisomers" are stereoisomers that have at least two asymmetric atoms, but which are not mirror-images of each other. The absolute stereochemistry is specified according to the Cahn-Ingold-Prelog R-S system. When a compound is an enantiomer, the stereochemistry at each chiral carbon can be specified by either R or S. Resolved compounds whose absolute configuration is unknown can be designated (+) or (-) depending on the direction (dextro- or levorotatory) which they rotate plane polarized light at the wavelength of the sodium D line. Certain of the compounds described herein contain one or more asymmetric centers and can thus give rise to enantiomers, diastereomers, and other stereoisomeric forms that can be defined, in terms of absolute stereochemistry at each asymmetric atom, as (R)- or (S)-. The present chemical entities, pharmaceutical compositions and methods are meant to include all such possible isomers, including racemic mixtures, optically substantially pure forms and intermediate mixtures. Optically active (R)- and (S)- isomers can be prepared, for example, using chiral synthons or chiral reagents, or resolved using conventional techniques. When the compounds described herein contain olefinic double bonds or other centers of geometric asymmetry, and unless otherwise indicated, it is intended that the compounds include both E and Z geometric isomers.

"Enantiomeric purity" as used herein refers to the relative amounts, expressed as a percentage, of the presence of a specific enantiomer relative to the other enantiomer. For example, if a compound, which can potentially have an (R)- or an (S)- isomeric configuration, is present as a racemic mixture, the enantiomeric purity is about 50% with respect to either the (R)- or (S)- isomer. If that compound has one isomeric form predominant over the other, for example, 80% (S)- and 20% (R)-, the enantiomeric purity of the compound with respect to the (S)- isomeric form is 80%. The enantiomeric purity of a compound can be determined in a number of ways known in the art, including but not limited to chromatography using a chiral support, polarimetric measurement of the rotation of polarized light, nuclear magnetic resonance spectroscopy using chiral shift reagents which include but are not limited to lanthanide containing chiral complexes or the Pirkle alcohol, or derivatization of a compounds using a chiral compound such as Mosher's acid followed by chromatography or nuclear magnetic resonance spectroscopy.

In certain embodiment, the term "tautomer" is a type of isomer that includes two or more interconvertable compounds resulting from at least one formal migration of a hydrogen atom and at least one change in valency {e.g., a single bond to a double bond, a triple bond to a single bond, or vice versa). "Tautomerization" includes prototropic or proton-shift tautomerization, which is considered a subset of acid-base chemistry. "Prototropic tautomerization" or "proton-shift tautomerization" involves the migration of a proton accompanied by changes in bond order. The exact ratio of

the tautomers depends on several factors, including temperature, solvent, and pH. Where tautomerization is possible {e.g., in solution), a chemical equilibrium of tautomers can be reached. Tautomerizations {i.e., the reaction providing a tautomeric pair) can be catalyzed by acid or base, or can occur without the action or presence of an external agent. Exemplary tautomerizations include, but are not limited to, keto-to-enol; amide-to-imide; lactam- to-lactim; enamine-to-imine; and enamine-to-(a different) enamine tautomerizations. A specific example of keto-enol tautomerization is the interconversion of pentane-2,4-dione and 4-hydroxypent-3-en-2-one tautomers. Another example of tautomerization is phenol-keto tautomerization. A specific example of phenol-keto tautomerization is the interconversion of pyridin-4-ol and pyridin-4(IH)-one tautomers.

Unless otherwise stated, structures depicted herein are also meant to include compounds which differ only in the presence of one or more isotopically enriched atoms. For example, compounds having the present structures except for the replacement of a hydrogen by a deuterium or tritium, or the replacement of a carbon by $^{13}C$- or $^{14}C$-enriched carbon are within the scope of this disclosure.

The disclosure also embraces isotopically labeled compounds which are identical to those recited herein, except that one or more atoms are replaced by an atom having an atomic mass or mass number different from the atomic mass or mass number usually found in nature. Examples of isotopes that can be incorporated into disclosed compounds include isotopes of hydrogen, carbon, nitrogen, oxygen, phosphorus, fluorine and chlorine, such as $^{2}H$, $^{3}H$, $^{13}C$, $^{14}C$, $^{15}N$, $^{18}O$, $^{17}O$, $^{31}P$, $^{32}P$, $^{35}S$, $^{18}F$, and $^{36}C1$, respectively. Certain isotopically labeled disclosed compounds (e.g., those labeled with $^{3}H$ and $^{14}C$) are useful in compound and/or substrate tissue distribution assays. Tritiated (i.e., $^{3}H$) and carbon-14 (i.e., $^{14}C$) isotopes can allow for ease of preparation and detectability. Further, substitution with heavier isotopes such as deuterium (i. e., $^{2}H$) can afford certain therapeutic advantages resulting from greater metabolic stability (e.g. , increased in vivo half-life or reduced dosage requirements). Isotopically labeled disclosed compounds can generally be prepared by substituting an isotopically labeled reagent for a non-isotopically labeled reagent. In certain embodiment, provided herein are compounds that can also contain unnatural proportions of atomic isotopes at one or more of atoms that constitute such compounds. All isotopic variations of the compounds as provided herein, whether radioactive or not, are encompassed within the scope of the present disclosure.

"Pharmaceutically acceptable carrier" or "pharmaceutically acceptable excipient" includes any and all solvents, dispersion media, coatings, antibacterial and antifungal agents, isotonic and absorption delaying agents and the like. The use of such media and agents for pharmaceutically active substances is well known in the art. Except insofar as any conventional media or agent is incompatible with the active ingredient, its use in the therapeutic compositions as provided herein is contemplated. Supplementary active ingredients can also be incorporated into the pharmaceutical compositions.

In the present application, the term "tumor" generally refers to any neoplastic cell growth and proliferation, malignant or benign, as well as any pre-cancerous and cancerous cells and tissues. In the present application, the term "neoplastic" refers to any form of abnormally regulated or unregulated cell growth, malignant or benign, resulting in abnormal tissue growth. Thus, "neoplastic cells" include malignant and benign cells with abnormally regulated or unregulated cell growth. The terms "cancer", "cancerous", "cell proliferative disorder", "proliferative disorder" and "tumor" are not mutually exclusive when referred to In the present application. In certain embodiment, a tumor may refer to a mass of flesh containing a plurality of cancer cells, e.g., cells exhibiting characteristics of any of the cancers described In the present application. In the present application, a tumor may be a solid tumor or a non-solid tumor (eg, hematological tumor, lymphoma). The term "solid tumor" generally refers to an abnormal growth or mass of tissue that usually does not contain cysts or fluid areas. Solid tumors can be benign (noncancerous) or malignant (cancerous). In the present application, the tumor may be a tumor with abnormal expression of PD-1 or PD-L1 in cells and tissues. In the present application, the tumor may be a tumor with abnormal expression or abnormal activity of TGFβ or TGFβR in cells and tissues. In the present application, the tumor may be a tumor with abnormal expression or activity of PI3K in cells and tissues.

In the present application, the term refers to a point where cancer has a reduced response to the treatment, for example, until the cancer does not respond to treatment. Cancer can be resistant at the beginning of treatment or it becomes resistant during the treatment. The cancer subject may have one or more mutations caused to become resistant to treatment, or subject can form such mutation during the treatment. The term "refractory" can refer to cancers for which treatment (e.g., chemotherapeutic drugs, biologics, and/or radiation therapy) has proven ineffective. Refractory cancers can shrink, but not to the extent that treatment is definitively effective. Typically, however, the tumor remains the same size as it was before treatment (stable disease), or grows (progressive disease).

In the present application, the terms "treatment" and "treating" generally refer to a method of obtaining a beneficial or desired result, including, but not limited to, a therapeutic benefit. A therapeutic benefit includes, but is not limited to, eradicating, inhibiting, reducing or ameliorating the underlying disorder being treated. Additionally, therapeutic benefit is achieved by eradicating, reducing or ameliorating one or more physiological symptoms associated with the underlying disorder such that improvement is observed in a patient, but the patient may still suffer from the underlying disorder.

In the present application, the terms "prevention" and "preventing" generally refer to methods of obtaining beneficial or desired results, including but not limited to prophylactic benefits. For prophylactic benefit, pharmaceutical compositions may be administered to patients at risk of developing a particular disease or to patients reporting one or more physical symptoms of a disease, even if the disease has not yet been diagnosed.

The term "subject" or "patient" is contemplated to humans (i.e., a male or female of any age group, e.g., a pediatric subject (e.g., infant, child, adolescent) or adult subject (e.g., young adult, middle-aged adult or senior adult)) and/or other primates (e.g., cynomolgus monkeys, rhesus monkeys); mammals, including commercially relevant mammals such as cattle, pigs, horses, sheep, goats, cats, and/or dogs; and/or birds, including commercially relevant birds such as chickens, ducks, geese, quail, and/or turkeys.

In the present application, the terms "about" or "approximately" generally refer to an acceptable error for a particular value as determined by one of ordinary skill in the art, depending in part on the manner in which the value was measured or determined. In certain embodiment, the term "about" or "approximately" generally refers to 1, 2, 3 or 4 standard deviations. In certain embodiment, the term "about" or "approximately" generally refers to 50%, 20%, 15%, 10%, 9%, 8%, 7%, 6%, 5% of a given value or range , 4%, 3%, 2%, 1%, 0.5% or 0.05%.

In the present application, the term "comprising" or "comprising" generally refers to an open form, and it should be understood that other substances not mentioned may also be included. For example, a drug combination comprising a phosphoinositide 3-kinase (PI3K) inhibitor and a second therapeutic agent, wherein the second therapeutic agent is an inhibitor of an immune checkpoint, a TGFβ inhibitor, a bifunctional immune checkpoint/ TGFβ inhibitors or combinations thereof. Wherein, "comprising" should be understood as containing other substances besides the phosphoinositide 3-kinase (PI3K) inhibitor and the second therapeutic agent.

## Details of invention

### Pharmaceutical combination

For one aspect, the present application provides a pharmaceutical combination comprising a phosphoinositide 3-kinase (PI3K) inhibitor and a second therapeutic agent, wherein the second therapeutic agent is an immune checkpoint inhibitor, a TGFβ inhibitor, a bifunctional immune checkpoint/ TGFβ inhibitors or combinations thereof.

In certain embodiment, wherein the immune checkpoint inhibitor comprises an agent capable of blocking the interaction between programmed death 1 (PD-1) and programmed death ligand 1 (PD-L1).

In certain embodiment, wherein the immune checkpoint inhibitor comprises a programmed death ligand 1 (PD-L1) and/or programmed death 1 (PD-1 ) inhibitor. For example, human PD-1 inhibitor, for example human PD-L1 inhibitor. As another example, the immune checkpoint inhibitor may comprise an anti-PD-L1 antibody or an antigen-binding fragment thereof, or an anti-PD-1 antibody or an antigen-binding fragment thereof. In certain embodiment, the inhibitor of PD-1 or PD-L1 can be an antibody molecule against PD-1 or PD-L1. PD-1 or PD-L1 inhibitors can be administered alone or in combination with other immune checkpoint modulators, for example, with inhibitors of LAG-3, TIM-3, or CTLA-4.

In certain embodiment, wherein the combination comprises:

1) the combination of PD-L1 inhibitor and PI3K inhibitor;
2) the combination of TGFβ inhibitor and PI3K inhibitor;
3) the combination of PD-1 inhibitor, TGFβ inhibitor and PI3K inhibitor;
4) the combination of PD-L1 inhibitor, TGFβ inhibitor and PI3K inhibitor;
5) the combination of PD-L1/TGFβ dual inhibitor and PI3K inhibitor; or
6) the combination of PD-1/TGFβ dual inhibitor and PI3K inhibitor.

In certain embodiment, wherein the inhibitors of immune checkpoints include nucleic acids(e.g. dsRNA, siRNA or sh RNA), polypeptide(e.g. soluble ligands, antibodies or antigen-binding fragments thereof, immuneoadhesins, fusion proteins, oligopeptides and other molecules) or compound. For example, antibody is selected from: human antibody, humanized antibody, chimeric antibody, multispecific antibody, monoclonal antibody and polyclonal antibody. For example, the antigen-binding fragment is selected from Fab, Fab', $F(ab')_2$, Fv, scFv, bispecific antibody, Fd, dAb, VHH, large antibody and complementarity determining region (CDR) fragments.

In certain embodiment, the antibody or antigen-binding fragment thereof comprises a heavy chain variable region (VH) comprising HCDR1, HCDR2 and HCDR3.

In certain embodiment, the antibody or antigen-binding fragment thereof further comprises or does not comprise a CH1 domain.

In certain embodiment, the antibody or antigen-binding fragment thereof further comprises or does not comprise CH2 and CH3 domains. For example, the antibody or antigen-binding fragment comprises VH-CH2-CH3 domain.

In certain embodiment, the antibody or antigen-binding fragment thereof further comprises an antibody heavy

chain constant region

In certain embodiment, the antibody heavy chain constant region is a human antibody heavy chain constant region. For example, the human antibody heavy chain constant region is selected from constant regions derived from the group consisting of IgGl, IgG2, IgG3, IgG4 and variants thereof.

In certain embodiment, the antibody or antigen-binding fragment thereof further comprises a light chain variable region (VL) comprising LCDR1, LCDR2, LCDR3.

For example, the antibody or antigen-binding fragment comprise a heavy chain variable region (VL) comprising HCDR1, HCDR2, HCDR3, and a light chain variable region (VL) comprising LCDR1, LCDR2, LCDR3.

In certain embodiment, the antibody or antigen-binding fragment comprises a light chain constant region (CL). For example, the antibody or antigen-binding fragment comprises a heavy chain comprising VH and CH1, and light chain comprising VL and CL. For example, the antibody or antigen-binding fragment comprises a heavy chain comprising VH, CH1, CH2 and CH3, and antibody light chain comprising VL and CL.

In certain embodiment, the checkpoint inhibitor could be PD-1/PD/L1 inhibitor. The PD-1/PD/L1 inhibitor comprise but not limited to those described in US 7,488,802, US 7,943,743, US 8,008,449, US 8,168,757, US 8,217,149, US 8,609,089, US 2010/028330, US 2012/0114649, WO 2003/042402, WO 2008/156712, WO 2010/089411, WO 2010/036959, WO 2011/066342, WO 2011/159877, WO 2011/082400 and WO 2011/161699 those described in, all of which are incorporated by reference into this application in their entirety.

In certain embodiment, checkpoint modulate could be PD-1 inhibitor. For example, PD-1 modulate could be anti-PD-1 antibody.

For example, the anti-PD-1 antibody can be Nivolumab. Alternative names for Nivolumab include MDX-1106, MDX-1106-04, ONO-4538, or BMS-936558, and the CAS registry number is: 946414-94-4. Nivolumab is a full-length human IgG4 monoclonal antibody that specifically blocks PD-1. Nivolumab (clone 5C4) and other human monoclonal antibodies that specifically bind PD-1 are disclosed in US 8,008,449 and WO 2006/121168.

For example, the anti-PD-1 antibody can be Pembrolizumab. Pembrolizumab (trade name KEYTRUDA, formerly Lambrolizumab, also known as Merck 3745, MK-3475 or SCH-900475) is a humanized IgG4 monoclonal antibody that binds to PD-1. Pembrolizumab is disclosed, e.g., in Hamid, O. et al. (2013) New England Journal of Medicine 369(2): 134-44, WO 2009/114335, and US 8,354,509.

For example, the anti-PD-1 antibody can be Pidilizumab. Pidilizumab (CT-011; CureTech) is a humanized IgG1k monoclonal antibody that binds to PD-1. Pidilizumab and other humanized anti-PD-1 monoclonal antibodies are disclosed in WO 2009/101611. Other anti-PD1 antibodies are disclosed in US 8,609,089, US 2010/028330 and/or US 2012/0114649.

For example, anti-PD1 antibody could be AMP-514(Amplimmune).

For example, the PD-1 inhibitor may be an immunoadhesin (e.g., an immunoadhesin comprising an extracellular or PD-1 binding portion of PDL1 or PDL2 fused to a constant region (e.g., an Fc region of an immunoglobulin sequence)).

In certain embodiment, checkpoint modulate is PD-L1 inhibitor. For example, checkpoint modulate could be anti PD-L1 antibody or its antigen-binding fragment. "PD-L1 antibody or antigen-binding fragment thereof' may include any anti-PD-L1 antibody or antigen-binding fragment thereof described in the art.

In certain embodiment, the anti-PD-L1 antibody or antigen-binding fragment thereof comprises a heavy chain variable region of an antibody, wherein the heavy chain variable region comprises HCDR1, HCDR2, HCDR3, which has at least about 80% (e.g. about 85%, 90%, or 95%)sequence identity to HCDR1, HCDR2, HCDR3, respectively, of atezolizumab, avelumab, BMS-936559, MPDL3280A (RG7446), or durvalumab (MEDI-4736).

In certain embodiment, the heavy chain is respectively associated with a heavy chain of the following molecule having at least about 80% (e.g. about 85%, 90%, or 95%) sequence identity: Atezolizumab, Avelumab, BMS-936559, MPDL3280A, or durvalumab.

In certain embodiment, the anti-PD-L1 antibody or antigen-binding fragment thereof comprises a heavy chain of an antibody, wherein the heavy chain has at least about 80% (e.g. about 85%, 90%, or 95%) sequence identity to the heavy chain, respectively, of atezolizumab, avelumab, BMS-936559, MPDL3280A (RG7446), or durvalumab (MEDI-4736).

In certain embodiment, the anti-PD-L1 antibody or antigen-binding fragment thereof further comprises an antibody light chain variable region, wherein the light chain variable region comprises LCDR1, LCDR2, LCDR3, each of which has at least about 80% (e.g. about 85%, 90%, or 95%) sequence identity to LCDR1, LCDR2, LCDR3, respectively, of atezolizumab, avelumab, BMS-936559, MPDL3280A, or durvalumab.

For example, the anti-PD-L1 antibody or antigen-binding fragment thereof comprises a heavy chain variable region and a light chain variable region of an antibody, wherein the heavy chain variable region comprises HCDR1, HCDR2, HCDR3, wherein the light chain can be The variable regions comprise LCDR1, LCDR2, LCDR3, each of which has at least about 80% (e.g., about 85%, 90%, or 95%) sequence identity to HCDR1, HCDR2, HCDR3, LCDR1, LCDR2, LCDR3, respectively, of: Atezolizumab, Avelumab, BMS-936559, MPDL3280A (RG7446), or durvalumab (MEDI4736).

In certain embodiment, the light chain variable region have at least about 80% (e.g., about 85%, 90%, or 95%)

sequence identity to the light chain variable region of atezolizumab, avelumab, BMS-936559, MPDL3280A, or durvalumab, respectively.

For example, the anti-PD-L1 antibody or antigen-binding fragment thereof comprises a heavy chain variable region and a light chain variable region, wherein is respectively associated with the heavy chain variable region and the light chain variable region of the following molecule having at least about 80%(e.g., about 85%, 90%, or 95%) sequence identity: Atezolizumab, Avelumab, BMS-936559, MPDL3280A(RG7446) or durvalumab(RG7446).

In certain embodiment, the anti-PD-L1 antibody or antigen-binding fragment thereof comprises a light chain, wherein the light chain having at least about 80%(e.g., about 85%, 90%, or 95%) sequence identity: Atezolizumab, Avelumab, BMS-936559, MPDL3280A or durvalumab.

For example, the anti-PD-L1 antibody or antigen-binding fragment thereof comprises a heavy chain and a light chain, wherein is respectively associated with the heavy chain and the light chain of the following molecule having at least about 80%(e.g., about 85%, 90%, or 95%) sequence identity: Atezolizumab, Avelumab, BMS-936559, MPDL3280A(RG7446) or durvalumab(RG7446).

In certain embodiment, the anti-PD-L1 antibody or antigen-binding fragment thereof comprises: Atezolizumab, Avelumab, BMS-936559, MPDL3280A or durvalumab, or an antigen-binding fragment thereof, or a variant or biosimilar thereof, or a combination thereof

In certain embodiment, the anti-PD-L1 antibody can also be a commercially available or published PD-L1 antibody. Including but not limited to, such as PD-L1 antibody BMS-936559, MPDL3280A, MEDI4736, MSB0010718C (see US2014341917, US20130034559, US8779108) and so on.

For example, an anti-PD-L1 antibody is MDX-1105. MDX-1105, also known as BMS-936559, an anti-PD-L1 antibody as described in WO 2007/005874. For example, the anti-PD-L1 antibody is YW243.55.S70. The YW243.55.S70 antibody is an anti-PD-L1 antibody as described in WO 2010/077634. The heavy and light chain variable region sequences of YW243.55.S70 are also described in WO2010/077634. For example, the anti-PD-L1 antibody is MDPL3280A (Genentech/Roche). MDPL3280A is a human Fc-optimized IgG1 monoclonal antibody that binds to PD-L1. MDPL3280A and other human monoclonal antibodies to PD-L1 are described in US 7,943,743 and US2012/0039906. For example, an anti-PD-L1 antibody is Avelumab (MSB0010718C). MSB0010718C (also known as A09-246-2; Merck Serono) is a monoclonal antibody that binds to PD-L1. Other humanized anti-PD-L1 antibodies are disclosed in WO 2013/079174. For example, an anti-PD-L1 antibody is durvalumab (CAS Number: 1428935-60-7). For example, the anti-PD-L1 antibody is Atezolizumab (CAS Number: 1380723-44-3).

In certain embodiment, the anti-PD-L1 antibody or antigen-binding fragment thereof comprises a heavy chain variable region of an antibody, wherein the heavy chain variable region comprises one or more heavy chain variable region CDRs (HCDRs) selected from the group consisting of: (a) HCDR1 having at least about 70% sequence identity to the amino acid sequence shown in SEQ ID NO: 1; (b) having at least about 70% sequence identity to SEQ ID NO:2 HCDR2 of sequence identity; and (c) HCDR3 having at least about 70% sequence identity to SEQ ID NO:3.

In certain embodiment, the anti-PD-L1 antibody or antigen-binding fragment thereof comprises an antibody heavy chain variable region, wherein the heavy chain variable region comprises one or more HCDRs selected from the group consisting of: (a) HCDR1 having the amino acid sequence shown in SEQ ID NO: 1 or having no more than 2 amino acid differences from the amino acid sequence shown in SEQ ID NO: 1 obtained through amino acid addition, elimination or substitution reactions HCDR1; (b) HCDR2 having the amino acid sequence shown in SEQ ID NO: 2 or HCDR2 having no more than 2 amino acid differences from the amino acid sequence shown in SEQ ID NO: 2 obtained through amino acid addition, elimination or substitution reactions; and (c) HCDR3 having the amino acid sequence shown in SEQ ID NO: 3 or HCDR3 having no more than 2 amino acid differences from the amino acid sequence shown in SEQ ID NO: 3 obtained through amino acid addition, elimination or substitution reactions.

In certain embodiment, the anti-PD-L1 antibody or antigen-binding fragment thereof comprises a heavy chain variable region of an antibody, wherein the heavy chain variable region comprises HCDR1, HCDR2, HCDR3, the said HCDR1 comprises an amino acid sequence with at least about 70% sequence identity to the amino acid sequence shown in SEQ ID NO: 1, said HCDR2 comprises an amino acid sequence with at least about 70% sequence identity to the amino acid sequence shown in SEQ ID NO: 2, wherein the HCDR3 comprises an amino acid sequence having at least about 70% sequence identity to the amino acid sequence set forth in SEQ ID NO:3.

In certain embodiment, the anti-PD-L1 antibody or antigen-binding fragment thereof comprises a heavy chain variable region of an antibody, wherein the heavy chain variable region comprises HCDR1, HCDR2, HCDR3, the said HCDR1 comprises the amino acid sequence shown in SEQ ID NO: 1 or an amino acid sequence obtained by amino acid addition, elimination or substitution reaction with no more than 2 amino acid differences from the amino acid sequence shown in SEQ ID NO: 1; HCDR2 comprises the amino acid sequence shown in SEQ ID NO: 2 or an amino acid sequence obtained by amino acid addition, elimination or substitution reaction with no more than 2 amino acid differences from the amino acid sequence shown in SEQ ID NO: 2; the HCDR3 comprising the amino acid sequence shown in SEQ ID NO: 3 or an amino acid sequence obtained by amino acid addition, elimination or substitution reaction and having no more than 2 amino acid differences from the amino acid sequence shown in SEQ ID NO: 3.

In certain embodiment, the anti-PD-L1 antibody or antigen-binding fragment thereof comprises an antibody heavy chain variable region, wherein the heavy chain variable region comprises an amino acid sequence selected from the group consisting of : (a) the amino acid sequence set forth in SEQ ID NO: 4; (b) an amino acid sequence at least about 85%, 90%, 95% or 99% sequence identity to the amino acid sequence set forth in SEQ ID NO: 4; and (c) an amino acid sequence having 1 or more differences from the amino acid sequence shown in SEQ ID NO: 4 obtained by addition, elimination or substitution reaction.

In certain embodiment, the TGFβ inhibitor binds to TGFβ1, TGFβ2 or TGFβ3.

In certain embodiment, the TGFβ inhibitor binds to TGFβ or transforming growth factor β receptor (TGFβR).

For example, the TGFβ inhibitor binds to TGFβ type I receptor (TGFβRI), TGFβ type II receptor (TGFβRII) or TGFβ type III receptor (TGFβRIII).

In certain embodiment, the TGFβ inhibitor comprises: 1) an anti-TGFβ antibody or an antigen-binding fragment thereof; 2) an anti-TGFβR antibody or an antigen-binding fragment thereof; 3) small molecule TGFβ inhibitors; 4) proteins comprising transforming growth factor β receptor II (TGFβRII) or functionally active fragments thereof, or variants or biosimilars thereof, or combinations thereof.

In certain embodiment, anti-TGFβ antibody or antigen-binding fragment thereof comprises an antibody heavy chain variable region, wherein said heavy chain variable region comprises HCDR1, HCDR2, HCDR3, each of which has at least about 80%(e.g. about 85%, 90%, or 95%) sequence identity with HCDR1, HCDR2, and HCDR3 of the fresolimumab molecule(CAS Number: 948564-73-6)).

In certain embodiment, the heavy chain variable region has at least about 80%(e.g. about 85%, 90%, or 95%) sequence identity to the heavy chain variable region of Fresolimumab.

In certain embodiment, the heavy chain has at least about 80%(e.g. about 85%, 90%, or 95%) sequence identity to the heavy chain variable region of Fresolimumab.

In certain embodiment, the anti-TGFβ antibody or antigen-binding fragment thereof comprises an antibody's light chain variable region, wherein said light chain variable region comprises LCDR1, LCDR2 , LCDR3, each of which has at least about 80% (e.g. about 85%, 90%, or 95%) sequence identity with LCDR1, LCDR2, and LCDR3 of the Fresolimumab.

For example, the anti-TGFβ antibody or antigen-binding fragment thereof comprises a heavy chain variable region and a light chain variable region of an antibody, wherein the heavy chain variable region comprises HCDR1, HCDR2, HCDR3, wherein the light chain variable region comprises LCDR1 , LCDR2, LCDR3, each of which may have at least about 80% (eg, 85%, 90%, or 95%) sequence identity with HCDR1, HCDR2, HCDR3, LCDR1, LCDR2, LCDR3 of the Fresolimumab molecule, respectively.

In certain embodiment, the light chain variable region has at least about 80% sequence identity to the light chain variable region.

For example, the anti-TGFβ antibody or antigen-binding fragment thereof comprises a heavy chain variable region and a light chain variable region of an antibody, each of which can be at least about (eg, 85%, 90%, or 95%) sequence identity with such heavy chain variable region and light chain variable region of Fresolimumab molecule.

In certain embodiment, the light chain has at least about 80% sequence identity to the light chain variable region.

For example, the anti-TGFβ antibody or antigen-binding fragment thereof comprises a heavy chain and a light chain of the antibody, each of which may have at least about 80% (e.g., 85%, 90% or 95%) sequence identity with the heavy chain and light chain of the Fresolimumab molecule, respectively.

In certain embodiment, the anti-TGFβ antibody or antigen-binding fragment thereof comprises: Fresolimumab, or an antigen-binding fragment thereof, or a variant or biosimiliar thereof.

In certain embodiment, the small molecule TGFβ inhibitor comprises the following compound: SB525334, SD-208, SB431542, LY2109761, LY2157299, GW788388, RepSox, SIS3, LDN-193189, EW-7197, LY364947, or a combination thereof.

In certain embodiment, the TGFβRII comprises a polypeptide having a wild-type human TGFβ receptor type 2 isoform A sequence(For example, the amino acid sequence of NCBI reference sequence (Ref Seq) accession number NP_001020018), or a polypeptide having a wild-type human TGFβ receptor type 2 isoform B sequence(For example, the amino acid sequence of NCBI reference sequence (Ref Seq) accession number NP_003233), or a polypeptide having a sequence substantially identical to an amino acid sequence thereof.

In certain embodiment, wherein said TGFβ RII or its functionally active fragment comprises: human TGFβRII extracellular domain (ECD), any part of NCBI Ref Seq NO. NP_001020018 or NCBI Ref Seq NO. NP_003233, or an amino acid sequence substantially identical to sequence((e.g., about 80%, 85%, 90% or 95%)).

In certain embodiment, wherein the TGFβRII or a functionally active fragment thereof comprises:

(a) the amino acid sequence of SEQ ID NO: 6;

(b) an amino acid sequence having at least about 85% sequence identity to the amino acid sequence set forth in SEQ ID NO: 6; Or

(c) has an amino acid sequence in which one or more amino acids are added, deleted and/or substituted as compared to the amino acid sequence shown in SEQ ID NO: 6.

In certain embodiment, wherein the bifunctional immune checkpoint/TGF β inhibitor is a fusion protein.
In certain embodiment, wherein the PD-L1/TGFβ dual inhibitor or PD-1/TGFβ dual inhibitor is a fusion protein.
In certain embodiment, wherein the PD-L1//TGF β dual inhibitor comprises a PD-L1 targeting moiety and a TGF β receptor domain comprising transforming growth factor β receptor II (TGFβ RII) or a functionally active fragment thereof.
In certain embodiment, wherein the PD-L1//TGF β dual inhibitor comprises a polypeptide, wherein the polypeptide comprises at least: (i) a heavy chain variable region of an anti-PD-L1 antibody; And (ii) TGFβII or a functionally active fragment thereof.
In certain embodiment, wherein the anti-PD-L1 antibody heavy chain variable region comprises HCDR1, HCDR2, HCDR3; Wherein the HCDR1 comprises an amino acid sequence having at least about 70% sequence identity to the amino acid sequence set forth in SEQ ID NO: 1, the HCDR2 comprises an amino acid sequence having at least about 70% sequence identity to the amino acid sequence set forth in SEQ ID NO: 2, and the HCDR3 comprises an amino acid sequence having at least about 70% sequence identity to the amino acid sequence set forth in SEQ ID NO: 3.
In certain embodiment, wherein the anti-PD-L1 antibody heavy chain variable region comprises HCDR1, HCDR2, HCDR3, and the HCDR1 comprises the amino acid sequence shown in SEQ ID NO: 1 or an amino acid sequence obtained by amino acid addition, elimination or substitution reaction and having no more than 2 amino acid differences from the amino acid sequence shown in SEQ ID NO: 1; The HCDR2 comprises the amino acid sequence represented by SEQ ID NO: 2 or an amino acid sequence obtained by amino acid addition, elimination or substitution reaction and having no more than 2 amino acid differences from the amino acid sequence represented by SEQ ID NO: 2; The HCDR3 comprises an amino acid sequence represented by SEQ ID NO: 3 or an amino acid sequence obtained by an amino acid addition, elimination or substitution reaction and having no more than 2 amino acid differences from the amino acid sequence represented by SEQ ID NO: 3.
In certain embodiment, wherein the anti-PD-L1 antibody heavy chain variable region comprises an amino acid sequence selected from the group consisting of: (a) the amino acid sequence set forth in SEQ ID NO: 4; (b) an amino acid sequence having at least about 85%, 90%, 95%, or 99% sequence identity to the amino acid sequence set forth in SEQ ID NO: 4; (c) an amino acid sequence having 1 or more differences from the amino acid sequence shown in SEQ ID NO: 4 obtained by addition, elimination or substitution.
In certain embodiment, wherein the TGFβRII or a functionally active fragment thereof comprises:

(a) the amino acid sequence of SEQ ID NO: 6;
(b) an amino acid sequence having at least about 85% sequence identity to the amino acid sequence set forth in SEQ ID NO: 6; or
(c) an amino acid sequence in which one or more amino acids are added, deleted and/or substituted as compared to the amino acid sequence shown in SEQ ID NO: 6

In certain embodiment, wherein the polypeptide further comprises a linker that links the C-terminus of the heavy chain variable region of the anti-PD-L1 antibody or antigen-binding fragment thereof to the N-terminus of the TGFβRII or functionally active fragment thereof.
In certain embodiment, wherein the polypeptide further comprising CH2, CH3 domains, said polypeptide comprising the heavy chain variable region (VH) of anti-PD-L1 antibody, CH2, CH3 domains and TGF βRII or a functionally active fragment thereof in sequence from N-terminus to C-terminus, said CH3 domain having its C-terminus connected to the N-terminus of said TGF βRII or a functionally active fragment thereof by a linker.
In certain embodiment, wherein the CH2, CH3 domains are derived from IgG.
In certain embodiment, wherein the linker is a peptide linker.
In certain embodiment, wherein the amino acid sequence of the peptide linker is $(G_4S)_x$, wherein x is any integer from 3 to 6.
In certain embodiment, wherein the peptide linker comprises an amino acid sequence selected from the group consisting of: (a) the amino acid sequence set forth in SEQ ID NO: 5; (b) an amino acid sequence having at least about 85%, 90%, 95%, or 99% sequence identity to the amino acid sequence set forth in SEQ ID NO: 5; And (c) an amino acid sequence having 1 or more differences from the amino acid sequence shown in SEQ ID NO: 5 obtained by addition, elimination or substitution reaction.
In certain embodiment, wherein the polypeptide comprises an amino acid sequence selected from the group consisting of: (a) the amino acid sequence set forth in SEQ ID NO: 7; (b) an amino acid sequence having at least about 85%, 90%, 95%, or 99% sequence identity to the amino acid sequence set forth in SEQ ID NO: 7; (c) an amino acid sequence having 1 or more differences from the amino acid sequence shown in SEQ ID NO: 7 obtained by addition,

elimination or substitution reactions.

In certain embodiment, wherein the PD-L1//TGFβ dual inhibitor comprises two polypeptides aforementioned.

In certain embodiment, wherein the PD-L1/TGFβ dual inhibitor comprises a first polypeptide and a second polypeptide, wherein the first polypeptide comprises at least: (i) a heavy chain of an anti-PD-L1 antibody variable region; and (ii) TGFβRII or a functionally active fragment thereof; wherein the second polypeptide comprises at least the light chain variable region of an anti-PD-L1 antibody; wherein the heavy chain variable region of the first polypeptide and the light chain variable regions of the second polypeptide are capable of specifically binding to PD-L1.

In certain embodiment, wherein the first polypeptide further comprises a linker, said linker connects the C-terminus of the heavy chain variable region of the anti-PD-L1 antibody or antigen-binding fragment thereof with the TGFβRII or the N-terminus of its functionally active fragments.

In certain embodiment, wherein the PD-L1/TGFβ dual inhibitor comprises a first polypeptide and a second polypeptide, wherein, the first polypeptide comprises the heavy chain variable region (VH) of the anti-PD-L1 antibody, the CH1 domain, and TGFβRII or its functionally active fragment sequentially from the N-terminus to the C-terminus, and the C-terminus of the CH1 domain is connected to the TGFβRII or its functionally active fragment N-terminus is connected by a linker; and the second polypeptide comprises a light chain variable region (VL) and a light chain constant region (CL) of an anti-PD-L1 antibody from the N-terminus to the C-terminus ).

In certain embodiment, wherein the PD-L1/TGFβ dual inhibitor comprises a first polypeptide and a second polypeptide, wherein, the first polypeptide comprises the heavy chain variable region (VH) of the anti-PD-L1 antibody, the CH1 , CH2, CH3 domain, and TGFβRII or its functionally active fragment sequentially from the N-terminus to the C-terminus, and the C-terminus of the CH3 domain is connected to the TGFβRII or its functionally active fragment N-terminus is connected by a linker; and the second polypeptide comprises a light chain variable region (VL) and a light chain constant region (CL) of an anti-PD-L1 antibody from the N-terminus to the C-terminus ).

In certain embodiment, wherein said linker is a peptide linker.

In certain embodiment, wherein the first polypeptide has at least about 80% sequence identity to a polypeptide having the same function as: M7824 or SHR-1701.

In certain embodiment, wherein the second polypeptide has at least about 80% sequence identity to a polypeptide having the same function as: M7824 or SHR-1701.

In certain embodiment, wherein the PD-L1/TGFβ dual inhibitor comprises M7824, SHR-1702, or variant or biosimilar or their combination.

In certain embodiment, a PI3K inhibitor can be a compound that inhibits one or more PI3K isoforms, e.g., α, β, δ, or γ isoforms. In one embodiment, the PI3K inhibitor may be a compound that inhibits theα, β, δ, or γ isoforms of PI3K. In another embodiment, the PI3K inhibitor may be a compound that inhibits the β, δ, or γ isoforms of PI3K. In another embodiment, the PI3K inhibitor may be a compound that inhibits the δ and γ isoforms of PI3K, ie, a PI3K δ/γ dual inhibitor.

In certain embodiment, wherein the said PI3K inhibitors comprise compounds as formula(IA-I), (IA-II), (IA-III), (IA-IV):

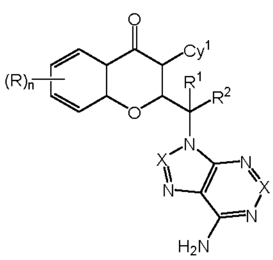
(IA-I)

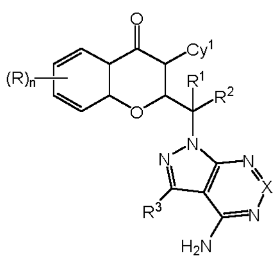
(IA-II)

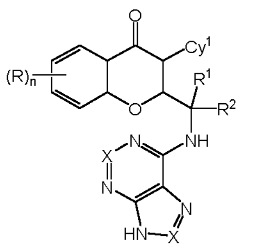
(IA-III)

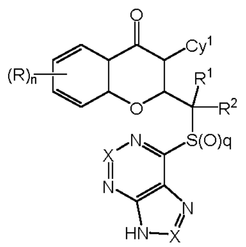
(IA-IV)

or their enantiomers, mixtures of enantiomers, mixtures of two or more diastereoisomers, isotopic variants, pharmaceutically acceptable salts, solvates, hydrates or prodrug, wherein,

each occurrence of R is independently selected from hydrogen, halogen, -$OR^a$, CN, substituted or unsubstituted $C_{1-6}$ alkyl, substituted or unsubstituted $C_{2-6}$ alkenyl, substituted or unsubstituted $C_{2-6}$ alkynyl, substituted or unsubstituted $C_{3-8}$ cycloalkyl, and substituted or unsubstituted heterocyclic group;

$R^1$ and $R^2$ may be the same or different and are independently selected from hydrogen, halogen, and substituted or unsubstituted $C_{1-6}$ alkyl, or both $R^1$ and$R^2$ directly bound to a common atom, may be joined to form an oxo group (=O) or a substituted or unsubstituted saturated or unsaturated 3-10 member ring, including the carbon atom to which $R^1$ and $R^2$ are bound, which may optionally include one or more heteroatoms which may be the same or different and are selected from O, $NR^a$ and S;

$Cy^1$ is selected from substituted or unsubstituted $C_{3-8}$ cycloalkyl, substituted or unsubstituted 3 to 15 membered heterocyclic groups having at least one heteroatom selected from N, O and S, substituted or unsubstituted $C_{6-20}$ aryl and substituted or unsubstituted 5 to 14 membered heteroaryl monocyclic groups with one or more heteroatoms selected from N, O and S;

each occurrence of $R^a$ may be the same or different and are independently selected from hydrogen, halogen, hydroxy, cyano, substituted or unsubstituted $C_{1-6}$alkyl, - $NR^cR^d$ , wherein $R^c$ and $R^d$ are independently hydrogen, halogen, hydroxy, cyano, substituted or unsubstituted $C_{1-6}$alkyl, and $C_1$-$_6$alkoxy, and -$OR^c$ , wherein $R^c$ is substituted or unsubstituted $C_{1-6}$alkyl ,

n is an integer from 1 to 4;

q is 0, 1 or 2;

each occurrence of X is independently selected from $CR^3$ or N;

and each occurrence of $R^3$ is independently selected from hydrogen, hydroxy, halogen, carboxyl, cyano, nitro, substituted or unsubstituted $C_{1-8}$ alkyl, substituted or unsubstituted $C_{1-8}$ alkoxy, substituted or unsubstituted $C_{2-10}$ alkenyl, substituted or unsubstituted $C_{2-10}$ alkynyl, substituted or unsubstituted $C_{6-20}$ aryl, substituted or unsubstituted $C_{6-20}$ aryl $C_{1-8}$alkyl, substituted or unsubstituted $C_{3-20}$ cycloalkyl, substituted or unsubstituted $C_{3-20}$ cycloalkyl $C_{1-8}$ alkyl, substituted or unsubstituted $C_{3-8}$ cycloalkenyl $C_{1-8}$ alkyl, substituted or unsubstituted $C_{3-8}$ cycloalkene, substituted or unsubstituted 5-14-membered heteroaryl with one or more heteroatoms selected from N, O and S, substituted or unsubstituted with one or more heteroatoms selected from N, O, S 5-14 membered heteroaryl $C_{1-8}$ alkyl, substituted or unsubstituted 3 to 15 membered heterocycles having at least one heteroatom selected from N, O and S, substituted or unsubstituted 3 to 15 membered heterocyclyl $C_{1-8}$ alkyl rings having at least one heteroatom selected from N, O and S, substituted or unsubstituted guanidine, - $COOR^x$, -$C(O)R^x$, -$C(S)R^x$, -$C(O)NR^xR^y$, -$C(O)ONR^xR^y$, -$NR^yR^z$, -$NR^xCONR^yR^z$, - $N(R^x)SOR^y$, - $N(R^x)SO_2R^y$, -(=N-$N(R^x)R^y$), - $NR^xC(O)OR^y$, -$NR^xR^y$, -$NR^xC(O)R^y$-, - $NR^xC(S)R^y$ -$NR^xC(S)NR^yR^z$, -$SONR^xR^y$-, -$SO_2NR^xR^y$-, -$OR^x$, -$OR^xC(O)NR^yR^z$, - $OR^xC(O)OR^y$-, -$OC(O)R^x$, -$OC(O)NR^xR^y$, - $R^xNR^yC(O)R^z$, -$R^xOR^y$, -$R^xC(O)OR^y$, - $R^xC(O)NR^yR^z$, -$R^xC(O)R^x$, -$R^xOC(O)R^y$, -$SR^x$, -$SOR^x$, - $SO_2R^x$, -$ONO_2$,

wherein $R^x$, $R^y$ and $R^z$ in each of the above groups can be hydrogen, substituted or unsubstituted $C_{1-8}$ alkyl, substituted or unsubstituted $C_{1-8}$ alkoxy, substituted or unsubstituted $C_{2-10}$ alkenyl, substituted or unsubstituted $C_{2-12}$ alkynyl, substituted or unsubstituted $C_{6-20}$ aryl, substituted or unsubstituted $C_{6-20}$ aryl $C_{1-8}$ alkyl , substituted or unsubstituted $C_{3-20}$ cycloalkyl, substituted or unsubstituted $C_{3-20}$ cycloalkyl$C_{1-8}$ alkyl, substituted or unsubstituted $C_{3-8}$ cycloalkenyl, substituted or unsubstituted 5 to 14 membered heteroaryl with one or more heteroatoms selected from N, O and S, substituted or unsubstituted heteroaryl with one or more heteroatoms selected from N, O and S atomic 5 to 14 membered heteroaryl $C_{1-8}$ alkyl, substituted or unsubstituted 3 to 15 membered heterocycle having at least one heteroatom selected from N, O and S, substituted or unsubstituted 3 to 15 membered heterocyclyl $C_{1-8}$ alkyl ring having at least one heteroatom selected from N, O and S or substituted or unsubstituted amino, or any two of $R^x$, $R^y$ and $R^z$ may be joined to form a substituted or unsubstituted saturated or unsaturated 3-10 membered ring, which may optionally include heteroatoms which may be the same or different and are selected from O, $NR^x$ or S, wherein $R^x$ is hydrogen or substituted or unsubstituted alkyl.

In certain embodiment, the term "substituted" generally refers to substitution with any one or any combination of the following substituents, which may be the same or different and independently selected from the following substituents: hydrogen, hydroxyl, halogen, carboxyl, cyano, nitro, =O group, =S group, $C_1$-$C_8$ alkyl, $C_1$-$C_8$ alkoxy, $C_2$-$C_{10}$ alkenyl, $C_2$-$C_{12}$ alkynyl, $C_6$-$C_{20}$ aryl, $C_6$-$C_{20}$ aryl ($C_1$-$C_8$ alkyl ), $C_3$-$C_{12}$ cycloalkyl, $C_3$-$C_{12}$ cycloalkyl ($C_1$-$C_8$ alkyl), $C_3$-$C_8$ cycloalkenyl, $C_3$-$C_8$ cycloalkenyl ($C_1$-$C_8$ alkyl); 5-14 membered heteroaryl A group, wherein the heteroatom is selected from N, O and S; 5-14 membered heteroaryl ($C_1$-$C_8$ alkyl), wherein the heteroatom is selected from N, O and S; 3-15 membered heterocyclic ring, Wherein the heteroatom is selected from N, O and S; and 3-15 membered heterocyclic group (Ci-Cs alkyl), wherein the heteroatom is selected from N, O and S.

In certain embodiment, guanidine, -$COOR^x$, -$C(O)R^x$, -$C(S)R^x$, -$C(O)NR^xR^y$, -$C(O)ONR^xR^y$, - $NR^yR^z$, -$NR^x$-$CONR^yR^z$, -$N(R^x)SOR^y$, -$N(R^x)SO_2R^y$, -(=N-$N(R^x)R^y$), -$NR^xC(O)OR^y$, -$NR^xR^y$, - $NR^xC(O)R^y$-, -$NR^xC(S)R^y$, -$NR^xC(S)NR^yR^z$, -$SONR^xR^y$-, -$SO_2NR^xR^y$-, -$OR^x$, -$OR^xC(O)NR^yR^z$, - $OR^xC(O)OR^y$-, -$OC(O)R^x$, -$OC(O)NR^xR^y$, -$R^xNR^yC(O)R^z$, - $R^xOR^y$, -$R^xC(O)OR^y$, -$R^xC(O)NR^yR^z$, - $R^xC(O)R^x$, -$R^xOC(O)R^x$, -$SR^x$, -$SOR^x$, -$SO_2R^x$ and -$ONO_2$, wherein each of the above groups $R^x$, $R^y$ and $R^z$ can be hydrogen, $C_1$-$C_8$ alkyl, $C_1$-$C_8$ alkoxy, $C_2$-$C_{10}$ alkenyl, $C_2$-$C_{12}$ alkynyl, $C_6$-$C_{20}$ aryl, $C_6$-$C_{20}$aryl ($C_1$-$C_8$ alkyl), $C_3$-$C_{12}$ cycloalkyl, $C_3$-$C_{12}$ cycloalkyl ($C_1$-$C_8$alkyl), $C_3$-$C_8$ cycloalkenyl, amino; 5-14 membered heteroaryl, wherein the heteroatom is selected from N, O and S; 5-14 membered heteroaryl ($C_1$-$C_8$ alkyl), wherein said heteroatoms are selected from N, O and S; 3-15 membered heterocyclic rings, wherein said heteroatoms are selected from N, O and S; 3-15 membered heterocyclic group ($C_1$-$C_8$ alkyl), or any two of $R^x$, $^{Ry}$ and $R^z$ can be connected to form a saturated or unsaturated 3-10 membered ring, which can optionally include the same or different heteroatoms selected from O, $NR^x$ or S, wherein $R^x$ is hydrogen or $C_{1-6}$ alkyl.

In certain embodiment, wherein the PI3K inhibitor comprises compounds as formula (IA-V):

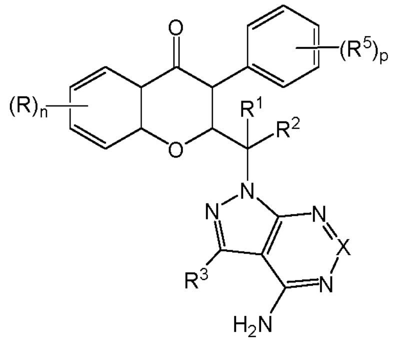

(IA-V)

or their enantiomers, mixtures of enantiomers, mixtures of two or more diastereoisomers, isotopic variants, pharmaceutically acceptable salts, solvates, hydrates or prodrug, wherein

each occurrence of R is independently selected from hydrogen, halogen, -$OR^a$, CN, substituted or unsubstituted $C_{1-6}$ alkyl, substituted or unsubstituted $C_{2-6}$ alkenyl, substituted or unsubstituted $C_{2-6}$ alkynyl, substituted or unsubstituted $C_{3-8}$ cycloalkyl, and substituted or unsubstituted heterocyclic group;

$R^1$ and $R^2$ may be the same or different and are independently selected from hydrogen, halogen, and substituted or unsubstituted $C_{1-6}$ alkyl, or both $R^1$ and$R^2$ directly bound to a common atom, may be joined to form an oxo group (=O) or a substituted or unsubstituted saturated or unsaturated 3-10 member ring, including the carbon atom to which $R^1$ and $R^2$ are bound, which may optionally include one or more heteroatoms which may be the same or different and are selected from O, $NR^a$ and S;

each occurrence of X is independently selected from $CR^3$ or N; and

each occurrence of $R^3$ is independently selected from hydrogen, hydroxy, halogen, carboxyl, cyano, nitro, substituted or unsubstituted $C_{1-8}$ alkyl, substituted or unsubstituted $C_{1-8}$ alkoxy, substituted or unsubstituted $C_{2-10}$ alkenyl, substituted or unsubstituted $C_{2-10}$ alkynyl, substituted or unsubstituted $C_{6-20}$ aryl, substituted or unsubstituted $C_{6-20}$ aryl $C_{1-8}$alkyl, substituted or unsubstituted $C_{3-20}$ cycloalkyl, substituted or unsubstituted $C_{3-20}$ cycloalkyl $C_{1-8}$ alkyl, substituted or unsubstituted $C_{3-8}$ cycloalkenyl $C_{1-8}$ alkyl, substituted or unsubstituted $C_{3-8}$ cycloalkene, substituted or unsubstituted 5-14-membered heteroaryl with one or more heteroatoms selected from N, O and S, substituted or unsubstituted with one or more heteroatoms selected from N, O, S 5-14 membered heteroaryl $C_{1-8}$ alkyl, substituted or unsubstituted 3 to 15 membered heterocycles having at least one heteroatom selected from N, O and S, substituted or unsubstituted 3 to 15 membered heterocyclyl $C_{1-8}$ alkyl rings having at least one heteroatom selected from N, O and S, substituted or unsubstituted guanidine, - $COOR^x$, -$C(O)R^x$, -$C(S)R^x$, -$C(O)NR^xR^y$, -$C(O)ONR^xR^y$, -$NR^yR^z$, -$NR^xCONR^yR^z$, - $N(R^x)SOR^y$, - $N(R^x)SO_2R^y$, -(=N-$N(R^x)R^y$), - $NR^xC(O)OR^y$, -$NR^xR^y$, -$NR^xC(O)R^y$-, - $NR^xC(S)R^y$ -$NR^xC(S)NR^yR^z$, -$SONR^xR^y$-, -$S0_2NR^xR^y$-, -$OR^x$, -$OR^xC(O)NR^yR^z$, - $OR^xC(O)OR^y$-, -$OC(O)R^x$, -$OC(O)NR^xR^y$, - $R^xNR^yC(O)R^z$, -$R^xOR^y$, -$R^xC(O)OR^y$, - $R^xC(O)NR^yR^z$, -$R^xC(0)R^x$, -$R^xOC(O)R^y$, -$SR^x$, -$SOR^x$, - $SO_2R^x$, -$ONO_2$,

wherein $R^x$, $R^y$ and $R^z$ in each of the above groups can be hydrogen, substituted or unsubstituted $C_{1-8}$ alkyl, substituted or unsubstituted $C_{1-8}$ alkoxy, substituted or unsubstituted $C_{2-10}$ alkenyl, substituted or unsubstituted $C_{2-12}$ alkynyl, substituted or unsubstituted $C_{6-20}$ aryl, substituted or unsubstituted $C_{6-20}$ aryl $C_{1-8}$ alkyl , substituted or unsubstituted $C_{3-20}$ cycloalkyl, substituted or unsubstituted $C_{3-20}$ cycloalkyl$C_{1-8}$ alkyl, substituted or unsubstituted $C_{3-8}$ cycloalkenyl, substituted or unsubstituted 5 to 14 membered heteroaryl with one or more heteroatoms selected from N, O and S, substituted or unsubstituted heteroaryl with one or more heteroatoms selected from N, O and S atomic 5 to 14 membered heteroaryl $C_{1-8}$ alkyl, substituted or unsubstituted 3 to 15 membered heterocycle having at least one heteroatom selected from N, O and S, substituted or unsubstituted 3 to 15 membered heterocyclyl $C_{1-8}$ alkyl ring having at least one heteroatom selected from N, O and S or substituted or unsubstituted amino, or any two of $R^x$, $R^y$ and $R^z$ may be joined to form a substituted or unsubstituted saturated or unsaturated 3-10 membered ring, which may optionally include heteroatoms which may be the same or different and are selected from O, $NR^x$ or S, wherein $R^x$ is hydrogen or substituted or unsubstituted alkyl,

each occurrence of $R^5$ is hydrogen, $C_{1-6}$ alkyl or halogen,

n is 0, 1, 2, 3 or 4; and

Pis 0, 1, 2, 3, 4 or 5.

In certain embodiment, wherein the PI3K inhibitor comprises compounds as formula (IA-VI):

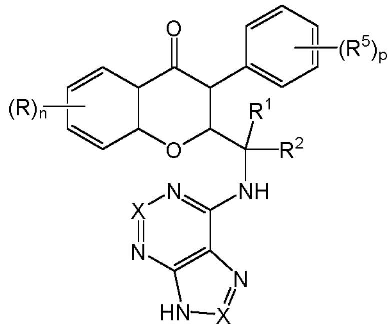

(IA-VI)

or their enantiomers, mixtures of enantiomers, mixtures of two or more diastereoisomers, isotopic variants, pharmaceutically acceptable salts, solvates, hydrates or prodrug, wherein

each occurrence of R is independently selected from hydrogen, halogen, -OR$^a$, CN, substituted or unsubstituted $C_{1-6}$ alkyl, substituted or unsubstituted $C_{2-6}$ alkenyl, substituted or unsubstituted $C_{2-6}$ alkynyl, substituted or unsubstituted $C_{3-8}$ cycloalkyl, and substituted or unsubstituted heterocyclic group;

$R^1$ and $R^2$ may be the same or different and are independently selected from hydrogen, halogen, and substituted or unsubstituted $C_{1-6}$ alkyl, or both $R^1$ and$R^2$ directly bound to a common atom, may be joined to form an oxo group (=O) or a substituted or unsubstituted saturated or unsaturated 3-10 member ring, including the carbon atom to which $R^1$ and $R^2$ are bound, which may optionally include one or more heteroatoms which may be the same or different and are selected from O, NR$^a$ and S;

each occurrence of X is independently selected from CR$^3$ or N; and

each occurrence of $R^3$ is independently selected from hydrogen, hydroxy, halogen, carboxyl, cyano, nitro, substituted or unsubstituted $C_{1-8}$ alkyl, substituted or unsubstituted $C_{1-8}$ alkoxy, substituted or unsubstituted $C_{2-10}$ alkenyl, substituted or unsubstituted $C_{2-10}$ alkynyl, substituted or unsubstituted $C_{6-20}$ aryl, substituted or unsubstituted $C_{6-20}$ aryl $C_{1-8}$alkyl, substituted or unsubstituted $C_{3-20}$ cycloalkyl, substituted or unsubstituted $C_{3-20}$ cycloalkyl $C_{1-8}$ alkyl, substituted or unsubstituted $C_{3-8}$ cycloalkenyl $C_{1-8}$ alkyl, substituted or unsubstituted $C_{3-8}$ cycloalkene, substituted or unsubstituted 5-14-membered heteroaryl with one or more heteroatoms selected from N, O and S, substituted or unsubstituted with one or more heteroatoms selected from N, O, S 5-14 membered heteroaryl $C_{1-8}$ alkyl, substituted or unsubstituted 3 to 15 membered heterocycles having at least one heteroatom selected from N, O and S, substituted or unsubstituted 3 to 15 membered heterocyclyl $C_{1-8}$ alkyl rings having at least one heteroatom selected from N, O and S, substituted or unsubstituted guanidine, - COOR$^x$, -C(O)R$^x$, -C(S)R$^x$, -C(O)NR$^x$R$^y$, -C(O)ONR$^x$R$^y$, -NR$^y$R$^z$, -NR$^x$CONR$^y$R$^z$, - N(R$^x$)SOR$^y$, - N(R$^x$)$SO_2$R$^y$, -(=N-N(R$^x$)R$^y$), - NR$^x$C(O)OR$^y$, -NR$^x$R$^y$, -NR$^x$C(O)R$^y$-, - NR$^x$C(S)R$^y$ -NR$^x$C(S)NR$^y$R$^z$, -SONR$^x$R$^y$-, -S0$_2$NR$^x$R$^y$-, -OR$^x$, -OR$^x$C(O)NR$^y$R$^z$, - OR$^x$C(O)OR$^y$-, -OC(O)R$^x$, -OC(O)NR$^x$R$^y$, - R$^x$NR$^y$C(O)R$^z$, -R$^x$OR$^y$, -R$^x$C(O)OR$^y$, - R$^x$C(O)NR$^y$R$^z$, -R$^x$C(0)R$^x$, -R$^x$OC(O)R$^y$, -SR$^x$, -SOR$^x$, - $SO_2$R$^x$, -ONO$_2$,

wherein R$^x$, R$^y$ and R$^z$ in each of the above groups can be hydrogen, substituted or unsubstituted $C_{1-8}$ alkyl, substituted or unsubstituted $C_{1-8}$ alkoxy, substituted or unsubstituted $C_{2-10}$ alkenyl, substituted or unsubstituted $C_{2-12}$ alkynyl, substituted or unsubstituted $C_{6-20}$ aryl, substituted or unsubstituted $C_{6-20}$ aryl $C_{1-8}$ alkyl , substituted or unsubstituted $C_{3-20}$ cycloalkyl, substituted or unsubstituted $C_{3-20}$ cycloalkyl$C_{1-8}$ alkyl, substituted or unsubstituted $C_{3-8}$ cycloalkenyl, substituted or unsubstituted 5 to 14 membered heteroaryl with one or more heteroatoms selected from N, O and S, substituted or unsubstituted heteroaryl with one or more heteroatoms selected from N, O and S atomic 5 to 14 membered heteroaryl $C_{1-8}$ alkyl, substituted or unsubstituted 3 to 15 membered heterocycle having at least one heteroatom selected from N, O and S, substituted or unsubstituted 3 to 15 membered heterocyclyl $C_{1-8}$ alkyl ring having at least one heteroatom selected from N, O and S or substituted or unsubstituted amino, or any two of R$^x$, R$^y$ and R$^z$ may be joined to form a substituted or unsubstituted saturated or unsaturated 3-10 membered ring, which may optionally include heteroatoms which may be the same or different and are selected from O, NR$^x$ or S, wherein R$^x$ is hydrogen or substituted or unsubstituted alkyl,

each occurrence of $R^5$ is hydrogen, $C_{1-6}$ alkyl or halogen,

n is 0, 1, 2, 3 or 4; and

Pis 0, 1, 2, 3, 4 or 5.

For example, wherein R is hydrogen, halogen, substituted or unsubstituted $C_{1-6}$ alkyl or OR$^a$.

In certain embodiment, wherein R$^a$ is alkyl.

For example, wherein Cy$^1$ is selected from:

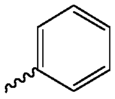 、 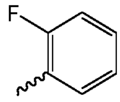 、 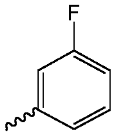 、 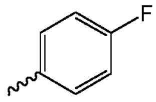 、 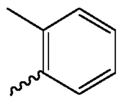 or 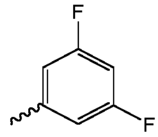 .

For example, wherein $R^1$ and $R^2$ each independently represent hydrogen or substituted or unsubstituted $C_{1-6}$ alkyl.

For example, wherein $R^3$ is iodo, cyano, substituted unsubstituted alkyl, substituted or unsubstituted alkynyl, substituted or unsubstituted aryl, substituted or unsubstituted heteroaryl.

For example, wherein X is $CR^3$ and each occurrence of $R^3$ is independently hydrogen, halogen, hydroxyl or $NH_2$, for example,wherein $R^3$ is hydrogen.

For example, wherein said PI3K inhibitors comprise:

2-[(6-Amino-9H-purin-9-yl) methyl]-6-bromo-3-phenyl-4H-chromen-4-one;
6-Bromo-2-(morpholinomethyl)-3-phenyl-4H-chromen-4-one;
6-Bromo-2-(morpholinomethyl)-3-phenyl-4H-chromen-4-one hydrochloride;
2-[(6-Amino-9H-purin-9-yl) methyl]-3-phenyl-4H-chromen-4-one;
2-(Morpholinomethyl)-3-phenyl-4H-chromen-4-one;
2-(Morpholinomethyl)-3-phenyl-4H-chromen-4-one hydrochloride;
2-[(1H-Benzo[d]imidazol-l-yl) methyl]-6-bromo-3-phenyl-4H-chromen-4-one;
6-Bromo-2-[(4-methyl-lH-benzo[d]imidazol-l-yl) methyl]-3-phenyl-4H-chromen-4-one;
2- [( 1 H-benzo [d] imidazol- 1 -yl) methyl] -3-phenyl-4H-chromen-4-one;
2-[(4-methyl- 1 H-benzo [d] imidazol- 1 -yl)methyl] -3-phenyl-4H-chromen-4-one;
2-[(6-Chloro-9H-purin-9-yl)methyl]-3-phenyl-4H-chromen-4-one;
6-Bromo-2-[(6-chloro-9H-purin-9-yl)methyl]-3-phenyl-4H-chromen-4-one;
2-((9H-Purin-6-ylthio)methyl)-3-phenyl-4H-chromen-4-one;
2- [( 1 H-Imidazol- 1 -yl)methyl] -3-phenyl-4H-chromen-4-one ;
2-[(9H-Purin-6-ylthio)methyl]-6-bromo-3-phenyl-4H-chromen-4-one;
2-((4-Amino-lH-pyrazolo[3,4-d]pyrimidin-l-yl)methyl)-6-bromo-3-phenyl-4H-chromen-4-one ;
2-[(6-Amino-9H-purin-9-yl)methyl]-6-bromo-3-(4-fluorophenyl)-4H-chromen-4-one ;
2- [(6-Arnino-9H-purin-9-yl)methyl]-3-(4-fluorophenyl)-4H-chromen-4-one ; 6-Bromo-3-(4-fluorophenyl)-2-(morpholinomethyl)-4H-chromen-4-one ; 6-Bromo-3-(4-fluorophenyl)-2-(morpholinomethyl)-4H-chromen-4-one hydro chloride;
3- (4-fluorophenyl)-2-(morpholinomethyl)-4H-chromen-4-one ; 3-(4-fluorophenyl)-2-(morpholinomethyl)-4H-chromen-4-one hydrochloride ;
2-[(6-Amino-9H-purin-9-yl)methyl]-6-bromo-3-o-tolyl-4H-chromen-4-one;
7-[(6-Bromo-4-oxo-3-phenyl-4H-chromen-2-yl)methyl]-l,3-dimethyl-lH-purine- 2,6(3H,7H)-dione;
2-(l-(6-Amino-9H-purin-9-yl)ethyl)-6-bromo-3-phenyl-4H-chromen-4-one;
2-(l-(9H-Purin-6-ylthio)ethyl)-6-bromo-3-phenyl-4H-chromen-4-one;
2-( 1 -(6-Amino-9H-purin-9-yl)ethyl)-3-phenyl-4H-chromen-4-one;
(S)-2-( 1 -(9H-purin-6-ylamino)ethyl)-6-bromo-3-phenyl-4H-chromen-4-one ;
2-((9H-purin-6-ylamino)methyl)-6-bromo-3-phenyl-4H-chromen-4-one ;
2-( 1 -(4-amino- 1 H-pyrazolo [3 ,4-d]pyrimidin- 1 -yl)ethyl)-6-bromo-3-phenyl-4H-chromen-4- one ;
2-((6-Amino-9H-purin-9-yl)methyl)-6-methoxy-3-phenyl-4H-chromen-4-one;
2-(l-(6-Amino-9H-purin-9-yl)ethyl)-6-bromo-3-(2-fluorophenyl)-4H-chromen-4-one;
2-((6-Amino-9H-purin-9-yl)methyl)-6-bromo-3-(2-fluorophenyl)-4H-chromen-4-one ;
2-( 1 -(4-Amino- 1 H-pyrazolo [3,4-d]pyrimidin- 1 -yl)ethyl)-3 -phenyl-4H-chromen-4-one ;
2-( 1 -(6-Amino-9H-purin-9-yl) propyl)-3-phenyl-4H-chromen-4-one; 2-(l-(6-Amino-9H-purin-9-yl)ethyl)-3 -(3 -fluorophenyl)-4H-chromen-4-one;
2-((6-Amino-9H-purin-9-yl)methyl)-3-(2-fluorophenyl)-4H-chromen-4-one ;
2-( 1 -(6-Amino-9H-purin-9-yl)ethyl)-3-(2-fluorophenyl)-4H-chromen-4-one ;
2-(l-(6-Amino-9H-purin-9-yl)propyl)-3-(2-fluorophenyl)-4H-chromen-4-one;
2-(l-(6-amino-9H-purin-9-yl)propyl)-3-(3-fluorophenyl)-4H-chromen-4-one ;
2-(l-(6-Amino-9H-purin-9-yl)propyl)-3-(4-fluorophenyl)-4H-chromen-4-one ;
2-(l-(6-amino-9H-purin-9-yl)propyl)-6-fluoro-3-phenyl-4H-chromen-4-one;
2-(l-(6-Amino-9H-purin-9-yl)ethyl)-3-(4-fluorophenyl)-4H-chromen-4-one;
2-(l-(6-Amino-9H-purin-9-yl)ethyl)-6-fluoro-3-phenyl-4H-chromen-4-one;

2-(l-(4-Amino-3-(3-methoxyphenyl)-lH-pyrazolo[3,4-d]pyrimidin-l-yl)ethyl)-3-phenyl-4H-chromen-4-one;
2-( 1 -(4-Amino-3-(3-hydroxyphenyl)- 1 H-pyrazolo [3 ,4-d]pyrimidin- 1 -yl)ethyl)-3 -phenyl-4H- chromen-4-one;
2-((9H-purin-6-ylamino)methyl)-3-phenyl-4H-chromen-4-one;
2-( 1 -(6-Amino-9H-purin-9-yl)ethyl)-3-o-tolyl-4H-chromen-4-one;
2-((9H-purin-6-ylamino)methyl)-3-(2-fluorophenyl)-4H-chromen-4-one;
2-((9H-purin-6-ylamino)methyl)-3-(3-fluorophenyl)-4H-chromen-4-one;
(S)-2-(l-(9H-purin-6-ylamino)ethyl)-3-(3-fluorophenyl)-4H-chromen-4-one;
2-(l-(6-amino-9H-purin-9-yl)ethyl)-6-fluoro-3-(2-fluorophenyl)-4H-chromen-4-one;
2-(l-(6-Amino-9H-purin-9-yl)ethyl)-3-(3,5-difluorophenyl)-4H-chromen-4-one;
2-(l-(6-amino-9H-purin-9-yl)ethyl)-6-fluoro-3-(3-fluorophenyl)-4H-chromen-4-one;
2-(l-(4-amino-3-(3-methoxyphenyl)-lH-pyrazolo[3,4-d]pyrimidin-l-yl)ethyl)-3-(3-fluorophenyl)-4H-chromen-4-one;
2-( 1 -(4-amino-3-(3-hydroxyphenyl)- 1 H-pyrazolo [3, 4-d] pyrimidin-l-yl) ethyl)-3-(3-fluorophenyl)-4H-chromen-4-one;
2-((4-Amino-3-(3-methoxyphenyl)-lH-pyrazolo [3, 4-d] pyrimidin-l-yl) methyl)-3-phenyl- 4H-chromen-4-one;
2-((4-Amino-3-(3-hydroxyphenyl)-lH-pyrazolo [3, 4-d] pyrimidin-l-yl) methyl)-3-phenyl- 4H-chromen-4-one;
2-((4-amino-3-(3-methoxyphenyl)-lH-pyrazolo [3, 4-d] pyrimidin-l-yl) methyl)-3-(3-fluorophenyl)-4H-chromen-4-one;
2-((4-amino-3-(3-hydroxyphenyl)-lH-pyrazolo [3, 4-d] pyrimidin-l-yl) methyl)-3-(3-fluorophenyl)-4H-chromen-4-one;
(R)-2-( 1 -(9H-purin-6-ylamino) ethyl)-3-(3-fluorophenyl)-4H-chromen-4-one;
(S)-2-(l-(9H-purin-6-ylamino) ethyl)-6-fluoro-3-phenyl-4H-chromen-4-one;
2-((4-Amino-3-iodo-lH-pyrazolo [3, 4-d] pyrimidin-l-yl) methyl)-3-phenyl-4H-chromen-4-one;
2-(l-(4-amino-3-iodo-lH-pyrazolo [3, 4-d] pyrimidin-l-yl) ethyl)-3-phenyl-4H-chromen-4- one;
2-(l-(4-amino-3-iodo-lH-pyrazolo [3, 4-d] pyrimidin-l-yl) ethyl)-3-(3-fluorophenyl)-4H-chromen-4-one;
2-((4-amino-3-iodo-lH-pyrazolo [3, 4-d] pyrimidin-l-yl) methyl)-6-fluoro-3-phenyl-4H-chromen-4-one;
2-(l-(4-amino-3-iodo-lH-pyrazolo [3, 4-d] pyrimidin-l-yl) ethyl)-6-fluoro-3-phenyl-4H-chromen-4-one;
2-(l-(4-amino-3-iodo-lH-pyrazolo [3, 4-d] pyrimidin-l-yl) ethyl)-6-fluoro-3-(3- fluorophenyl)-4H-chromen-4-one;
2-(l-(4-ammo-3-iodo-lH-pyrazolo [3, 4-d] pyrimidin-l-yl) propyl)-3-(3-fluorophenyl)-4H-chromen-4-one;
2-((4-amino-3-(pyridin-3-yl)-lH-pyrazolo [3, 4-d] pyrimidin-l-yl) methyl)-3-phenyl-4H-chromen-4-one;
2-((4-amino-3-(3-hydroxyprop-l-ynyl)-lH-pyrazolo [3, 4-d] pyrimidin-l-yl) methyl)-3- phenyl-4H-chromen-4-one;
2-((4-amino-3-(lH-pyrazol-4-yl)-lH-pyrazolo [3, 4-d] pyrimidin-l-yl) methyl)-3-phenyl-4H-chromen-4-one;
2-((4-amino-3-(3-(hydroxymethyl) phenyl)- lH-pyrazolo [3, 4-d] pyrimidin-l-yl) methyl)-3-phenyl-4H-chromen-4-one;
2-((4-amino-3-(lH-indazol-4-yl)-lH-pyrazolo [3, 4-d] pyrimidin-l-yl) methyl)-3-phenyl-4H-chromen-4-one;
2-((4-amino-3-(3-fluorophenyl)-lH-pyrazolo [3, 4-d] pyrimidin-l-yl) methyl)-3-phenyl-4H-chromen-4-one
2-((4-amino-3-(3-hydroxypropyl)-l H-pyrazolo [3, 4-d] pyrimidin-l-yl) methyl)-3-phenyl- 4H-chromen-4-one;
N-(3-(4-amino-l-((4-oxo-3-phenyl-4H-chromen-2-yl) methyl)- lH-pyrazolo [3, 4-d] pyrimidin-3-yl) phenyl) acetamide;
2-((4-amino-3-(3-fluoro-5-methoxyphenyl)-l H-pyrazolo [3, 4-d] pyrimidin-l-yl) methyl)-3-phenyl-4H-chromen-4-one;
2-((4-amino-3-(3-fluoro-5-hydroxyphenyl)-lH-pyrazolo [3, 4-d] pyrimidin-l-yl) methyl)-3-phenyl-4H-chromen-4-one;
2-((4-amino-3-(3-fluoro-5-methoxyphenyl)- 1 H-pyrazolo [3, 4-d] pyrimidin-l-yl) methyl)-3-(3-fluorophenyl)-4H-chromen-4-one;
2-((4-amino-3-(3-fluoro-5-hydroxyphenyl)-lH-pyrazolo [3, 4-d] pyrimidin-l-yl) methyl)-3- (3-fluorophenyl)-4H-chromen-4-one;
2-( 1 -(4-amino-3-( 1 H-pyrazol-4-yl)- 1 H-pyrazolo [3, 4-d] pyrimidin-l-yl) ethyl)-3-phenyl-4H- chromen-4-one;
2-(l-(4-amino-3-(lH-indazol-6-yl)-lH-pyrazolo [3, 4-d] pyrimidin-l-yl) ethyl)-3-phenyl-4H-chromen-4-one;
2-(l-(4-amino-3-(3-hydroxy-3-methylbut-l-ynyl)-lH-pyrazolo [3, 4-d] pyrimidin-l-yl) ethyl)-3-phenyl-4H-chromen-4-one;
2-( 1 -(4-amino-3-( 1 H-pyrazol-4-yl)- 1 H-pyrazolo [3, 4-d] pyrimidin-l-yl) ethyl)-3-(3-fluorophenyl)-4H-chromen-4-one;
(S)-2-( 1 -(9H-purin-6-ylamino) ethyl)-3-phenyl-4H-chromen-4-one;
(S)-2-( 1 -(9H-purin-6-ylamino) ethyl)-6-fluoro-3-(3-fluorophenyl)-4H-chromen-4-one;
2-((4-amino-3-(lH-indazol-6-yl)-lH-pyrazolo [3, 4-d] pyrimidin-l-yl) methyl)-3-phenyl-4H-chromen-4-one;
2-(l-(4-amino-3-(3-fluoro-5-methoxyphenyl)-lH-pyrazolo [3, 4-d] pyrimidin-l-yl) ethyl)-3- (3-fluorophenyl)-4H-chromen-4-one;
2-(l-(4-amino-3-(3-fluoro-5-hydroxyphenyl)-lH-pyrazolo [3, 4-d] pyrimidin-l-yl) ethyl)-3- (3-fluorophenyl)-4H-chromen-4-one;
2-(l-(4-amino-3-(lH-indazol-4-yl)-lH-pyrazolo [3, 4-d] pyrimidin-l-yl) ethyl)-3-(3-fluorophenyl)-4H-chromen-4-one;
2-(l-(4-amino-3-(3, 5-dimethyl-lH-pyrazol-4-yl)-lH-pyrazolo [3, 4-d] pyrimidin-l-yl) ethyl)-3-(3-fluorophenyl)-4H-chromen-4-one;
2-(l-(4-amino-3-(3-methyl-lH-indazol-6-yl)-lH-pyrazolo [3, 4-d] pyrimidin-l-yl) ethyl)-3- (3-fluorophenyl)-4H-chromen-4-one;

2-(l-(4-amino-3-(lH-indazol-6-yl)-lH-pyrazolo [3, 4-d] pyrimidin-l-yl) ethyl)-3-(3-fluorophenyl)-4H-chromen-4-one;
2-(l-(4-amino-3-(2-(hydroxymethyl) phenyl)- lH-pyrazolo [3, 4-d] pyrimidin-l-yl) ethyl)-3- (3-fluorophenyl)-4H-chromen-4-one;
2-(l-(4-amino-3-(4-fluoro-3-methoxyphenyl)-lH-pyrazolo [3, 4-d] pyrimidin-l-yl) ethyl)-3- (3-fluorophenyl)-4H-chromen-4-one;
2-(l-(4-amino-3-(4-fluoro-3-hydroxyphenyl)-lH-pyrazolo [3, 4-d] pyrimidin-l-yl) ethyl)-3- (3-fluorophenyl)-4H-chromen-4-one;
2-(l-(4-amino-3-(3-hydroxyprop-l-ynyl)-lH-pyrazolo [3, 4-d] pyrimidin-l-yl) ethyl)-3-(3-fluorophenyl)-4H-chromen-4-one;
2-(l-(4-amino-3-(3-fluoro-4-methoxyphenyl)-lH-pyrazolo [3, 4-d] pyrimidin-l-yl) ethyl)-3- (3-fluorophenyl)-4H-chromen-4-one;
2-(l-(4-amino-3-(3-fluoro-4-hydroxyphenyl)-lH-pyrazolo [3, 4-d] pyrimidin-l-yl) ethyl)-3- (3-fluorophenyl)-4H-chromen-4-one;
2-(l-(4-amino-3-(3-chloro-5-methoxyphenyl)-1H-pyrazolo [3, 4-d] pyrimidin-l-yl) ethyl)-3- (3-fluorophenyl)-4H-chromen-4-one;
2-(1-(4-amino-3-(3-chloro-5-hydroxyphenyl)-lH-pyrazolo [3, 4-d] pyrimidin-l-yl) ethyl)-3- (3-fluorophenyl)-4H-chromen-4-one;
2- (l-(4-amino-3-(3-(trifluoromethoxy) phenyl)- lH-pyrazolo [3, 4-d] pyrimidin-l-yl) ethyl)-3- (3-fluorophenyl)-4H-chromen-4-one;
2-(l-(4-amino-3-(4-methoxyphenyl)-1H-pyrazolo [3, 4-d] pyrimidin-l-yl) ethyl)-3-(3-fluorophenyl)-4H-chromen-4-one;
2-(l-(4-amino-3-(4-hydroxyphenyl)-lH-pyrazolo [3, 4-d] pyrimidin-l-yl) ethyl)-3-(3-fluorophenyl)-4H-chromen-4-one;
2-((6-amino-9H-purin-9-yl) methyl)-3 -(3 -fluorophenyl)-4H-chromen-4-one;
2-(l-(4-amino-3-(4-fluoro-2-methoxyphenyl)-lH-pyrazolo [3, 4-d] pyrimidin-l-yl) ethyl)-3- (3-fluorophenyl)-4H-chromen-4-one;
2-(l-(4-amino-3-(4-fluoro-2-hydroxyphenyl)-lH-pyrazolo [3, 4-d] pyrimidin-l-yl) ethyl)-3- (3-fluorophenyl)-4H-chromen-4-one;
2-((4-amino-3-(3-aminophenyl)-lH-pyrazolo [3, 4-d] pyrimidin-l-yl) methyl)-3-phenyl-4H-chromen-4-one;
2-((4-amino-3-(3-methyl-lH-indazol-6-yl)-lH-pyrazolo [3, 4-d] pyrimidin-l-yl) methyl)-3-phenyl-4H-chromen-4-one;
2-(l-(4-amino-3-(2-aminopyrimidin-5-yl)-lH-pyrazolo [3, 4-d] pyrimidin-l-yl) ethyl)-3-(3-fluorophenyl)-4H-chromen-4-one;
2-(l-(4-amino-3-(lH-indol-6-yl)-lH-pyrazolo [3, 4-d] pyrimidin-l-yl) ethyl)-3-(3-fluorophenyl)-4H-chromen-4-one;
2-(l-(4-amino-3-(4-chloro-3-methoxyphenyl)-lH-pyrazolo [3, 4-d] pyrimidin-l-yl) ethyl)-3- (3-fluorophenyl)-4H-chromen-4-one;
2-(l-(4-amino-3-(4-chloro-3-hydroxyphenyl)-lH-pyrazolo [3, 4-d] pyrimidin-l-yl) ethyl)-3- (3-fluorophenyl)-4H-chromen-4-one;
2-(l-(4-amino-3-(2-chloro-5-methoxyp enyl)-lH-pyrazolo [3, 4-d] pyrimidin-l-yl) ethyl)-3- (3-fluorophenyl)-4H-chromen-4-one;
2-(l-(4-amino-3-(2-chloro-5-hydroxyphenyl)-lH-pyrazolo [3, 4-d] pyrimidin-l-yl) ethyl)-3- (3-fluorophenyl)-4H-chromen-4-one;
2-(l-(4-amino-3-(3, 4-dimethoxyphenyl)-lH-pyrazolo [3, 4-d] pyrimidin-l-yl) ethyl)-3-(3-fluorophenyl)-4H-chromen-4-one;
2-(l-(4-amino-3-(3, 4-dihydroxyphenyl)- 1 H-pyrazolo [3, 4-d] pyrimidin-l-yl) ethyl)-3-(3-fluorophenyl)-4H-chromen-4-one;
2-((4-amino-3-(3-methyl- 1 H-indazol-6-yl)- 1 H-pyrazolo [3, 4-d] pyrimidin-l-yl) methyl)-3-(3-fluorophenyl)-4H-chromen-4-one;
2-(l-(4-amino-3-(lH-indol-5-yl)-lH-pyrazolo [3, 4-d] pyrimidin-l-yl) ethyl)-3-(3-fluorophenyl)-4H-chromen-4-one;
2-(l-(4-Amino-3-(3-methyl-lH-indol-5-yl)-lH-pyrazolo [3, 4-d] pyrimidin-l-yl) ethyl)-3-(3-fluorophenyl)-4H-chromen-4-one;
tert-butyl-(5-(4-amino-l-(l-(3-(3-fluorophenyl)-4-oxo-4H-chromen-2-yl) ethyl)- 1 H-pyrazolo [3, 4-d] pyrimidin-3-yl) thiophen-2-yl) methylcarbamate
2-(l-(4-amino-3-(5-(aminomethyl) thiophen-2-yl)-lH-pyrazolo [3, 4-d] pyrimidin-l-yl) ethyl)-3-(3-fluorophenyl)-4H-chromen-4-one;
2-((4-amino-3-(3-methyl- 1 H-indazol-6-yl)- 1 H-pyrazolo [3, 4-d] pyrimidin-l-yl) methyl)-6-fluoro-3-phenyl-4H-chromen-4-one;
2-( 1 -(4-amino-3-(3-methyl- 1 H-indazol-6-yl)- 1 H-pyrazolo [3, 4-d] pyrimidin-l-yl) ethyl)-3-phenyl-4H-chromen-4-one;
2-( 1 -(4-amino-3-(3-methyl- 1 H-indazol-6-yl)- 1 H-pyrazolo [3, 4-d] pyrimidin-l-yl) ethyl)-6-fluoro-3-phenyl-4H-chromen-4-one;

2-( 1 -(4-amino-3-(3-methyl- lH-indazol-5-yl)- 1 H-pyrazolo [3, 4-d] pyrimidin-l-yl) ethyl)-3-(3-fluorophenyl)-4H-chromen-4-one;
N-(4-(4-amino-l-(l-(3-(3-fluorophenyl)-4-oxo-4H-chromen-2-yl) ethyl)- 1 H-pyrazolo [3, 4-d] pyrimidin-3-yl) phenyl) acetamide;
2-(l-(4-amino-3-(4-aminophenyl)-l H-pyrazolo [3, 4-d] pyrimidin-l-yl) ethyl)-3-(3-fluorophenyl)-4H-chromen-4-one;
2-(l-(4-amino-3-(3-methyl-lH-indazol-6-yl)-lH-pyrazolo [3, 4-d] pyrimidin-l-yl) ethyl)-6-fluoro-3 -(3 -fluorophenyl)-4H-chromen-4-one;
2- (l-(4-amino-3-(2, 3-dihydrobenzofuran-5-yl)-l H-pyrazolo [3, 4-d] pyrimidin-l-yl) ethyl)-3-(3-fluorophenyl)-4H-chromen-4-one;
2-(l-(4-amino-3-(3-ethyl-lH-indazol-6-yl)-lH-pyrazolo [3, 4-d] pyrimidin-l-yl) ethyl)-3-(3-fluorophenyl)-4H-chromen-4-one;
2-( 1 -(4-amino-3-(3-methyl- 1 H-indol-6-yl)- 1 H-pyrazolo [3, 4-d] pyrimidin-l-yl) ethyl)-3-(3- fluorophenyl)-4H-chromen-4-one;
2-( 1 -(4-amino-3-(2-methoxypyrimidin-5-yl)- 1 H-pyrazolo [3, 4-d] pyrimidin-l-yl) ethyl)-3-(3-fluorophenyl)-4H-chromen-4-one;
4- (4-amino- 1-( 1 -(3-(3-fluorophenyl)-4-oxo-4H-chromen-2-yl) ethyl)- 1 H-pyrazolo [3, 4-d] pyrimidin-3-yl) thiophene-2-carbaldehyde;
2-(l-(4-amino-3-(5-(hydroxymethyl) thiophen-3-yl)-lH-pyrazolo [3, 4-d] pyrimidin-l-yl) ethyl)-3-(3-fluorophenyl)-4H-chromen-4-one;
2-(l-(4-amino-3-(2-methyl-l1H-benzo[d]imidazol-5-yl)-lH-pyrazolo [3, 4-d] pyrimidin-l-yl) ethyl)-3-(3-fluorophenyl)-4H-chromen-4-one;
2-( 1 -(4-amino-3-(3-methyl- 1 H-indazol-6-yl)- 1 H-pyrazolo [3, 4-d] pyrimidin-l-yl) propyl)-3- (3-fluorophenyl)-4H-chromen-4-one;
2-(l-(4-amino-3-(3-methyl-lH-indol-6-yl)-lH-pyrazolo [3, 4-d] pyrimidin-l-yl) ethyl)-3-(3-fluorophenyl)-4H-chromen-4-one;
2-((6-amino-9H-purin-9-yl) methyl)-6-fluoro-3-phenyl-4H-chromen-4-one;
2-((6-amino-9H-purin-9-yl) methyl)-6-fluoro-3 -(3 -fluorophenyl)-4H-chromen-4-one;
2-((4-amino-3-(3-fluoro-5-methoxyphenyl)-lH-pyrazolo [3, 4-d] pyrimidin-l-yl) methyl)-6-fluoro-3-(3-fluorophenyl)-4H-chromen-4-one;
2-((4-amino-3-(3-fluoro-5-hydroxyphenyl)-lH-pyrazolo [3, 4-d] pyrimidin-l-yl) methyl)-6-fluoro-3 -(3 -fluorophenyl)-4H-chromen-4-one;
2-(l-(4-amino-3-(3-methoxyphenyl)-lH-pyrazolo [3, 4-d] pyrimidin-l-yl) ethyl)-6-fluoro-3- (3-fluorophenyl)-4H-chromen-4-one;
2-(l-(4-amino-3-(3-hydroxyphenyl)-lH-pyrazolo [3, 4-d] pyrimidin-l-yl) ethyl)-6-fluoro-3- (3-fluorophenyl)-4H-chromen-4-one;
2-((9H-purin-6-ylamino) methyl)-6-fluoro-3-(3-fluorophenyl)-4H-chromen-4-one;
2-((9H-purin-6-ylamino) methyl)-6-fluoro-3-phenyl-4H-chromen-4-one;
(R)-2-( 1 -(9H-purin-6-ylamino) ethyl)-6-fluoro-3-(3-fluorophenyl)-4H-chromen-4-one;
2-((4-amino-3-(lH-pyrazol-4-yl)-lH-pyrazolo [3, 4-d] pyrimidin-l-yl) methyl)-6-fluoro-3-phenyl-4H-chromen-4-one;
2-(l-(4-amino-3-(3, 5-difluoro-4-methoxyphenyl)-lH-pyrazolo [3, 4-d] pyrimidin-l-yl) ethyl)-3-(3-fluorophenyl)-4H-chromen-4-one;
2-(l-(4-amino-3-(3, 5-difluoro-4-hydroxyphenyl)-lH-pyrazolo [3, 4-d] pyrimidin-l-yl) ethyl)-3-(3-fluorophenyl)-4H-chromen-4-one;
2-((4-amino-3-(3, 5-difluoro-4-methoxyphenyl)-lH-pyrazolo [3, 4-d] pyrimidin-l-yl) methyl)-6-fluoro-3-(3-fluorophenyl)-4H-chromen-4-one;
2-((4-amino-3-(3, 5-difluoro-4-hydroxyphenyl)-lH-pyrazolo [3, 4-d] pyrimidin-l-yl) methyl)-6-fluoro-3-(3-fluorophenyl)-4H-chromen-4-one;
(+)-2-(l-(4-amino-3-(3-methyl-lH-indazol-6-yl)-lH-pyrazolo [3, 4-d] pyrimidin-l-yl) ethyl)- 3-(3-fluorophenyl)-4H-chromen-4-one;
(-)-2-(l-(4-amino-3-(3-methyl-lH-indazol-6-yl)-lH-pyrazolo [3, 4-d] pyrimidin-l-yl) ethyl)- 3-(3-fluorophenyl)-4H-chromen-4-one;
2-(l-(4-amino-3-(3, 5-dimethoxyphenyl)-lH-pyrazolo [3, 4-d] pyrimidin-l-yl) ethyl)-3-(3-fluorophenyl)-4H-chromen-4-one;
2-(l-(4-amino-3-(4-methoxy-3, 5-dimethylphenyl)-lH-pyrazolo [3, 4-d] pyrimidin-l-yl) ethyl)-3-(3-fluorophenyl)-4H-chromen-4-one;
2- (l-(4-amino-3-(2-fluoro-5-isopropoxyphenyl)-lH-pyrazolo [3, 4-d] pyrimidin-l-yl) ethyl)-3- (3-fluorophenyl)-4H-chromen-4-one;
2-(l-(4-amino-3-(2, 3-dihydrobenzo[b] [1, 4] dioxin-6-yl)-lH-pyrazolo [3, 4-d] pyrimidin-l- yl) ethyl)-3-(3-fluorophenyl)-4H-chromen-4-one;
2-(l-(4-amino-3-(l-benzyl-lH-pyrazol-4-yl)-lH-pyrazolo [3, 4-d] pyrimidin-l-yl) ethyl)-3- (3-fluorophenyl)-4H-chromen-4-one;
2-(l-(4-amino-3-(2-methylpyridin-4-yl)-lH-pyrazolo [3, 4-d] pyrimidin-l-yl) ethyl)-3-(3-fluorophenyl)-4H-chromen-4-one;
2-(l-(4-amino-3-(3, 4-dihydro-2H-benzo[b] [1, 4] dioxepin-7-yl)-lH-pyrazolo [3, 4-d] pyrimidin-l-yl) ethyl)-3-(3-fluorophenyl)-4H-chromen-4-one;
2-(l-(4-amino-3-(6-morpholinopyridin-3-yl)-lH-pyrazolo [3, 4-d] pyrimidin-l-yl) ethyl)-3- (3-fluorophenyl)-4H-chromen-4-one;
2-(l-(4-amino-3-(dibenzo [b, d] furan-4-yl)-lH-pyrazolo [3, 4-d] pyrimidin-l-yl) ethyl)-3-(3-fluorophenyl)-4H-chromen-4-one;
2-(l-(4-amino-3-(4-phenoxyphenyl)-lH-pyrazolo [3, 4-d] pyrimidin-l-yl) ethyl)-3-(3-fluorophenyl)-4H-chromen-4-one;
2-(l-(4-amino-3-(4-(benzyloxy)-3-chlorophenyl)-lH-pyrazolo [3, 4-d] pyrimidin-l-yl) ethyl)-3-(3-fluorophenyl)-4H-chromen-4-one;
2- (l-(4-amino-3-(3-chloro-4-isopropoxyphenyl)-lH-pyrazolo [3, 4-d] pyrimidin-l-yl) ethyl)-3- (3-fluorophenyl)-4H-chromen-4-one;
2-(l-(4-amino-3-(3-(dimethylamino) phenyl)- lH-pyrazolo [3, 4-d] pyrimidin-l-yl) ethyl)-3- (3-fluorophenyl)-4H-chromen-4-one;
2-(l-(4-amino-3-(4-ethoxy-3-fluorophenyl)-lH-pyrazolo [3, 4-d] pyrimidin-l-yl) ethyl)-3-(3-fluorophenyl)-4H-chromen-4-one;
2-(l-(4-amino-3-(4-isopropoxyphenyl)-lH-pyrazolo [3, 4-d] pyrimidin-l-yl) ethyl)-3-(3-fluorophenyl)-4H-chromen-4-one;
2- (l-(4-amino-3-(4-(trifluoromethoxy) phenyl)- lH-pyrazolo [3, 4-d] pyrimidin-l-yl) ethyl)-3- (3-fluorophenyl)-4H-chromen-4-one;
2-(l-(3-(4-acetylphenyl)-4-amino-lH-pyrazolo [3, 4-d] pyrimidin-l-yl) ethyl)-3-(3-fluorophenyl)-4H-chromen-4-one;
2-(l-(4-amino-3-(4-(benzyloxy) phenyl)- lH-pyrazolo [3, 4-d] pyrimidin-l-yl) ethyl)-3-(3-fluorophenyl)-4H-chromen-4-one;
2-(l-(4-amino-3-(4-(dimethylamino) phenyl)- lH-pyrazolo [3, 4-d] pyrimidin-l-yl) ethyl)-3- (3-fluorophenyl)-4H-chromen-4-one;
2-(l-(4-amino-3-(4-(methylsulfonyl) phenyl)- lH-pyrazolo [3, 4-d] pyrimidin-l-yl) ethyl)-3- (3-fluorophenyl)-4H-chromen-4-one;
2-( 1 -(4-amino-3-(3-ethoxyphenyl)- 1 H-pyrazolo [3, 4-d] pyrimidin-l-yl) ethyl)-3-(3-fluorophenyl)-4H-chromen-4-one;
2-(l-(4-amino-3-(benzo[b]thiophen-2-yl)-lH-pyrazolo [3, 4-d] pyrimidin-l-yl) ethyl)-3-(3-fluorophenyl)-4H-chromen-4-one;
2-(l-(4-amino-3-(5-chlorothiophen-2-yl)-lH-pyrazolo [3, 4-d] pyrimidin-l-yl) ethyl)-3-(3-fluorophenyl)-4H-chromen-4-one;
2-(l-(4-amino-3-(3, 5-dimethylisoxazol-4-yl)-lH-pyrazolo [3, 4-d] pyrimidin-l-yl) ethyl)-3- (3-fluorophenyl)-4H-chromen-4-one;
2-(l-(4-amino-3-(3-propoxyphenyl)-lH-pyrazolo [3, 4-d] pyrimidin-l-yl) ethyl)-3-(3-fluorophenyl)-4H-chromen-4-one;
2~(l-(4-amino-3-(furan-2-yl)-lH-pyrazolo [3, 4-d] pyrimidin-l-yl) ethyl)-3-(3-fluorophenyl)-4H-chromen-4-one;
2-(l-(4-amino-3-(4-ethoxyphenyl)-lH-pyrazolo [3, 4-d] pyrimidin-l-yl) ethyl)-3-(3-fluorophenyl)-4H-chromen-4-one;
2-(l-(4-amino-3-(3-chloro-4-methoxyphenyl)-lH-pyrazolo [3, 4-d] pyrimidin-l-yl) ethyl)-3- (3-fluorophenyl)-4H-chromen-4-one;
2- (l-(4-amino-3-(3-fluoro-4-isopropoxyphenyl)-lH-pyrazolo [3, 4-d] pyrimidin-l-yl) ethyl)-3- (3-fluorophenyl)-4H-chromen-4-one;
2-(l-(4-amino-3-(6-fluoropyridin-3-yl)-lH-pyrazolo [3, 4-d] pyrimidin-l-yl) ethyl)-3-(3-fluorophenyl)-4H-chromen-4-one;
2-(l-(4-amino-3-(pyrimidin-5-yl)-lH-pyrazolo [3, 4-d] pyrimidin-l-yl) ethyl)-3-(3-fluorophenyl)-4H-chromen-4-one;
2-(l-(4-amino-3-(3-(methoxymethyl) phenyl)- lH-pyrazolo [3, 4-d] pyrimidin-l-yl) ethyl)-3- (3-fluorophenyl)-4H-chromen-4-one;
2-(l-(4-amino-3-(6-hydroxynaphthalen-2-yl)-lH-pyrazolo [3, 4-d] pyrimidin-l-yl) ethyl)-3- (3-fluorophenyl)-4H-chromen-4-one;
2-( 1 -(4-amino-3-(3-isopropoxyphenyl)- lH-pyrazolo [3, 4-d] pyrimidin-l-yl) ethyl)-3-(3-fluorophenyl)-4H-chromen-4-one;
2-(l-(4-amino-3-(l-methyl-lH-pyrazol-4-yl)-lH-pyrazolo [3, 4-d] pyrimidin-l-yl) ethyl)-3- (3-fluorophenyl)-4H-chromen-4-one;
6-Fluoro-3-(3-fluorophenyl)-2-(l-(4-methoxyphenylamino) ethyl)-4H-chromen-4-one;
2-(l-(4-Chloro-7H-pyrrolo [2, 3-d] pyrimidin-7-yl) emyl)-3-(3-fluorophenyl)-4H-chromen-4-one; 2-(l-(4-Chloro-lH-pyrazolo [3, 4-b] pyridin-l-yl) ethyl)-3-(3-fluorophenyl)-4H-chromen-4-one;
2-(l-(4-Chloro-lH-pyrazolo [3, 4-d] pyrimidin-l-yl) ethyl)-3-(3-fluorophenyl)-4H-chromen-4- one;
2-(l-(4-Chloro-5H-pyrrolo [3, 2-d] pyrimidin-5-yl) ethyl)-3-(3-fluorophenyl)-4H-chromen-4-one;
2-(l-(4-amino-3-(l, 3-dimethyl-lH-indazol-6-yl)-lH-pyrazolo [3, 4-d] pyrimidin-l-yl) ethyl)-3-(3-fluorophenyl)-4H-chromen-4-one;
2-(l-(4-amino-3-(2, 3-dimethyl-2H-indazol-6-yl)-lH-pyrazolo [3, 4-d] pyrimidin-l-yl) ethyl)-3-(3-fluorophenyl)-4H-chromen-4-one;
2-( 1 -(4-amino-3-(6-methoxynaphthalen-2-yl)- 1 H-pyrazolo [3, 4-d] pyrimidin-l-yl) ethyl)-3-(3-fluorophenyl)-4H-chromen-4-one;
2-(l-(4-amino-3-(benzo[b]thiophen-3-yl)-lH-pyrazolo [3, 4-d] pyrimidin-l-yl) ethyl)-3-(3-fluorophenyl)-4H-chromen-4-one;
2- (l-(4-amino-3-(2, 4-dimethoxypyrimidin-5-yl)-l H-pyrazolo [3, 4-d] pyrimidin-l-yl) ethyl)-3- (3-fluorophenyl)-4H-chromen-4-one;
2- ( 1 -(4-amino-3-(6-ethoxynaphthalen-2-yl)- 1 H-pyrazolo [3, 4-d] pyrimidin-l-yl) ethyl)-3-(3- fluorophenyl)-4H-chromen-4-one;
3- (4-amino-l-(l-(3-(3-fluorophenyl)-4-oxo-4H-chromen-2-yl) ethyl)- 1 H-pyrazolo [3, 4-d] pyrimidin-3-yl)-N-cyclopropylbenzamide;
2-(l-(4-amino-3-(3-(morpholine-4-carbonyl) phenyl)- 1 H-pyrazolo [3, 4-d] pyrimidin-l-yl) ethyl)-3-(3-fluorophenyl)-4H-chromen-4-one;
2-(l-(4-amino-3-(3-(difluoromethoxy) phenyl)- 1 H-pyrazolo [3, 4-d] pyrimidin-l-yl) ethyl)-3-(3-fluorophenyl)-4H-chromen-4-one;
5- (4-amino-l-(l-(3-(3-fluorophenyl)-4-oxo-4H-chromen-2-yl) ethyl)- 1 H-pyrazolo [3, 4-d] pyrimidin-3-yl) furan-2-carbaldehyde and pharmaceutically acceptable salts thereof;
or their enantiomers, mixtures of enantiomers, mixtures of two or more diastereoisomers, isotopic variants, pharmaceutically acceptable salts, solvates, hydrates or pro-drug.

In certain embodiment, wherein said PI3K inhibitor comprise: 2-(1-(9H-purin--6-ylamino) propyl group)-3-(3-fluorophenyl)-4H-chromen-4-one or its enantiomers, mixtures of enantiomers, mixtures of two or more diastereoisomers, isotopic variants, pharmaceutically acceptable salts, solvates, hydrates or prodrugs.

In certain embodiment, wherein said PI3K inhibitor comprise: (S)-2-(1-(9H-purin--6-ylamino) propyl group)-3-(3-fluorophenyl)--4H-chromen--4-one (Tenalisib, also named CN401 ) or its pharmaceutically acceptable salts, solvates, hydrates or prodrugs.

In certain embodiment, the pharmaceutical combination may include an anti-PD-L1 antibody or its antigen-binding fragment thereof and PI3K inhibitor.

For example, the pharmaceutical combination may include: (i) an anti-PD-L1 antibody or antigen-binding fragment thereof comprising atezolizumab, avelumab, BMS-936559, MPDL3280A or durvalumab or an antigen thereof binding fragment;(ii) PI3Kδ/γ dual inhibitor, said PI3Kδ/γ dual inhibitor comprise 2-(1-(9H-purin--6-ylamino) propyl group)-3-(3-fluorophenyl)-4H-chromen-4-one or its enantiomers, mixtures of enantiomers, mixtures of two or more diastereoisomers, isotopic variants, pharmaceutically acceptable salts, solvates, hydrates or prodrugs.

For another example, said pharmaceutical combination may include:(i) anti-PD-L1 antibody selected from the following group: Atezolizumab, Avelumab, BMS-936559, MPDL3280A, durvalumab or its combination;(ii) 2-(1-(9H-purin--6-ylamino) propyl group)-3-(3-fluorophenyl)-4H-chromen-4-one or its pharmaceutically acceptable salts, solvates, hydrates or prodrugs.

In certain embodiment, said pharmaceutical combination may include TGF β inhibitor and PI3K inhibitor.

For example, the pharmaceutical combination may include: (i) TGFβ inhibitors, said TGFβ inhibitors include: anti-TGFβ antibodies or antigen-binding fragments thereof, small molecule TGFβ inhibitors or combinations thereof, said TGFβ antibodies or antigen-binding fragments thereof including Fresolimumab or an antigen-binding fragment thereof, the small molecule TGFβ inhibitors include SB525334, SD-208, SB431542, LY2109761, LY2157299 (Galunisertib), GW788388, RepSox, SIS3, LDN-193189, EW-7197, LY364947 or combinations thereof;(ii) PI3K8/y dual inhibitor, said PI3Kδ/γ dual inhibitor comprises 2-(1-(9H-purin--6-ylamino) propyl group)-3-(3-fluorophenyl)-4H-chromen-4-one or its enantiomers, mixtures of enantiomers, mixtures of two or more diastereoisomers, isotopic variants, pharmaceutically acceptable salts, solvates, hydrates or prodrugs.

For another example, the pharmaceutical combination may comprise Fresolimumab or its antigen-binding fragment and (S)-2-(1-(9H-purin--6-ylamino) propyl group)-3-(3-fluorophenyl)-4H-chromen-4-one or a pharmaceutically acceptable salt, solvate, hydrate or prodrug thereof.

For another example, the pharmaceutical combination may comprise Fresolimumab or its antigen-binding fragment, SB431542 and (S)-2-(1-(9H-purin--6-ylamino) propyl group)-3-(3-fluorophenyl)-4H-chromen-4-one or a pharmaceutically acceptable salt, solvate, hydrate or prodrug thereof.

In certain embodiment, the pharmaceutical combination may comprise PD-1 inhibitor, TGF β inhibitor and PI3K inhibitor.

For example, the pharmaceutical combination may comprise (i) an anti-PD-1 antibody or an antigen-binding fragment thereof, the anti-PD-1 antibody or an antigen-binding fragment thereof including Nivolumab, Pembrolizumab, Pidilizumab antibody or AMP-514 or antigen-binding fragment thereof; (ii) PI3K8/y dual inhibitors , said PI3K8/y dual inhibitor comprising 2-(1-(9H-purin--6-ylamino) propyl group)-3-(3-fluorophenyl)-4H-chromen-4-one, or its enantiomer, a mixture of enantiomers, a mixture of two or more diastereoisomers, or isotopic variants; or pharmaceutically acceptable salts, solvates, hydrates or prodrugs thereof; (iii) anti-TGFβ antibodies or antigen-binding fragments thereof, small molecule TGFβ inhibitors or combinations thereof, said TGFβ antibodies or antigens-binding fragments include Fresolimumab or antigen-binding fragments thereof, and said small molecule TGFβ inhibitors include SB525334, SD-208, SB431542, LY2109761, LY2157299 (Galunisertib), GW788388, RepSox, SIS3, LDN-193189, EW-7197, LY364947 or their combination.

For another example, the pharmaceutical combination may include: (i) an anti-PD-1 antibody selected from the group consisting of nivolumab, pembrolizumab, Pidilizumab or AMP-514 or a combination thereof; (ii) (S)-2-(1-(9H-purin--6-ylamino) propyl group)-3-(3-fluorophenyl)-4H-chromen-4-one or a pharmaceutically acceptable salt thereof, a solvate, hydrate or prodrug; (iii) Fresolimumab or an antigen-binding fragment thereof and/or SB431542.

In one embodiment, the pharmaceutical combination may comprise PD-1 inhibitor, TGF β inhibitor and PI3K inhibitor.

For example, the pharmaceutical combination may comprise (i) an anti-PD-L1 antibody or antigen-binding fragment thereof comprising atezolizumab, avelumab, BMS-936559, MPDL3280A or durvalumab or an antigen-binding fragments; (ii) PI3Kδ/γ dual inhibitors comprising 2-(1-(9H-purin--6-ylamino) propyl group)-3-(3-fluorophenyl)-4H-chromen-4-one, or an enantiomer, a mixture of enantiomers, a mixture of two or more diastereoisomers, or an isotopic variant thereof; or a pharmaceutically acceptable salts, solvates, hydrates or prodrugs; (iii) anti-TGFβ antibodies or antigen-binding fragments thereof comprising Fresolimumab or antigen-binding fragments thereof.

For another example, the pharmaceutical combination may include: (i) an anti-PD-L1 antibody selected from the group consisting of Atezolizumab, Avelumab, BMS-936559, MPDL3280A or durvalumab or a combination thereof; (ii) (S)- 2-(1-(9H-purin--6-ylamino) propyl group)-3-(3-fluorophenyl)-4H-chromen-4-one or its pharmaceutically acceptable salt, solvate, hydrate or prodrug (iii) Fresolimumab or an antigen-binding fragment thereof.

In one embodiment, the pharmaceutical combination may comprise the PD-L1/TGFβ dual inhibitor and a PI3K inhibitor.

In certain embodiment, the PD-L1/TGFβ dual inhibitors may be those described in PCT/CN2019124535, all of which are incorporated by reference into this application. For example, the PD-L1/TGFβ dual inhibitor may be WBP1126 (also known as CN202), or a variant or biosimilar thereof. The WBP1126 may comprise two polypeptides, the polypeptides sequentially comprise the heavy chain variable region (VH), CH2, CH3 domains of the anti-PD-L1 antibody from the N-terminus to the C-terminus, and TGFβRII or a functionally active fragment thereof. The C-terminus of the CH3 domain is connected to the N-terminus of the TGFβRII or its functionally active fragment through a linker; for example, the polypeptide may have the amino acid sequence shown in Table 1:

Table 1

| WBP1126-U15T1.G1-1.uIgG1 | | SEQ ID NO | Amino acid sequence |
|---|---|---|---|
| VHH | HCDR1 | 1 | GHFSNLAVN |
| | HCDR2 | 2 | GILWSGGSTFYADSVKG |
| | HCDR3 | 3 | GTN |
| | VHH | 4 | EVQLVESGGGLVQPGGSLRLSCAASGHFSNLAVNWFRQAPGKERELVAGILWSGGSTFYADSVKGRFTISRGNAENMLYLQMNSLRAEDTAVYYCNTGTNWGQGTLVTVSS |
| linker | | 5 | GGGGSGGGGSGGGGSGGGGS |

(continued)

| WBP1126-U15T1.G1-1.uIgG1 | SEQ ID NO | Amino acid sequence |
|---|---|---|
| TGFβRII extracellular domain | 6 | IPPHVQKSVNNDMIVTDNNGAVKFPQLCKFC DVRFSTCDNQKSCMSNCSITSICEKPQEVCVA VWRKNDENITLETVCHDPKLPYHDFILEDAAS PKCIMKEKKKPGETFFMCSCSSDECNDNIIFSE EYNTSNPD |
| Sequence full length | 7 | EVQLVESGGGLVQPGGSLRLSCAASGHFSNLA VNWFRQAPGKERELVAGILWSGGSTFYADSV KGRFTISRGNAENMLYLQMNSLRAEDTAVYY CNTGTNWGQGTLVTVSSEPKSCDKTHTCPPC PAPELLGGPSVFLFPPKPKDTLMISRTPEVTCV VVDVSHEDPEVKFNWYVDGVEVHNAKTKPR EEQYNSTYRVVSVLTVLHQDWLNGKEYKCK VSNKALPAPIEKTISKAKGQPREPQVYTLPPSR EEMTKNQVSLTCLVKGFYPSDIAVEWESNGQP ENNYKTTPPVLDSDGSFFLYSKLTVDKSRWQ QGNVFSCSVMHEALHNHYTQKSLSLSPGGGG SGGGGSGGGGSGGGGSIPPHVQKSVNNDMIV TDNNGAVKFPQLCKFCDVRFSTCDNQKSCMS NCSITSICEKPQEVCVAVWRKNDENITLETVC HDPKLPYHDFILEDAASPKCIMKEKKKPGETF FMCSCSSDECNDNIIFSEEYNTSNPD |

In certain embodiment, wherein the PD-L1/TGFβ dual inhibitor comprises a first polypeptide and a second polypeptide, wherein the first polypeptide comprises at least: (i) a heavy chain of an anti-PD-L1 antibody variable region; and (ii) TGFβRII or a functionally active fragment thereof; wherein the second polypeptide comprises at least the light chain variable region of an anti-PD-L1 antibody; wherein the heavy chain variable region of the first polypeptide and the light chain variable regions of the second polypeptide are capable of specifically binding to PD-L1.

In certain embodiment, wherein PD-L1/TGFβ dual inhibitor further comprises a linker, said linker connects the C-terminus of the heavy chain variable region of the anti-PD-L1 antibody or antigen-binding fragment thereof with the TGFβRII or the N-terminus of its functionally active fragments.

In certain embodiment, wherein the PD-L1/TGFβ dual inhibitor comprises a first polypeptide and a second polypeptide:

wherein the first polypeptide comprises the heavy chain variable region (VH) of the anti-PD-L1 antibody, the CH1 domain, and TGFβRII or its functionally active fragment, and the C-terminus of the CH1 domain is connected to the The TGFβRII or its functionally active fragment N-terminus is connected by a linker; and the second polypeptide comprises a light chain variable region (VL) and a light chain constant region (CL) of an anti-PD-L1 antibody from the N-terminus to the C-terminus.

In certain embodiment, wherein the PD-L1/TGFβ dual inhibitor comprises a first polypeptide and a second polypeptide:

The first polypeptide comprises the heavy chain variable region (VH), CH1, CH2, CH3 domains and TGFβRII or its functionally active fragments of the anti-PD-L1 antibody sequentially from the N-terminus to the C-terminus, and the CH3 domain The C-terminus is connected to the N-terminus of the TGFβRII or its functionally active fragment through a linker; and the second polypeptide comprises a light chain variable region (VL) and a light chain of an anti-PD-L1 antibody from the N-terminus to the C-terminus Constant region (CL).

In certain embodiment, wherein said linker is a peptide linker.

In certain embodiment, wherein the first polypeptide has at least about 80% sequence identity to a polypeptide having the same function as: M7824 or SHR-1701.

In certain embodiment, wherein the second polypeptide has at least about 80% sequence identity to a polypeptide having the same function as: M7824 or SHR-1701.

In certain embodiment, the PD-L1/TGFβ dual inhibitors may be those described in PCT/EP2015052781 and PCT/CN2018086451, all of which are incorporated by reference into this application.

In certain embodiment, wherein the PD-L1/TGFβ dual inhibitor comprises M7824, SHR-1702, or variant or biosimilar or their combination.

In certain embodiment, the PD-L1/TGFβ dual inhibitor and the PI3K inhibitor may not be mixed with each other in the drug combination.

In certain embodiment, the PD-L1/TGFβ dual inhibitor and the PI3K inhibitor can be independently contained in separate containers.

Pharmaceutical composition and Uses

In another aspect, the present application provides a pharmaceutical composition, the pharmaceutical combination comprises the aforementioned PI3K inhibitor and a second therapeutic agent, and optionally one or more pharmaceutically acceptable carriers or excipients

In certain embodiment, the second therapeutic agent is the aforementioned PD-L1/TGFβ dual inhibitor.

In certain embodiment, the PI3K inhibitor is the aforementioned PI3Kδ/γ dual inhibitor.

In some embodiment, the pharmaceutical composition comprises a PD-L1/TGFβ dual inhibitor and a PI3K8/y dual inhibitor.

For example, the pharmaceutical composition may comprise:

i) WBP1126 or a variant or biosimilar thereof; and
ii) Tenalisib or a pharmaceutically acceptable salt, solvate, hydrate or prodrug thereof.

In certain embodiment, the second therapeutic agent is the aforementioned anti-PD-L1 antibody, and the PI3K inhibitor is the aforementioned PI3K8/y dual inhibitor.

For example, the pharmaceutical composition may include: i) nivolumab, pembrolizumab, pidilizumab, or combinations thereof; and ii) tenalisib or a pharmaceutically acceptable salt, solvate, or hydrate thereof or prodrug.

In certain embodiment, the second therapeutic agent is the aforementioned PD-L1/TGFβ dual inhibitor, and the PI3K inhibitor is a PI3Kα inhibitor.

For example, the pharmaceutical composition may comprise:

i) WBP1126 or a variant or biosimilar thereof; and
ii) Alplisib or its pharmaceutically acceptable salt, solvate, hydrate or prodrug.

In certain embodiment, wherein said second therapeutic agent and said PI3K inhibitor are present in a single or separate dosage form.

In certain embodiment, wherein the pharmaceutical composition includes, each unit dosage form (unit dosage form) contains 1-1000 mg dose of the first therapeutic agent, such as PI3K inhibitor active substance (such as CN401 (Tenalisib)), For example 1, 2, 3, 4, 5, 6, 7, 8, 9, 10, 11, 12, 13, 14, 15, 16, 17, 18, 19, 20, 25, 30, 40, 50, 60, 70, 80, 90, 100, 110, 120, 130, 140, 150, 160, 170, 180, 190, 200, 250, 300, 350, 400, 450, 500, 600, 700, 800, 900, 1000mg or a value between any two of the above values; and, each unit dosage form (unit dosage form) contains a second therapeutic agent at a dose of 1-1000 mg, such as a PD-L1 inhibitor, a TGFβ inhibitor, a PD-L1 inhibitor, PD-L1/TGFβ dual inhibitors (such as CN202 (WBP1126)) or PD-1/TGFβ dual inhibitors or a combination thereof, such as 1, 2, 3, 4, 5, 6, 7, 8, 9, 10, 11, 12, 13, 14, 15, 16, 17, 18, 19, 20, 25, 30, 40, 50, 60, 70, 80, 90, 100, 110, 120, 130, 140, 150, 160, 170, 180, 190, 200, 250, 300, 350, 400, 450, 500, 600, 700, 800, 900, 1000 mg or a value between any two of the above values.

In certain embodiment, the pharmaceutical composition can further include a third active substance.

In certain embodiment, wherein the third active substance may be selected from vincristine, vinblastine, vindesine, etoposide, docetaxel, paclitaxel (such as nab-paclitaxel), irinotecan, vinorelbine, mitoxantrone, vinflunine, topotecan

or any combination thereof.

In another aspect, the present application provides a method for treating and/or preventing tumors, which comprises administering to a subject in need: an effective amount of the aforementioned pharmaceutical combination, or the aforementioned pharmaceutical composition.

In certain embodiment, the method includes administering to a subject in need: an effective amount of the aforementioned PI3K inhibitor and the aforementioned immune checkpoint inhibitor.

In certain embodiment, the method comprises administering to a subject in need: an effective amount of the aforementioned PI3K inhibitor and the aforementioned TGFβ inhibitor.

In certain embodiment, the method includes administering to a subject in need: an effective amount of the aforementioned PI3K inhibitor, the aforementioned TGFβ inhibitor, and the aforementioned immune checkpoint inhibitor.

For example, the method may comprise administering to a subject in need: an effective amount of the aforementioned PI3K inhibitor, such as the aforementioned (S)- 2-(1-(9H-purin--6-ylamino) propyl group)-3-(3-fluorophenyl)-4H-chromen-4-one or a pharmaceutically acceptable salt thereof and the aforementioned anti-PD-L1 antibody, for example, Atezolizumab, Avelumab, BMS-936559, MPDL3280A, durvalumab, or a combination of them.

For example, the method may comprise administering to a subject in need: an effective amount of the aforementioned PI3K inhibitor, such as the aforementioned (S)- 2-(1-(9H-purin--6-ylamino) propyl group)-3-(3-fluorophenyl)-4H-chromen-4-one or a pharmaceutically acceptable salt thereof and the aforementioned TGFβ inhibitors, for example, Fresolimumab, SB525334 or a combination thereof.

For example, the method may comprise administering to a subject in need: an effective amount of the aforementioned PI3K inhibitor, such as the aforementioned (S)- 2-(1-(9H-purin--6-ylamino) propyl group)-3-(3-fluorophenyl)-4H-chromen-4-one or a pharmaceutically acceptable salt thereof; the aforementioned TGFβ inhibitors, for example, Fresolimumab, SB525334 or their combination; the aforementioned anti-PD-L1 Antibodies, e.g., Atezolizumab, Avelumab, BMS-936559, MPDL3280A, durvalumab, or combinations thereof.

For example, the method may comprise administering to a subject in need: an effective amount of the aforementioned PI3K inhibitor, such as the aforementioned (S)- 2-(1-(9H-purin--6-ylamino) propyl group)-3-(3-fluorophenyl)-4H-chromen-4-one or a pharmaceutically acceptable salt thereof; the aforementioned TGFβ inhibitors, for example, Fresolimumab, SB525334 or their combination; the aforementioned anti-PD-L1 Antibodies, e.g. Nivolumab, pembrolizumab, pidilizumab, or a combination thereof.

In certain embodiment, the method includes administering to a subject in need: an effective amount of the aforementioned PI3K inhibitor and the aforementioned PD-L1/TGFβ dual inhibitor.

For example, the method may comprise administering to a subject in need: an effective amount of the aforementioned PI3K inhibitor, such as the aforementioned (S)- 2-(1-(9H-purin--6-ylamino) propyl group)-3-(3-fluorophenyl)-4H-chromen-4-one, and the aforementioned PD-L1/TGFβ dual inhibitors, such as WBP1126, M7824, SHR-1701, or variants or biosimilars thereof, or a combination thereof.

In certain embodiment, the PI3K inhibitor can be administered simultaneously with the PD-L1/TGFβ dual inhibitor.

In certain embodiment, the PI3K inhibitor can be administered after the PD-L1/TGFβ dual inhibitor.

In certain embodiment, the PI3K inhibitor can be administered before the PD-L1/TGFβ dual inhibitor.

In some embodiment, it also includes administering an effective amount of the aforementioned small molecule TGFβ inhibitor to a subject in need.

In certain embodiment, the tumor may include solid tumors and non-solid tumors.

In certain embodiment, the tumor may comprise a tumor with abnormal expression of PI3K.

In some embodiment, the tumor may include a tumor with abnormal expression of PD-L1.

In certain embodiment, the tumor may include a tumor with abnormal expression of TGFβ.

In certain embodiment, the tumor may include a tumor of the gastrointestinal tract, melanoma, or lymphoma.

In certain embodiment, the methods of the present application delay resistance compared to the time at which resistance would normally develop when a subject is treated with either agent or inhibitor alone as a monotherapy. In certain embodiment, resistance is delayed by at least 2 weeks, e.g., at least 2 weeks, 4 weeks, 1 month, 2 months, 3 months, 4 months, 5 months, 6 months, 8 months, 10 months, 12 months, 1 year, 2 years, 4 years, 6 years, 8 years or longer. In certain embodiment, according to the method, the remission (e.g., complete remission or partial remission) is prolonged compared to the time period that remission would typically last if the subject was treated with either agent or inhibitor alone as monotherapy. In certain embodiment, the remission (e.g., complete remission or partial remission) is prolonged by at least 2 weeks, e.g., at least 2 weeks, 4 weeks, 1 month, 2 months, 3 months, 4 months, 5 months, 6 months, 8 months, 10 months, 12 months, 1 year, 2 years, 4 years, 6 years, 8 years or longer.

In certain embodiment, administrating a PI3K inhibitor or a second agent to the treatment regimen increases or restores sensitivity to the agent that the cancer is resistant to. For example, in certain embodiment, adding a second agent to the treatment regimen increases or restores sensitivity to a cancer-resistant PI3K inhibitor.

In certain embodiment, wherein the remission of the cancer in the subject is prolonged.

In certain embodiment, wherein the subject experiences a complete remission of cancer.

In certain embodiment, wherein the level of minimal residual disease (MRD) is reduced.

In certain embodiment, the methods in the present application comprise selecting a subject for treatment with a combination of a PI3K inhibitor and a second agent. In certain embodiment, a subject (e.g., a patient with a cancer, such as a cancer described herein) is selected for treatment with the combination based on the MRD in the subject. In certain embodiment, selection is based on the presence of MRD above a preselected level (e.g., 1 malignant cell in 100 normal cells, 1 malignant cell in 1000 normal cells, or 1 in 10,000 normal cells with 1 malignant tumor cell). Methods for monitoring minimal residual disease negativity (MRD) are known in the art. See, e.g., Zhou, J. et al., Blood, 2007, 110:1607-1611. Such methods include DNA-based tests or RNA-based tests. In certain embodiment, MRD is monitored using flow cytometry, sequencing, or PCR.

In another aspect, the application provides the aforementioned pharmaceutical combination or the aforementioned pharmaceutical composition in the preparation of medicines for treating and/or preventing tumors.

For example, the use of the aforementioned PI3K inhibitors and the aforementioned immune checkpoint inhibitors in the preparation of drugs for treating and/or preventing tumors. For example, the use of the aforementioned PI3K inhibitor and the aforementioned TGFβ inhibitor in the preparation of medicaments for treating and/or preventing tumors. For example, the use of the aforementioned PI3K inhibitors, the aforementioned immune checkpoint inhibitors and the aforementioned TGFβ inhibitors in the preparation of drugs for treating and/or preventing tumors.

In another aspect, the present application provides the aforementioned pharmaceutical combination or the aforementioned pharmaceutical composition for treating and/or preventing tumors.

In another aspect, the present application provides a method for inhibiting tumor growth, the method comprising exposing the tumor to the aforementioned pharmaceutical combination or the aforementioned pharmaceutical composition.

In certain embodiment, the method comprises exposing the tumor to an aforementioned PI3K inhibitor and an aforementioned inhibitor of an immune checkpoint. In certain embodiment, the method comprises contacting the tumor with the aforementioned PI3K inhibitor and the aforementioned TGFβ inhibitor. In certain embodiment, the method comprises contacting the tumor with an aforementioned PI3K inhibitor, an aforementioned inhibitor of an immune checkpoint, and an aforementioned TGFβ inhibitor.

In another aspect, the present application provides a method for inhibiting tumor growth, the method comprising exposing the tumor to the aforementioned PI3K inhibitor and the aforementioned dual PD-L1/TGFβ inhibitor.

In another aspect, the present application provides a kit, which includes: (1) a first container, and the aforementioned PI3K inhibitor located in the first container; (2) a second container, and the aforementioned PD-L1/TGFβ dual inhibitors located in the second container.

In another aspect, the present application provides a kit, which may include: (1) a first container, and the aforementioned PI3K inhibitor located in the first container; (2) a second container, and the aforementioned immune checkpoint inhibitor or the aforementioned TGFβ inhibitor located in the second container.

In certain embodiment, the kit further includes (3) a third container, and the aforementioned immune checkpoint inhibitor or the aforementioned TGFβ inhibitor located in the third container, the agent in the third container different from the medicament in the second container.In certain embodiment, the dosage form of the medicine in the kit can be oral dosage form or injection dosage form

In another aspect, the present application provides a method for inhibiting tumor growth, said method comprising exposing the tumor to the aforementioned PI3K inhibitor and the aforementioned dual PD-L1/TGFβ inhibitor.

In another aspect, the present application provides a kit, which includes: (1) a first container, and the aforementioned PI3K inhibitor located in the first container; (2) a second container, and the aforementioned PD-L1/TGFβ dual inhibitors located in the second containers.

In another aspect, the present application provides a kit, which includes: (1) a first container, and the aforementioned PI3K inhibitor located in the first container; (2) a second container, and the aforementioned TGFβ inhibitors located in the second containers.

In certain embodiment, the kit further includes (3) a third container, and the aforementioned immune checkpoint inhibitor or the aforementioned TGFβ inhibitor located in the third container, the agent in the third container is different from the medicament in the second container.

In certain embodiment, the dosage form of the medicine in the kit can be oral dosage form or injection dosage form.

In certain embodiment, the kit can also contain instructions.

Without intending to be limited by any theory, the following examples are only to illustrate the pharmaceutical combination, preparation method and use of the present application, and not to limit the scope of the invention

**Example**

Materials and method

The animal experiment was carried out in accordance with the requirements of AAALAC, and was approved by Bikai Animal Experiment Center IACUC to conduct. 6-8 week-old Balb/c mice (16-20 grams) were purchased from Beijing Weitong Lihua Experimental Animal Technology Co., Ltd. Each mouse was subcutaneously inoculated with 500,000 A20 cells, and inoculated on the right rear side of the back of the mouse. When the volume of the tumor grew to about 60mm$^3$, the mice were randomly assigned to the control group and the experimental group.

Micewere euthanized whnmorbidityor weightloss>20% was observed. Tumors were measured with acaliper twice weekly until final sacrifice. When at umor size reached approximately 2000 mm $^3$ or there were animal health issues(20% area of atumor ulcerated), the animals would be euthanized an date of death recorded. Solid tumor volumes were estimated from two dimensional tumor measurements and calculated according to the following equation:

$$\text{Tumor volume(mm 3)} = [\text{length(mm) x width2 (mm 2)}] /2$$

The percent median regression for a group at a given day was then obtained by taking the median of the individual percent regression calculated for each animal of the group at this day. The day of calculation was determined on the day when $\Delta T/\Delta C$ (i.e., the ratio of medians of tumor volume changes from baseline between the treated and control groups)was calculated, except when median percent regression was not representative of the activity of the group. In that case, the day was determined on the first day when the median percent regression was maximal. Regressions were defined as partial (PR) if the tumor volume decreased to 50% of the tumor volume at the start of treatment. Complete regression(CR) was considered to have been achieved when tumor volume was below 14mm$^3$ or could not be recorded.

RTV: Relative Tumor Volume. RTV=$V_t/V_0$。$V_t$: Tumor volume at the end of an experimental period (treatment group). $V_0$: tumor volume at the beginning of the experiment

TGI：Tumor Growth Inhibition value. TGI=[1-RTV(experimental group)/RTV(control group)]*100%

Statistical analysis

A two-way ANOVA type with factors treatment an day(repeated) was performed on tumor volume changes from baseline. In case of significant treatment day interaction or treatment effects, it was followed by a contrast analysis with Bonferroni-Holm correction for multiplicity to compareall treated groups to the control group at each day from day 8 to 27. Tumor volume changes from baseline were calculated for each animal and each day by subtracting the tumor volume on the day of first treatment from the tumor volume on the specified observation day. As heterogeneity of variances was observed between groups, the compound symmetric(CS) covariance structure with group= option was chosen for the ANOVA type model (SAS Institute Inc.(2008) SAS/STAT 9.2User's Guide by CaryNC). All statistical analyses were performed using SAS version v9.2 software. A probability less than5% ($p<0.05$).

**Example 1 Study of the combination of PI3K inhibitors with TGF$\beta$/PDL1 bispecific antibody with different dosage**

Example 1.1 Study on the combination of CN401 (Tenalisib) and CN202 (WBP1126) (Smg/kg) in the A20 mouse B-cell lymphoma model (see Table 2 for the test design)

6-8 weeks old balb/c mice (18-20g) were purchased from Beijing Weitong Lihua Experimental Animal Technology Co., Ltd., and each mouse was subcutaneously inoculated with 500,000 A20 cells on the right rear side of the mouse's back. Treatment was initiated when tumors grew to a volume of approximately 62 mm$^3$. CN401 was orally administered at 150 mg/kg twice a day for 3 weeks.

CN202 was administered by intraperitoneal injection, 3 times a week at a dose of 5 mg/kg for 3 weeks. The control group was treated with vehicle (0.5% MC and PBS) without active product. Tumor size and body weight were measured three times a week. This animal experiment was carried out in accordance with the requirements of AAALAC, and was approved by Bikai Animal Experiment Center IACUC to conduct this animal experiment.

Table 2

| Group | Amount | Method of administration |
|---|---|---|
| 1 | 8 | Vehicle control group (PBS i.p. tiw×3weeks +0.5%MC p.o. bid×3 weeks) |
| 2 | 8 | CN401 150mg/kg, p.o. bid×3 weeks |
| 3 | 8 | CN202 5mg/kg, i.p. tiw×3 weeks |
| 4 | 8 | CN401 150mg/kg, p.o. bid×3 weeks +CN202 5mg/kg, i.p. tiw×3 weeks |

The results of tumor growth curves and body weight growth curves are shown in Figure 1A-Figure 1F; TGI, p-value and number of complete responding mice in the treatment group compared with the blank group at day 22 are shown in Table 3.

Result: In this study, the combination of CN401 and CN202 had a synergistic effect on tumor suppression in the A20 mouse B-cell lymphoma model (p=0.0001, TGI=89%, CR=4/8). The mice showed good tolerance to the dosing regimen.

Table 3

| Group# | TGI% | p value (vs vehicle control group) | CR (Number of mice with tumor regression /total amount of mice in each group) |
|---|---|---|---|
| CN401 150mg/kg | 55 | 0.034 | 2/8 |
| CN202 5mg/kg | 31 | 0.27 | 1/8 |
| CN401 150mg/kg+CN202 5mg/kg | 89 | 0.00010 | 4/8 |

Example 1.2 Study on the combination of CN401 and CN202 (15mg/kg) in the A20 mouse B-cell lymphoma model (see Table 4 for the test design)

Referring to the experimental method of Example 1.1, the difference is that the dosage of CN202 is replaced by 15 mg/kg from 5 mg/kg.

Table 4

| Group | Amount | Method of administration |
|---|---|---|
| 1 | 8 | Vehicle control group (PBS i.p. tiw×3weeks +0.5%MC p.o. bid×3 weeks) |
| 2 | 8 | CN401 150mg/kg, p.o. bid×3 weeks |
| 3 | 8 | CN202 15mg/kg, i.p. tiw×3 weeks |
| 4 | 8 | CN401 150mg/kg, p.o. bid×3 weeks +CN202 15mg/kg, i.p. tiw×3 weeks |

The results of tumor growth curves and body weight growth curves are shown in Figure 2A-Figure 2F; TGI, p-value and number of complete responding mice in the treatment group compared with the blank group at day 22 are shown in Table 5.

Result: In this study, the combination of CN401 and CN202 had a synergistic effect on tumor suppression in the A20 mouse B-cell lymphoma model (p=0.023, TGI=71%, CR=5/8). The mice showed good tolerance to the dosing regimen.

Table 5

| Group# | TGI% | p value (vs vehicle control group) | CR (Number of mice with tumor regression /total amount of mice in each group) |
|---|---|---|---|
| CN401 150mg/kg | 55 | 0.034 | 2/8 |
| CN202 15mg/kg | 58 | 0.014 | 2/8 |

(continued)

| Group# | TGI% | p value (vs vehicle control group) | CR (Number of mice with tumor regression /total amount of mice in each group) |
|---|---|---|---|
| CN401 150mg/kg+CN202 15mg/kg | 71 | 0.023 | 5/8 |

**Example 2 Study of the combination of TGFβ/PDL1 bispecific antibody with different forms of PI3K inhibition**

**Example 2.1 Study of the combination of CN202(WBP1126) with CN401(Tenalisib) (see Table 6 for the test design)**

6-8 weeks old balb/c mice (18-20g) were purchased from Beijing Weitong Lihua Experimental Animal Technology Co., Ltd., and each mouse was subcutaneously inoculated with 500,000 A20 cells on the right rear side of the mouse's back. Treatment was initiated when tumors grew to a volume of approximately 80 $mm^3$.

CN202 was administered by intraperitoneal injection, 3 times a week at a dose of 5 mg/kg for 3 weeks. CN401 was orally administered at 150 mg/kg twice a day for 3 weeks.The control group was treated with vehicle (0.5% MC and PBS) without active product. Tumor size and body weight were measured three times a week. This animal experiment was carried out in accordance with the requirements of AAALAC, and was approved by Bikai Animal Experiment Center IACUC to conduct this animal experiment.

Table 6

| Group | Amount | Method of administration |
|---|---|---|
| 1 | 7 | Vehicle control group (PBS i.p. tiw×3weeks +0.5%MC p.o. bid×3 weeks) |
| 2 | 7 | CN202 5mg/kg i.p. tiw×3 weeks |
| 3 | 7 | CN401 150mg/kg p.o. bid×3 weeks |
| 4 | 7 | CN202 5mg/kg i.p. tiw×3 weeks +CN401 150mg/kg p.o. bid×3 weeks |

The results of tumor growth curves and body weight growth curves are shown in Figure 4A-Figure 4F; TGI, p-value and number of complete responding mice in the treatment group compared with the blank group at day 22 are shown in Table 7.

Result: In this study, the combination of CN202 and CN401 had a synergistic effect on tumor suppression in the A20 mouse B-cell lymphoma model (p=0.0009, TGI=85%, CR=6/7). The mice showed good tolerance to the dosing regimen.

Table 7

| Group | TGI% | P value (vs vehicle control group) | CR(Number of mice with tumor regression /total amount of mice in each group) |
|---|---|---|---|
| CN202 5mg/kg | 68 | 0.004 | 1/7 |
| CN401 150mg/kg | 45 | 0.028 | 0/7 |
| CN202 5mg/kg + CN401 150mg/kg | 85 | 0.0009 | 6/7 |

**Example 2.2 Study of the combination of CN202(WBP1126) with Alpelisib (see Table 8 for the test design)**

Referring to the experimental method of Example 2.1, the difference is that the PI3K inhibitor was replaced by oral administration of CN401 (Tenalisib) at 150 mg/kg twice a day for 3 weeks to Alpelisib at 50 mg/kg once a day for 3 weeks.

Table 8

| Group | Amount | Plan for Administration |
|---|---|---|
| 1 | 7 | Vehicle control group (PBS i.p. tiw×3 weeks +0.5%MC p.o. bid×3 weeks) |

(continued)

| Group | Amount | Plan for Administration |
|---|---|---|
| 2 | 7 | CN202 5mg/kg i.p. tiw×3 weeks |
| 3 | 7 | Alpelisib 50mg/kg p.o. qd×3 weeks |
| 4 | 7 | CN202 5mg/kg i.p. tiw×3 weeks + Alpelisib 50mg/kg p.o. qd×3 weeks |

The results of tumor growth curves and body weight growth curves are shown in Figure 5A-Figure 5F; TGI, p-value and number of complete responding mice in the treatment group compared with the blank group at day 21 are shown in Table 9.

Result: In this study, the combination of CN202 and Alpelisib had a synergistic effect on tumor suppression in the A20 mouse B-cell lymphoma model (p=0.0002, TGI=85%, CR=3/7). The mice showed good tolerance to the dosing regimen.

Table 9

| Group# | TGI% | p value (vs vehicle control group) | CR(Number of mice with tumor regression /total amount of mice in each group) |
|---|---|---|---|
| CN202 5mg/kg | 68 | 0.004 | 1/7 |
| Alpelisib 50mg/kg | 37 | 0.161 | 0/7 |
| CN202 5mg/kg + Alpelisib 50mg/kg | 85 | 0.0002 | 3/7 |

**Example 3 Study of the combination of CN401 with anti-PD-L1 antibody in the A20 mouse B-cell lymphoma model (see Table 8 for the test design)**

6-8 weeks old balb/c mice (18-20g) were purchased from Beijing Weitong Lihua Experimental Animal Technology Co., Ltd., and each mouse was subcutaneously inoculated with 500,000 A20 cells on the right rear side of the mouse's back. Treatment was initiated when tumors grew to a volume of approximately 62 $mm^3$.

CN401 was orally administered at 150 mg/kg twice a day for 3 weeks. Anti-PD-L1 antibody(Atezolizumab) was administered by intraperitoneal injection, 3 times a week at a dose of 5 mg/kg for 3 weeks. The control group was treated with vehicle (0.5% MC and PBS) without active product. Tumor size and body weight were measured three times a week. This animal experiment was carried out in accordance with the requirements of AAALAC, and was approved by Bikai Animal Experiment Center IACUC to conduct this animal experiment.

Table 10

| Group | Amount | Method of administration |
|---|---|---|
| 1 | 8 | Vehicle control group(PBS i.p. biw×3weeks +0.5%MC p.o. bid×3 weeks) |
| 2 | 8 | CN401 150mg/kg, p.o. bid×3 weeks |
| 3 | 8 | Anti-PD-L1 Ab (atezolizumab) 5mg/kg, i.p. biw×3 weeks |
| 4 | 8 | CN401 150mg/kg, p.o. bid×3 weeks + anti-PD-L1 Ab (atezolizumab) 5mg/kg, i.p. biw×3 weeks |

The results of tumor growth curves and body weight growth curves are shown in Figure 6A-Figure 6F; TGI, p-value and number of complete responding mice in the treatment group compared with the blank group at day 22 are shown in Table 11.

Result: In this study, the combination of CN401 and anti-PD-L1 antibody had a synergistic effect on tumor suppression in the A20 mouse B-cell lymphoma model (p=0.026, TGI=63%, CR=3/8). The mice showed good tolerance to the dosing regimen

. 表11

| Group# | TGI% | p value (vs vehicle control group) | CR(Number of mice with tumor regression /total amount of mice in each group) |
|---|---|---|---|
| CN401 150mg/kg | 55 | 0.034 | 2/8 |
| Anti-PD-L1 Ab 5mg/kg | 15 | 0.57 | 0/8 |
| CN401 150mg/kg+anti-PD-L1 Ab 5mg/kg | 63 | 0.026 | 3/8 |

**Example 4 Study of combination of CN202 and nab-pac and the combination of CN401, CN202 and nab-pac in EMT-6 tumor model (see Table 12 for experimental design)**

6-8 weeks old balb/c mice (18-20g) were purchased from Beijing Weitong Lihua Experimental Animal Technology Co., Ltd., and each mouse was subcutaneously inoculated with 500,000 EMT-6 cells(mouse triple-negative breast cancer cells), inoculated in the 2-3 pairs of mammary gland fat pads on the left side of the mouse chest from top to bottom. CN401 was administered orally at 150 mg/kg twice daily for 4 weeks. CN202 and nab-paclitaxel were administered by intraperitoneal injection. CN202 was administered 3 times a week at a dose of 15 mg/kg for 2 weeks; with the same dose, CN202 was administered once a week for 2 weeks. Nab-paclitaxel was administered twice weekly at a dose of 10 mg/kg for 2 weeks. The control group was treated with vehicle (0.5% MC and PBS) without active product. Tumor size and body weight were measured three times a week. This animal experiment was carried out in accordance with the requirements of AAALAC, and was approved by Bikai Animal Experiment Center IACUC to conduct this animal experiment.

Table 12

| Group | Amount | Group | Plan of administration |
|---|---|---|---|
| 1 | 8 | Vehicle control group | Vehical (PBS i.p., three times a week×2 week +0.5%MC oral twice a day×2week) |
| 2 | 8 | CN202 15mg/kg | CN202 15mg/kg, i.p., (three times a week × 2 week+ once a week × 2 week) |
| 3 | 8 | CN401 150mg/kg | CN401 150 mg/kg, oral, twice a week × 4 week |
| 4 | 8 | Nab-paclitaxel 10mg/kg | Nab-paclitaxel 10 mg/kg, i.p., twice a week × 2 week |
| 5 | 8 | CN202 15mg/kg+CN401 150mg/kg | CN202 15 mg/kg, i.p., (three times a week × 2 week+ once a week × 2 week) + CN401 150 mg/kg, oral, twice a week × 4 week |
| 6 | 8 | CN202 15 mg/kg + Nab-paclitaxel 10 mg/kg | CN202 15 mg/kg, i.p., (three times a week × 2 week+ once a week × 2 week) + Nab-paclitaxel 10mg/kg, i.p., twice a week × 2 week |
| 7 | 8 | CN202 15 mg/kg +CN401 150 mg/kg + Nab-paclitaxel 10 mg/kg | CN202 15 mg/kg, i.p., (three times a week × 2 week+ once a week × 2 week) + CN401 150 mg/kg, oral, twice a day ×4 week+ Nab-paclitaxel 10mg/kg, i.p., twice a week × 2 week |

The results of tumor growth curve and body weight growth curve are shown in FIG. 7.

Results: This study found that in the orthotopic model of triple-negative breast cancer in EMT-6 mice, the combination of CN202 and nab-paclitaxel had a certain synergistic effect on tumor suppression (p=0.06, TGI=48% (at 14 days), CR=3/8 (at 80 days)); CN401, CN202 combined with nab-paclitaxel had a good synergistic effect on tumor suppression (p=0.02, TGI=56% (at 14 days), CR=4/8_ (within 80 days)). The dosing regimen was well tolerated by mice. The results of the tumor growth curve and body weight growth curve are shown in Figure 7A. According to the IACUC regulations of Bikai Animal Experiment Center, the individual tumor volume ≥ 2000mm$^3$ needs to be euthanized, and the results are shown in Figure 7B;comparing to the vehicle control group on the 14$^{th}$ day , p-values and the number of complete responding mice (observed up to 80 days) are shown in Table 13.

Table 13

| Group | TGI% | p value (vs vehicle control group) | CR(Number of mice with tumor regression /total amount of mice in each group) |
|---|---|---|---|
| CN202 15 mg/kg | 42 | 0.11 | 0/8 |
| CN401 150 mg/kg | 3 | 0.87 | 0/8 |
| Nab-paclitaxel 10 mg/kg | 2 | 0.9 | 0/8 |
| CN202 15 mg/kg +CN401 150 mg/kg | 28 | 0.3 | 1/8 |
| CN202 15 mg/kg + Nab-paclitaxel 10 mg/kg | 48 | 0.06 | 3/8 |
| CN202 15 mg/kg +CN401 150 mg/kg + Nab-paclitaxel 10 mg/kg | 56 | 0.02 | 4/8 |

**Claims**

1. A pharmaceutical combination, comprising a phosphoinositide 3-kinase (PI3K) inhibitor and a second therapeutic agent, wherein the second therapeutic agent is an immune checkpoint inhibitor, a TGFβ inhibitor, a bifunctional immune checkpoint TGFβ inhibitors or combinations thereof.

2. The pharmaceutical combination according to claim 1, wherein the immune checkpoint inhibitor comprises an agent capable of blocking the interaction between programmed death 1 (PD-1) and programmed death ligand 1 (PD-L1) .

3. The pharmaceutical combination according to any one of claims 1-2, wherein the immune checkpoint inhibitor comprises a programmed death ligand 1 (PD-L1) and/or programmed death 1 (PD-1 ) inhibitor.

4. The pharmaceutical combination according to any one of claims 1-3, wherein the bifunctional immune checkpoint/TGFβ inhibitor comprises a PD-L1/TGFβ dual inhibitor, or a PD-1/TGFβ dual inhibitor.

5. The pharmaceutical combination according to any one of claims 1-4, wherein the combination comprises:

   1) the combination of PD-L1 inhibitor and PI3K inhibitor;
   2) the combination of TGFβ inhibitor and PI3K inhibitor;
   3) the combination of PD-1 inhibitor, TGFβ inhibitor and PI3K inhibitor;
   4) the combination of PD-L1 inhibitor, TGFβ inhibitor and PI3K inhibitor;
   5) the combination of PD-L1/TGFβ dual inhibitor and PI3K inhibitor; or
   6) the combination of PD-1/TGFβ dual inhibitor and PI3K inhibitor.

6. The pharmaceutical combination according to any one of claims 1-5, wherein the immune checkpoint inhibitor comprises a nucleic acid, an antibody or an antigen-binding fragment thereof, a fusion protein or a chemical compound.

7. The pharmaceutical combination according to claim 6, wherein said antibody is selected from the group consisting of: human antibody, humanized antibody, chimeric antibody, multi-specific antibody, monoclonal antibody and polyclonal antibody.

8. The pharmaceutical combination according to any one of claims 6-7, wherein the antigen-binding fragment is selected from Fab, Fab', $F(ab')_2$, Fv, scFv, bispecific antibody, Fd, dAb, VHH, large antibody and complementarity determining region (CDR) fragments.

9. The pharmaceutical combination according to any one of claims 6-8, wherein the antibody or antigen-binding fragment thereof comprises a heavy chain variable region (VH) comprising HCDR1, HCDR2 and HCDR3.

**10.** The pharmaceutical combination according to any one of claims 6-9, wherein the antibody or antigen-binding fragment thereof further comprises or does not comprise a CH1 domain.

**11.** The pharmaceutical combination according to claim 10, wherein the antibody or antigen-binding fragment thereof further comprises or does not comprise CH2 and CH3 domains.

**12.** The pharmaceutical combination according to any one of claims 6-9, wherein the antibody or antigen-binding fragment thereof further comprises an antibody heavy chain constant region.

**13.** The pharmaceutical combination according to claim 12, wherein the antibody heavy chain constant region is a human antibody heavy chain constant region.

**14.** The pharmaceutical combination according to claim 13, wherein the human antibody heavy chain constant region is selected from constant regions derived from the group consisting of IgGl, IgG2, IgG3, IgG4 and variants thereof.

**15.** The pharmaceutical combination according to any one of claims 6-14, wherein the antibody or antigen-binding fragment thereof further comprises a light chain variable region (VL) comprising LCDR1, LCDR2, LCDR3.

**16.** The pharmaceutical combination according to claim 15, wherein the antibody or antigen-binding fragment thereof further comprises a light chain constant region (CL).

**17.** The pharmaceutical combination according to any one of claims 1-16, wherein the immune checkpoint inhibitor comprises an anti-PD-L1 antibody or an antigen-binding fragment thereof, or an anti-PD-1 antibody or an antigen-binding fragment thereof.

**18.** The pharmaceutical combination according to claim 17, wherein the anti-PD-L1 antibody or antigen-binding fragment thereof comprises a heavy chain variable region of an antibody, wherein the heavy chain variable region comprises HCDR1, HCDR2, HCDR3, which has at least about 80% sequence identity to HCDR1, HCDR2, HCDR3, respectively, of atezolizumab, avelumab, BMS-936559, MPDL3280A (RG7446), or durvalumab (MEDI-4736).

**19.** The pharmaceutical combination according to any one of claims 17-18, wherein the anti-PD-L1 antibody or antigen-binding fragment thereof comprises an antibody heavy chain variable region, wherein the heavy chain variable region is separately associated with the heavy chain variable regions of the following molecules have at least about 80% sequence identity: atezolizumab, avelumab, BMS-936559, MPDL3280A, or durvalumab.

**20.** The pharmaceutical composition according to any one of claims 17-19, wherein the anti-PD-L1 antibody or antigen-binding fragment thereof comprises a heavy chain of an antibody, wherein the heavy chain is respectively associated with a heavy chain of the following molecule having at least about 80% sequence identity: Atezolizumab, Avelumab, BMS-936559, MPDL3280A, or durvalumab.

**21.** The pharmaceutical combination according to any one of claims 17-20, wherein the anti-PD-L1 antibody or antigen-binding fragment thereof further comprises an antibody light chain variable region, wherein the light chain variable region comprises LCDR1, LCDR2, LCDR3, each of which has at least about 80% sequence identity to LCDR1, LCDR2, LCDR3, respectively, of atezolizumab, avelumab, BMS-936559, MPDL3280A, or durvalumab.

**22.** The pharmaceutical combination according to any one of claims 17-21, wherein the anti-PD-L1 antibody or antigen-binding fragment thereof further comprises an antibody light chain variable region, wherein the light chain variable region have at least about 80% sequence identity to the light chain variable region of atezolizumab, avelumab, BMS-936559, MPDL3280A, or durvalumab, respectively.

**23.** The pharmaceutical combination according to any one of claims 17-22, wherein the anti-PD-L1 antibody or antigen-binding fragment thereof further comprises a light chain of the antibody, wherein the light chain is respectively associated with the light chain of the following molecule having at least about 80% sequence identity: Atezolizumab, Avelumab, BMS-936559, MPDL3280A or durvalumab.

**24.** The pharmaceutical combination according to any one of claims 17-23, wherein the anti-PD-L1 antibody or antigen-binding fragment thereof comprises: Atezolizumab, Avelumab, BMS-936559, MPDL3280A or durvalumab, or an antigen-binding fragment thereof , or a variant or biosimilar thereof, or a combination thereof.

25. The pharmaceutical combination according to claim 17, wherein the anti-PD-L1 antibody or antigen-binding fragment thereof comprises a heavy chain variable region of an antibody, wherein the heavy chain variable region comprises one or more heavy chain variable region CDRs (HCDRs) selected from the group consisting of: (a) HCDR1 having at least about 70% sequence identity to the amino acid sequence shown in SEQ ID NO: 1; (b) having at least about 70% sequence identity to SEQ ID NO:2 HCDR2 of sequence identity; and (c) HCDR3 having at least about 70% sequence identity to SEQ ID NO:3.

26. The pharmaceutical combination according to claim 25, wherein the anti-PD-L1 antibody or antigen-binding fragment thereof comprises an antibody heavy chain variable region, wherein the heavy chain variable region comprises one or more HCDRs selected from the group consisting of: (a) HCDR1 having the amino acid sequence shown in SEQ ID NO: 1 or having no more than 2 amino acid differences from the amino acid sequence shown in SEQ ID NO: 1 obtained through amino acid addition, elimination or substitution reactions HCDR1; (b) HCDR2 having the amino acid sequence shown in SEQ ID NO: 2 or HCDR2 having no more than 2 amino acid differences from the amino acid sequence shown in SEQ ID NO: 2 obtained through amino acid addition, elimination or substitution reactions; and (c) HCDR3 having the amino acid sequence shown in SEQ ID NO: 3 or HCDR3 having no more than 2 amino acid differences from the amino acid sequence shown in SEQ ID NO: 3 obtained through amino acid addition, elimination or substitution reactions.

27. The pharmaceutical combination according to claim 17, wherein the anti-PD-L1 antibody or antigen-binding fragment thereof comprises a heavy chain variable region of an antibody, wherein the heavy chain variable region comprises HCDR1, HCDR2, HCDR3, the said HCDR1 comprises an amino acid sequence with at least about 70% sequence identity to the amino acid sequence shown in SEQ ID NO: 1, said HCDR2 comprises an amino acid sequence with at least about 70% sequence identity to the amino acid sequence shown in SEQ ID NO: 2, wherein the HCDR3 comprises an amino acid sequence having at least about 70% sequence identity to the amino acid sequence set forth in SEQ ID NO:3.

28. The pharmaceutical combination according to claim 27, wherein the anti-PD-L1 antibody or antigen-binding fragment thereof comprises a heavy chain variable region of an antibody, wherein the heavy chain variable region comprises HCDR1, HCDR2, HCDR3, the said HCDR1 comprises the amino acid sequence shown in SEQ ID NO: 1 or an amino acid sequence obtained by amino acid addition, elimination or substitution reaction with no more than 2 amino acid differences from the amino acid sequence shown in SEQ ID NO: 1; HCDR2 comprises the amino acid sequence shown in SEQ ID NO: 2 or an amino acid sequence obtained by amino acid addition, elimination or substitution reaction with no more than 2 amino acid differences from the amino acid sequence shown in SEQ ID NO: 2; the HCDR3 comprising the amino acid sequence shown in SEQ ID NO: 3 or an amino acid sequence obtained by amino acid addition, elimination or substitution reaction and having no more than 2 amino acid differences from the amino acid sequence shown in SEQ ID NO: 3.

29. The pharmaceutical combination according to claim 28, wherein the anti-PD-L1 antibody or antigen-binding fragment thereof comprises an antibody heavy chain variable region, wherein the heavy chain variable region comprises an amino acid sequence selected from the group consisting of: (a) the amino acid sequence set forth in SEQ ID NO: 4; (b) an amino acid sequence at least about 85%, 90%, 95% or 99% sequence identity to the amino acid sequence set forth in SEQ ID NO: 4; and (c) an amino acid sequence having 1 or more differences from the amino acid sequence shown in SEQ ID NO: 4 obtained by addition, elimination or substitution reaction.

30. The pharmaceutical combination according to any one of claims 1-29, wherein the TGFβ inhibitor binds to TGFβ1, TGFβ2 or TGFβ3.

31. The pharmaceutical combination according to any one of claims 1-30, wherein the TGFβ inhibitor binds to TGFβ or transforming growth factor β receptor (TGFβR).

32. The pharmaceutical combination according to any one of claims 1-31, wherein the TGFβ inhibitor binds to TGFβ type I receptor (TGFβRI), TGFβ type II receptor (TGFβRII) or TGFβ type III receptor (TGFβRIII).

33. The pharmaceutical combination according to any one of claims 1-32, wherein the TGFβ inhibitor comprises: 1) an anti-TGFβ antibody or an antigen-binding fragment thereof; 2) an anti-TGFβR antibody or an antigen-binding fragment thereof; 3) small molecule TGFβ inhibitors; 4) proteins comprising transforming growth factor β receptor II (TGFβRII) or functionally active fragments thereof, or variants or biosimilars thereof, or combinations thereof.

**34.** The pharmaceutical combination according to claim 33, wherein said anti-TGFβ antibody or antigen-binding fragment thereof comprises an antibody heavy chain variable region, wherein said heavy chain variable region comprises HCDR1, HCDR2, HCDR3, each of which has at least about 80% sequence identity with HCDR1, HCDR2, and HCDR3 of the fresolimumab molecule.

**35.** The pharmaceutical combination according to any one of claims 33-34, wherein the anti-TGFβ antibody or its antigen-binding fragment comprises an antibody heavy chain variable region, wherein the heavy chain variable region has at least about 80% sequence identity to the heavy chain variable region of Fresolimumab.

**36.** The pharmaceutical combination according to any one of claims 33-34, wherein the anti-TGFβ antibody or its antigen-binding fragment comprises an antibody heavy chain, wherein the heavy chain has at least about 80% sequence identity to the heavy chain of Fresolimumab.

**37.** The pharmaceutical combination according to any one of claims 33-36, wherein said anti-TGFβ antibody or antigen-binding fragment thereof comprises an antibody's light chain variable region, wherein said light chain variable region comprises LCDR1, LCDR2 , LCDR3, each of which has at least about 80% sequence identity with LCDR1, LCDR2, and LCDR3 of the Fresolimumab.

**38.** The pharmaceutical combination according to any one of claims 33-37, wherein the anti-TGFβ antibody or its antigen-binding fragment comprises an antibody light chain variable region, wherein the light chain variable region has at least about 80% sequence identity to the light chain variable region.

**39.** The pharmaceutical combination of any one of claims 33-38, wherein the anti-TGF β antibody, or antigen-binding fragment thereof, comprises a light chain of an antibody, wherein the light chain has at least about 80% sequence identity with the light chain of a Fresolimumab molecule.

**40.** The pharmaceutical combination of any one of claims 33-39, wherein the anti-TGFβ antibody or antigen-binding fragment thereof comprises: Fresolimumab, or an antigen-binding fragment thereof, or a variant or biosimiliar thereof

**41.** The pharmaceutical combination of any one of claims 33-40, wherein the small molecule TGFβ inhibitor comprises the following compound: SB525334, SD-208, SB431542, LY2109761, LY2157299, GW788388, RepSox, SIS3, LDN-193189, EW-7197, LY364947, or a combination thereof

**42.** The pharmaceutical combination of any one of claims 33-41, wherein the TGF β RII comprises a polypeptide having a wild-type human TGF β receptor type 2 isoform A sequence, or a polypeptide having a wild-type human TGF β receptor type 2 isoform B sequence, or a polypeptide having a sequence substantially identical to an amino acid sequence thereof

**43.** The pharmaceutical combination of any one of claims 33-42, wherein the TGF βRII or a functionally active fragment thereof comprises a human TGF βRII ectodomain (ECD) or a sequence substantially identical to an amino acid sequence thereof

**44.** The pharmaceutical combination of claim 43, wherein the TGFβRII or a functionally active fragment thereof comprises:

(a) the amino acid sequence of SEQ ID NO: 6;
(b) an amino acid sequence having at least about 85% sequence identity to the amino acid sequence set forth in SEQ ID NO: 6; or
(c) has an amino acid sequence in which one or more amino acids are added, deleted and/or substituted as compared to the amino acid sequence shown in SEQ ID NO: 6.

**45.** The pharmaceutical combination of any one of claims 1-44, wherein the bifunctional immune checkpoint/TGF β inhibitor is a fusion protein.

**46.** The pharmaceutical combination of any one of claims 4-45, wherein the PD-L1/TGFβ dual inhibitor or PD-1/TGFβ dual inhibitor is a fusion protein.

**47.** The pharmaceutical combination of any one of claims 4-46, wherein the PD-L1//TGF β dual inhibitor comprises a

PD-L1 targeting moiety and a TGF β receptor domain comprising transforming growth factor β receptor II (TGFβ RII) or a functionally active fragment thereof.

**48.** The pharmaceutical combination of any one of claims 4-47, wherein the PD-L1//TGF β dual inhibitor comprises a polypeptide, wherein the polypeptide comprises at least: (i) a heavy chain variable region of an anti-PD-L1 antibody; And (ii) TGFβII or a functionally active fragment thereof.

**49.** The pharmaceutical combination of claim 48, wherein the anti-PD-L1 antibody heavy chain variable region comprises HCDR1, HCDR2, HCDR3; wherein the HCDR1 comprises an amino acid sequence having at least about 70% sequence identity to the amino acid sequence set forth in SEQ ID NO: 1, the HCDR2 comprises an amino acid sequence having at least about 70% sequence identity to the amino acid sequence set forth in SEQ ID NO: 2, and the HCDR3 comprises an amino acid sequence having at least about 70% sequence identity to the amino acid sequence set forth in SEQ ID NO: 3; or
wherein the HCDR1, HCDR2, HCDR3 have at least about 80% sequence identity to the HCDR1, HCDR2, HCDR3, respectively, of Atezolizumab, Avelumab, BMS-936559, MPDL3280A (RG7446), or durvalumab (MEDI-4736).

**50.** The pharmaceutical combination of claim 49, wherein the anti-PD-L1 antibody heavy chain variable region comprises HCDR1, HCDR2, HCDR3, and the HCDR1 comprises the amino acid sequence shown in SEQ ID NO: 1 or an amino acid sequence obtained by amino acid addition, elimination or substitution reaction and having no more than 2 amino acid differences from the amino acid sequence shown in SEQ ID NO: 1; The HCDR2 comprises the amino acid sequence represented by SEQ ID NO: 2 or an amino acid sequence obtained by amino acid addition, elimination or substitution reaction and having no more than 2 amino acid differences from the amino acid sequence represented by SEQ ID NO: 2; The HCDR3 comprises an amino acid sequence represented by SEQ ID NO: 3 or an amino acid sequence obtained by an amino acid addition, elimination or substitution reaction and having no more than 2 amino acid differences from the amino acid sequence represented by SEQ ID NO: 3.

**51.** The pharmaceutical combination of claim 50, wherein the anti-PD-L1 antibody heavy chain variable region comprises an amino acid sequence selected from the group consisting of: (a) the amino acid sequence set forth in SEQ ID NO: 4; (b) an amino acid sequence having at least about 85%, 90%, 95%, or 99% sequence identity to the amino acid sequence set forth in SEQ ID NO: 4; (c) an amino acid sequence having 1 or more differences from the amino acid sequence shown in SEQ ID NO: 4 obtained by addition, elimination or substitution; and (d) an amino acid sequence having at least about 80% sequence identity to the heavy chain variable region of Atezolizumab, Avelumab, BMS-936559, MPDL3280A, or durvalumab.

**52.** The pharmaceutical combination of claim 47, wherein the TGFβRII or a functionally active fragment thereof comprises:

(a) the amino acid sequence of SEQ ID NO: 6;
(b) an amino acid sequence having at least about 85% sequence identity to the amino acid sequence set forth in SEQ ID NO: 6; Or
(c) has an amino acid sequence in which one or more amino acids are added, deleted and/or substituted as compared to the amino acid sequence shown in SEQ ID NO: 6.

**53.** The pharmaceutical combination of claim 47, wherein the polypeptide further comprises a linker that links the C-terminus of the heavy chain variable region of the anti-PD-L1 antibody or antigen-binding fragment thereof to the N-terminus of the TGFβRII or functionally active fragment thereof.

**54.** The pharmaceutical combination according to claim 47, said polypeptide further comprising CH2, CH3 domains, said polypeptide comprising the heavy chain variable region (VH) of anti-PD-L1 antibody, CH2, CH3 domains and TGF βRII or a functionally active fragment thereof in sequence from N-terminus to C-terminus, said CH3 domain having its C-terminus connected to the N-terminus of said TGF βRII or a functionally active fragment thereof by a linker.

**55.** The pharmaceutical combination of claim 54, wherein the CH2, CH3 domains are derived from IgG.

**56.** The pharmaceutical combination of any one of claims 53-55, wherein the linker is a peptide linker.

**57.** The pharmaceutical combination of claim 56, wherein the amino acid sequence of the peptide linker is $(G_4S)_x$, wherein x is any integer from 3 to 6.

**58.** The pharmaceutical combination of claim 57, wherein the peptide linker comprises an amino acid sequence selected from the group consisting of: (a) the amino acid sequence set forth in SEQ ID NO: 5; (b) an amino acid sequence having at least about 85%, 90%, 95%, or 99% sequence identity to the amino acid sequence set forth in SEQ ID NO: 5; And (c) an amino acid sequence having 1 or more differences from the amino acid sequence shown in SEQ ID NO: 5 obtained by addition, elimination or substitution reaction.

**59.** The pharmaceutical combination of claims 48-58, wherein the polypeptide comprises an amino acid sequence selected from the group consisting of: (a) the amino acid sequence set forth in SEQ ID NO: 7; (b) an amino acid sequence having at least about 85%, 90%, 95%, or 99% sequence identity to the amino acid sequence set forth in SEQ ID NO: 7; (c) an amino acid sequence having 1 or more differences from the amino acid sequence shown in SEQ ID NO: 7 obtained by addition, elimination or substitution reactions.

**60.** The pharmaceutical combination according to any one of claims 1-59, wherein the PD-L1//TGFβ dual inhibitor comprises two polypeptides according to any one of claims 48-59.

**61.** The pharmaceutical combination according to any one of claims 1-60, wherein the PI3K inhibitor is a dual PI3K δ / γ inhibitor.

**62.** The pharmaceutical combination according to anyone of claims 1-61, wherein the said PI3K inhibitors comprise compounds as formula(IA-I), (IA-II), (IA-III), (IA-IV):

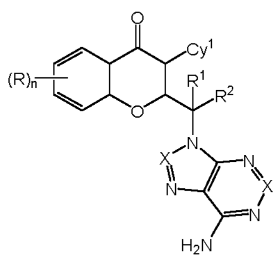
(IA-I)

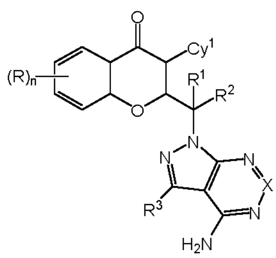
(IA-II)

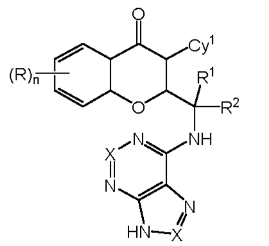
(IA-III)

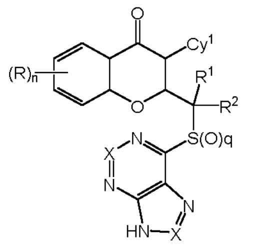
(IA-IV)

or their enantiomers, mixtures of enantiomers, mixtures of two or more diastereoisomers, isotopic variants, pharmaceutically acceptable salts, solvates, hydrates or prodrug, wherein,
each occurrence of R is independently selected from hydrogen, halogen, $-OR^a$, CN, substituted or unsubstituted $C_{1-6}$ alkyl, substituted or unsubstituted $C_{2-6}$ alkenyl, substituted or unsubstituted $C_{2-6}$ alkynyl, substituted or unsubstituted $C_{3-8}$ cycloalkyl, and substituted or unsubstituted heterocyclic group;
$R^1$ and $R^2$ may be the same or different and are independently selected from hydrogen, halogen, and substituted or unsubstituted $C_{1-6}$ alkyl, or both $R^1$ and $R^2$ directly bound to a common atom, may be joined to form an oxo group (=O) or a substituted or unsubstituted saturated or unsaturated 3-10 member ring, including the carbon atom to which $R^1$ and $R^2$ are bound, which may optionally include one or more heteroatoms which may be the same or different and are selected from O, $NR^a$ and S;
$Cy^1$ is selected from substituted or unsubstituted $C_{3-8}$ cycloalkyl, substituted or unsubstituted 3 to 15 membered heterocyclic groups having at least one heteroatom selected from N, O and S, substituted or unsubstituted $C_{6-20}$ aryl and substituted or unsubstituted 5 to 14 membered heteroaryl monocyclic groups with one or more heteroatoms selected from N, O and S;
each occurrence of $R^a$ may be the same or different and are independently selected from hydrogen, halogen, hydroxy, cyano, substituted or unsubstituted $C_{1-6}$alkyl, $-NR^cR^d$ , wherein $R^c$ and $R^d$ are independently hydrogen, halogen, hydroxy, cyano, substituted or unsubstituted $C_{1-6}$alkyl, and $C_{1-6}$alkoxy, and $-OR^c$ , wherein $R^c$ is substituted or unsubstituted $C_{1-6}$alkyl ,
n is an integer from 1 to 4;
q is 0, 1 or 2;
each occurrence of X is independently selected from $CR^3$ or N;
and each occurrence of $R^3$ is independently selected from hydrogen, hydroxy, halogen, carboxyl, cyano, nitro, substituted or unsubstituted $C_{1-8}$ alkyl, substituted or unsubstituted $C_{1-8}$ alkoxy, substituted or unsubstituted $C_{2-10}$ alkenyl, substituted or unsubstituted $C_{2-10}$ alkynyl, substituted or unsubstituted $C_{6-20}$ aryl, substituted or unsubstituted $C_{6-20}$ aryl $C_{1-8}$alkyl, substituted or unsubstituted $C_{3-20}$ cycloalkyl,
substituted or unsubstituted $C_{3-20}$ cycloalkyl $C_{1-8}$ alkyl, substituted or unsubstituted $C_{3-8}$ cycloalkenyl $C_{1-8}$ alkyl,

substituted or unsubstituted $C_{3-8}$ cycloalkene, substituted or unsubstituted 5-14-membered heteroaryl with one or more heteroatoms selected from N, O and S, substituted or unsubstituted with one or more heteroatoms selected from N, O, S 5-14 membered heteroaryl $C_{1-8}$ alkyl, substituted or unsubstituted 3 to 15 membered heterocycles having at least one heteroatom selected from N, O and S, substituted or unsubstituted 3 to 15 membered heterocyclyl $C_{1-8}$ alkyl rings having at least one heteroatom selected from N, O and S, substituted or unsubstituted guanidine, - $COOR^x$, $-C(O)R^x$, $-C(S)R^x$, $-C(O)NR^xR^y$, $-C(O)ONR^xR^y$, $-NR^yR^z$, $-NR^xCONR^yR^z$, - $N(R^x)SOR^y$, - $N(R^x)SO_2R^y$, $-(=N-N(R^x)R^y)$, - $NR^xC(O)OR^y$, $-NR^xR^y$, $-NR^xC(O)R^y$-, - $NR^xC(S)R^y$ $-NR^xC(S)NR^yR^z$, $-SONR^xR^y$-, $-S0_2NR^xR^y$-, $-OR^x$, $-OR^xC(O)NR^yR^z$, - $OR^xC(O)OR^y$-, $-OC(O)R^x$, $-OC(O)NR^xR^y$, - $R^xNR^yC(O)R^z$, $-R^xOR^y$, $-R^xC(O)OR^y$, - $R^xC(O)NR^yR^z$, $-R^xC(0)R^x$, $-R^xOC(O)R^y$, $-SR^x$, $-SOR^x$, - $SO_2R^x$, $-ONO_2$, wherein $R^x$, $R^y$ and $R^z$ in each of the above groups can be hydrogen, substituted or unsubstituted $C_{1-8}$ alkyl, substituted or unsubstituted $C_{1-8}$ alkoxy, substituted or unsubstituted $C_{2-10}$ alkenyl, substituted or unsubstituted $C_{2-12}$ alkynyl, substituted or unsubstituted $C_{6-20}$ aryl, substituted or unsubstituted $C_{6-20}$ aryl $C_{1-8}$ alkyl , substituted or unsubstituted $C_{3-20}$ cycloalkyl, substituted or unsubstituted $C_{3-20}$ cycloalkyl$C_{1-8}$ alkyl, substituted or unsubstituted $C_{3-8}$ cycloalkenyl, substituted or unsubstituted 5 to 14 membered heteroaryl with one or more heteroatoms selected from N, O and S, substituted or unsubstituted heteroaryl with one or more heteroatoms selected from N, O and S atomic 5 to 14 membered heteroaryl $C_{1-8}$ alkyl, substituted or unsubstituted 3 to 15 membered heterocycle having at least one heteroatom selected from N, O and S, substituted or unsubstituted 3 to 15 membered heterocyclyl $C_{1-8}$ alkyl ring having at least one heteroatom selected from N, O and S or substituted or unsubstituted amino, or any two of $R^x$, $R^y$ and $R^z$ may be joined to form a substituted or unsubstituted saturated or unsaturated 3-10 membered ring, which may optionally include heteroatoms which may be the same or different and are selected from O, $NR^x$ or S, wherein $R^x$ is hydrogen or substituted or unsubstituted alkyl.

**63.** The pharmaceutical combination according to any one of claims 1-62, wherein the PI3K inhibitor comprises compounds as formula (IA-V):

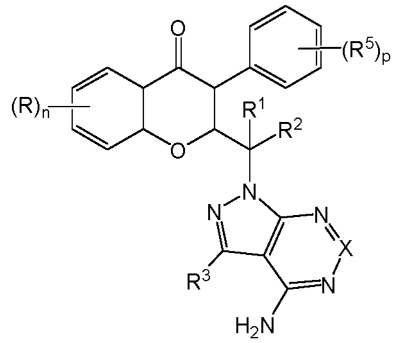

(IA-V)

or their enantiomers, mixtures of enantiomers, mixtures of two or more diastereoisomers, isotopic variants, pharmaceutically acceptable salts, solvates, hydrates or prodrug, wherein

each occurrence of R is independently selected from hydrogen, halogen, $-OR^a$, CN, substituted or unsubstituted $C_{1-6}$ alkyl, substituted or unsubstituted $C_{2-6}$ alkenyl, substituted or unsubstituted $C_{2-6}$ alkynyl, substituted or unsubstituted $C_{3-8}$ cycloalkyl, and substituted or unsubstituted heterocyclic group;

$R^1$ and $R^2$ may be the same or different and are independently selected from hydrogen, halogen, and substituted or unsubstituted $C_{1-6}$ alkyl, or both $R^1$ and$R^2$ directly bound to a common atom, may be joined to form an oxo group (=O) or a substituted or unsubstituted saturated or unsaturated 3-10 member ring, including the carbon atom to which $R^1$ and $R^2$ are bound, which may optionally include one or more heteroatoms which may be the same or different and are selected from O, $NR^a$ and S;

each occurrence of X is independently selected from $CR^3$ or N; and

each occurrence of $R^3$ is independently selected from hydrogen, hydroxy, halogen, carboxyl, cyano, nitro, substituted or unsubstituted $C_{1-8}$ alkyl, substituted or unsubstituted $C_{1-8}$ alkoxy, substituted or unsubstituted $C_{2-10}$ alkenyl, substituted or unsubstituted $C_{2-10}$ alkynyl, substituted or unsubstituted $C_{6-20}$ aryl, substituted or unsubstituted $C_{6-20}$ aryl $C_{1-8}$alkyl, substituted or unsubstituted $C_{3-20}$ cycloalkyl,

substituted or unsubstituted $C_{3-20}$ cycloalkyl $C_{1-8}$ alkyl, substituted or unsubstituted $C_{3-8}$ cycloalkenyl $C_{1-8}$ alkyl, substituted or unsubstituted $C_{3-8}$ cycloalkene, substituted or unsubstituted 5-14-membered heteroaryl with one or more heteroatoms selected from N, O and S, substituted or unsubstituted with one or more heteroatoms selected from N, O, S 5-14 membered heteroaryl C1-8 alkyl, substituted or unsubstituted 3 to 15 membered

heterocycles having at least one heteroatom selected from N, O and S, substituted or unsubstituted 3 to 15 membered heterocyclyl $C_{1-8}$ alkyl rings having at least one heteroatom selected from N, O and S, substituted or unsubstituted guanidine, - $COOR^x$, $-C(O)R^x$, $-C(S)R^x$, $-C(O)NR^xR^y$, $-C(O)ONR^xR^y$, $-NR^yR^z$, $-NR^xCONR^yR^z$, - $N(R^x)SOR^y$, - $N(R^x)SO_2R^y$, $-(=N-N(R^x)R^y)$, - $NR^xC(O)OR^y$, $-NR^xR^y$, $-NR^xC(O)R^y$-, - $NR^xC(S)R^y$ $-NR^xC(S)NR^yR^z$, $-SONR^xR^y$-, $-SO_2NR^xR^y$-, $-OR^x$, $-OR^xC(O)NR^yR^z$, - $OR^xC(O)OR^y$-, $-OC(O)R^x$, $-OC(O)NR^xR^y$, - $R^xNR^yC(O)R^z$, $-R^xOR^y$, $-R^xC(O)OR^y$, - $R^xC(O)NR^yR^z$, $-R^xC(O)R^x$, $-R^xOC(O)R^y$, $-SR^x$, $-SOR^x$, - $SO_2R^x$, $-ONO_2$, wherein $R^x$, $R^y$ and $R^z$ in each of the above groups can be hydrogen, substituted or unsubstituted $C_{1-8}$ alkyl, substituted or unsubstituted $C_{1-8}$ alkoxy, substituted or unsubstituted $C_{2-10}$ alkenyl, substituted or unsubstituted $C_{2-12}$ alkynyl, substituted or unsubstituted $C_{6-20}$ aryl, substituted or unsubstituted $C_{6-20}$ aryl $C_{1-8}$ alkyl , substituted or unsubstituted $C_{3-20}$ cycloalkyl, substituted or unsubstituted $C_{3-20}$ cycloalkyl$C_{1-8}$ alkyl, substituted or unsubstituted $C_{3-8}$ cycloalkenyl, substituted or unsubstituted 5 to 14 membered heteroaryl with one or more heteroatoms selected from N, O and S, substituted or unsubstituted heteroaryl with one or more heteroatoms selected from N, O and S atomic 5 to 14 membered heteroaryl $C_{1-8}$ alkyl, substituted or unsubstituted 3 to 15 membered heterocycle having at least one heteroatom selected from N, O and S, substituted or unsubstituted 3 to 15 membered heterocyclyl $C_{1-8}$ alkyl ring having at least one heteroatom selected from N, O and S or substituted or unsubstituted amino, or any two of $R^x$, $R^y$ and $R^z$ may be joined to form a substituted or unsubstituted saturated or unsaturated 3-10 membered ring, which may optionally include heteroatoms which may be the same or different and are selected from O, $NR^x$ or S, wherein $R^x$ is hydrogen or substituted or unsubstituted alkyl,

each occurrence of $R^5$ is hydrogen, $C_{1-6}$ alkyl or halogen,

n is 0, 1, 2, 3 or 4; and

Pis 0, 1, 2, 3, 4 or 5.

**64.** The pharmaceutical combination according to any one of claims 1-62, wherein the PI3K inhibitor comprises compounds as formula (IA-VI):

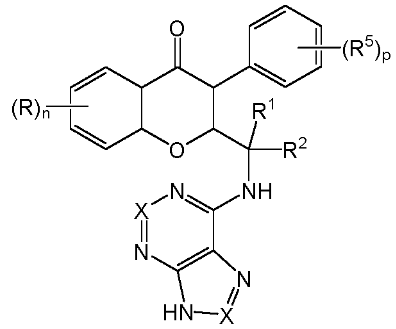

(IA-VI)

or their enantiomers, mixtures of enantiomers, mixtures of two or more diastereoisomers, isotopic variants, pharmaceutically acceptable salts, solvates, hydrates or prodrug, wherein

each occurrence of R is independently selected from hydrogen, halogen, $-OR^a$, CN, substituted or unsubstituted $C_{1-6}$ alkyl, substituted or unsubstituted $C_{2-6}$ alkenyl, substituted or unsubstituted $C_{2-6}$ alkynyl, substituted or unsubstituted $C_{3-8}$ cycloalkyl, and substituted or unsubstituted heterocyclic group;

$R^1$ and $R^2$ may be the same or different and are independently selected from hydrogen, halogen, and substituted or unsubstituted $C_{1-6}$ alkyl, or both $R^1$ and$R^2$ directly bound to a common atom, may be joined to form an oxo group (=O) or a substituted or unsubstituted saturated or unsaturated 3-10 member ring, including the carbon atom to which $R^1$ and $R^2$ are bound, which may optionally include one or more heteroatoms which may be the same or different and are selected from O, $NR^a$ and S;

each occurrence of X is independently selected from $CR^3$ or N; and

each occurrence of $R^3$ is independently selected from hydrogen, hydroxy, halogen, carboxyl, cyano, nitro, substituted or unsubstituted $C_{1-8}$ alkyl, substituted or unsubstituted $C_{1-8}$ alkoxy, substituted or unsubstituted $C_{2-10}$ alkenyl, substituted or unsubstituted $C_{2-10}$ alkynyl, substituted or unsubstituted $C_{6-20}$ aryl, substituted or unsubstituted $C_{6-20}$ aryl $C_{1-8}$alkyl, substituted or unsubstituted $C_{3-20}$ cycloalkyl,

substituted or unsubstituted $C_{3-20}$ cycloalkyl $C_{1-8}$ alkyl, substituted or unsubstituted $C_{3-8}$ cycloalkenyl $C_{1-8}$ alkyl, substituted or unsubstituted $C_{3-8}$ cycloalkene, substituted or unsubstituted 5-14-membered heteroaryl with one or more heteroatoms selected from N, O and S, substituted or unsubstituted with one or more heteroatoms selected from N, O, S 5-14 membered heteroaryl $C_{1-8}$ alkyl, substituted or unsubstituted 3 to 15 membered heterocycles having at least one heteroatom selected from N, O and S, substituted or unsubstituted 3 to 15

membered heterocyclyl $C_{1-8}$ alkyl rings having at least one heteroatom selected from N, O and S, substituted or unsubstituted guanidine, - $COOR^x$, $-C(O)R^x$, $-C(S)R^x$, $-C(O)NR^xR^y$, $-C(O)ONR^xR^y$, $-NR^yR^z$, $-NR^xCONR^yR^z$, - $N(R^x)SOR^y$, - $N(R^x)SO_2R^y$, $-(=N-N(R^x)R^y)$, - $NR^xC(O)OR^y$, $-NR^xR^y$, $-NR^xC(O)R^y-$, - $NR^xC(S)R^y$ $-NR^xC(S)NR^yR^z$, $-SONR^xR^y-$, $-S0_2NR^xR^y-$, $-OR^x$, $-OR^xC(O)NR^yR^z$, - $OR^xC(O)OR^y-$, $-OC(O)R^x$, $-OC(O)NR^xR^y$, - $R^xNR^yC(O)R^z$, $-R^xOR^y$, $-R^xC(O)OR^y$, - $R^xC(O)NR^yR^z$, $-R^xC(0)R^x$, $-R^xOC(O)R^y$, $-SR^x$, $-SOR^x$, - $SO_2R^x$, $-ONO_2$, wherein $R^x$, $R^y$ and $R^z$ in each of the above groups can be hydrogen, substituted or unsubstituted $C_{1-8}$ alkyl, substituted or unsubstituted $C_{1-8}$ alkoxy, substituted or unsubstituted $C_{2-10}$ alkenyl, substituted or unsubstituted $C_{2-12}$ alkynyl, substituted or unsubstituted $C_{6-20}$ aryl, substituted or unsubstituted $C_{6-20}$ aryl $C_{1-8}$ alkyl , substituted or unsubstituted $C_{3-20}$ cycloalkyl, substituted or unsubstituted $C_{3-20}$ cycloalkyl$C_{1-8}$ alkyl, substituted or unsubstituted $C_{3-8}$ cycloalkenyl, substituted or unsubstituted 5 to 14 membered heteroaryl with one or more heteroatoms selected from N, O and S, substituted or unsubstituted heteroaryl with one or more heteroatoms selected from N, O and S atomic 5 to 14 membered heteroaryl $C_{1-8}$ alkyl, substituted or unsubstituted 3 to 15 membered heterocycle having at least one heteroatom selected from N, O and S, substituted or unsubstituted 3 to 15 membered heterocyclyl $C_{1-8}$ alkyl ring having at least one heteroatom selected from N, O and S or substituted or unsubstituted amino, or any two of $R^x$, $R^y$ and $R^z$ may be joined to form a substituted or unsubstituted saturated or unsaturated 3-10 membered ring, which may optionally include heteroatoms which may be the same or different and are selected from O, $NR^x$ or S, wherein $R^x$ is hydrogen or substituted or unsubstituted alkyl,
each occurrence of $R^5$ is hydrogen, $C_{1-6}$ alkyl or halogen,
n is 0, 1, 2, 3 or 4; and
Pis 0, 1, 2, 3, 4 or 5.

**65.** The pharmaceutical combination according to any one of claims 1-64, wherein R is hydrogen, halogen, substituted or unsubstituted $C_{1-6}$ alkyl or $OR^a$.

**66.** The pharmaceutical combination according to 62, wherein $R^a$ is alkyl.

**67.** The pharmaceutical combination according to any one of claims 62-66, wherein $Cy^1$ is selected from:

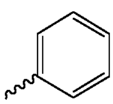 、 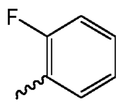 、 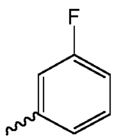 、 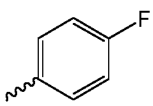 、 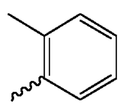 or 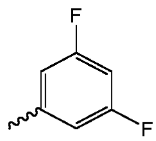 .

**68.** The pharmaceutical combination according to any one of claims 62-67, wherein $R^1$ and $R^2$ each independently represent hydrogen or substituted or unsubstituted $C_{1-6}$ alkyl.

**69.** The pharmaceutical combination according to any one of claims 62-68, wherein $R^3$ is iodo, cyano, substituted unsubstituted alkyl, substituted or unsubstituted alkynyl, substituted or unsubstituted aryl, substituted or unsubstituted heteroaryl.

**70.** The pharmaceutical combination according to any one of claims 62-69, wherein X is $CR^3$ and each occurrence of $R^3$ is independently hydrogen, halogen, hydroxyl or $NH_2$.

**71.** The pharmaceutical combination according to any one of claims 62-70, wherein $R^3$ is hydrogen.

**72.** The pharmaceutical combination according to any one of claims 1-71, wherein said PI3K inhibitors comprise:

2-[(6-Amino-9H-purin-9-yl) methyl]-6-bromo-3-phenyl-4H-chromen-4-one;
6-Bromo-2-(morpholinomethyl)-3-phenyl-4H-chromen-4-one;
6-Bromo-2-(morpholinomethyl)-3-phenyl-4H-chromen-4-one hydrochloride;
2-[(6-Amino-9H-purin-9-yl) methyl]-3-phenyl-4H-chromen-4-one;
2-(Morpholinomethyl)-3-phenyl-4H-chromen-4-one;
2-(Morpholinomethyl)-3-phenyl-4H-chromen-4-one hydrochloride;
2-[(1H-Benzo[d]imidazol-1-yl) methyl]-6-bromo-3-phenyl-4H-chromen-4-one;
6-Bromo-2-[(4-methyl-1H-benzo[d]imidazol-1-yl) methyl]-3-phenyl-4H-chromen-4-one;
2- [( 1 H-benzo [d] imidazol- 1 -yl) methyl] -3-phenyl-4H-chromen-4-one;
2-[(4-methyl- 1 H-benzo [d] imidazol- 1 -yl)methyl] -3-phenyl-4H-chromen-4-one;

2-[(6-Chloro-9H-purin-9-yl)methyl]-3-phenyl-4H-chromen-4-one;
6-Bromo-2-[(6-chloro-9H-purin-9-yl)methyl]-3-phenyl-4H-chromen-4-one;
2-((9H-Purin-6-ylthio)methyl)-3-phenyl-4H-chromen-4-one;
2- [( 1 H-Imidazol- 1 -yl)methyl] -3-phenyl-4H-chromen-4-one ;
2-[(9H-Purin-6-ylthio)methyl]-6-bromo-3-phenyl-4H-chromen-4-one;
2-((4-Amino-1H-pyrazolo[3,4-d]pyrimidin-1-yl)methyl)-6-bromo-3-phenyl-4H-chromen-4-one ;
2-[(6-Amino-9H-purin-9-yl)methyl]-6-bromo-3-(4-fluorophenyl)-4H-chromen-4-one ;
2- [(6-Arnino-9H-purin-9-yl)methyl]-3-(4-fluorophenyl)-4H-chromen-4-one ; 6-Bromo-3-(4-fluorophenyl)-2-(morpholinomethyl)-4H-chromen-4-one ; 6-Bromo-3-(4-fluorophenyl)-2-(morpholinomethyl)-4H-chromen-4-one hydro chloride;
3- (4-fluorophenyl)-2-(morpholinomethyl)-4H-chromen-4-one ; 3-(4-fluorophenyl)-2-(morpholinomethyl)-4H-chromen-4-one hydrochloride ;
2-[(6-Amino-9H-purin-9-yl)methyl]-6-bromo-3-o-tolyl-4H-chromen-4-one;
7-[(6-Bromo-4-oxo-3-phenyl-4H-chromen-2-yl)methyl]-1,3-dimethyl-1H-purine- 2,6(3H,7H)-dione;
2-(1-(6-Amino-9H-purin-9-yl)ethyl)-6-bromo-3-phenyl-4H-chromen-4-one;
2-(1-(9H-Purin-6-ylthio)ethyl)-6-bromo-3-phenyl-4H-chromen-4-one;
2-( 1 -(6-Amino-9H-purin-9-yl)ethyl)-3-phenyl-4H-chromen-4-one;
(S)-2-( 1 -(9H-purin-6-ylamino)ethyl)-6-bromo-3-phenyl-4H-chromen-4-one ;
2-((9H-purin-6-ylamino)methyl)-6-bromo-3-phenyl-4H-chromen-4-one ;
2-( 1 -(4-amino- 1 H-pyrazolo [3 ,4-d]pyrimidin- 1 -yl)ethyl)-6-bromo-3-phenyl-4H-chromen-4- one ;
2-((6-Amino-9H-purin-9-yl)methyl)-6-methoxy-3-phenyl-4H-chromen-4-one;
2-(1-(6-Amino-9H-purin-9-yl)ethyl)-6-bromo-3-(2-fluorophenyl)-4H-chromen-4-one;
2-((6-Amino-9H-purin-9-yl)methyl)-6-bromo-3-(2-fluorophenyl)-4H-chromen-4-one ;
2-( 1 -(4-Amino- 1 H-pyrazolo [3,4-d]pyrimidin- 1 -yl)ethyl)-3 -phenyl-4H-chromen-4-one ;
2-( 1 -(6-Amino-9H-purin-9-yl) propyl)-3-phenyl-4H-chromen-4-one; 2-(1-(6-Amino-9H-purin-9-yl)ethyl)-3 -(3 -fluorophenyl)-4H-chromen-4-one;
2-((6-Amino-9H-purin-9-yl)methyl)-3-(2-fluorophenyl)-4H-chromen-4-one ;
2-( 1 -(6-Amino-9H-purin-9-yl)ethyl)-3-(2-fluorophenyl)-4H-chromen-4-one ;
2-(1-(6-Amino-9H-purin-9-yl)propyl)-3-(2-fluorophenyl)-4H-chromen-4-one;
2-(1-(6-amino-9H-purin-9-yl)propyl)-3-(3-fluorophenyl)-4H-chromen-4-one;
2-(1-(6-Amino-9H-purin-9-yl)propyl)-3-(4-fluorophenyl)-4H-chromen-4-one ;
2-(1-(6-amino-9H-purin-9-yl)propyl)-6-fluoro-3-phenyl-4H-chromen-4-one;
2-(1-(6-Amino-9H-purin-9-yl)ethyl)-3-(4-fluorophenyl)-4H-chromen-4-one;
2-(1-(6-Amino-9H-purin-9-yl)ethyl)-6-fluoro-3-phenyl-4H-chromen-4-one;
2-(1-(4-Amino-3-(3-methoxyphenyl)-1H-pyrazolo[3,4-d]pyrimidin-1-yl)ethyl)-3-phenyl-4H-chromen-4-one;
2-( 1 -(4-Amino-3-(3-hydroxyphenyl)- 1 H-pyrazolo [3 ,4-d]pyrimidin- 1 -yl)ethyl)-3 -phenyl-4H- chromen-4-one;
2-((9H-purin-6-ylamino)methyl)-3-phenyl-4H-chromen-4-one;
2-( 1 -(6-Amino-9H-purin-9-yl)ethyl)-3-o-tolyl-4H-chromen-4-one;
2-((9H-purin-6-ylamino)methyl)-3-(2-fluorophenyl)-4H-chromen-4-one;
2-((9H-purin-6-ylamino)methyl)-3-(3-fluorophenyl)-4H-chromen-4-one;
(S)-2-(1-(9H-purin-6-ylamino)ethyl)-3-(3-fluorophenyl)-4H-chromen-4-one;
2-(1-(6-amino-9H-purin-9-yl)ethyl)-6-fluoro-3-(2-fluorophenyl)-4H-chromen-4-one;
2-(1-(6-Amino-9H-purin-9-yl)ethyl)-3-(3,5-difluorophenyl)-4H-chromen-4-one;
2-(1-(6-amino-9H-purin-9-yl)ethyl)-6-fluoro-3-(3-fluorophenyl)-4H-chromen-4-one;
2-(1-(4-amino-3-(3-methoxyphenyl)-1H-pyrazolo[3,4-d]pyrimidin-1-yl)ethyl)-3-(3-fluorophenyl)-4H-chromen-4-one;
2-( 1 -(4-amino-3-(3-hydroxyphenyl)- 1 H-pyrazolo [3, 4-d] pyrimidin-1-yl) ethyl)-3-(3-fluorophenyl)-4H-chromen-4-one;
2-((4-Amino-3-(3-methoxyphenyl)-lH-pyrazolo [3, 4-d] pyrimidin-1-yl) methyl)-3-phenyl- 4H-chromen-4-one;
2-((4-Amino-3-(3-hydroxyphenyl)-lH-pyrazolo [3, 4-d] pyrimidin-1-yl) methyl)-3-phenyl- 4H-chromen-4-one;
2-((4-amino-3-(3-methoxyphenyl)-lH-pyrazolo [3, 4-d] pyrimidin-1-yl) methyl)-3-(3-fluorophenyl)-4H-chromen-4-one;
2-((4-amino-3-(3-hydroxyphenyl)-lH-pyrazolo [3, 4-d] pyrimidin-1-yl) methyl)-3-(3-fluorophenyl)-4H-chromen-4-one;
(R)-2-( 1 -(9H-purin-6-ylamino) ethyl)-3-(3-fluorophenyl)-4H-chromen-4-one;
(S)-2-(1-(9H-purin-6-ylamino) ethyl)-6-fluoro-3-phenyl-4H-chromen-4-one;
2-((4-Amino-3-iodo-lH-pyrazolo [3, 4-d] pyrimidin-1-yl) methyl)-3-phenyl-4H-chromen-4-one;
2-(1-(4-amino-3-iodo-1H-pyrazolo [3, 4-d] pyrimidin-1-yl) ethyl)-3-phenyl-4H-chromen-4- one;

2-(1-(4-amino-3-iodo-1H-pyrazolo [3, 4-d] pyrimidin-1-yl) ethyl)-3-(3-fluorophenyl)-4H-chromen-4-one;
2-((4-amino-3-iodo-lH-pyrazolo [3, 4-d] pyrimidin-1-yl) methyl)-6-fluoro-3-phenyl-4H-chromen-4-one;
2-(1-(4-amino-3-iodo-1H-pyrazolo [3, 4-d] pyrimidin-1-yl) ethyl)-6-fluoro-3-phenyl-4H-chromen-4-one;
2-(1-(4-amino-3-iodo-1H-pyrazolo [3, 4-d] pyrimidin-1-yl) ethyl)-6-fluoro-3-(3- fluorophenyl)-4H-chromen-4-one;
2-(1-(4-ammo-3-iodo-1H-pyrazolo [3, 4-d] pyrimidin-1-yl) propyl)-3-(3-fluorophenyl)-4H-chromen-4-one;
2-((4-amino-3-(pyridin-3-yl)-lH-pyrazolo [3, 4-d] pyrimidin-1-yl) methyl)-3-phenyl-4H-chromen-4-one;
2-((4-amino-3-(3-hydroxyprop-1-ynyl)-1H-pyrazolo [3, 4-d] pyrimidin-1-yl) methyl)-3- phenyl-4H-chromen-4-one;
2-((4-amino-3-(lH-pyrazol-4-yl)-lH-pyrazolo [3, 4-d] pyrimidin-1-yl) methyl)-3-phenyl-4H-chromen-4-one;
2-((4-amino-3-(3-(hydroxymethyl) phenyl)- 1H-pyrazolo [3, 4-d] pyrimidin-1-yl) methyl)-3-phenyl-4H-chromen-4-one;
2-((4-amino-3-(lH-indazol-4-yl)-lH-pyrazolo [3, 4-d] pyrimidin-1-yl) methyl)-3-phenyl-4H-chromen-4-one;
2-((4-amino-3-(3-fluorophenyl)-lH-pyrazolo [3, 4-d] pyrimidin-1-yl) methyl)-3-phenyl-4H-chromen-4-one
2-((4-amino-3-(3-hydroxypropyl)-l H-pyrazolo [3, 4-d] pyrimidin-1-yl) methyl)-3-phenyl- 4H-chromen-4-one;
N-(3-(4-amino-1-((4-oxo-3-phenyl-4H-chromen-2-yl) methyl)- 1H-pyrazolo [3, 4-d] pyrimidin-3-yl) phenyl) acetamide;
2-((4-amino-3-(3-fluoro-5-methoxyphenyl)-1 H-pyrazolo [3, 4-d] pyrimidin-1-yl) methyl)-3-phenyl-4H-chromen-4-one;
2-((4-amino-3-(3-fluoro-5-hydroxyphenyl)-lH-pyrazolo [3, 4-d] pyrimidin-1-yl) methyl)-3-phenyl-4H-chromen-4-one;
2-((4-amino-3-(3-fluoro-5-methoxyphenyl)- 1 H-pyrazolo [3, 4-d] pyrimidin-1-yl) methyl)-3-(3-fluorophenyl)-4H-chromen-4-one;
2-((4-amino-3-(3-fluoro-5-hydroxyphenyl)-lH-pyrazolo [3, 4-d] pyrimidin-1-yl) methyl)-3- (3-fluorophenyl)-4H-chromen-4-one;
2-( 1 -(4-amino-3-( 1 H-pyrazol-4-yl)- 1 H-pyrazolo [3, 4-d] pyrimidin-1-yl) ethyl)-3-phenyl-4H- chromen-4-one;
2-(1-(4-amino-3-(1H-indazol-6-yl)-1H-pyrazolo [3, 4-d] pyrimidin-1-yl) ethyl)-3-phenyl-4H-chromen-4-one;
2-(1-(4-amino-3-(3-hydroxy-3-methylbut-1-ynyl)-1H-pyrazolo [3, 4-d] pyrimidin-1-yl) ethyl)-3-phenyl-4H-chromen-4-one;
2-( 1 -(4-amino-3-( 1 H-pyrazol-4-yl)- 1 H-pyrazolo [3, 4-d] pyrimidin-1-yl) ethyl)-3-(3-fluorophenyl)-4H-chromen-4-one;
(S)-2-( 1 -(9H-purin-6-ylamino) ethyl)-3-phenyl-4H-chromen-4-one;
(S)-2-( 1 -(9H-purin-6-ylamino) ethyl)-6-fluoro-3-(3-fluorophenyl)-4H-chromen-4-one;
2-((4-amino-3-(lH-indazol-6-yl)-lH-pyrazolo [3, 4-d] pyrimidin-1-yl) methyl)-3-phenyl-4H-chromen-4-one;
2-(1-(4-amino-3-(3-fluoro-5-methoxyphenyl)-1H-pyrazolo [3, 4-d] pyrimidin-1-yl) ethyl)-3- (3-fluorophenyl)-4H-chromen-4-one;
2-(1-(4-amino-3-(3-fluoro-5-hydroxyphenyl)-1H-pyrazolo [3, 4-d] pyrimidin-1-yl) ethyl)-3- (3-fluorophenyl)-4H-chromen-4-one;
2-(1-(4-amino-3-(1H-indazol-4-yl)-1H-pyrazolo [3, 4-d] pyrimidin-1-yl) ethyl)-3-(3-fluorophenyl)-4H-chromen-4-one;
2-(1-(4-amino-3-(3, 5-dimethyl-lH-pyrazol-4-yl)-lH-pyrazolo [3, 4-d] pyrimidin-1-yl) ethyl)-3-(3-fluorophenyl)-4H-chromen-4-one;
2-(1-(4-amino-3-(3-methyl-1H-indazol-6-yl)-1H-pyrazolo [3, 4-d] pyrimidin-1-yl) ethyl)-3- (3-fluorophenyl)-4H-chromen-4-one;
2-(1-(4-amino-3-(1H-indazol-6-yl)-1H-pyrazolo [3, 4-d] pyrimidin-1-yl) ethyl)-3-(3-fluorophenyl)-4H-chromen-4-one;
2-(1-(4-amino-3-(2-(hydroxymethyl) phenyl)- 1H-pyrazolo [3, 4-d] pyrimidin-1-yl) ethyl)-3- (3-fluorophenyl)-4H-chromen-4-one;
2-(1-(4-amino-3-(4-fluoro-3-methoxyphenyl)-1H-pyrazolo [3, 4-d] pyrimidin-1-yl) ethyl)-3- (3-fluorophenyl)-4H-chromen-4-one;
2-(1-(4-amino-3-(4-fluoro-3-hydroxyphenyl)-1H-pyrazolo [3, 4-d] pyrimidin-1-yl) ethyl)-3- (3-fluorophenyl)-4H-chromen-4-one;
2-(1-(4-amino-3-(3-hydroxyprop-1-ynyl)-1H-pyrazolo [3, 4-d] pyrimidin-1-yl) ethyl)-3-(3-fluorophenyl)-4H-chromen-4-one;
2-(1-(4-amino-3-(3-fluoro-4-methoxyphenyl)-1H-pyrazolo [3, 4-d] pyrimidin-1-yl) ethyl)-3- (3-fluorophenyl)-4H-chromen-4-one;
2-(1-(4-amino-3-(3-fluoro-4-hydroxyphenyl)-1H-pyrazolo [3, 4-d] pyrimidin-1-yl) ethyl)-3- (3-fluorophenyl)-4H-chromen-4-one;
2-(1-(4-amino-3-(3-chloro-5-methoxyphenyl)-1H-pyrazolo [3, 4-d] pyrimidin-1-yl) ethyl)-3- (3-fluorophenyl)-4H-chromen-4-one;
2-(1-(4-amino-3-(3-chloro-5-hydroxyphenyl)-1H-pyrazolo [3, 4-d] pyrimidin-1-yl) ethyl)-3- (3-fluorophenyl)-4H-chromen-4-one;
2- (1-(4-amino-3-(3-(trifluoromethoxy) phenyl)- 1H-pyrazolo [3, 4-d] pyrimidin-1-yl) ethyl)-3- (3-fluorophenyl)-4H-chromen-4-one;
2-(1-(4-amino-3-(4-methoxyphenyl)-1H-pyrazolo [3, 4-d] pyrimidin-1-yl) ethyl)-3-(3-fluorophenyl)-4H-chromen-4-one;
2-(1-(4-amino-3-(4-hydroxyphenyl)-1H-pyrazolo [3, 4-d] pyrimidin-1-yl) ethyl)-3-(3-fluorophenyl)-4H-chromen-4-one;
2-((6-amino-9H-purin-9-yl) methyl)-3 -(3 -fluorophenyl)-4H-chromen-4-one;
2-(1-(4-amino-3-(4-fluoro-2-methoxyphenyl)-1H-pyrazolo [3, 4-d] pyrimidin-1-yl) ethyl)-3- (3-fluorophenyl)-4H-chromen-4-one;
2-(1-(4-amino-3-(4-fluoro-2-hydroxyphenyl)-1H-pyrazolo [3, 4-d] pyrimidin-1-yl) ethyl)-3- (3-fluorophenyl)-4H-chromen-4-one;
2-((4-amino-3-(3-aminophenyl)-lH-pyrazolo [3, 4-d] pyrimidin-1-yl) methyl)-3-phenyl-4H-chromen-4-one;
2-((4-amino-3-(3-methyl-lH-indazol-6-yl)-lH-pyrazolo [3, 4-d] pyrimidin-1-yl) methyl)-3-phenyl-4H-chromen-4-one;
2-(1-(4-amino-3-(2-aminopyrimidin-5-yl)-1H-pyrazolo [3, 4-d] pyrimidin-1-yl) ethyl)-3-(3-fluorophenyl)-4H-chromen-4-one;
2-(1-(4-amino-3-(1H-indol-6-yl)-1H-pyrazolo [3, 4-d] pyrimidin-1-yl) ethyl)-3-(3-fluorophenyl)-4H-chromen-4-one;
2-(1-(4-amino-3-(4-chloro-3-methoxyphenyl)-1H-pyrazolo [3, 4-d] pyrimidin-1-yl) ethyl)-3- (3-fluorophenyl)-4H-chromen-4-one;
2-(1-(4-amino-3-(4-chloro-3-hydroxyphenyl)-1H-pyrazolo [3, 4-d] pyrimidin-1-yl) ethyl)-3- (3-fluorophenyl)-4H-chromen-4-one;
2-(1-(4-amino-3-(2-chloro-5-methoxyp enyl)-lH-pyrazolo [3, 4-d] pyrimidin-1-yl) ethyl)-3- (3-fluorophenyl)-4H-chromen-4-one;
2-(1-(4-amino-3-(2-chloro-5-hydroxyphenyl)-1H-pyrazolo [3, 4-d] pyrimidin-1-yl) ethyl)-3- (3-fluorophenyl)-4H-chromen-4-one;
2-(1-(4-amino-3-(3, 4-dimethoxyphenyl)-lH-pyrazolo [3, 4-d] pyrimidin-1-yl) ethyl)-3-(3-fluorophenyl)-4H-chromen-4-one;
2-(1-(4-amino-3-(3, 4-dihydroxyphenyl)- 1 H-pyrazolo [3, 4-d] pyrimidin-1-yl) ethyl)-3-(3-fluorophenyl)-4H-chromen-4-one;
2-((4-amino-3-(3-methyl- 1 H-indazol-6-yl)- 1 H-pyrazolo [3, 4-d] pyrimidin-1-yl) methyl)-3-(3-fluorophenyl)-4H-chromen-4-one;
2-(1-(4-amino-3-(1H-indol-5-yl)-1H-pyrazolo [3, 4-d] pyrimidin-1-yl) ethyl)-3-(3-fluorophenyl)-4H-chromen-4-one;
2-(1-(4-Amino-3-(3-methyl-1H-indol-5-yl)-1H-pyrazolo [3, 4-d] pyrimidin-1-yl) ethyl)-3-(3-fluorophenyl)-4H-chromen-4-one;
tert-butyl-(5-(4-amino-1-(1-(3-(3-fluorophenyl)-4-oxo-4H-chromen-2-yl) ethyl)- 1 H-pyrazolo [3, 4-d] pyrimidin-3-yl) thiophen-2-yl) methylcarbamate
2-(1-(4-amino-3-(5-(aminomethyl) thiophen-2-yl)-lH-pyrazolo [3, 4-d] pyrimidin-1-yl) ethyl)-3 -(3 -fluorophenyl)-4H-chromen-4-one;
2-((4-amino-3-(3-methyl- 1 H-indazol-6-yl)- 1 H-pyrazolo [3, 4-d] pyrimidin-1-yl) methyl)-6-fluoro-3-phenyl-4H-chromen-4-one;
2-( 1 -(4-amino-3-(3-methyl- 1 H-indazol-6-yl)- 1 H-pyrazolo [3, 4-d] pyrimidin-1-yl) ethyl)-3-phenyl-4H-chromen-4-one;
2-( 1 -(4-amino-3-(3-methyl- 1 H-indazol-6-yl)- 1 H-pyrazolo [3, 4-d] pyrimidin-1-yl) ethyl)-6-fluoro-3-phenyl-4H-chromen-4-one;
2-( 1 -(4-amino-3-(3-methyl- 1H-indazol-5-yl)- 1 H-pyrazolo [3, 4-d] pyrimidin-1-yl) ethyl)-3-(3-fluorophenyl)-4H-chromen-4-one;
N-(4-(4-amino-1-(1-(3-(3-fluorophenyl)-4-oxo-4H-chromen-2-yl) ethyl)- 1 H-pyrazolo [3, 4-d] pyrimidin-3-yl) phenyl) acetamide;
2-(1-(4-amino-3-(4-aminophenyl)-1 H-pyrazolo [3, 4-d] pyrimidin-1-yl) ethyl)-3-(3-fluorophenyl)-4H-chromen-4-one;
2-(1-(4-amino-3-(3-methyl-1H-indazol-6-yl)-1H-pyrazolo [3, 4-d] pyrimidin-1-yl) ethyl)-6-fluoro-3 -(3 -fluorophenyl)-4H-chromen-4-one;
2- (1-(4-amino-3-(2, 3-dihydrobenzofuran-5-yl)-1 H-pyrazolo [3, 4-d] pyrimidin-1-yl) ethyl)-3-(3-fluorophenyl)-4H-chromen-4-one;
2-(1-(4-amino-3-(3-ethyl-1H-indazol-6-yl)-1H-pyrazolo [3, 4-d] pyrimidin-1-yl) ethyl)-3-(3-fluorophenyl)-4H-chromen-4-one;
2-( 1 -(4-amino-3-(3-methyl- 1 H-indol-6-yl)- 1 H-pyrazolo [3, 4-d] pyrimidin-1-yl) ethyl)-3-(3- fluorophenyl)-4H-chromen-4-one;
2-( 1 -(4-amino-3-(2-methoxypyrimidin-5-yl)- 1 H-pyrazolo [3, 4-d] pyrimidin-1-yl) ethyl)-3-(3-fluorophenyl)-4H-chromen-4-one;
4- (4-amino- 1-( 1 -(3-(3-fluorophenyl)-4-oxo-4H-chromen-2-yl) ethyl)- 1 H-pyrazolo [3, 4-d] pyrimidin-3-yl) thiophene-2-carbaldehyde;
2-(1-(4-amino-3-(5-(hydroxymethyl) thiophen-3-yl)-1H-pyrazolo [3, 4-d] pyrimidin-1-yl) ethyl)-3-(3-fluorophenyl)-4H-chromen-4-one;
2-(1-(4-amino-3-(2-methyl-1H-benzo[d]imidazol-5-yl)-1H-pyrazolo [3, 4-d] pyrimidin-1-yl) ethyl)-3-(3-fluorophenyl)-4H-chromen-4-one;
2-( 1 -(4-amino-3-(3-methyl- 1 H-indazol-6-yl)- 1 H-pyrazolo [3, 4-d] pyrimidin-1-yl) propyl)-3- (3-fluorophenyl)-4H-chromen-4-one;
2-(1-(4-amino-3-(3-methyl-1H-indol-6-yl)-1H-pyrazolo [3, 4-d] pyrimidin-1-yl) ethyl)-3-(3-fluorophenyl)-4H-chromen-4-one;
2-((6-amino-9H-purin-9-yl) methyl)-6-fluoro-3-phenyl-4H-chromen-4-one;
2-((6-amino-9H-purin-9-yl) methyl)-6-fluoro-3 -(3 -fluorophenyl)-4H-chromen-4-one;
2-((4-amino-3-(3-fluoro-5-methoxyphenyl)-lH-pyrazolo [3, 4-d] pyrimidin-1-yl) methyl)-6-fluoro-3 -(3 -fluorophenyl)-4H-chromen-4-one;
2-((4-amino-3-(3-fluoro-5-hydroxyphenyl)-lH-pyrazolo [3, 4-d] pyrimidin-1-yl) methyl)-6-fluoro-3 -(3 -fluorophenyl)-4H-chromen-4-one;
2-(1-(4-amino-3-(3-methoxyphenyl)-1H-pyrazolo [3, 4-d] pyrimidin-1-yl) ethyl)-6-fluoro-3- (3-fluorophenyl)-4H-chromen-4-one;
2-(1-(4-amino-3-(3-hydroxyphenyl)-1H-pyrazolo [3, 4-d] pyrimidin-1-yl) ethyl)-6-fluoro-3- (3-fluorophenyl)-4H-chromen-4-one;
2-((9H-purin-6-ylamino) methyl)-6-fluoro-3-(3-fluorophenyl)-4H-chromen-4-one;
2-((9H-purin-6-ylamino) methyl)-6-fluoro-3-phenyl-4H-chromen-4-one;
(R)-2-( 1 -(9H-purin-6-ylamino) ethyl)-6-fluoro-3-(3-fluorophenyl)-4H-chromen-4-one;
2-((4-amino-3-(lH-pyrazol-4-yl)-lH-pyrazolo [3, 4-d] pyrimidin-1-yl) methyl)-6-fluoro-3-phenyl-4H-chromen-4-one;
2-(1-(4-amino-3-(3, 5-difluoro-4-methoxyphenyl)-1H-pyrazolo [3, 4-d] pyrimidin-1-yl) ethyl)-3-(3-fluorophenyl)-4H-chromen-4-one;
2-(1-(4-amino-3-(3, 5-difluoro-4-hydroxyphenyl)-lH-pyrazolo [3, 4-d] pyrimidin-1-yl) ethyl)-3-(3-fluorophenyl)-4H-chromen-4-one;
2-((4-amino-3-(3, 5-difluoro-4-methoxyphenyl)-1H-pyrazolo [3, 4-d] pyrimidin-1-yl) methyl)-6-fluoro-3-(3-fluorophenyl)-4H-chromen-4-one;
2-((4-amino-3-(3, 5-difluoro-4-hydroxyphenyl)-lH-pyrazolo [3, 4-d] pyrimidin-1-yl) methyl)-6-fluoro-3-(3-fluorophenyl)-4H-chromen-4-one;
(+)-2-(1-(4-amino-3-(3-methyl-1H-indazol-6-yl)-1H-pyrazolo [3, 4-d] pyrimidin-1-yl) ethyl)- 3-(3-fluorophenyl)-4H-chromen-4-one;
(-)-2-(l-(4-amino-3-(3-methyl-lH-indazol-6-yl)-lH-pyrazolo [3, 4-d] pyrimidin-1-yl) ethyl)- 3-(3-fluorophenyl)-4H-chromen-4-one;
2-(1-(4-amino-3-(3, 5-dimethoxyphenyl)-lH-pyrazolo [3, 4-d] pyrimidin-1-yl) ethyl)-3-(3-fluorophenyl)-4H-chromen-4-one;
2-(1-(4-amino-3-(4-methoxy-3, 5-dimethylphenyl)-lH-pyrazolo [3, 4-d] pyrimidin-1-yl) ethyl)-3 -(3 -fluorophenyl)-4H-chromen-4-one;
2- (1-(4-amino-3-(2-fluoro-5-isopropoxyphenyl)-1H-pyrazolo [3, 4-d] pyrimidin-1-yl) ethyl)-3- (3-fluorophenyl)-4H-chromen-4-one;
2-(1-(4-amino-3-(2, 3-dihydrobenzo[b] [1, 4] dioxin-6-yl)-lH-pyrazolo [3, 4-d] pyrimidin-1-yl) ethyl)-3-(3-fluorophenyl)-4H-chromen-4-one;
2-(1-(4-amino-3-(1-benzyl-1H-pyrazol-4-yl)-1H-pyrazolo [3, 4-d] pyrimidin-1-yl) ethyl)-3- (3-fluorophenyl)-4H-chromen-4-one;
2-(1-(4-amino-3-(2-methylpyridin-4-yl)-1H-pyrazolo [3, 4-d] pyrimidin-1-yl) ethyl)-3-(3-fluorophenyl)-4H-chromen-4-one;
2-(1-(4-amino-3-(3, 4-dihydro-2H-benzo[b] [1, 4] dioxepin-7-yl)-lH-pyrazolo [3, 4-d] pyrimidin-1-yl) ethyl)-3-(3-fluorophenyl)-4H-chromen-4-one;

2-(1-(4-amino-3-(6-morpholinopyridin-3-yl)-1H-pyrazolo [3, 4-d] pyrimidin-1-yl) ethyl)-3- (3-fluorophenyl)-4H-chromen-4-one;
2-(1-(4-amino-3-(dibenzo [b, d] furan-4-yl)-lH-pyrazolo [3, 4-d] pyrimidin-1-yl) ethyl)-3-(3-fluorophenyl)-4H-chromen-4-one;
2-(1-(4-amino-3-(4-phenoxyphenyl)-1H-pyrazolo [3, 4-d] pyrimidin-1-yl) ethyl)-3-(3-fluorophenyl)-4H-chromen-4-one;
2-(1-(4-amino-3-(4-(benzyloxy)-3-chlorophenyl)-1H-pyrazolo [3, 4-d] pyrimidin-1-yl) ethyl)-3-(3-fluorophenyl)-4H-chromen-4-one;
2- (1-(4-amino-3-(3-chloro-4-isopropoxyphenyl)-1H-pyrazolo [3, 4-d] pyrimidin-1-yl) ethyl)-3- (3-fluorophenyl)-4H-chromen-4-one;
2-(1-(4-amino-3-(3-(dimethylamino) phenyl)- 1H-pyrazolo [3, 4-d] pyrimidin-1-yl) ethyl)-3- (3-fluorophenyl)-4H-chromen-4-one;
2-(1-(4-amino-3-(4-ethoxy-3-fluorophenyl)-1H-pyrazolo [3, 4-d] pyrimidin-1-yl) ethyl)-3-(3-fluorophenyl)-4H-chromen-4-one;
2-(1-(4-amino-3-(4-isopropoxyphenyl)-1H-pyrazolo [3, 4-d] pyrimidin-1-yl) ethyl)-3-(3-fluorophenyl)-4H-chromen-4-one;
2- (1-(4-amino-3-(4-(trifluoromethoxy) phenyl)- 1H-pyrazolo [3, 4-d] pyrimidin-1-yl) ethyl)-3- (3-fluorophenyl)-4H-chromen-4-one;
2-(1-(3-(4-acetylphenyl)-4-amino-1H-pyrazolo [3, 4-d] pyrimidin-1-yl) ethyl)-3-(3-fluorophenyl)-4H-chromen-4-one;
2-(1-(4-amino-3-(4-(benzyloxy) phenyl)- 1H-pyrazolo [3, 4-d] pyrimidin-1-yl) ethyl)-3-(3-fluorophenyl)-4H-chromen-4-one;
2-(1-(4-amino-3-(4-(dimethylamino) phenyl)- 1H-pyrazolo [3, 4-d] pyrimidin-1-yl) ethyl)-3- (3-fluorophenyl)-4H-chromen-4-one;
2-(1-(4-amino-3-(4-(methylsulfonyl) phenyl)- 1H-pyrazolo [3, 4-d] pyrimidin-1-yl) ethyl)-3- (3-fluorophenyl)-4H-chromen-4-one;
2-( 1 -(4-amino-3-(3-ethoxyphenyl)- 1 H-pyrazolo [3, 4-d] pyrimidin-1-yl) ethyl)-3-(3-fluorophenyl)-4H-chromen-4-one;
2-(1-(4-amino-3-(benzo[b]thiophen-2-yl)-1H-pyrazolo [3, 4-d] pyrimidin-1-yl) ethyl)-3-(3-fluorophenyl)-4H-chromen-4-one;
2-(1-(4-amino-3-(5-chlorothiophen-2-yl)-1H-pyrazolo [3, 4-d] pyrimidin-1-yl) ethyl)-3-(3-fluorophenyl)-4H-chromen-4-one;
2-(1-(4-amino-3-(3, 5-dimethylisoxazol-4-yl)-lH-pyrazolo [3, 4-d] pyrimidin-1-yl) ethyl)-3- (3-fluorophenyl)-4H-chromen-4-one;
2-(1-(4-amino-3-(3-propoxyphenyl)-1H-pyrazolo [3, 4-d] pyrimidin-1-yl) ethyl)-3-(3-fluorophenyl)-4H-chromen-4-one;
2~(1-(4-amino-3-(furan-2-yl)-1H-pyrazolo [3, 4-d] pyrimidin-1-yl) ethyl)-3-(3-fluorophenyl)-4H-chromen-4-one;
2-(1-(4-amino-3-(4-ethoxyphenyl)-1H-pyrazolo [3, 4-d] pyrimidin-1-yl) ethyl)-3-(3-fluorophenyl)-4H-chromen-4-one;
2-(1-(4-amino-3-(3-chloro-4-methoxyphenyl)-1H-pyrazolo [3, 4-d] pyrimidin-1-yl) ethyl)-3- (3-fluorophenyl)-4H-chromen-4-one;
2- (1-(4-amino-3-(3-fluoro-4-isopropoxyphenyl)-1H-pyrazolo [3, 4-d] pyrimidin-1-yl) ethyl)-3- (3-fluorophenyl)-4H-chromen-4-one;
2-(1-(4-amino-3-(6-fluoropyridin-3-yl)-1H-pyrazolo [3, 4-d] pyrimidin-1-yl) ethyl)-3-(3-fluorophenyl)-4H-chromen-4-one;
2-(1-(4-amino-3-(pyrimidin-5-yl)-1H-pyrazolo [3, 4-d] pyrimidin-1-yl) ethyl)-3-(3-fluorophenyl)-4H-chromen-4-one;
2-(1-(4-amino-3-(3-(methoxymethyl) phenyl)- 1H-pyrazolo [3, 4-d] pyrimidin-1-yl) ethyl)-3- (3-fluorophenyl)-4H-chromen-4-one;
2-(1-(4-amino-3-(6-hydroxynaphthalen-2-yl)-1H-pyrazolo [3, 4-d] pyrimidin-1-yl) ethyl)-3- (3-fluorophenyl)-4H-chromen-4-one;
2-( 1 -(4-amino-3-(3-isopropoxyphenyl)- 1H-pyrazolo [3, 4-d] pyrimidin-1-yl) ethyl)-3-(3-fluorophenyl)-4H-chromen-4-one;
2-(1-(4-amino-3-(1-methyl-1H-pyrazol-4-yl)-1H-pyrazolo [3, 4-d] pyrimidin-1-yl) ethyl)-3- (3-fluorophenyl)-4H-chromen-4-one;
6-Fluoro-3-(3-fluorophenyl)-2-(1-(4-methoxyphenylamino) ethyl)-4H-chromen-4-one;
2-(1-(4-Chloro-7H-pyrrolo [2, 3-d] pyrimidin-7-yl) emyl)-3-(3-fluorophenyl)-4H-chromen-4-one; 2-(1-(4-Chloro-1H-pyrazolo [3, 4-b] pyridin-1-yl) ethyl)-3-(3-fluorophenyl)-4H-chromen-4-one;

2-(1-(4-Chloro-1H-pyrazolo [3, 4-d] pyrimidin-1-yl) ethyl)-3-(3-fluorophenyl)-4H-chromen-4- one;
2-(1-(4-Chloro-5H-pyrrolo [3, 2-d] pyrimidin-5-yl) ethyl)-3-(3-fluorophenyl)-4H-chromen-4-one;
2-(1-(4-amino-3-(1, 3-dimethyl-lH-indazol-6-yl)-lH-pyrazolo [3, 4-d] pyrimidin-1-yl) ethyl)-3-(3-fluorophenyl)-4H-chromen-4-one;
2-(1-(4-amino-3-(2, 3-dimethyl-2H-indazol-6-yl)-lH-pyrazolo [3, 4-d] pyrimidin-1-yl) ethyl)-3-(3-fluorophenyl)-4H-chromen-4-one;
2-( 1 -(4-amino-3-(6-methoxynaphthalen-2-yl)- 1 H-pyrazolo [3, 4-d] pyrimidin-1-yl) ethyl)-3-(3-fluorophenyl)-4H-chromen-4-one;
2-(1-(4-amino-3-(benzo[b]thiophen-3-yl)-1H-pyrazolo [3, 4-d] pyrimidin-1-yl) ethyl)-3-(3-fluorophenyl)-4H-chromen-4-one;
2- (1-(4-amino-3-(2, 4-dimethoxypyrimidin-5-yl)-1 H-pyrazolo [3, 4-d] pyrimidin-1-yl) ethyl)-3- (3-fluorophenyl)-4H-chromen-4-one;
2- ( 1 -(4-amino-3-(6-ethoxynaphthalen-2-yl)- 1 H-pyrazolo [3, 4-d] pyrimidin-1-yl) ethyl)-3-(3- fluorophenyl)-4H-chromen-4-one;
3- (4-amino-1-(1-(3-(3-fluorophenyl)-4-oxo-4H-chromen-2-yl) ethyl)- 1 H-pyrazolo [3, 4-d] pyrimidin-3-yl)-N-cyclopropylbenzamide;
2-(1-(4-amino-3-(3-(morpholine-4-carbonyl) phenyl)- 1 H-pyrazolo [3, 4-d] pyrimidin-1-yl) ethyl)-3-(3-fluorophenyl)-4H-chromen-4-one;
2-(1-(4-amino-3-(3-(difluoromethoxy) phenyl)- 1 H-pyrazolo [3, 4-d] pyrimidin-1-yl) ethyl)-3-(3-fluorophenyl)-4H-chromen-4-one;
5- (4-amino-1-(1-(3-(3-fluorophenyl)-4-oxo-4H-chromen-2-yl) ethyl)- 1 H-pyrazolo [3, 4-d] pyrimidin-3-yl) furan-2-carbaldehyde and pharmaceutically acceptable salts thereof;
or their enantiomers, mixtures of enantiomers, mixtures of two or more diastereoisomers, isotopic variants, pharmaceutically acceptable salts, solvates, hydrates or pro-drug.

**73.** The pharmaceutical combination according to any one of claims 1-72, wherein said PI3K inhibitor comprise: 2-(1-(9H-purin--6-ylamino) propyl group)-3-(3-fluorophenyl)-4H-chromen-4-one or its enantiomers, mixtures of enantiomers, mixtures of two or more diastereoisomers, isotopic variants, pharmaceutically acceptable salts, solvates, hydrates or prodrugs.

**74.** The pharmaceutical combination according to any one of claims 1-73, wherein said PI3K inhibitor comprise: (S)-2-(1-(9H-purin--6-ylamino) propyl group)-3-(3-fluorophenyl)--4H-chromen--4-one or its pharmaceutically acceptable salts, solvates, hydrates or prodrugs.

**75.** The pharmaceutical combination according to any one of claims 1-74, comprising a PD-L1/TGFβ dual inhibitor and a PI3K inhibitor.

**76.** The pharmaceutical combination according to any one of claims 75, wherein the PD-L1/TGFβ dual inhibitor comprises an anti-PD-L1 antibody or an antigen-binding fragment thereof and TGFβRII or a functionally active fragment thereof.

**77.** The pharmaceutical combination according to any one of claim 76, wherein the PD-L1/TGFβ dual inhibitor comprises two polypeptides comprising an amino acid sequence selected from the group consisting of: (a) the amino acid sequence shown in SEQ ID NO :7; (b) an amino acid sequence having at least 85%, 90%, 95% or 99% sequence identity with the amino acid sequence shown in SEQ ID NO: 7; (c) an amino acid sequence having one or more differences from the amino acid sequence shown in SEQ ID NO:7 obtained by addition, elimination or substitution reaction.

**78.** The pharmaceutical combination according to claim 75, wherein the PI3K inhibitor is a dual PI3K δ/γ inhibitor.

**79.** The pharmaceutical combination according to claim 78, wherein the PI3K inhibitor is (S)-2-(1-(9H-purin--6-ylamino) propyl group)-3-(3-fluorophenyl)--4H-chromen--4-one or its pharmaceutically acceptable salts, solvates, hydrates or prodrugs.

**80.** The pharmaceutical combination according to any one of claims 1-79, wherein the second therapeutic agent and the PI3K inhibitor are not intermixed in the pharmaceutical combination.

**81.** The pharmaceutical combination according to any one of claims 1-80, wherein the second therapeutic agent and the PI3K inhibitor are each independently present in separate containers.

**82.** The pharmaceutical composition according to any one of claims 1-81, said pharmaceutical combination may further comprise a third active substance.

**83.** The pharmaceutical composition according to claim 82, wherein the third active substance is selected from vincristine, vinblastine, vindesine, etoposide, docetaxel, paclitaxel (such as albumin bound paclitaxel), irinotecan, vinorelbine, mitoxantrone, vinflunine, topotecan, or any combination thereof.

**84.** A pharmaceutical composition comprising the PI3K inhibitor of any one of claims 1-83 and a second therapeutic agent, and optionally one or more pharmaceutically acceptable carriers or excipients agent.

**85.** The pharmaceutical composition according to claim 84, wherein the second therapeutic agent is the PD-L1/TGFβ dual inhibitor of any one of claims 46-60.

**86.** The pharmaceutical composition according to any one of claims 84-85, wherein the PI3K inhibitor is the dual PI3K delta/gamma inhibitor according to any one of claims 61-74.

**87.** The pharmaceutical composition according to any one of claims 84-86, wherein the second therapeutic agent and the PI3K inhibitor are present in a single or separate dosage form.

**88.** A method for treating and/or preventing tumors, comprising administering to a subject in need: an effective amount of the drug combination according to any one of claims 1-81, or pharmaceutical composition of any one of claims 84-87.

**89.** The method of claim 88, comprising administering to a subject in need: an effective amount of the PI3K inhibitor of any one of claims 61-74; and PD-L1/TGFβ dual inhibitor of any one of claims 46-60.

**90.** The method of claim 89, wherein the PI3K inhibitor is administered simultaneously with the PD-L1/TGFβ dual inhibitor.

**91.** The method of claim 90, wherein the PI3K inhibitor is administered after the PD-L1/TGFβ dual inhibitor.

**92.** The method of claim 89, wherein the PI3K inhibitor is administered before the PD-L1/TGFβ dual inhibitor.

**93.** The method of claim 89, further comprising administering an effective amount of the small molecule TGFβ inhibitor of claim 41 to a subject in need thereof.

**94.** The method of any one of claims 88-93, wherein the tumor comprises solid tumors and non-solid tumors.

**95.** The method of any one of claims 88-94, wherein the tumor comprises a tumor with aberrant expression of PI3K.

**96.** The method of any one of claims 88-95, wherein the tumor comprises a tumor with aberrant expression of PD-L1.

**97.** The method of any one of claims 88-96, wherein the tumor comprises a tumor with aberrant expression of TGFβ

**98.** The method of any one of claims 88-97, wherein the tumor comprises a tumor of the digestive tract, melanoma, or lymphoma.

**99.** Use of the pharmaceutical combination according to any one of claims 1-83 or the pharmaceutical composition according to any one of claims 84-87 in the preparation of medicines for treating and/or preventing tumors.

**100.** Use of the pharmaceutical combination according to any one of claims 1-83 or the pharmaceutical composition according to any one of claims 84-87 for treating and/or preventing tumors.

**101.** A method of inhibiting tumor growth comprising contacting a tumor with the pharmaceutical combination of any one of claims 1-83 or the pharmaceutical composition of any one of claims 84-87.

**102.** A method of inhibiting tumor growth, said method comprising contacting a tumor with the PI3K inhibitor of any one of claims 61-74 and the PD-L1/TGFβ of any one of claims 46-60 dual inhibitors.

**103.** A kit comprising: (1) a first container, and the PI3K inhibitor of any one of claims 61-74 in the first container; (2) a second container, and the PD-L1/TGFβ dual inhibitor according to any one of claims 46-60 located in the second container.

**104.** A kit comprise: (1) a first container, and the PI3K inhibitor of any one of claims 61-74 in the first container; (2) a second container, and the immune checkpoint inhibitor according to any one of claims 17-29 or the TGFβ inhibitor according to any one of claims 30-44 located in the second container.

**105.** The kit according to claim 102, further comprising (3) a third container, and the immune checkpoint inhibitor according to any one of claims 17-29 in the third container or the TGFβ inhibitor of any one of claims 30-44, wherein the agent in the third container is different from the agent in the second container.

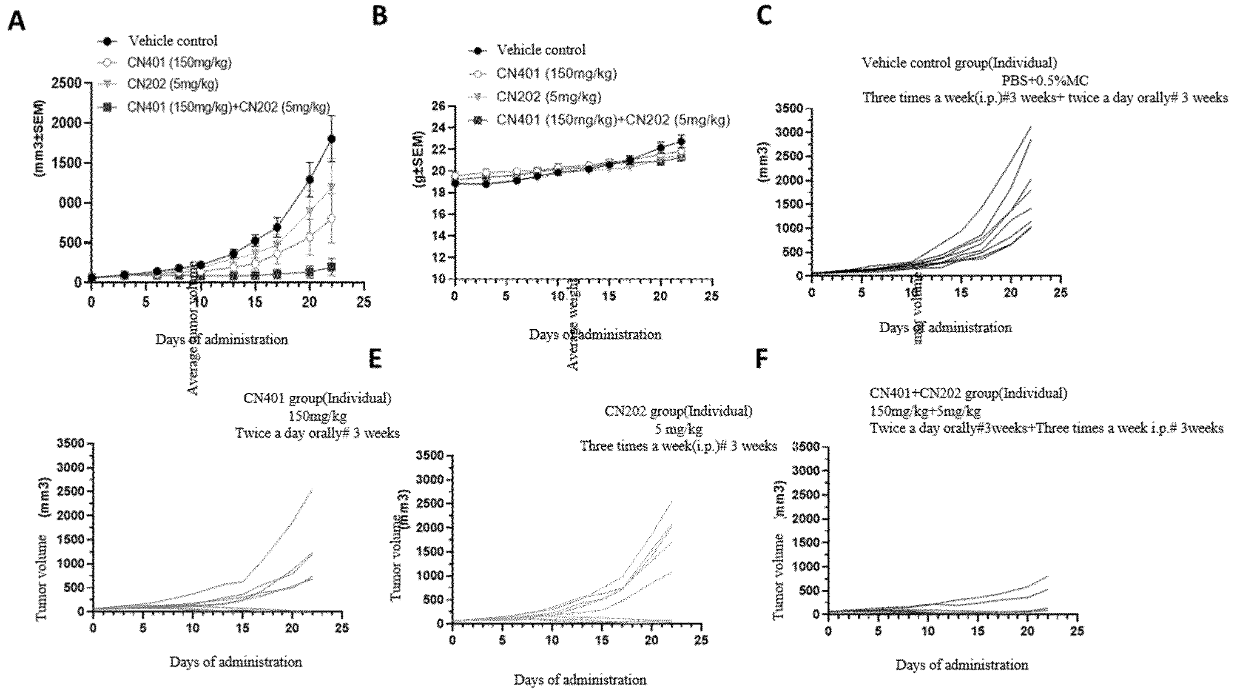


Figure 1

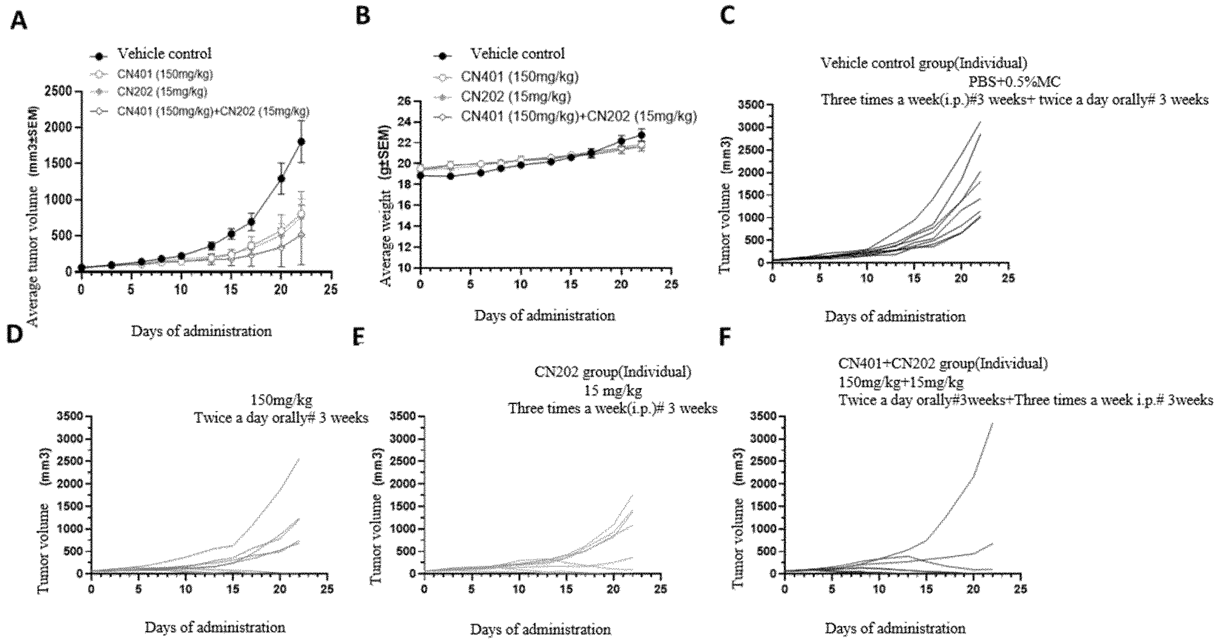


Figure 2

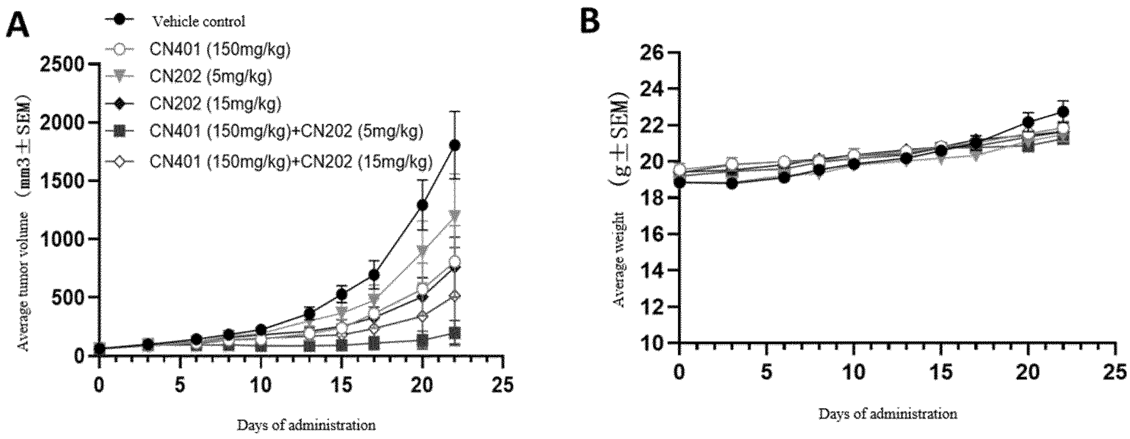


Figure 3

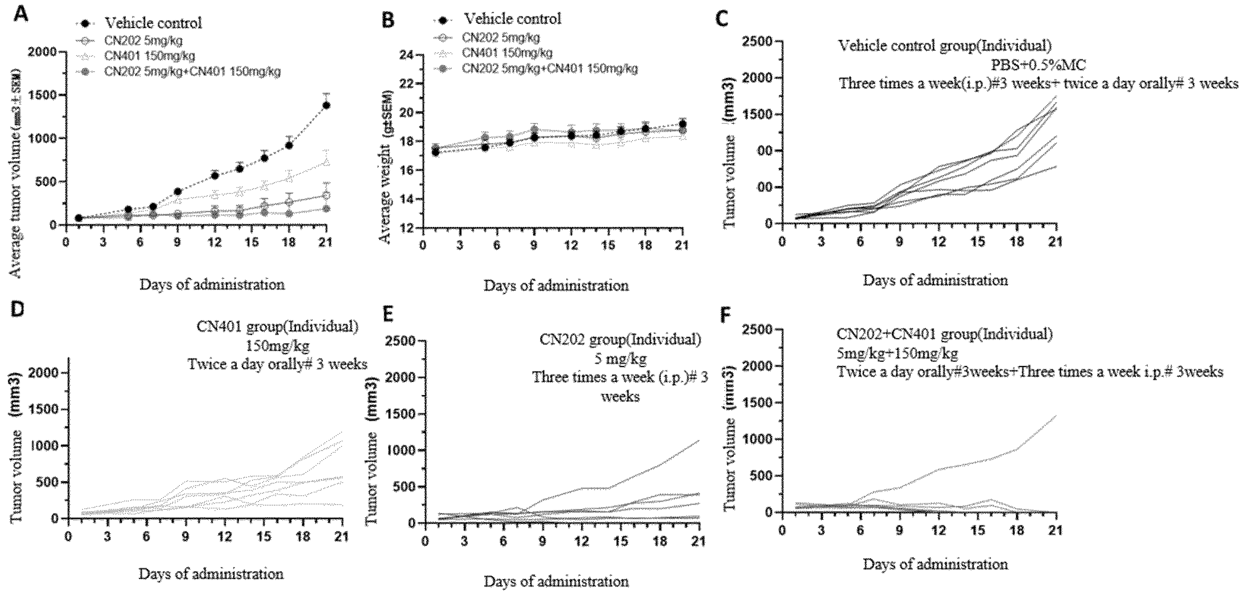


Figure 4

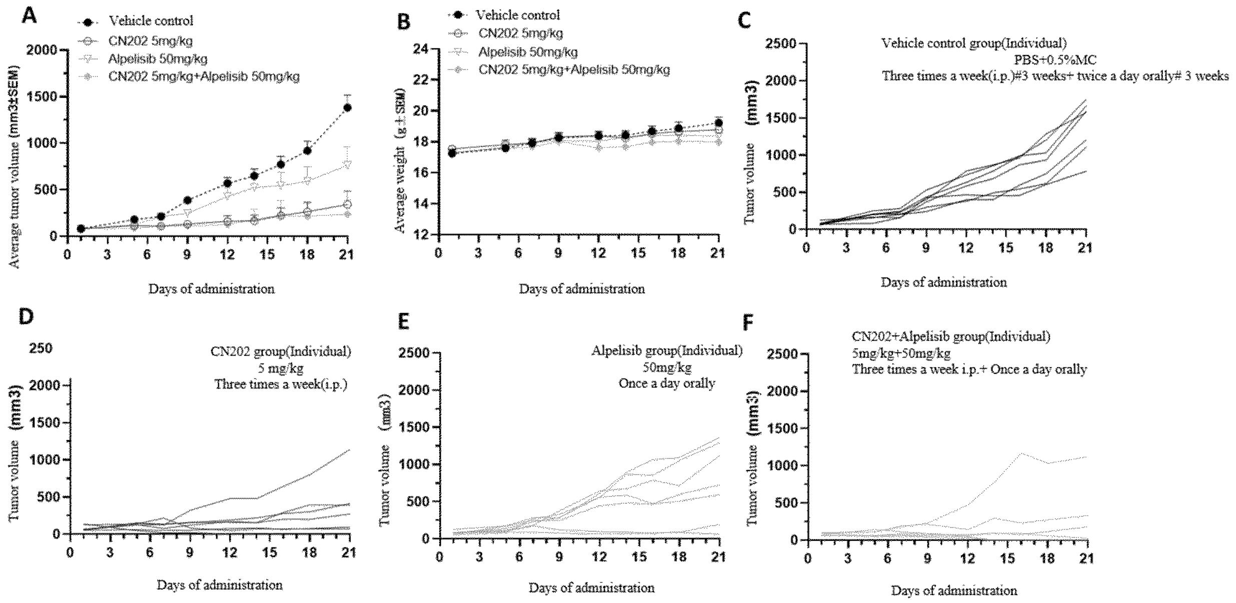


Figure 5

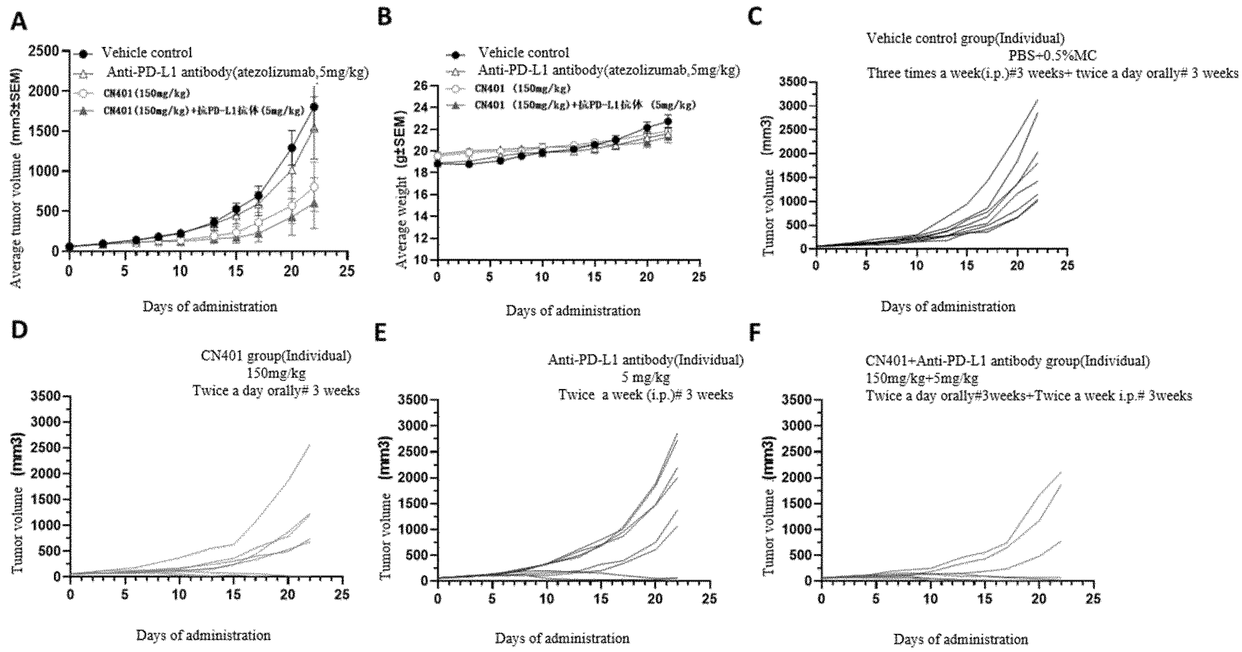


Figure 6

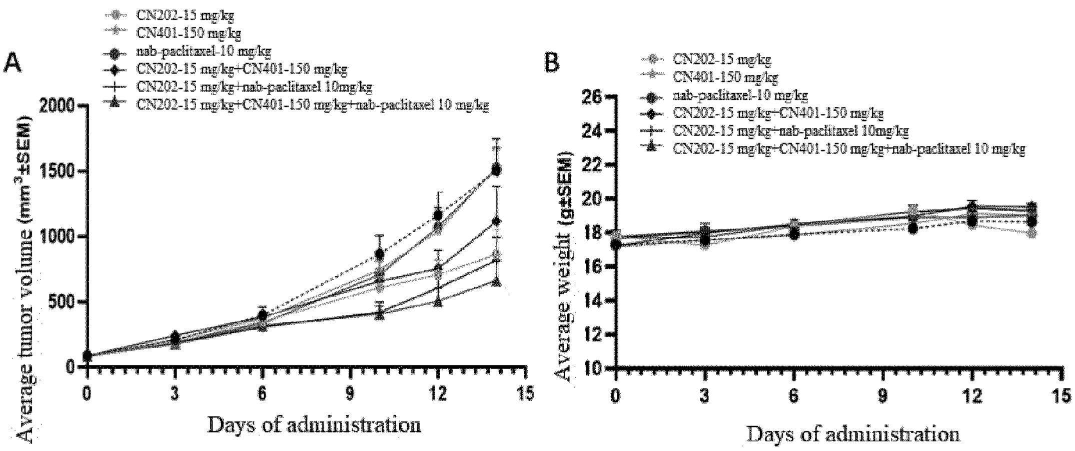


Figure 7A

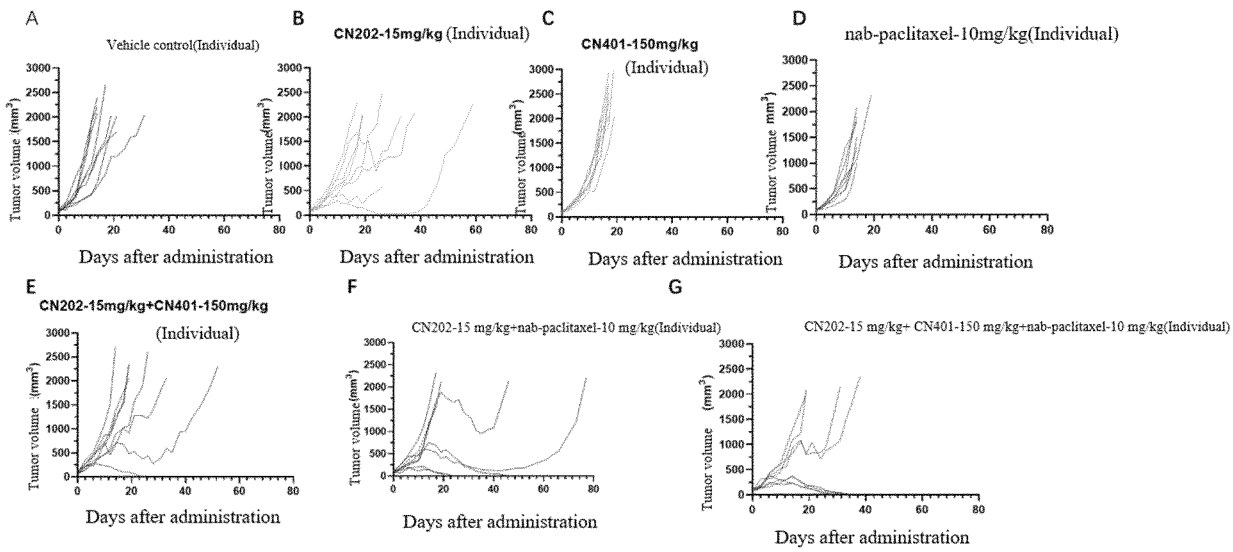


Figure 7 B

**INTERNATIONAL SEARCH REPORT**

International application No.
**PCT/CN2022/075642**

**A. CLASSIFICATION OF SUBJECT MATTER**

A61K 31/519(2006.01)i; A61K 31/522(2006.01)i; A61P 35/00(2006.01)i

According to International Patent Classification (IPC) or to both national classification and IPC

**B. FIELDS SEARCHED**

Minimum documentation searched (classification system followed by classification symbols)

A61K; A61P

Documentation searched other than minimum documentation to the extent that such documents are included in the fields searched

Electronic data base consulted during the international search (name of data base and, where practicable, search terms used)

CNABS; CNTXT; USTXT; EPTXT; WOTXT; CNKI; 万方, WANFANG; ISI WEB OF KNOWLEDGE; NCBI; STN: 申请人, 发明人, 磷酸肌醇3-激酶, PI3K, 抑制剂, 转化生长因子β, TGFβ, PD-1, PD-L1, Atezolizumab, Avelumab, BMS-936559, MPDL3280A, durvalumab, Fresolimumab, 式IA化合物, 2-(1-(9H-嘌呤-6-基氨基)丙基)-3-3(3-氟苯基)-4H-色烯-4-酮, CN401, Tenalisib, CN202, WBP1126, Alpelisib, (2S)-1-N-[4-甲基-5-[2-(1,1,1-三氟-2-甲基丙烷-2-基]吡咯烷-1,2-二甲酰胺, 紫杉醇, 癌症, 肿瘤, tumor, cancer

**C. DOCUMENTS CONSIDERED TO BE RELEVANT**

| Category* | Citation of document, with indication, where appropriate, of the relevant passages | Relevant to claim No. |
|---|---|---|
| X | US 2018222982 A1 (DRANOFF, G. et al.) 09 August 2018 (2018-08-09) claims 1-46, and description, paragraphs 335-340, 643-665, and 773-778 | 1-3, 5-44, 61, 80-84, 87, 88, 94-101 |
| Y | US 2018222982 A1 (DRANOFF, G. et al.) 09 August 2018 (2018-08-09) claims 1-46, and description, paragraphs 335-340, 643-665, and 773-778 | 4. 45-60, 62-79, 85-86, 89-93, 102-105 |
| Y | DAVID, J. M. et al. "A novel bifunctional anti-PD-L1/TGF-β Trap fusion protein (M7824) efficiently reverts mesenchymalization of human lung cancer cells" *OncoImmunology,* Vol. 6, No. 10, 11 August 2017 (2017-08-11), e1349589, pp. 1-16 | 4, 46-60, 75-79, 85-86, 89-93, 102-105 |
| Y | US 2020289520 A1 (RHIZEN PHARMACEUTICALS SA) 17 September 2020 (2020-09-17) claims 1-18, and description, paragraph 25 | 62-74, 79, 86, 89-93, 102-105 |
| X | KR 102195221 B1 (SEOUL NATIONAL UNIVERSITY R&DB FOUNDATION) 24 December 2020 (2020-12-24) claim 1 | 1-16, 80-84, 88, 94, 99-101 |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **25 April 2022** | **07 May 2022** |

| Name and mailing address of the ISA/CN | Authorized officer |
|---|---|
| **China National Intellectual Property Administration (ISA/CN)**<br>**No. 6, Xitucheng Road, Jimenqiao, Haidian District, Beijing 100088, China** | |
| Facsimile No. **(86-10)62019451** | Telephone No. |

Form PCT/ISA/210 (second sheet) (January 2015)

**INTERNATIONAL SEARCH REPORT**

International application No.
**PCT/CN2022/075642**

**C. DOCUMENTS CONSIDERED TO BE RELEVANT**

| Category* | Citation of document, with indication, where appropriate, of the relevant passages | Relevant to claim No. |
|---|---|---|
| X | WO 2017007658 A1 (RIGEL PHARMACEUTICALS, INC.) 12 January 2017 (2017-01-12) claims 1-7 | 1-29, 80-84, 88, 94, 99-101 |
| X | WANG, Manni et al. "Immune checkpoint blockade and its combination therapy with small-molecule inhibitors for cancer treatment" *BBA-Reviews on Cancer,* Vol. 1871, 31 December 2018 (2018-12-31), pp. 199-224 | 1-16, 80-84, 88, 94, 99-101 |
| Y | US 2015272936 A1 (RHIZEN PHARMACEUTICALS SA) 01 October 2015 (2015-10-01) claims 1-34 | 62-74, 79, 86, 89-93, 102-105 |
| A | CN 111712243 A (ARRAY BIOPHARMA INC.) 25 September 2020 (2020-09-25) entire document | 1-105 |
| A | US 2015361070 A1 (GILEAD SCIENCES, INC.) 17 December 2015 (2015-12-17) entire document | 1-105 |
| A | WO 2020039097 A1 (BIOMEDICAL RESEARCH FOUNDATION OF THE ACADEMY OF ATHENS (BRFAA) et al.) 27 February 2020 (2020-02-27) entire document | 1-105 |
| A | WO 2020072445 A1 (VERASTEM, INC.) 09 April 2020 (2020-04-09) entire document | 1-105 |

Form PCT/ISA/210 (second sheet) (January 2015)

**INTERNATIONAL SEARCH REPORT**

International application No.
**PCT/CN2022/075642**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed:
      ☑ in the form of an Annex C/ST.25 text file.
      ☐ on paper or in the form of an image file.
   b. ☐ furnished together with the international application under PCT Rule 13*ter*.1(a) for the purposes of international search only in the form of an Annex C/ST.25 text file.
   c. ☐ furnished subsequent to the international filing date for the purposes of international search only:
      ☐ in the form of an Annex C/ST.25 text file (Rule 13*ter*.1(a)).
      ☐ on paper or in the form of an image file (Rule 13*ter*.1(b) and Administrative Instructions, Section 713).
2. ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.
3. Additional comments:

Form PCT/ISA/210 (continuation of first sheet) (January 2015)

**INTERNATIONAL SEARCH REPORT**

International application No.
**PCT/CN2022/075642**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **88-98,100-102**
because they relate to subject matter not required to be searched by this Authority, namely:

[1] Claims 88-98 and 100-102 relate to a method for treating or preventing tumors, belonging to a method for treating diseases set forth in PCT Rule 39.1(iv), and therefore, an international search is made on the basis of a corresponding pharmaceutical use of a drug composition.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

Form PCT/ISA/210 (continuation of first sheet) (January 2015)

**INTERNATIONAL SEARCH REPORT**
**Information on patent family members**

International application No.
**PCT/CN2022/075642**

| Patent document cited in search report | Publication date (day/month/year) | Patent family member(s) | Publication date (day/month/year) |
|---|---|---|---|
| US 2018222982 A1 | 09 August 2018 | JP 2021119167 A | 12 August 2021 |
| | | WO 2017019896 A1 | 02 February 2017 |
| | | JP 2018522027 A | 09 August 2018 |
| | | EP 3328418 A1 | 06 June 2018 |
| | | CN 108025051 A | 11 May 2018 |
| US 2020289520 A1 | 17 September 2020 | BR 112020011167 A2 | 17 November 2020 |
| | | CN 111770776 A | 13 October 2020 |
| | | CO 2020006886 A2 | 31 August 2020 |
| | | CA 3084905 A1 | 13 June 2019 |
| | | SG 11202005208Q A | 29 July 2020 |
| | | CL 2020001482 A1 | 20 November 2020 |
| | | IL 275028 D0 | 30 July 2020 |
| | | WO 2019111185 A1 | 13 June 2019 |
| | | EP 3720557 A1 | 14 October 2020 |
| | | JP 2021505571 A | 18 February 2021 |
| | | KR 20200096781 A | 13 August 2020 |
| | | AU 2018378415 A1 | 23 July 2020 |
| | | EA 202091082 A1 | 23 October 2020 |
| KR 102195221 B1 | 24 December 2020 | US 2021198378 A1 | 01 July 2021 |
| WO 2017007658 A1 | 12 January 2017 | None | |
| US 2015272936 A1 | 01 October 2015 | JP 2015536973 A | 24 December 2015 |
| | | CN 104870017 A | 26 August 2015 |
| | | US 2019083475 A1 | 21 March 2019 |
| | | EA 201590721 A1 | 30 September 2015 |
| | | WO 2014072937 A1 | 15 May 2014 |
| | | CN 111904962 A | 10 November 2020 |
| | | CA 2889905 A1 | 15 May 2014 |
| | | JP 2018203760 A | 27 December 2018 |
| | | KR 20210010958 A | 28 January 2021 |
| | | US 2020237735 A1 | 30 July 2020 |
| | | EP 2916868 A1 | 16 September 2015 |
| | | US 2017319558 A1 | 09 November 2017 |
| | | HK 1214159 A1 | 22 July 2016 |
| | | KR 20150079745 A | 08 July 2015 |
| CN 111712243 A | 25 September 2020 | None | |
| US 2015361070 A1 | 17 December 2015 | NZ 726055 A | 27 April 2018 |
| | | AR 100806 A1 | 02 November 2016 |
| | | WO 2015191743 A1 | 17 December 2015 |
| | | JP 2017521380 A | 03 August 2017 |
| | | CA 2952018 A1 | 17 December 2015 |
| | | AU 2015274626 A1 | 24 November 2016 |
| | | ES 2763104 T3 | 27 May 2020 |
| | | EA 201692266 A1 | 30 June 2017 |
| | | KR 20170015508 A | 08 February 2017 |
| | | BR 112016028876 A2 | 22 August 2017 |
| | | MX 2016016538 A | 01 May 2017 |
| | | CN 106459012 A | 22 February 2017 |
| | | TW 201625590 A | 16 July 2016 |
| | | SG 11201609527P A | 29 December 2016 |

Form PCT/ISA/210 (patent family annex) (January 2015)

**INTERNATIONAL SEARCH REPORT**
**Information on patent family members**

International application No.
**PCT/CN2022/075642**

| Patent document cited in search report | Publication date (day/month/year) | Patent family member(s) | Publication date (day/month/year) |
|---|---|---|---|
| | | EP 3154961 A1 | 19 April 2017 |
| | | IL 248789 D0 | 31 January 2017 |
| WO 2020039097 A1 | 27 February 2020 | US 2021246120 A1 | 12 August 2021 |
| | | CA 3110311 A1 | 27 February 2020 |
| | | CN 113272294 A | 17 August 2021 |
| | | AU 2019326768 A1 | 18 March 2021 |
| | | EP 3841097 A1 | 30 June 2021 |
| WO 2020072445 A1 | 09 April 2020 | US 2021338681 A1 | 04 November 2021 |
| | | EP 3860609 A1 | 11 August 2021 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 4751180 A
- US 4935233 A
- WO 8809344 A
- WO 9954440 A
- US 7488802 B
- US 7943743 B
- US 8008449 B
- US 8168757 B
- US 8217149 B
- US 8609089 B
- US 2010028330 A
- US 20120114649 A
- WO 2003042402 A
- WO 2008156712 A
- WO 2010089411 A
- WO 2010036959 A
- WO 2011066342 A
- WO 2011159877 A
- WO 2011082400 A
- WO 2011161699 A
- WO 2006121168 A
- WO 2009114335 A
- US 8354509 B
- WO 2009101611 A
- US 2014341917 A
- US 20130034559 A
- US 8779108 B
- WO 2007005874 A
- WO 2010077634 A
- US 20120039906 A
- WO 2013079174 A
- CN 2019124535 W
- EP 2015052781 W
- CN 2018086451 W


### Non-patent literature cited in the description


- **KNIGHT et al.** *Cell,* 2006, vol. 125 (4), 733-47
- **VANHAESEBROECK et al.** *Current Topic Microbiol. Immunol.,* 2010, vol. 347, 1-19
- **CLAYTON et al.** *J Exp Med.,* 2002, vol. 196 (6), 753-19 63
- **FUNG-LEUNG.** *Cell Signal.,* 2011, vol. 23 (4), 603-8
- **OKKENHAUG et al.** *Science,* 2002, vol. 297 (5583), 1031-34
- **LI et al.** *Annu. Rev. Immunol.,* 2006, vol. 24, 99-146
- **BORDER et al.** *Nature,* 1990, vol. 346, 371-374
- **BORDER et al.** *Nature,* 1992, vol. 360, 361-364
- **ISAKA et al.** *KidneyInt.,* 1999, vol. 55, 465-475
- **SHARMA et al.** *Diabetes,* vol. 45, 522
- **XIN et al.** *Transplantation,* 2004, vol. 15, 1433
- **BENIGNI et al.** *J. Am. Soc. Nephrol.,* 2003, vol. 14, 1816
- **KATSO et al.** *Annu. Rev. Cell Dev. Biol.,* 2001, vol. 17, 615-675
- **FOSTER, F.M. et al.** *J Cell Sci,* 2003, vol. 116, 3037-3040
- **ALTSCHUL et al.** *Nucleic Acids Res.,* 1977, vol. 25, 3389
- **ALTSCHUL et al.** *J. Mol. Biol.,* 1990, vol. 215, 403
- **WARD et al.** Binding Actida of a Repertoire ofSingle Immunoglobulin. variable Domains Secreted From Escherichia coli. *Nature,* 1989, vol. 341, 544-546
- **HUSTON et al.** Protein Engineering of Antibody Binding Sites: Recovery of SpecificActivity in an Anti-Digoxin Single-Chain Fv Analogue Produced in Escherichiacoli. *Proc. Natl. Acad. Sci. USA,* 1988, vol. 85, 5879-5883
- **NGUYEN V. K. et al.** *The EMBO Journal,* 2000, vol. 19, 921-930
- **MUYLDERMANS S.** *J Biotechnol,* 2001, vol. 74, 277-302
- **VANLANDSCHOOT P. et al.** *Antiviral Research,* 2011, vol. 92, 389-407
- **KABAT et al.** Immunology protein sequence. United States National Institution of Health, 1991
- **A1-LAZIKANI et al.** *JMol Biol,* 1997, vol. 273, 927-48
- **MATHIEU DONDELINGER et al.** Understanding the Significance and Imperial of Antibody Numbering and Antigen-Binding Surface/Residue Definition. *Front.Immunol.,* 16 October 2018
- **DALL'ACQUA et al.** *Biochem.,* 1998, vol. 37, 9266-9273

- **CHEADLE et al.** *Mol Immunol,* 1992, vol. 29, 21-30
- **RAAG ; WHITLOW.** *FASEB,* 1995, vol. 9 (1), 73-80
- **SAMBROOK et al.** Molecular Cloning: A Laboratory Manual. Cold Spring Harbor Laboratory Press, 2001
- **BERGE et al.** *J. Pharmaceutical Sciences,* 1977, vol. 66, 1-19
- **BUNDGARD, H.** Design of Prodrugs. Elsevier, 1985, 7-9, 21-24
- **HIGUCHI, T. et al.** Pro-drugs as Novel Delivery Systems. *A. C. S. Symposium Series,* vol. 14
- Bioreversible Carriers in Drug Design. American Pharmaceutical Association and Pergamon Press, 1987
- Burger's Medicinal Chemistry and Drug Discovery. 1995, vol. 172-178, 949-982
- Design of Prodrugs. Elselvier, 1985
- *CHEMICAL ABSTRACTS,* 946414-94-4
- **HAMID, O. et al.** *New England Journal of Medicine,* 2013, vol. 369 (2), 134-44
- *CHEMICAL ABSTRACTS,* 1428935-60-7
- *CHEMICAL ABSTRACTS,* 1380723-44-3
- *CHEMICAL ABSTRACTS,* 948564-73-6
- **ZHOU, J. et al.** *Blood,* 2007, vol. 110, 1607-1611